# EUROPEAN PATENT SPECIFICATION

(11) **EP 1 370 531 B1**
(45) Date of publication and mention of the grant of the patent: **28.02.2007**
(21) Application number: 02715515.9
(22) Date of filing: 18.01.2002
(51) Int. Cl.: C07D 213/81, C07D 471/04, C07C 229/36, C07D 401/12, C07D 213/89, A61P 37/00

(54) **PHENYLALANINE ENAMIDE DERIVATIVES POSSESSING A CYCLOBUTENE GROUP, FOR USE AS INTEGRIN INHIBITORS**
PHENYLALANINENAMIDDERIVATE MIT EINER CYCLOBUTENGRUPPE ZUR VERWENDUNG ALS INTEGRININHIBITOREN
DERIVES DE PHENYLALANINE-ENAMIDE TRAITANT UN GROUPE DE CYCLOBUTENE ET UTILISES COMME INHIBITEURS D'INTEGRINE

(30) Priority: 22.02.2001 GB 0104418; 08.06.2001 GB 0114000; 16.11.2001 GB 0127562
(43) Date of publication of application: 17.12.2003
(60) Divisional application: 06077243.1
(73) Proprietor: Celltech R & D Limited, Slough, Berkshire SL1 3WE (GB)
(72) Inventor: BAILEY, Stuart, Celltech R & D Limited, Slough, Berkshire SL1 3WE (GB); BROWN, Julien, Alistair, Celltech R & D Limited, Slough, Berkshire SL1 3WE (GB); BRAND, Stephen, Celltech R & D Limited, Slough, Berkshire SL1 3WE (GB); JOHNSON, James, Andrew, Celltech R & D Limited, Slough, Berkshire SL1 3WE (GB); PORTER, John, Robert, Celltech R & D Limited, Slough, Berkshire SL1 3WE (GB); HEAD, John, Clifford, 208 Bath Road, Slough Berks SL1 3WE (GB)
(74) Representative: Mercer, Christopher Paul
(86) International application number: PCT/GB2002/000206
(87) International publication number: WO 2002/068393

(56) References cited:
- WO-A-00/32575
- WO-A-00/73260
- WO-A-01/79173

## Description

This invention relates to a series of phenylalanine enamide derivatives, to compositions containing them, to processes for their preparation, and to their use in medicine.

Over the last few years it has become increasingly clear that the physical interaction of inflammatory leukocytes with each other and other cells of the body plays an important role in regulating immune and inflammatory responses [Springer, T. A., Nature, 346, 425, (1990); Springer, T. A., Cell, 76, 301, (1994)]. Specific cell surface molecules collectively referred to as cell adhesion molecules mediate many of these interactions.

The adhesion molecules have been sub-divided into different groups on the basis of their structure. One family of adhesion molecules which is believed to play a particularly important role in regulating immune and inflammatory responses is the integrin family. This family of cell surface glycoproteins has a typical non-covalently linked heterodimer structure. At least 16 different integrin alpha chains and 8 different integrin beta chains have been identified [Newman, P. *et al,* Molecular Medicine Today, 304, (1996)]. The members of the family are typically named according to their heterodimer composition although trivial nomenclature is widespread in the field. Thus the integrin α4β1 consists of the integrin alpha 4 chain associated with the integrin beta 1 chain, but is also widely referred to as Very Late Antigen 4 or VLA-4. Not all of the potential pairings of integrin alpha and beta chains have yet been observed in nature and the integrin family has been subdivided into a number of subgroups based on the pairings that have been recognised to date [Sonnenberg, A., Current Topics in Microbiology and Immunology, 184, 7, (1993)].

The importance of integrin function in normal physiological responses is highlighted by two human deficiency diseases in which integrin function is defective. Thus in the disease termed Leukocyte Adhesion Deficiency (LAD) there is a defect in one of the families of integrins expressed on leukocytes [Marlin, S. D. *et al,* J. Exp. Med. 164, 855, (1986)]. Patients suffering from this disease have a reduced ability to recruit leukocytes to inflammatory sites and suffer recurrent infections, which in extreme cases may be fatal. In the case of patients suffering from the disease termed Glanzman's thrombasthenia (a defect in a member of the beta 3 integrin family) there is a defect in blood clotting (Hodivala-Dilke, K. M., J. Clin. Invest. 103, 229, (1999)].

The potential to modify integrin function in such a way as to beneficially modulate cell adhesion has been extensively investigated in animal models using specific antibodies and peptides that block various functions of these molecules [e.g. Issekutz, T. B., J. Immunol. 149, 3394, (1992); Li, Z. *et al,* Am. J. Physiol. 263, L723, (1992); Mitjans, F. *et al,* J. Cell Sci. 108, 2825, (1995); Brooks, P. C. *et al,* J. Clin. Invest. 96, 1815, (1995); Binns, R. M. *et al,* J. Immunol. 157, 4094, (1996); Hammes, H.-P. *et al,* Nature Medicine 2, 529, (1996); Srivata, S. *et al,* Cardiovascular Res. 36, 408 (1997)]. In particular an anti α₄β₇-antibody has demonstrated both clinical and histologic improvement of inflammatory activity and disease in a non-human primate model of inflammatory bowel disease [Hesterberg, P.E. *et al,* Gastroenterol, 111, 1373-80 (1996)]. A number of monoclonal antibodies which block integrin function are currently being investigated for their therapeutic potential in human disease, and one, ReoPro, a chimeric antibody against the platelet integrin αIIbβ3 is in use as a potent antithrombotic agent for use in patients with cardiovascular complications following coronary angioplasty.

Integrins recognize both cell surface and extracellular matrix ligands, and ligand specificity is determined by the particular alpha-beta subunit combination of the molecule [Newman, P., *ibid*]. One particular integrin subgroup of interest involves the α4 chain which can pair with two different beta chains β1 and β7 [Sonnenberg, A., *ibid*]. The α4β1 pairing occurs on many circulating leukocytes (for example lymphocytes, monocytes, eosinophils and basophils) although it is absent or only present at low levels on circulating neutrophils. α4β1 binds to an adhesion molecule (Vascular Cell Adhesion Molecule-1 also known as VCAM-1) frequently up-regulated on endothelial cells at sites of inflammation [Osborne, L., Cell, 62, 3, (1990)]. The molecule has also been shown to bind to at least three sites in the matrix molecule fibronectin [Humphries, M. J. *et al,* Ciba Foundation Symposium, 189, 177, (1995)]. Based on data obtained with monoclonal antibodies in animal models it is believed that the interaction between α4β1 and ligands on other cells and the extracellular matrix plays an important role in leukocyte migration and activation [Yednock, T. A. *et al,* Nature, 356, 63, (1992); Podolsky, D. K. *et al,* J. Clin. Invest. 92, 372, (1993); Abraham, W. M. *et al,* J. Clin. Invest. 93, 776, (1994)].

The integrin generated by the pairing of α4 and β7 has been termed LPAM-1 [Holzmann, B. and Weissman, I. L., EMBO J. 8, 1735, (1989)]. The α4β7 pairing is expressed on certain sub-populations of T and B lymphocytes and on eosinophils [Erle, D. J. *et al,* J. Immunol. 153, 517 (1994)]. Like α4β1, α4β7 binds to VCAM-1 and fibronectin. In addition, α4β7 binds to an adhesion molecule believed to be involved in the homing of leukocytes to mucosal tissue such as gastrointestinal mucosa termed MAdCAM-1 [Berlin, C. *et al,* Cell, 74, 185, (1993)]. MAdCAM-1 is preferentially expressed in the gastrointestinal track. The interaction between α4β7 and MAdCAM-1 may also be important at sites of inflammation outside of mucosal tissue [Yang, X.-D. *et al,* PNAS, 91, 12604, (1994)].

Regions of the peptide sequence recognized by α4β1 and α4β7 when they bind to their ligands have been identified. α4β1 seems to recognise LDV, IDA or REDV peptide sequences in fibronectin and a QIDSP sequence in VCAM-1 [Humphries. M. J. *et al, ibid*] whilst α4β7 recognises a LDT sequence in MAdCAM-1 [Birskin, M. J. *et al,* J. lmmunol. 156, 719, (1996)). There have been several reports of inhibitors of these interactions being designed from modifications of these short peptide sequences [Cardarelli, P. M. *et al,* J. Biol. Chem., 269, 18668, (1994); Shorff, H. N.*et al,* Biorganic Med. Chem. Lett., 6, 2495, (1996); Vanderslice, P. *et al,* J. Immunol., 158, 1710, (1997)]. It has also been reported that a short peptide sequence derived from the α4β1 binding site in fibronectin can inhibit a contact hypersensitivity reaction in a trinitrochlorobenzene sensitised mouse [Ferguson, T. A., *et al,* PNAS, 88, 8072, (1991)].

WO00/73260 discloses squaric acid derivatives able to inhibit the binding of integrins to their ligands and are of use in the prophylaxis and treatment of immune or inflammatory disorders, or disorders involving the inappropriate growth or migration of cells.

WO00/32575 discloses alkanoic acid derivatives able to inhibit the binding of α4 integrins to their ligands and are of use in the prophylaxis and treatment of immune or inflammatory disorders.

WO01/79173 discloses enamine derivatives able to inhibit the binding of integrins to their ligands and are of use in the prophylaxis and treatment of immune or inflammatory disorders, or disorders involving the inappropriate growth or migration of cells.

Since the alpha 4 subgroup of integrins are predominantly expressed on leukocytes their inhibition can be expected to be beneficial in a number of immune or inflammatory disease states. However, because of the ubiquitous distribution and wide range of functions performed by other members of the integrin family it is important to be able to identify selective inhibitors of the alpha 4 subgroup.

We have now found a group of compounds which are potent and selective inhibitors of α4 integrins. Members of the group are able to inhibit α4 integrins such as α4β1 and/or α4β7 at concentrations at which they generally have no or minimal inhibitory action on α integrins of other subgroups. These compounds possess the additional advantages of good pharmacokinetic properties, especially low plasma clearance and good absorption properties making them particularly suitable for oral dosing.

Thus according to one aspect of the invention we provide a compound of formula (1): 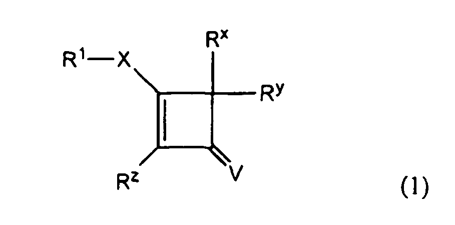 wherein
R¹ is a group Ar¹ L²Ar²Alk- in which:
Ar¹ is an optionally substituted aromatic or heteroaromatic group; wherein the optional substituents are selected from one, two or three halogen atoms, and/or C₁₋₆alkyl, C₃₋₈cycloalkyl, C₁₋₆hydroxyalkyl, carboxyC₁₋₆alkyl, C₁₋₆alkylthio, carboxyC₁₋₆alkylthio, C₁₋₆alkoxy, hydroxyC₁₋₆alkoxy, haloC₁₋₆alkyl, haloC₁₋₆alkoxy, C₁₋₆alkylamino, -NH₂, aminoC₁₋₆alkyl, C₁₋₆dialkylamino, C₁₋₆alkylaminoC₁₋₆alkyl, C₁₋₆dialkylaminoC₁₋₆alkyl, aminoC₁₋₆alkoxy, C₁₋₆alkylaminoC₁₋₆alkoxy, C₁₋₆dialkylaminoC₁₋₆alkoxy, nitro, cyano, amidino, -OH, HC(O)-, -CO₂H, -CO₂R⁵, C₁₋₆alkanoyl, -SH, thioC₁₋₆alkyl, -SO₃H, -SO₃R⁵, C₁₋₆alkylsulphinyl, C₁₋₆alkylsulphonyl, -SO₂NH₂, C₁₋₆alkylaminosulphonyl, C₁₋₆dialkylaminosulphonyl, phenylamino-sulphonyl, -CONH₂, C₁₋₆alkylaminocarbonyl, C₁₋₆dialkylaminocarbonyl, aminoC₁₋₆alkylaminocarbonyl, C₁₋₆alkylaminoC₁₋₆alkylaminocarbonyl, C₁₋₆dialkyl-aminoC₁₋₆alkylaminocarbonyl, aminocarbonylamino, C₁₋₆alkylamino-carbonylamino, C₁₋₆dialkylamino-carbonylamino, C₁₋₆alkylamino-cabonylC₁₋₆alkylamino, aminothiocarbonylamino, C₁₋₆alkylaminothiocarbonylamino, C₁₋₆dialkylaminothiocarbonylamino, C₁₋₆alkylaminothio-carbonylC₁₋₆alkylamino, C₁₋₆alkylsulphonylamino, C₁₋₆dialkyl-sulphonylamino, -NHSO₂NH₂, C₁₋₆alkylaminosulphonylamino, C₁₋₆dialkyl-aminosulphonylamino, C₁₋₆alkanoylamino, aminoC₁₋₆alkanoylamino, C₁₋₆dialkylaminoC₁₋₆alkanoylamino, C ₁₋₆alkanoylaminoC₁₋₆alkyl, C₁₋₆alkanoylaminoC₁₋₆alkylamino or C₁₋₆alkoxycarbonyl-amino groups;
R⁵ is a hydrogen atom, or a C₁₋₆alkyl or C₃₋₈cycloalkyl group;
L² is a covalent bond or a group L^{2a} or -(Alk³)L^{2a}, where Alk³ is an optionally substituted aliphatic or heteroaliphatic chain and L^{2a} is a -O- or -S- atom or a -C(O)-, - C(O)O-, -OC(O)-, -C(S)-, -S(O)-, -S(O)₂-, -N(R⁸)- [where R⁸ is a hydrogen atom or a straight or branched C₁₋₆alkyl group], -CON(R⁸)-, -OC(O)N(R⁸)-, -CSN(R⁸)-, - N(R⁸)CO-, -N(R⁸)C(O)O-, -N(R⁸)CS-, -S(O)₂N(R⁸)-, -N(R⁸)S(O)₂-, -N(R⁸)O-, -ON(R⁸)-, -N(R⁸)N(R⁸)-, -N(R⁸)CON(R⁸)-, -N(R⁸)CSN(R⁸)-, or -N(R⁸)SO₂N(R⁸)-group; optional substituents that may be present on Alk³ are selected from one, two or three substituents where each substituent may be the same or different and is selected from halogen atoms, or -OH, -CO₂H, -CO₂R⁹, where R⁹ is a straight or branched C₁₋₆alkyl group, -CONHR⁹, -CON(R⁹)₂, -COR⁹, C₁₋₆alkoxy, thiol, -S(O)R⁹, -S(O)₂R⁹, C₁₋₆alkylthio, amino, -NHR⁹ or -N(R⁹)₂ groups;
Ar² is an optionally substituted arylene or heteroarylene group; wherein the optional substituents which may be present on Ar² are selected from halogen atoms, or C₁₋₆alkyl, haloC₁₋₆alkyl, C₁₋₆alkoxy, haloC₁₋₆alkoxy, -CN, -CO₂CH₃, -NO₂, -NH₂, - NR⁵R⁶ and -N(R⁵)COCH₃ groups;
R⁶ is as defined for R⁵;
and Alk is a chain
-CH₂-CH(R)-, -CH=C(R)- or 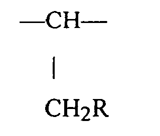 in which R is a carboxylic acid (-CO₂H) or a tetrazole, phosphonic acid, phosphinic acid, sulphonic acid, sulphinic acid, boronic acid or an acylsulphonamide biostere thereof or a -CO₂Alk⁷ or -CONR⁵R⁶ group;
   Alk⁷ is straight or branched C₁₋₈alkyl, C₂₋₈ alkenyl, C₂₋₈alkynyl, C₃₋₈cycloalkyl, C₃₋₈heterocycloalkyl, 1-methylpyrrolidinyl, 1-methyl-piperidinyl, 5-methyl-2-oxo-[1,3]dioxol-4-yl, C₃₋₈cycloalkylC₁₋₈alkyl, C₃₋₈heterocycloalkylC₁₋₈alkyl, C₁₋₆alkyloxyC₁₋₆alkyl, hydroxyC₁₋₆alkyl, C₁₋₆alkylthioC₁₋₆alkyl, C₁₋₆alkylsulfinylC₁₋₆alkyl, C₁₋₆alkylsulfonylC₁₋₆alkyl, C₃₋₈cycloalkyloxyC₁₋₆alkyl, C₃₋₈cycloalkylthioC₁₋₆alkyl, C₃₋₈cycloalkyl-sulfinylC₁₋₆alkyl, C₃₋₈cycloalkylsulfonylC₁₋₆alkyl, C₁₋₆alkyloxycarbonylC₁₋₆alkyl, C₁₋₆alkyloxycarbonylC₁₋₆alkenyl, C₁₋₆alkyloxycarbonyloxyC₁₋₆alkyl, C₁₋₆alkyloxycarbonyloxyC₁₋₆alkenyl, C₃₋₈cycloalkyloxycarbonyloxyC₁₋₆alkyl, N-di-C₁₋₈alkylaminoC₁₋₈alkyl, N-C₆₋₁₂aryl-N-C₁₋₆alkylaminoC₁₋₆alkyl, N-di-C₁₋₈alkyl-carbamoylC₁₋₈alkyl, C₆₋₁₂arylC₁₋₆alkyl, heteroC₆₋₁₀arylC₁₋₆alkyl, C₆₋₁₂aryl, C₆₋₁₂aryloxyC₁₋₈alkyl, C₆₋₁₂arylthioC₁₋₈alkyl, C_{6- 12}arylsulfinylC₁₋₈alkyl, C₆₋₁₂arylsulfonylC₁₋₈ alkyl, C₁₋₈alkanoyloxyC₁₋₈alkyl, C₄₋₈imidoC₁₋₈alkyl, C₆₋₁₂aroyloxyC₁₋₈ alkyl, or a triglyceride;
X is an -N(R²)- group in which:
   R² is a hydrogen atom or a C₁₋₆alkyl group;
   V is an oxygen (O) atom;
R^{z} is a hydrogen or halogen atom or a C₁₋₆alkyl group, or a group L¹(Alk¹)ₙR³ in which:
   L¹ is a covalent bond or an -O-, -S- or -Se- atom or -S(O)- or -N(R⁸)- group;
   Alk¹ is a C₁₋₆alkylene chain;
   R³ is a hydrogen atom or C₃₋₁₀cycloaliphatic group, or an optionally substituted C₃₋₁₀heterocycloaliphatic, C₆₋₁₂aromatic or C₁₋₉heteroaromatic group; the optional substituents which may be present on such heterocycloaliphatic groups are the R^{x} and R^{y} spiro-linked heterocycloaliphatic group substituents; optional substituents which may be present on such aromatic and heteroaromatic groups are selected from halogen atoms, or C₁₋₆alkyl, haloC₁₋₆alkyl, C₁₋₆alkoxy or haloC₁₋₆alkoxy groups;
   n is zero or the integer 1;
   R^{x} and R^{y} are joined together to form an optionally substituted spiro linked C₃₋₁₀cycloaliphatic or C₃₋₁₀heterocycloaliphatic group; optional substituents which may be present on such spiro linked groups are selected from halogen atoms, C₁₋₆alkyl groups, C₁₋₆alkoxy groups, haloC₁₋₆alkoxy groups, -CN, -CO₂CH₃, -NO₂ and -N(R¹¹)₂; when the spiro linked heterocycloaliphatic group contains a nitrogen atom this may be substituted by a group -(L⁶)ₚ(Alk⁵)_{q}R¹² where L⁶ is -C(O)- or -S(O)₂-, Alk⁵ is a C₁₋₆alkylene chain, or a heteroC₁₋₆alkylene chain and R¹² is a hydrogen atom or an optionally substituted phenyl ring, wherein the optional phenyl substituents are selected from halogen atoms, or C₁₋₆alkyl, haloC₁₋₆alkyl, C₁₋₆alkoxyl, haloC₁₋₆alkoxy, -CN, - CO₂CH₃, -NO₂, -NH₂, -NR⁵R⁶, -N(R⁵)COCH₃, or phenyl, furyl, thienyl, imidazolyl, pyridyl or pyrimidinyl groups;
   R¹¹ is a hydrogen atom, or a C₁₋₆alkyl or C₃₋₈cycloalkyl group; and the salts, solvates, hydrates and N-oxides thereof.

It will be appreciated that compounds of formula (1) may have one or more chiral centres, and exist as enantiomers or diastereomers. The invention is to be understood to extend to all such enantiomers, diastereomers and mixtures thereof, including racemates. Formula (1) and the formulae hereinafter are intended to represent all individual isomers and mixtures thereof, unless stated or shown otherwise. In addition, compounds of formula (1) may exist as tautomers, for example keto (CH₂C=O)-enol (CH=CHOH) tautomers. Formula (1) and the formulae hereinafter are intended to represent all individual tautomers and mixtures thereof, unless stated otherwise.

Optionally substituted aromatic groups represented by Ar¹ when present in the group R¹ include for example optionally substituted monocyclic or bicyclic fused ring C₆₋₁₂aromatic groups, such as phenyl, 1- or 2-naphthyl, 1- or 2-tetrahydronaphthyl, indanyl or indenyl groups.

Optionally substituted heteroaromatic groups represented by the group Ar¹ when present in the group R¹ include for example optionally substituted C₁₋₉heteroaromatic groups containing for example one, two, three or four heteroatoms selected from oxygen, sulphur or nitrogen atoms. In general, the heteroaromatic groups may be for example monocyclic or bicyclic fused ring heteroaromatic groups. Monocyclic heteroaromatic groups include for example five- or six-membered heteroaromatic groups containing one, two, three or four heteroatoms selected from oxygen, sulphur or nitrogen atoms. Bicyclic heteroaromatic groups include for example eight- to thirteen-membered fused-ring heteroaromatic groups containing one, two or more heteroatoms selected from oxygen, sulphur or nitrogen atoms.

Particular examples of heteroaromatic groups of these types include pyrrolyl, furyl, thienyl, imidazolyl, N-C₁₋₆alkylimidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, 1,3,4-thiadiazole, pyridyl, pyrimidinyl, pyridazinyl, pyrazinyl, 1,3,5-triazinyl, 1,2,4-triazinyl, 1,2,3-triazinyl, benzofuryl, [2,3-dihydro]benzofuryl, [2,3-dihydro]benzothienyl, benzothienyl, benzotriazolyl, indolyl, isoindolyl, benzimidazolyl, imidazo[1,2-a]pyridyl, benzothiazolyl, benzoxazolyl, benzisoxazolyl, benzopyranyl, [3,4-dihydro]benzopyranyl, quinazolinyl, quinoxalinyl, naphthyridinyl, e.g. 2,6-naphthyridinyl, or 2,7-naphthyridinyl, pyrido[3,4-b]pyridyl, pyrido[3,2-b]pyridyl, pyrido[4,3-b]pyridyl, quinolinyl, isoquinolinyl, tetrazolyl, 5,6,7,8-tetrahydroquinolinyl, 5,6,7,8-tetrahydroisoquinolinyl, and imidyl, e.g. succinimidyl, phthalimidyl, or naphthalimidyl such as 1,8-naphthalimidyl.

When R⁴, R⁵, R⁶, R⁷ and/or R⁸ is present as a C₁₋₆alkyl group it may be a straight or branched C₁₋₆alkyl group, e.g. a C₁₋₃alkyl group such as a methyl, ethyl or i-propyl group. C₃₋₈cycloalkyl groups represented by, R⁴, R⁵, R⁶ and/or R⁷ include C₃₋₆cycloalkyl groups e.g. cyclopropyl, cyclobutyl, cyclopentyl and cyclohexyl groups. Optional substituents which may be present on such alkyl or cycloalkyl groups include for example one, two or three substituents which may be the same or different selected from halogen atoms, for example fluorine, chlorine, bromine or iodine atoms, or hydroxy or C₁₋₆alkoxy e.g. methoxy or ethoxy groups.

When the groups R⁵ and R⁶ or R⁶ and R⁷ are both C₁₋₆alkyl groups these groups may be joined, together with the N atom to which they are attached, to form a heterocyclic ring. Such heterocyclic rings may be optionally interrupted by a further heteroatom selected from -O-, -S- or - N(R5)-. Particular examples of such heterocyclic rings include piperidinyl, morpholinyl, thiomorpholinyl, pyrrolidinyl, imidazolidinyl and piperazinyl rings.

Ar¹ in compounds of the invention may be optionally substituted by one, two, three or more halogen atoms, e.g. fluorine, chlorine, bromine or iodine atoms, and/or C₁₋₆alkyl, e.g. methyl, ethyl, n-propyl, i-propyl, n-butyl or t-butyl, C₃₋₈cycloalkyl, e.g. cyclopropyl, cyclobutyl, cyclopentyl or cyclohexyl, C₁₋₆hydroxyalkyl, e.g. hydroxymethyl, hydroxyethyl or -C(OH)(CF₃)₂, carboxyC₁₋₆alkyl, e.g. carboxyethyl, C₁₋₆alkylthio e.g. methylthio or ethylthio, carboxyC₁₋₆alkylthio, e.g. carboxymethylthio, 2-carboxyethylthio or 3-carboxypropylthio, C₁₋₆alkoxy, e.g. methoxy or ethoxy, hydroxyC₁₋₆alkoxy, e.g. 2-hydroxyethoxy, haloC₁₋₆alkyl, e.g. -CF₃, -CHF₂, -CH₂F, haloC₁₋₆alkoxy, e.g. -OCF₃, -OCHF₂, - OCH₂F, C₁₋₆alkylamino, e.g. methylamino or ethylamino, amino (-NH₂), aminoC₁₋₆alkyl, e.g. aminomethyl or aminoethyl, C₁₋₆dialkylamino, e.g. dimethylamino or diethylamino, C₁₋₆alkylaminoC₁₋₆alkyl, e.g. ethylaminoethyl, C₁₋₆dialkylaminoC₁₋₆alkyl, e.g. diethylaminoethyl, aminoC₁₋₆alkoxy, e.g. aminoethoxy, C₁₋₆alkylaminoC₁₋₆alkoxy, e.g. methylaminoethoxy, C₁₋₆dialkylaminoC₁₋₆alkoxy, e.g. dimethylaminoethoxy, diethylaminoethoxy, diisopropylaminoethoxy or dimethylaminopropoxy, nitro, cyano, amidino, hydroxyl (-OH), formyl [HC(O)-], carboxyl (-CO₂H), - CO₂R⁵ e.g. -CO₂CH₃ or -CO₂C(CH₃)₃, C₁₋₆alkanoyl e.g. acetyl, thiol (-SH), thioC₁₋₆alkyl, e.g. thiomethyl or thioethyl, sulphonyl (-SO₃H), -SO₃R⁵, C₁₋₆alkylsulphinyl, e.g. methylsulphinyl, C₁₋₆alkylsulphonyl, e.g. methylsulphonyl, aminosulphonyl (-SO₂NH₂), C₁₋₆alkylaminosulphonyl, e.g. methylaminosulphonyl or ethylaminosulphonyl, C₁₋₆dialkylaminosulphonyl, e.g. dimethylaminosulphonyl or diethylaminosulphonyl, phenylaminosulphonyl, carboxamido (-CONH₂), C₁₋₆alkylaminocarbonyl, e.g. methylaminocarbonyl or ethylaminocarbonyl, C₁₋₆dialkylaminocarbonyl, e.g. dimethylaminocarbonyl or diethylaminocarbonyl, aminoC₁₋₆alkylaminocarbonyl, e.g. aminoethylaminocarbonyl, C₁₋₆alkylaminoC₁₋₆alkylaminocarbonyl, e.g. ethylaminoethylaminocarbonyl, C₁₋₆dialkylaminoC₁₋₆alkylaminocarbonyl, e.g. diethylaminoethylaminocarbonyl, aminocarbonylamino, C₁₋₆alkylaminocarbonylamino, e.g. methylaminocarbonylamino or ethylaminocarbonylamino, C₁₋₆dialkylaminocarbonylamino, e.g. dimethylaminocarbonylamino or diethylaminocarbonylamino, C₁₋₆alkylaminocabonylC₁₋₆alkylamino, e.g. methylaminocarbonylmethylamino, aminothiocarbonylamino, C₁₋₆alkylaminothiocarbonylamino, e.g. methylaminothiocarbonylamino or ethylaminothiocarbonylamino, C₁₋₆dialkylaminothiocarbonylamino, e.g. dimethylaminothiocarbonylamino or diethylaminothiocarbonylamino, C₁₋₆alkylaminothiocarbonylC₁₋₆alkylamino, e.g. ethylaminothiocarbonylmethylamino, C₁₋₆alkylsulphonylamino, e.g. methylsulphonylamino or ethylsulphonylamino, C₁₋₆dialkylsulphonylamino. e.g. dimethylsulphonylamino or diethylsulphonylamino, aminosulphonylamino (-NHSO₂NH₂), C₁₋₆alkylaminosulphonylamino, e.g. methylaminosulphonylamino or ethylaminosulphonylamino, C₁₋₆dialkylaminosulphonylamino, e.g. dimethylaminosulphonylamino or diethylaminosulphonylamino, C₁₋₆alkanoylamino, e.g. acetylamino, aminoC₁₋₆alkanoylamino e.g. aminoacetylamino, C₁₋₆dialkylaminoC₁₋₆alkanoylamino, e.g. dimethylaminoacetylamino, C₁₋₆alkanoylaminoC₁₋₆alkyl, e.g. acetylaminomethyl, C₁₋₆alkanoylaminoC₁₋₆alkylamino, e.g. acetamidoethylamino, C₁₋₆alkoxycarbonylamino, e.g. methoxycarbonylamino, ethoxycarbonylamino or t-butoxycarbonylamino groups.

L² when present as part of the group R¹ in compounds of the invention may be a linker atom or group L^{2a} or a linker -(Alk³)L^{2a}-, where Alk³ is an optionally substituted aliphatic or heteroaliphatic chain
as described below and L^{2a} is a -O- or -S- atom or a -C(O)-, -C(O)O-, -OC(O)-, - C(S)-, -S(O)-, -S(O)₂-, -N(R⁸)- [where R⁸ is a hydrogen atom or a straight or branched C₁₋₆alkyl group], -CON(R⁸)-, -OC(O)N(R⁸)-, -CSN(R⁸)-, -N(R⁸)CO-, - N(R⁸)C(O)O-, -N(R⁸)CS-, -S(O)₂N(R⁸)-, -N(R⁸)S(O)₂-, -N(R⁸)O-, - ON(R8)-, -N(R8)N(R⁸)-, -N(R⁸)CON(R⁸)-, -N(R⁸)CSN(R⁸)-, or -N(R⁸)SO₂N(R⁸)-group.

When AIk³ is present as an optionally substituted aliphatic chain it may be an optionally substituted C₁₋₁₀aliphatic chain. Particular examples include optionally substituted straight or branched chain C₁₋₆alkylene, C₂₋₆alkenylene or C₂₋₆alkynylene chains.

Particular examples of aliphatic chains represented by Alk¹ include optionally substituted -CH₂-, -(CH₂)₂-, -CH(CH₃)CH₂-, -(CH₂)₂CH₂-, - (CH₂)₃CH₂-, -CH(CH₃)(CH₂)₂-, -CH₂CH(CH₃)CH₂-, -C(CH₃)₂CH₂-, - CH₂C(CH₃)₂CH₂-, -(CH₂)₂C(CH₃)₂CH₂-, -(CH₂)₄CH₂-, -(CH₂)₅CH₂-, - CHCH-, -CHCHCH₂-, -CH₂CHCH-, -CHCHCH₂CH₂-, -CH₂CHCHCH₂-, - (CH₂)₂CHCH-, -CC-, -CCCH₂-, -CH₂CC-, -CCCH₂CH₂-, -CH₂CCCH₂- or - (CH₂)₂CC- chains.

Heteroaliphatic chains represented by Alk³ include the aliphatic chains just described for Alk³ but with each additionally containing one, two, three or four heteroatoms or heteroatom-containing groups. Particular heteroatoms or groups include atoms or groups L⁵ where L⁵ is as defined above for L^{2a}. Each L⁵ atom or group may
interrupt the aliphatic chain, or may be positioned at its terminal carbon atom to connect the chain to an adjoining atom or group. Particular examples include optionally substituted -CH₂L⁵-, -CH₂CH₂L⁵-, -L⁵CH₂-, - L⁵CH₂CH₂-, -L⁵CH(CH₃)CH₂-, -L⁵CH₂CH(CH₃)CH₂-, - L⁵CH₂CH₂CH(CH₃)-, -L⁵C(CH₃)₂CH₂-, -CH₂L⁵CH₂CH₂-, -(CH₂)₂L⁵CH₂-, - (CH₂)₃L⁵CH₂-, -L⁵(CH₂)₃-, -L⁵(CH₂)₄-, -CH₂L⁵CH₂CHL⁵CH₂- and - (CH₂)₂L⁵CH₂CH₂- chains.

Optionally substituted arylene groups represented by Ar² when present as part of the group R¹ include those aromatic groups as previously described for Ar¹.

Optionally substituted heteroarylene groups represented by Ar² when present as part of the group R¹ include those heteroaromatic groups as previously described for Ar¹.

Each divalent arylene or heteroarylene group represented by Ar² may be attached to the remainder of the molecule through any available ring carbon or nitrogen atoms.

The optional substituents which may be present on aliphatic or heteroaliphatic chains represented by Alk³ include one, two or three substituents where each substituent may be the same or different and is selected from halogen atoms, e.g. fluorine, chlorine, bromine or iodine atoms, or -OH, -CO₂H, -CO₂R⁹, where R⁹ is a straight or branched C₁₋₆alkyl group, -CONHR⁹, -CON(R⁹)₂, -COR⁹, e.g. -COCH₃, C₁₋₆alkoxy, e.g. methoxy or ethoxy, thiol, -S(O)R⁹, -S(O)₂R⁹, C₁₋₆alkylthio e.g. methylthio or ethylthio, amino, -NHR⁹ or -N(R⁹)₂ groups. Where two R⁹ groups are present in any of the above substituents these may be the same or different.

When the group R² is present in compounds of the invention as a C₁₋₆alkyl group it may be for example a straight or branched C₁₋₆alkyl group e.g. a C₁₋₃alkyl group such as a methyl or ethyl group.

When the group R is present in R¹ in compounds of the invention as a derivative of a carboxylic acid it may be for example -CO₂Alk⁷ or -CONR⁵R⁶ group as defined herein. When R is a biostere of a carboxylic acid it may be for example a tetrazole or other acid such as phosphonic acid, phosphinic acid, sulphonic acid, sulphinic acid or boronic acid or an acylsulphonamide group.

Esters (-CO₂Alk⁷) and amide (-CONR⁵R⁶) derivatives of the carboxylic acid group (-CO₂H) in compounds of formula (1) may advantageously be used as prodrugs of the active compound. Such prodrugs are compounds which undergo biotransformation to the corresponding carboxylic acid prior to exhibiting their pharmacological effects and the invention particularly extends to prodrugs of the acids of formula (1). Such prodrugs are well known in the art, see for example International Patent Application No. WO00/23419, Bodor, N. (Alfred Benzon Symposium, 1982, 17, 156-177), Singh, G. et al (J. Sci. Ind. Res., 1996, 55, 497-510) and Bundgaard, H., (Design of Prodrugs, 1985, Elsevier, Amsterdam).

Esterified carboxyl groups represented by the group -CO₂Alk⁷ include groups wherein Alk⁷ is a straight or branched C₁₋₈alkyl group such as a methyl, ethyl, n-propyl, i-propyl, n-butyl, i-butyl, s-butyl, t-butyl, pentyl or neopentyl group; a C₂₋₈alkenyl group such as a propenyl e.g. 2-propenyl or butenyl e.g. 2-butenyl or 3-butenyl group, a C₂₋₈alkynyl group such as a ethynyl, propynyl e.g. 2-propynyl or butynyl e.g. 2-butynyl or 3-butynyl group, a C₃₋₈cycloalkyl group such as a cyclopropyl, cyclobutyl, cyclopentyl, cyclohexyl or cycloheptyl group; a C₃₋₈heterocycloalkyl group such as a tetrahydrofuanyl e.g. tetrahydrofuran-3-yl, pyrrolidinyl e.g. 1-methylpyrrolidinyl such as 1-methylpyrrolidin-3-yl, piperidinyl e.g. 1-methylpiperidinyl such as 1-methylpiperidin-4-yl, tetrahydropyranyl e.g. tetrahydropyran-4-yl or 2-oxo-[1,3]dioxol-4-yl e.g. 5-methyl-2-oxo-[1,3]dioxol-4-yl group; a C₃₋₈cycloalkylC₁₋₈alkyl group such as a cyclopentylmethyl, cyclohexylmethyl or cyclohexylethyl group; a C₃₋₈heterocycloalkylC₁₋₈alkyl group such as a morpholinyl-N-ethyl, thiomorpholinyl-N-methyl, pyrrolidinyl-N-ethyl, pyrrolidinyl-N-propyl, piperidinyl-N-ethyl, pyrazolidinyl-N-methyl or piperazinyl-N-ethyl group; a C₁₋₆alkyloxyC₁₋₆alkyl group such as a methyloxyethyl or propyloxyethyl group; a hydroxyC₁₋₆alkyl group such as a hydroxyethyl e.g. 2-hydroxyethyl or hydroxypropyl e.g. 2-hydroxypropyl, 3-hydroxypropyl or 2,3-dihydroxypropyl group; a C₁₋₆alkylthioC₁₋₆alkyl group such as an ethylthioethyl group; a C₁₋₆alkylsulfinylC₁₋₆alkyl group such as an methylsulfinylethyl group; a C₁₋₆alkylsulfonylC₁₋₆alkyl group such as an methylsulfonylmethyl group; a C₃₋₈cycloalkyloxyC₁₋₆alkyl group such as a cyclohexyloxymethyl group; a C₃₋₈cycloalkylthioC₁₋₆alkyl group such as a cyclopentylthiomethyl group; a C₃₋₈cycloalkylsulfinylC₁₋₆alkyl group such as a cyclopentyl-sulfinylmethyl group; a C₃₋₈cycloalkylsulfonylC₁₋₆alkyl group such as a cyclopentylsulfonylmethyl group; a C₁₋₆alkyloxycarbonylC₁₋₆alkyl group such as isobutoxy-carbonylpropyl group; a C₁₋₆alkyloxycarbonylC₁₋₆alkenyl group such as isobutoxycarbonylpentenyl group; a C₁₋₆alkyloxycarbonyloxyC₁₋₆alkyl group such as an ethyloxycarbonyloxymethyl or isopropoxycarbonyloxyethyl e.g 1-(isopropoxycarbonyloxy)ethyl or 2-(isopropoxycarbonyloxy)ethyl group; a C₁₋₆alkyloxycarbonyloxyC₁₋₆alkenyl group such as a isopropoxycarbonyloxybutenyl group, a C₃₋₈cycloalkyloxycarbonyloxyC₁₋₆alkyl group such as a cyclohexyloxycarbonyloxyethyl, e.g. a 2-(cyclohexyloxycarbonyloxy)ethyl group, a N-di-C₁₋₈alkylaminoC₁₋₈alkyl group such as a N-dimethylaminoethyl or N-diethylaminoethyl group; a N-C₆₋₁₂aryl-N-C₁₋₆alkylaminoC₁₋₆alkyl group such as a N-phenyl-N-methylaminomethyl group; a N-di-C₁₋₈alkylcarbamoylC₁₋₈alkyl group such as a N-diethylcarbamoylmethyl group; a C₆₋₁₂arylC₁₋₆alkyl group such as a benzyl, phenylethyl, phenylpropyl, 1-naphthylmethyl or 2-naphthylmethyl group; a heteroC₆₋₁₀arylC₁₋₆alkyl group, such as a pyridinylmethyl e.g. pyridin-4-ylmethyl or imidazolylethyl e.g. 2-imidazol-1-ylethyl group; a C₆₋₁₂aryl group such as a phenyl, 1-naphthyl or 2-naphthyl group; a C₆₋₁₂aryloxyC₁₋₈alkyl group such as a phenyloxymethyl, phenyloxyethyl, 1-naphthyloxymethyl, or 2-naphthyloxymethyl group; a C₆₋₁₂arylthioC₁₋₈alkyl group such as a phenylthioethyl group; a C₆₋₁₂arylsulfinylC₁₋₈alkyl group such as a phenyl-sulfinylmethyl group; a C₆₋₁₂arylsulfonylC₁₋₈alkyl group such as a phenylsulfonylmethyl group; a C₁₋₈alkanoyloxyC₁₋₈alkyl group, such as a acetoxymethyl, ethoxycarbonyloxyethyl, pivaloyloxymethyl, propionyloxyethyl or propionyloxypropyl group; a C₄₋₈imidoC₁₋₈alkyl group such as a succinimidomethyl or phthalamidoethyl group; a C₆₋₁₂aroyloxyC_{1- 8}alkyl group such as a benzoyloxyethyl or benzoyloxypropyl group or a triglyceride such as a 2-substituted triglyceride e.g. a 1,3-di-C₁₋₈alkylglycerol-2-yl group such as a 1,3-diheptylglycerol-2-yl group.

It will be appreciated that in the forgoing list of Alk⁷ groups the point of attachment to the remainder of the compound of formula (1) is via the last described part of the Alk⁷ group. Thus, for example a methoxyethyl group would be attached by the ethyl group, whilst a morpholinyl-N-ethyl group would be attached via the N-ethyl group.

It will be further appreciated that in the forgoing list of Alk⁷ groups, where not specifically mentioned, alkyl groups may be replaced by alkenyl or alkynyl groups where such groups are as previously defined for Alk¹. Additionally these alkyl, alkenyl or alkynyl groups may optionally be interrupted by one, two or three linker atoms or groups where such linker atoms and groups are as previously defined for L^{2a}.

Further prodrugs of compounds of formula (1) include cyclic esters where X is a -N(R²)- group in which R² becomes a C₁₋₆alkyl joining chain, especially a -CH₂- or -CH₂CH₂- chain, which is also connected to the acid group R to form a cyclic ester of formula (1a):

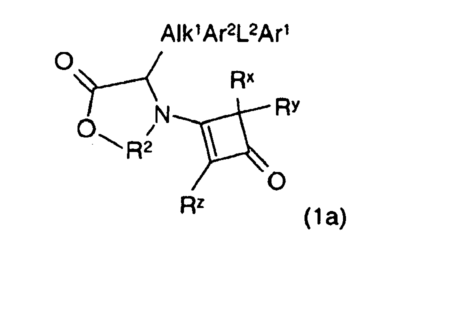

Cycloaliphatic groups represented by the group R³ when present in the group R^{z} in compounds of the invention are C₃₋₁₀cycloaliphatic groups. Particular examples include C₃₋₁₀cycloalkyl, e.g. C₃₋₈cycloalkyl or C₃₋₁₀cycloalkenyl, e.g C₃₋₈cycloalkenyl groups.

Optionally substituted heterocycloaliphatic groups represented by the group R³ when present in the group R^{z} are optionally substituted C₃₋₁₀heterocycloaliphatic groups. Particular examples include optionally substituted C₃₋₁₀heterocycloalkyl, e.g. C₃₋₇heterocycloalkyl, or C₃₋₁₀heterocycloalkenyl, e.g. C₃₋₇hetercycloalkenyl groups, each of said groups containing one, two, three or four heteroatoms or heteroatom-containing groups L⁵ as defined above.

Particular examples of cycloaliphatic, and heterocyclo-aliphatic groups represented by the group R³ include optionally substituted cyclopropyl, cyclobutyl, cyclopentyl, cyclohexyl, cycloheptyl, cyclooctyl, cyclopropenyl, cyclobutenyl, cyclopentenyl, cyclohexenyl, cycloheptenyl, cyclooctenyl, 2-cyclobuten-1-yl, 2-cyclopenten-1-yl, 3-cyclopenten-1-yl, tetrahydrofuranyl, tetrahydrothiophenyl, tetrahydrothiophene-1-oxide, tetrahydrothiophene-1,1-dioxide, pyrroline, e.g. 2- or 3-pyrrolinyl, pyrrolidinyl, pyrrolidinone, oxazolidinyl, oxazolidinone, dioxolanyl, e.g. 1,3-dioxolanyl, imidazolinyl, e.g. 2-imidazolinyl, imidazolidinyl, pyrazolinyl, e.g. 2-pyrazolinyl, pyrazolidinyl, pyranyl, e.g. 2- or 4-pyranyl, tetrahydropyranyl, tetrahydrothiopyranyl, tetrahydrothiopyran-1-oxide, tetrahydrothiopyran-1,1-dioxide, piperidinyl, piperidinone, dioxanyl e.g. 1,3-dioxanyl or 1,4-dioxanyl, morpholinyl, morpholinone, dithianyl, e.g. 1,3-dithianyl or 1,4-dithianyl, thiomorpholinyl, piperazinyl, 1,3,5-trithianyl, oxazinyl, e.g. 2H-1,3-, 6H-1,3-, 6H-1,2-, 2H-1,2- or 4H-1,4- oxazinyl, 1,2,5-oxathiazinyl, isoxazinyl, e.g. o-or p-isoxazinyl, oxathiazinyl, e.g. 1,2,5 or 1,2,6-oxathiazinyl, or 1,3,5,-oxadiazinyl groups.

Aliphatic or heteroaliphatic chains represented by Alk⁵ include those chains described above for Alk³.

When the group R³ is an optionally substituted C₆₋₁₂ aromatic or C₁₋₉ heteroaromatic group it may be for example an aromatic or heteroaromatic group as described herein for the group Ar¹.

The groups R^{x} an R^{y} are joined together to form an optionally substituted spiro linked C₃₋₁₀ cycloaliphatic or C₃₋₁₀ heterocycloaliphatic group joined to the cyclobutenone ring as defined by formula (1).

The presence of certain substituents in the compounds of formula (1) may enable salts of the compounds to be formed. Suitable salts include pharmaceutically acceptable salts, for example acid addition salts derived from inorganic or organic acids, and salts derived from inorganic and organic bases.

Acid addition salts include hydrochlorides, hydrobromides, hydroiodides, alkylsulphonates, e.g. methanesulphonates, ethanesulphonates, or isothionates, arylsulphonates, e.g. p-toluenesulphonates, besylates or napsylates, phosphates, sulphates, hydrogen sulphates, acetates, trifluoroacetates, propionates, citrates, maleates, fumarates, malonates, succinates, lactates, oxalates, tartrates and benzoates.

Salts derived from inorganic or organic bases include alkali metal salts such as sodium or potassium salts, alkaline earth metal salts such as magnesium or calcium salts, and organic amine salts such as morpholine, piperidine, dimethylamine or diethylamine salts.

Particularly useful salts of compounds according to the invention include pharmaceutically acceptable salts, especially acid addition pharmaceutically acceptable salts.

In the compounds according to the invention the group R¹ is preferably an Ar¹L²Ar²Alk- group. In compounds of this type Ar¹ is preferably an optionally substituted phenyl, monocyclic heteroaromatic or bicyclic heteroaromatic group. Particularly useful monocyclic heteroaromatic groups are optionally substituted five- or six-membered heteroaromatic groups as described previously, especially five- or six-membered heteroaromatic groups containing one or two heteroatoms selected from oxygen, sulphur or nitrogen atoms. Nitrogen-containing groups are especially useful, particularly pyridyl or pyrimidinyl groups. Particularly useful substituents present on these monocyclic Ar¹ groups include halogen atoms or alkyl, haloalkyl, -OR⁵, -SR⁵, -NR⁵R⁶, -CO₂H, -CO₂CH₃, -NO₂, -N(R⁵)COR⁶ or -CN groups as described above in relation to the compounds of formula (1). Particularly useful bicyclic heteroaromatic groups represented by Ar¹ include optionally substituted ten-membered fused-ring heteroaromatic groups containing one, two or three, especially one or two heteroatoms, especially nitrogen atoms. Particular examples include optionally substituted naphthyridinyl, especially 2,6-naphthyridinyl, 2,7-naphthyridinyl, quinolinyl and isoquinolinyl, especially isoquinolin-1-yl groups. Particular optional substituents include those just described for monocyclic heteroaromatic groups. Additionally, in compounds according to the invention X is preferably an -N(R²)- group and V is preferably an oxygen atom.

A particularly useful group of compounds according to the invention has the formula (2a): 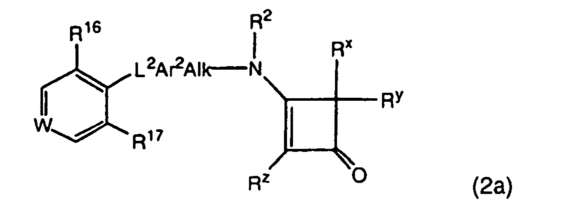 wherein -W= is -CR¹⁸=, -N= or -N(O)=;
R¹⁶, R¹⁷ and R¹⁸, which may be the same or different is each a hydrogen atom or an atom or group selected from the optional substituents an Ar¹ mentioned above; L², Ar², Alk, R², R^{x}, R^{y} and R^{z} are as defined for formula (1); and the salts, solvates, hydrates and N-oxides thereof.

In one preferred class of compounds of formula (2a) where W is a -CR¹⁸= group R¹⁸ is a hydrogen atom. In another preferred class of compounds R¹⁸ is a preferred atom or group as hereinafter defined for R¹⁶, especially a C₁₋₆alkoxy, especially a methoxy or ethoxy, group.

In another preferred class of compounds of formula (2a) W is a -N= or - N(O)= group.

R¹⁶ and R¹⁷ in compounds of formula (2a) is each preferably as particularly described above for compounds of formula (1), other than a hydrogen atom. Particularly useful R¹⁶ and R¹⁷ substituents include halogen atoms, especially fluorine or chlorine atoms, or C₁₋₆alkyl, especially methyl, ethyl or isopropyl, haloC₁₋₆alkyl especially halomethyl, most especially -CF₃, -CHF₂ or -CH₂F, C₁₋₆alkoxy especially methoxy or etoxy or haloC₁₋₆alkoxy especially halomethoxy, most especially -OCF₃, - OCHF₂ or -OCH₂F groups.

A further particularly useful group of compounds according to the invention has the formula (2b): 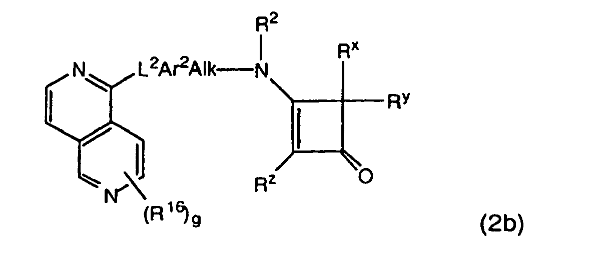 wherein g is the integer 1, 2, 3 or 4;
R¹⁶, is indepently an atom or group selected from the optional substituents an Ar¹ mentioned above
L², Ar², Alk, R², R^{x}, R^{y} and R^{z} are as defined for formula (1);
and the salts, solvates, hydrates and N-oxides thereof.

Particularly useful R¹⁶ substituents when present in compounds of formula (2b) include halogen atoms, especially fluorine, chlorine or bromine atoms, or C₁₋₆alkyl e.g. methyl, ethyl or isopropyl, haloC₁₋₆alkyl, especially halomethyl, most especially -CF₃, C₁₋₆alkoxyl, especially methoxy, haloC₁₋₆alkoxy, especially halomethoxy, most especially -OCF₃, -CN, - CO₂CH₃, -NO₂, amino (-NH₂), substituted amino (-NR⁵R⁶) especially - NHCH₃ and -N(CH₃)₂, -N(R⁵)COCH₃, especially -NHCOCH₃ groups or optionally substituted phenyl, furyl, thienyl, imidazolyl, pyridyl and pyrimidinyl groups.

A further particularly useful group of compounds according to the invention has the formula (2c): 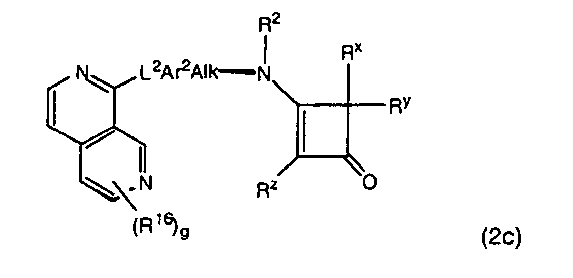 wherein R¹⁶, g, L², Ar², Alk, R², R^{x}, R^{y} and R^{z} are as defined for formula (2b);
and the salts, solvates, hydrates and N-oxides thereof.

A further particularly useful group of compounds according to the invention has the formula (2d): 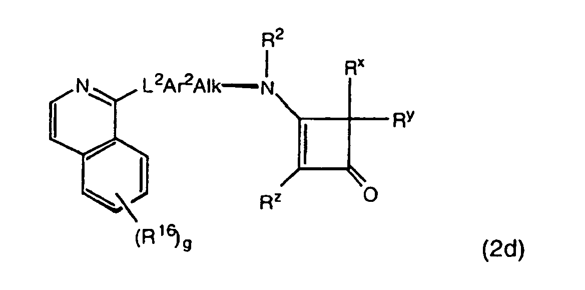 wherein R¹⁶, g, L², Ar², Alk, R², R^{x}, R^{y} and R^{z} are as defined for formula (2b):
and the salts, solvates, hydrates and N-oxides thereof.

In one preferred class of compounds of formula (2d) at least one R¹⁶ atom or group is present at the 3-position of the isoquinoline ring. In a preferred group of compounds of this class R¹⁶ is an optionally substituted phenyl ring. Optional substituents which may be present on the phenyl ring include halogen atoms, especially fluorine or chlorine atoms, or C₁₋₆alkyl, especially methyl, ethyl or isopropyl, haloC₁₋₆alkyl especially halomethyl, most especially -CF₃, -CHF₂ or -CH₂F, C₁₋₆alkoxy especially methoxy or etoxy or haloC₁₋₆alkoxy especially halomethoxy, most especially -OCF₃, - OCHF₂ or -OCH₂F groups.

It will be understood that compounds according to formulae (2a), (2b), (2c) and (2d) include, where applicable, the corresponding hydroxy tautomers.

Alk in compounds of the invention is preferably: 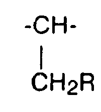 or, especially, -CH₂CH(R)-.

In one preferred class of compounds of formulae (1), (2a), (2b), (2c) and (2d) R is a -CO₂H group.

In another prefered class of compounds of formulae formulae (1), (2a), (2b), (2c) and (2d) R is an esterified carboxyl group of formula -CO₂Alk⁷ which may advantageously be used as a prodrug of the active compound. In this class of compound Alk⁷ is preferably a C₁₋₈alkyl group, especially a methyl, ethyl, propyl, i-propyl, butyl, t-butyl, pentyl or neopenyl group; a C₃₋₈cycloalkyl group, especially a cyclopropyl, cyclobutyl, cyclopentyl, cyclohexyl or cycloheptyl group; a substituted C₃₋₈heterocycloalkyl group especially a tetrahydrofuanyl e.g. tetrahydrofuran-3-yl, pyrrolidinyl e.g. 1-methylpyrrolidinyl such as 1-methylpyrrolidin-3-yl, piperidinyl e.g. 1-methylpiperidinyl such as 1-methylpiperidin-4-yl, tetrahydropyranyl e.g. tetrahydropyran-4-yl or 2-oxo-[1,3]dioxol-4-yl e.g. 5-methyl-2-oxo-[1,3]dioxol-4-yl group; a C₆₋₁₀aryl group, especially a phenyl group; a C₆₋₁₀arylC₁₋₆alkyl group, especially a benzyl group; a heteroC₆₋₁₀arylC₁₋₆alkyl group, especially a pyridinylC₁₋₃alkyl group such as pyridinylmethyl e.g. pyridin-4-ylmethyl or pyridinylethyl e.g. pyridine-4-ylethyl or a imidazolylC₁₋₃alkyl group such as imidazolylethyl e.g. 2-imidazol-1-ylethyl or imidazolylpropyl e.g. 2-imidazol-1-ylpropyl group; a hydroxyC₁₋₆alkyl group, especially a hydroxyethyl e.g. 2-hydroxyethyl or hydroxypropyl e.g. 3-hydroxypropyl or 2,3-dihydroxypropyl group; a C₃₋₈heterocycloalkylC₁₋₆alkyl group, especially a morpholinyl-N-ethyl group; a N-di-C₁₋₈alkylaminoC₁₋₈alkyl group, especially a N-dimethylaminoethyl or N-diethylaminoethyl group; or a C₁₋₆alkyloxyC₁₋₆alkyl group, especially a methyloxyethyl group. Especially preferred esterified carboxyl groups include -CO₂CH₃, - CO₂CH₂CH₃, -CO₂CH₂CH₂CH₃, -CO₂CH(CH₃)₂ and -CO₂C(CH₃)₃ groups. A most especially preferred esterified carboxyl group is - CO₂(hydroxyC₁₋₆alkyl), especially -CO₂CH₂CH₂OH.

In general in compounds of formula (1) when X is a -N(R²) group and in particular in compounds of formulae (2a), (2b), (2c) and (2d) R² is preferably a hydrogen atom.

In one preferred class of compounds of formula (2a) L² is preferably L^{2a} where L^{2a} is a -CON(R⁸)- group [where R⁸ is preferably a hydrogen atom or a C₁₋₃alkyl group], especially a -CONH- group or a -(Alk³)L^{2a}- group, especially a -(Alk³)O- group [where Alk³ is preferably a C₁₋₃alkyl group], most especially a -CH₂O- group. In this class of compounds -W= is preferably -N= or -N(O)=. Most preferably W is -N=.

In another preferred class of compounds of formula (2a) L² is preferably a covalent bond. In this class of compounds -W= is preferably -C(R¹⁸)=, where R¹⁸ is as hereinbefore generally and particularly defined.

In general in compounds of formulae (2b), (2c) and (2d) L² is preferably L^{2a} where L^{2a} is an -O- atom or -N(R⁸)- group [where R⁸ is preferably a hydrogen atom or a C₁₋₃alkyl group]. An especially useful -N(R⁸)- group is -NH-.

The group Ar² in compounds of formulae (1), (2a), (2b), (2c) and (2d) is preferably an optionally substituted phenylene or optionally substituted pyridinediyl group or formula: 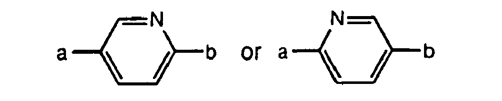 where a and b signify the points of attachment of L² and Alk respectively. Most preferably Ar² is an optionally substituted 1,4-phenylene group.

Optional substituents which may be present on Ar² in compounds of the invention are halogen atoms, especialy fluorine, chlorine or bromine atoms, or C₁₋₆alkyl e.g. methyl, ethyl or i-propyl, haloC₁₋₆alkyl especially halomethyl, most especialy -CF₃, C₁₋₆alkoxy especially methoxy or haloC₁₋₆alkoxy, especially halomethoxy, most especially -OCF₃, -CN, -CO₂CH₃, -NO₂, amino (-NH₂), substituted amino (NR⁵R⁶) especially -NHCH₃ and -N(CH₃)₂ and -N(R⁵)COCH₃, especially - NHCOCH₃ groups.

In another preferred class of compounds of formulae (1), (2a), (2b), (2c) and (2d) R^{z} is a hydrogen atom.

In another preferred class of compounds of formulae (1), (2a), (2b), (2c) and (2d) R^{z} is a halogen atom, especially a fluorine, chlorine, bromine or iodine atom, most especially a chlorine or bromine atom.

In another preferred class of compounds of formulae (1), (2a), (2b), (2c) and (2d) R^{z} is a group -L¹(Alk¹)ₙR³. In this class of compounds L¹ is a covalent bond or an -O-, -S- or -Se- atom or -S(O)- or -N(R⁸)-, especially -NH- or -N(CH₃)- group. Most preferably L¹ is a -S- atom or - S(O)- group. In this class of compounds R³ is a hydrogen atom or an optionally substituted C₃₋₁₀cycloaliphatic, especially C₃₋₇cycloalkyl group, most especially an optionally substituted cyclopentyl, cyclohexyl or cycloheptyl group; or an optionally substituted C₃₋₁₀heterocycloaliphatic, especially C₃₋₇heterocycloalkyl group, most especially an optionally substituted piperidinyl, piperazinyl, pyrrolidinyl, imidazolidinyl, dithianyl or pyrazolidinyl group, or an optionally substituted C₆₋₁₂aromatic group, preferably an optionally substituted phenyl group or an optionally substituted C₁₋₉heteroaromatic group, preferably an optionally substituted monocyclic C₁₋₉heteroaromatic group, most preferably a 5- or 6-membered monocyclic heteroaromatic group containing one, two , three or four heteroatoms selected from oxygen, sulphur or nitrogen atoms, especially an optionally substituted furyl, thienyl, imidazolyl e.g. 1-methylimidazol-2-yl, triazolyl, tetrazolyl, pyridyl, pyrimidinyl or pyrazinyl group. Optional substituents which may be present on such heterocycloaliphatic groups include those substituents as described hereinafter when R^{x} and R^{y} are joined to form an optionally substituted spiro linked heterocycloaliphatic group. Optional substituents which may be present on such aromatic and heteroaromatic groups are selected from halogen atoms, or C₁₋₆alkyl, halo C₁₋₆alkyl, C₁₋₆alkoxy or haloC₁₋₆alkoxy groups. In one preferred group of compounds of this class n is zero. In another preferred group of compounds of this class L¹ is a covalent bond and n is zero. In this group of compounds R³ is preferrably an optionally substituted C₃₋₁₀cycloaliphatic, C₃₋₁₀heterocycloaliphatic, C₆₋₁₂aromatic or monocyclic C₁₋₉heteroaromatic group as just described. In a further preferred group of compounds of this class n is the integer 1 and Alk¹ is a C₁₋₆alkylene chain, especially a -CH₂-, -CH₂CH₂- or -CH₂CH(CH₃)- chain. In a further preferred group of compounds of this class L¹ is a covalent bond, n is the integer 1 and Alk¹ is a C₁₋₆alkylene chain, especially a-CH₂-, -CH₂CH₂-, -CH₂CH₂CH₂- or -CH₂CH(CH₃)- chain. In a further preferred group of compounds of this class L¹ is a preferred atom or group as just described, most especially a -S- atom, n is the integer 1 and Alk¹ is a C₁₋₆alkylene chain, especially a -CH₂-, -CH₂CH₂-,-CH₂CH₂CH₂- or -CH₂CH(CH₃)- chain. In this class of compounds R³ is preferably a hydrogen atom.

Most especially useful R^{z} groups which may be present in compounds of the invention include a hydrogen or halogen atom, especially fluorine, chlorine, bomine or iodine atom or a group of formula -L¹(Alk¹)ₙR³ as just defined, especially an alkyl group as previously described or a hydroxyl (-OH); C₁₋₆alkoxymethoxy, ethoxy or i-propoxy; C₃₋₇cycloalkyl, especially cyclopentyl or cyclohexyl; C₁₋₆alkylsulfanyl, especially methyl- ethyl- or i-propylsulfanyl; C₁₋₆alkylsuffinyl, especially methyl- ethyl- or i-propylsulfinyl; C₃₋₇heterocycloalkyl, especially piperidinyl most especially piperidin-3-yl such as 1-methylpiperidin-3-yl or dithianyl especially [1,3]dithian-2-yl; C₆₋₁₂aryiselenenyl, especially phenylselenenyl; C₆₋₁₂arylsulfanyl, especially phenylsulfanyl or pentafluorophenylsulfanyl; monocyclic C₁₋₉heteroaromaticsulfanyl, especially tetrazol-5-ylsulfanyl most especially 1-methyl-1 H-terazol-5-ylsulfanyl or imidazolylsulfanyl especially imidazol-2-ylsulfanyl most especially 1-methyl-1H-imidazol-2-ylsulfanyl; monocyclic C₁₋₉heteroaromatic, especially pyridinyl most especially pyridin-3-yl, 1-methylpyridinium or pyrazinyl especially pyrazin-2-yl; or a C₆₋₁₂arylC₁₋₃alkyl, especially benzyl group.

In compounds of formulae (1), (2a), (2b), (2c) and (2d) R^{x} and R^{y} are joined to form an optional-substituted spiro linked cycloaliphatic group particularly a C₃₋₁₀cycloaliphatic group, most particularly a C₃₋₈cycloalkyl group, especially an optionally substituted cyclopentyl cyclohexyl, cycloheptyl or cyclooctyl group, or a C₃₋₈cycloalkenyl group, especially an optionally substituted cyclopentenyl, cyclohexenyl, cycloheptenyl or cyclooctenyl group group. Optional substituents which may be present on such spiro linked cycloaliphatic groups are halogen atoms, especially fluorine or chlorine atoms, C₁₋₆alkyl groups, especially methyl, ethyl, propyl or i-propyl, C₁₋₆alkoxy groups, especially methoxy or ethoxy, haloC₁₋₆alkoxy groups, especially -OCF₃, -CN, -CO₂CH₃, -NO₂ and substituted amino (-N(R¹¹)₂), especially -NHCH₃ and -N(CH₃)₂ groups. In a preferred group of compounds of this class R^{z} is a hydrogen atom. In another preferred group of compounds of this class R^{z} is an alkyl group as just described. In a further preferred group of compounds of this class R^{z} is a halogen atom, especially a fluorine, chlorine, bromine or iodine atom, most especially a chlorine or bromine atom, particularly a bromine atom. In a still further preferred group of compounds of this class R^{z} is a group -L¹(Alk¹)ₙR³ as just described.

In another preferred class of compounds of formulae (1), (2a), (2b), (2c) and (2d) R^{x} and R^{y} are joined to form an optionally substituted spiro linked heterocycloaliphatic group, particularly an optionally substituted C₃₋₁₀heterocycloaliphatic group, most particularly an optionally substituted C₃₋₇heterocycloalkyl group, especially an optionally substituted C₃₋₇heterocycloalkyl group containing one or two -O-, -S-, -S(O)-, -S(O)₂-, - NH- or -C(O)- heteroatoms or heteroatom-containing groups. Especially preferred optionally substituted heterocycloaliphatic groups include optionally substituted 5- and 6-membered heterocycloalkyl groups containing one heteroatom or heteroatom-containing group as just described, especially optionally substituted pyrrolidinyl, tetrahydrofuranyl, tetrahydrothiophenyl, tetrahydrothiophene-1-oxide, tetrahydrothiophene-1,1-dioxide, piperidinyl, tetrahydropyranyl, tetrahydrothiopyranyl tetrahydrothiopyran-1-oxide or tetrahydrothiopyran-1,1-dioxide groups. Optional substituents which may be present on such spiro linked heterocycloaliphatic groups are halogen atoms, especially fluorine or chlorine atoms, C₁₋₆alkyl groups, especially methyl, ethyl, propyl or i-propyl, C₁₋₆alkoxy groups, especially methoxy or ethoxy, haloC₁₋₆alkoxy groups, especially -OCF₃, -CN, -CO₂CH₃, -NO₂ and substituted amino (-N(R¹¹)₂), especially -NHCH₃ and -N(CH₃)₂ groups. In addition when the spiro linked heterocycloaliphatic group contains a nitrogen atom this may be substituted by a group -(L⁶)ₚ(Alk⁵)_{q}R¹² where L⁶ is -C(O)- or -S(O)₂-, Alk⁵ is a C₁₋₆alkylene chain, especially a -CH₂-, -(CH₂)₂- or-CH(CH₃)CH₂- chain or a heteroC₁₋₆alkylene chain, especially -CH₂L⁵-, -CH₂CH₂L⁵-, -L⁵CH₂- or -L⁵CH₂CH₂ chain where L⁵ is an -O- or -S- atom or -NH or -N(CH₃)- group and R¹² is a hydrogen atom or an optionally substituted phenyl ring. In one preferred group of compounds of this class R^{z} is a hydrogen atom. In another preferred group of compounds of this class R^{z} is an alkyl group as just described. In a further preferred group of compounds of this class R^{z} is a halogen atom, especially a fluorine, chlorine, bromine or iodine atom, most especially a chlorine or bromine atom. In a still further preferred group of compounds of this class R^{z} is a group -L¹(Alk¹)ₙR³ as just described.

Particularly useful compounds of the invention include:
(2*S*)-2-[(3-Oxospiro[3.5]non-1-en-1-yl)amino]-3-{4-[(3,5-dichloroisonicotinoyl)amino]phenyl}propanoic acid
(2*S*)-2-[(3-Oxospiro[3.5]non-1-en-1-yl)amino]-3-{4-[(3-methyl[2,7]naphthyridin-1-yl)oxy]phenyl}propanoic acid
(2*S*)-2-[(2-Bromo-3-oxospiro[3.5]non-1-en-1-yl)amino]-3-{4-[(3,5-dichloroisonicotinoyl)amino]phenyl}propanoic acid)amino]
(2*S*)-2-[(2-Bromo-3-oxospiro[3.5]non-1-en-1-yl)amino]-3-{4-[(2,7)naphthyridin-1-yloxy]phenyl}propanoic acid
(2*S*)-2-[(2-Bromo-3-oxo-7-oxaspiro[3.5]non-1-en-1-yl)amino]-3-{4-[(3,5-dichloroisonicotinoyl)amino]phenyl}propanoic acid
(2*S*)-2-{(2-Bromo-3-oxospiro[3.5]non-1-en-1-yl)amino}-3-(2,6-dimethoxy[1,1'-biphenyl]-4-yl)propanoic acid
(2*S*)-2-[(3-Oxospiro[3.6]dec-1-en-1-yl)amino]3-{4-[(3,5-dichloroisonicotinoyl)amino]phenyl}propanoic acid
(2*S*)-2-[(2-Bromo-3-oxospiro[3.6]dec-1-en-1-yl)amino]-3-{4-[(3,5-dichloroisonicotinoyl)amino]phenyl}propanoic acid
(2*S*)-2-[(2-Bromo-7-methoxy-3-oxospiro[3.5]non-1-en-1-yl)amino]-3-{4-[(3,5-dichloroisonicotinoyl)amino]phenyl}propanoic acid
(2*S*)-2-{(2-Bromo-3-oxospiro[3.5]non-1-en-1-yl)amino}-3-{4-[(3-methyl[2.7]naphthyridin-1-yl)oxy]phenyl}propanoic acid
(2*S*)-2-[(2-Chloro-3-oxospiro[3.5]non-1-en-1-yl)amino]-3-{4-[(3,5-dichloroisonicotinoyl)amino]phenyl}propanoic acid
(2S)-3-{4-[(3,5-Dichloroisonicotinoyl)amino]phenyl}-2-(3-oxo-2-pyridin-3-yl-spiro[3.5]non-1-en-1-ylamino)-propanoic acid
(2*S*)-2-[(2-lodo-3-oxospiro[3.5]non-1-en-1-yl)amino]-3-{4-[(3,5-dichloroisonicotinoyl)amino]phenyl}propanoic acid
(2*S*)-2-[(2-Bromo-3-oxospiro[3.5]non-1-en-1-yl)amino]-3-{5-[(3,5-dichloroisonicotinoyl)amino]pyridin-2-yl}propanoic acid
(2*S*)-2-[(2-Chloro-3-oxospiro[3.5]non-1-en-1-yl)amino]-3-{5-[(3,5-dichloroisonicotinoyl)amino]pyridin-2-yl}propanoic acid
(2*S*)-2-[(2-Chloro-3-oxo-7-oxa-spiro[3.5]non-1-en-1-yl)amino]-3-{4-[(3,5-dichloroisonicotinoyl)amino]phenyl}propanoic acid
(2S)-2-[(2-Chloro-3-oxo-spiro[3.6]dec-1-en-1-yl)amino]-3-{4-[(3,5-dichloroisonicotinoyl)amino]phenyl}propanoic acid
(2*S*)-2-[(2-Bromo-3,7,7-trioxo-7λ⁶-thia-spiro[3.5]non-1-en-1-yl)amino]-3-(4-[(3,5-dichloroisonicotinoyl)amino]phenyl}propanoic acid
(2*S*)-2-[(2-Chloro-3-oxo-spiro[3.4]oct-1-en-1-yl)amino]-3-{4-[(3,5-dichloroisonicotinoyl)amino]phenyl}propanoic acid
(2*S*)-2-[(2-Bromo-3-oxo-spiro[3.4]oct-1-en-1-yl)amino]-3-{4-[(3,5-dichloroisonicotinoyl)amino]phenyl}propanoic acid
(2*S*)-2-[(2-Methylsulfanyl-3-oxo-spiro[3.5]non-1-en-1-yl)amino]-3-{4-[(3,5-dichloroisonicotinoyl)amino]phenyl}propanoic acid
(2*S*)-2-(2-Fluoro-3-oxo-spiro[3.5]non-1-en-1-ylamino)3-{4-[(3,5-dichloroisonicotinoyl)amino]phenyl}propanoic acid
(2*S*)-2-[(2-Fluoro-3-oxo-7-oxa-spiro[3.5]non-1-en-1-yl)amino]-3-{4-[(3,5-dichloroisonicotinoyl)amino]phenyl}propanoic acid
(2*S*)-2-[(2-Isopropylsulfanyl-3-oxo-spiro[3.5]non-1-en-1-yl)amino]-3-(4-[(3,5-dichloroisonicotinoyl)amino]phenyl)propanoic acid
(2*S*)-2-[(2-isopropylsulfanyl-3-oxo-spiro[3.5]non-1-en-1-yl)amino]-3-[4-([2,7]naphthyridin-1-ylamino)phenyl]propanoic acid
(2*S*)-2-[(2-isopropylsulfanyl-3-oxo-7-oxa-spiro[3.5]non-1-en-1-yl)amino]-3-[4-([2,7]naphthyridin-1-ylamino)phenyl]propanoic acid
(2*S*)-2-[(2-Bromo-3-oxo-spiro[3.4]octa-1,6-dien-1-yl)amino]-3-{4-[(3,5-dichloroisonicotinoyl)amino]phenyl}propanoic acid
(*2S*)-*2*-[(7-Acetyl-2-bromo-3-oxo-7-aza-spiro[3.5]non-1-en-1-yl)amino]-3-{4-[(3,5-dichloroisonicotinoyl)amino]phenyl}propanoic acid
(*2S*)-2-{[2-(lsopropylsulfanyl)-3-oxo-7-oxaspiro[3.5]non-1-en-yl)]amino}-3-(2,6-dimethoxy[1,1'-biphenyl]-4-yl)propanoic acid
(2*S*)-2-[(2-Cyclohexyl-3-oxo-spiro[3.5]non-1-en-1-yl)amino]-3-{4-[(3,5-dichloroisonicotinoyl)amino]phenyl}propanoic acid
(*2S*)-3-{4-[(3,5-Dichloroisonicotinoyl)amino]phenyl}-2-(2-methyl-3-oxo-spiro[3.5]non-1-en-1-ylamino)propanoic acid
(2*S*)-3-(4-[(3,5,Dichloroisonicotinoyl)amino]phenyl)-2-(3-oxo-2,propylspiro[3.5]non-1-en-1-ylamino)propanoic acid
(2*S*)-3-{4-[(3,5-Dichloroisonicotinoyl)amino]phenyl}-2-(2-methyl-3-oxo-7-oxa-spiro[3.5]non-1-en-1-ylamino)propanoic acid
(2*S*)-3-{4-[(3,5-Dichloroisonicotinoyl)amino]phenyl}-2-(3-oxo-2-propyl-7-oxa-spiro[3.5]non-1-en-1-ylamino)propanoic acid
(2*S*)-3-{4-[(3,5-Dichloroisonicotinoyl)amino]phenyl}-2-(2-[1,3]dithian-2-yl-3-oxo-spiro[3.5]non-1-en-1-ylamino)propanoic acid
(2*S*)-3-{4-[(3,5-Dichloroisonicotinoyl)amino]phenyl}-2-(2-ethyl-3-oxo-spiro[3.5]non-1-en-1-ylamino)propanoic acid
(2*S*)-3-{4-[(3,5-Dichloro-1-oxy-pyridine-4-carbonyl)amino]phenyl}-2-(3-oxo-spiro[3.5]non-1-en-1-ylamino)propanoic acid
(*2S*)-2-(2-Bromo-3-oxo-spiro[3.5]non-1-en-1-ylamino)-3-{4-[(3,5-dichloro-1-oxy-pyridine-4-carbonyl)amino]phenyl}propanoic acid
(*2S*)-2-(2-Chloro-3-oxo-spiro[3.5]non-1-en-1-ylamino)-3-{4-[(3,5-dichloro-1-oxy-pyridine-4-carbonyl)amino]phenyl}propanoic acid
(*2S*)-2-(2-Bromo-3-oxo-spiro[3.5]non-1-en-1-ylamino)-3-[4-(3-methyl-[2,7]naphthyridin-1-ylamino)phenyl]propanoic acid
(*2S*)-2-(2-Bromo-3-oxo-spiro[3.5]non-1-en-1-ylamino)-3-[4-([2,7]naphthyridin-1-ylamino)phenyl]propanoic acid
(2*S*)-2-[(2-Bromo-3-oxo-spiro[3.4]oct-1-en-1-yl)amino]-3-{4-[(3,5-dichloroisonicotinoyl)amino]phenyl}propanoic acid
and the salts, solvates, hydrates, N-oxides and carboxylic acid esters thereof.

Particularly useful carboxylic acid esters thereof include the methyl, ethyl, propy, i-propyl and t-butyl esters.

Most especially useful compounds of the invention include:
(2*S*)-2-[(2-Bromo-3-oxospiro[3.5]non-1-en-1-yl)amino]-3-{4-[(3,5-dichloroisonicotinoyl)amino]phenyl}propanoic acid
(2*S*)-2-[(2-Bromo-3-oxo-7-oxaspiro[3.5]non-1-en-1-yl)amino]-3-{4-[(3,5-dichloroisonicotinoyl)amino]phenyl}propanoic acid
(2*S*)-2-[(2-Bromo-3-oxospiro[3.6]dec-1-en-1-yl)amino]-3-{4-[(3,5-dichloroisonicotinoyl)amino]phenyl}propanoic acid
(2*S*)-2-{(2-Bromo-3-oxospiro[3.5]non-1-en-1-yl)amino}-3-{4-[(3-methyl[2,7]naphthyridin-1-yl)oxy]phenyl}propanoic acid
(2*S*)-2-[(2-Chloro-3-oxospiro[3.5]non-1-en-1-yl)amino]-3-{4-[(3,5-dichloroisonicotinoyl)amino]phenyl}propanoic acid
(2S)-3-{4-[(3,5-Dichloroisonicotinoyl)amino]phenyl}-2-(3-oxo-2-pyridin-3-yl-spiro[3.5]non-1-en-1-ylamino)propanoic acid
(2*S*)-2-[(2-Iodo-3-oxospiro[3.5]non-1-en-1-yl)amino]-3-{4-[(3,5-dichloroisonicotinoyl)amino]phenyl}propanoic acid
(2*S*)-2-[(2-Bromo-3-oxospiro[3.5]non-1-en-1-yl)amino]-3-{5-[(3,5-dichloroisonicotinoyl)amino]pyridin-2-yl}propanoic acid
(2*S*)-2-[(2-Chloro-3-oxospiro[3.5]non-1-en-1-yl)amino]-3-{5-[(3,5-dichloroisonicotinoyl)amino]pyridin-2-yl}propanoic acid
(2*S*)-2-[(2-Chloro-3-oxo-7-oxa-spiro[3.5]non-1-en-1-yl)amino]-3-(4-[(3,5-dichloroisonicotinoyl)amino]phenyl}propanoic acid
(2*S*)-2-[(2-Chloro-3-oxo-spiro[3.6]dec-1-en-1-yl)amino]-3-{4-[(3,5-dichloroisonicotinoyl)amino]phenyl}propanoic acid
(2*S*)-2-[(2-Bromo-3,7,7-trioxo-7λ⁶-thia-spiro[3.5]non-1-en-1-yl)amino]-3-{4-[(3,5-dichloroisonicotinoyl)amino]phenyl}propanoic acid
(2*S*)-2-[(2-Chloro-3-oxo-spiro[3.4]oct-1-en-1-yl)amino]-3-{4-[(3,5-dichloroisonicotinoyl)amino]phenyl}propanoic acid
(2*S*)-2-[(2-Bromo-3-oxo-spiro[3.4]oct-1-en-1-yl)amino]-3-{4-[(3,5-dichloroisonicotinoyl)amino]phenyl}propanoic acid
(2*S*)-2-[(2-Methylsulfanyl-3-oxo-spiro[3.5]non-1-en-1-yl)amino]-3-{4-[(3,5-dichloroisonicotinoyl)amino]phenyl}propanoic acid
(2*S*)-2-(2-Fluoro-3-oxo-spiro[3.5]non-1-en-1-ylamino)3-{4-[(3,5-dichloroisonicotinoyl)amino]phenyl}propanoic acid
(2*S*)*-*2-[(2-Fluoro-3-oxo-7-oxa-spiro[3.5]non-1-en-1-yl)amino]-3-{4-[(3,5-dichloroisonicotinoyl)amino]phenyl}propanoic acid
(2*S*)-2-[(2-Isopropylsulfanyl-3-oxo-spiro[3.5]non-1-en-1-yl)amino]-3-{4-[(3,5-dichloroisonicotinoyl)amino]phenyl}propanoic acid
(2*S*)-2-[(2-Isopropylsulfanyl-3-oxo-spiro[3.5]non-1-en-1-yl)amino]-3-[4-([2,7]naphthyridin-1-ylamino)phenyl]propanoic acid
(2*S*)-2-[(2-Isopropylsulfanyl-3-oxo-7-oxa-spiro[3.5]non-1-en-1-yl)amino]-3-[4-([2,7]naphthyridin-1-ylamino)phenyl]propanoic acid
(2*S*)-2-[(2-Bromo-3-oxo-spiro[3.4]octa-1,6-dien-1-yl)amino]-3-{4-[(3,5-dichloroisonicotinoyl)amino]phenyl}propanoic acid
(2*S*)-2-[(7-Acetyl-2-bromo-3-oxo-7-aza-spiro[3.5]non-1-en-1-yl)amino]-3-{4-[(3,5-dichloroisonicotinoyl)amino]phenyl}propanoic acid
(2*S*)-2-[(2-Cyclohexyl-3-oxo-spiro[3.5]non-1-en-1-yl)amino]-3-{4-[(3,5-dichloroisonicotinoyl)amino]phenyl}propanoic acid
(2*S*)3-{4-[(3,5-Dichloroisonicotinoyl)amino]phenyl)-2-(2-methyl-3-oxo-spiro[3.5]non-1-en-1-ylamino)propanoic acid
(2*S*)-3-{4-[(3,5-Dichloroisonicotinoyl)amino]phenyl}-2-(3-oxo-2-propyl-spiro[3.5]non-1-en-1-ylamino)propanoic acid
(2S)-3-{4-[(3,5-Dichloroisonicotinoyl)amino]phenyl}-2-(2-[1,3]dithian-2-yl-3-oxo-spiro[3.5]non-1-en-1-ylamino)propanoic acid
(*2S*)-2-(2-Bromo-3-oxo-spiro[3.5]non-1-en-1-ylamino)-3-{4-[(3,5-dichloro-1-oxy-pyridine-4-carbonyl)amino]phenyl}propanoic acid
2-(2-Chloro-3-oxo-spiro[3.5]non-1-en-1-ylamino)-3-{4-[(3,5-dichloro-1-oxypyridine-4-carbonyl)amino]phenyl}propanoic acid
and the salts, solvates, hydrates, N-oxides and carboxylic acid esters thereof.

Particularly useful carboxylic acid esters thereof include the methyl, ethyl, propy, i-propyl and t-butyl esters.

Particularly useful ester prodrugs of compounds of the invention include:
Ethyl (2S)-2-(2-bromo-3-oxo-spiro[3.5]non-1-en-1-ylamino)-3-{4-[(3,5-dichloroisonicotinoyl)amino]phenyl}propanoate
Isopropyl (2S)-2-(2-bromo-3-oxo-spiro[3.5]non-1-en-1-ylamino)-3-{4-[(3,5-dichloroisonicotinoyl)amino]phenyl}propanoate
t-Butyl (2S)-2-(2-bromo-3-oxo-spiro[3.5]non-1-en-1-ylamino)-3-{4-[(3,5-dichloroisonicotinoyl)amino]phenyl}propanoate
1-Methyl-piperidin-4-yl (2*S*)-2-(2-bromo-3-oxo-spiro[3.5]non-1-en-1-ylamino)-3-{4-[(3,5-dichloroisonicotinoyl)amino]phenyl}propanoate
Phenyl (2*S*)-2-(2-bromo-3-oxo-spiro[3.5]non-1-en-1-ylamino)-3-{4-[(3,5-dichloroisonicotinoyl)amino]phenyl}propanoate
Cyclopentyl (2*S*)-2-(2-bromo-3-oxo-spiro[3.5]non-1-en-1-ylamino)-3-{4-[(3,5-dichloroisonicotinoyl)amino]phenyl}propanoate
2-Imidazol-1-yl-ethyl (2*S*)-2-(2-bromo-3-oxo-spiro[3.5]non-1-en-1-ylamino)-3-{4-[(3,5-dichloroisonicotinoyl)amino]phenyl}propanoate
Neopentyl (2*S*)-2-(2-bromo-3-oxo-spiro[3.5]non-1-en-1-ylamino)-3-{4-[(3,5-dichloroisonicotinoyl)amino]phenyl}propanoate
Tetrahydro-furan-3-yl (2*S*)-2-(2-bromo-3-oxo-spiro[3.5]non-1-en-1-ylamino)-3-{4-[(3,5-dichloroisonicotinoyl)amino]phenyl}propanoate
Pyridin-4-ylmethyl (2*S*)-2-(2-bromo-3-oxo-spiro[3.5]non-1-en-1-ylamino)-3-{4-[(3,5-dichloroisonicotinoyl)amino]phenyl}propanoate
Tetrahydropyran-4-yl (2*S*)-2-(2-bromo-3-oxo-spiro[3.5]non-1-en-1-ylamino)-3-{4-[(3,5-dichloroisonicotinoyl)amino]phenyl}propanoate
5-Methyl-2-oxo-[1,3]dioxol-4-ylmethyl (2*S*)-2-(2-bromo-3-oxo-spiro[3.5]non-1-en-1-ylamino)-3-{4-[(3,5-dichloroisonicotinoyl)amino]-phenyl}propanoate
1-Methyl-pyrrolidin-3-yl (2*S*)-2-(2-bromo-3-oxo-spiro[3.5]non-1-en-1-ylamino)-3-{4-[(3,5-dichloroisonicotinoyl)amino]phenyl}propanoate
2,3-Dihydroxypropyl (2*S*)-2-(2-bromo-3-oxo-spiro[3.5]non-1-en-1-ylamino)-3-{4-[(3,5-dichloroisonicotinoyl)amino]phenyl}propanoate
Tetrahydrofuran-2-ylmethyl (2*S*)-2-(2-bromo-3-oxo-spiro[3.5]non-1-en-1-ylamino)-3-{4-[(3,5-dichloroisonicotinoyl)amino]phenyl}propanoate
and the salts, solvates, hydrates and N-oxides thereof.

Compounds according to the invention are potent and selective inhibitors of α4 integrins. The ability of the compounds to act in this way may be simply determined by employing tests such as those described in the Examples hereinafter.

The compounds are of use in modulating cell adhesion and in particular are of use in the prophylaxis and treatment of diseases or disorders including inflammation in which the extravasation of leukocytes plays a role and the invention extends to such a use and to the use of the compounds for the manufacture of a medicament for treating such diseases or disorders,

Diseases or disorders of this type include inflammatory arthritis such as rheumatoid arthritis vasculitis or polydermatomyositis, multiple sclerosis, allograft rejection, diabetes, inflammatory dermatoses such as psoriasis or dermatitis, asthma and inflammatory bowel disease.

For the prophylaxis or treatment of disease the compounds according to the invention may be administered as pharmaceutical compositions, and according to a further aspect of the invention we provide a pharmaceutical composition which comprises a compound of formula (1) together with one or more pharmaceutically acceptable carriers, excipients or diluents.

Pharmaceutical compositions according to the invention may take a form suitable for oral, buccal, parenteral, nasal, topical or rectal administration, or a form suitable for administration by inhalation or insufflation.

For oral administration, the pharmaceutical compositions may take the form of, for example, tablets, lozenges or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, nonaqueous vehicles and preservatives. The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The compounds for formula (1) may be formulated for parenteral administration by injection e.g. by bolus injection or infusion. Formulations for injection may be presented in unit dosage form, e.g. in glass ampoule or multi dose containers, e.g. glass vials. The compositions for injection may take such forms as suspensions, solutions or emulsions in oily or aqueous vehicles, and may contain formulatory agents such as suspending, stabilising, preserving and/or dispersing agents. Alternatively, the active ingredient may be in powder form for constitution with a suitable vehicle, e.g. sterile pyrogen-free water, before use.

In addition to the formulations described above, the compounds of formula (1) may also be formulated as a depot preparation. Such long acting formulations may be administered by implantation or by intramuscular injection.

For nasal administration or administration by inhalation, the compounds for use according to the present invention are conveniently delivered in the form of an aerosol spray presentation for pressurised packs or a nebuliser, with the use of suitable propellant, e.g. dichlorodifluoromethane, trichloro-fluoromethane, dichlorotetrafluoroethane, carbon dioxide or other suitable gas or mixture of gases.

The compositions may, if desired, be presented in a pack or dispenser device which may contain one or more unit dosage forms containing the active ingredient. The pack or dispensing device may be accompanied by instructions for administration.

The quantity of a compound of the invention required for the prophylaxis or treatment of a particular condition will vary depending on the compound chosen, and the condition of the patient to be treated. In general, however, daily dosages may range from around 100ng/kg to 100mg/kg e.g. around 0.01mg/kg to 40mg/kg body weight for oral or buccal administration, from around 10ng/kg to 50mg/kg body weight for parenteral administration and around 0.05mg to around 1000mg e.g. around 0.5mg to around 1000mg for nasal administration or administration by inhalation or insufflation.

The compounds of the invention may be prepared by a number of processes as generally described below and more specifically in the Examples hereinafter. In the following process description, the symbols Ar¹, Ar², Alk, R¹, R², R³, L¹, L², Alk¹, R^{x}, R^{y}, R^{z} and n when used in the formulae depicted are to be understood to represent those groups described above in relation to formula (1) unless otherwise indicated. In the reactions described below, it may be necessary to protect reactive functional groups, for example hydroxy, amino, thio or carboxy groups, where these are desired in the final product, to avoid their unwanted participation in the reactions. Conventional protecting groups may be used in accordance with standard practice [see, for example, Green, T. W. in "Protective Groups in Organic Synthesis", John Wiley and Sons, 1999]. In some instances, deprotection may be the final step in the synthesis of a compound of formula (1) and the processes according to the invention described hereinafter are to be understood to extend to such removal of protecting groups. For convenience the processes described below all refer to a preparation of a compound of formula (1) but clearly the description applies equally to the preparation of compounds of formula (2).

Thus according to a further aspect of the invention, a compound of formula (1) in which R is a -CO₂H group may be obtained by hydrolysis of an ester of formula (1a): 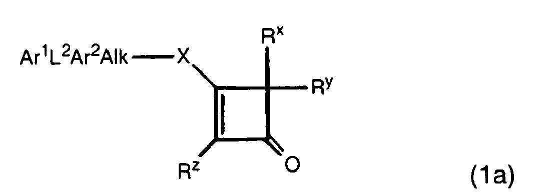 where Alk represents a group
-CH₂CH(CO₂Alk⁷)-, -CH=CH(CO₂Alk⁷)-, or 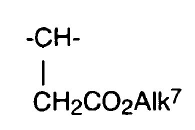 [where Alk⁷ is an alkyl group for example a C₁₋₆alkyl group].

The hydrolysis may be performed using either an acid or a base depending on the nature of Alk⁷, for example an organic acid such as trifluoroacetic acid or an inorganic base such as lithium, sodium or potassium hydroxide optionally in an aqueous organic solvent such as an amide e.g. a substituted amide such as dimethylformamide, an ether e.g. a cyclic ether such as tetrahydrofuran or dioxane or an alcohol e.g. methanol at a temperature from ambient to the reflux temperature. Where desired, mixtures of such solvents may be used.

According to a further aspect of the invention a compound of formula (1) may be prepared by condensation of a compound of formula (3): 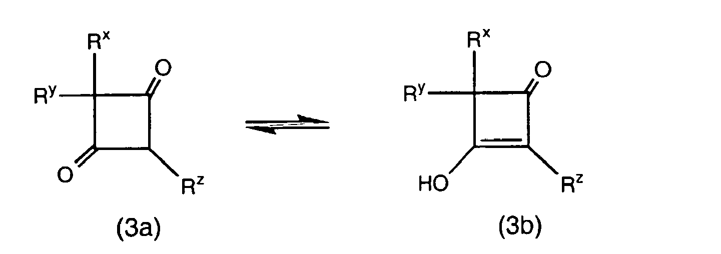 where compounds of formula (3) exist as two tautomeric isomers, (3a) and (3b) in solution with an amine of formula R¹R²NH, an alcohol of formula R¹OH or a thiol of formula R¹SH.

The reaction may be performed in an inert solvent or mixture of solvents, for example a hydrocarbon such as an aromatic hydrocarbon e.g. benzene or toluene and/or a halogenated hydrocarbon such as 1,2-dichloroethane, or dichloromethane at a temperature from 0°C to the reflux temperature. Where necessary, for example when a salt of an amine R¹R²NH is used, an organic base such as diisopropylethylamine can be added.

Any carboxylic acid group present in the intermediate of formula (3) or the amine R¹R²NH, alcohol R¹OH or thiol R¹SH may need to be protected during the displacement reaction, for example as an ethyl ester. The desired acid may then be obtained through subsequent hydrolysis, for example as particularly described above and generally described below.

The displacement reaction may also be carried out on an intermediate of formula 4 (see below) under the conditions just described.

Where desired the displacement reaction may also be performed on an intermediate of formulae (3), R¹R²NH, R¹OH or R¹SH which is linked, for example via its R, R¹ or R³ group, to a solid support, such as a polystyrene resin. After the reaction the desired compound of formula (1) may be displaced from the support by any convenient method, depending on the original linkage chosen.

Intermediates of formulae (3) R¹R²NH, R¹OH and R¹SH may be obtained from simpler, known compounds by one or more standard synthetic methods employing substitution, oxidation, reduction or cleavage reactions. Particular substitution approaches include conventional alkylation, arylation, heteroarylation, acylation, thioacylation, halogenation, sulphonylation, nitration, formylation and coupling procedures. It will be appreciated that these methods may also be used to obtain or modify other compounds of formulae (1) and (2a), (2b), (2c) and (2d) where appropriate functional groups exist in these compounds.

Thus intermediates of formula (3) may be obtained by hydrolysis of intermediates of formula (4): 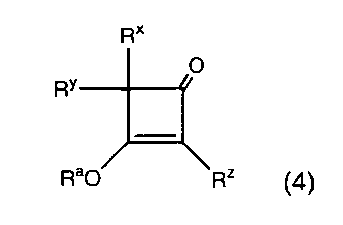 where R^{a} represents a C₁₋₆alkyl group or a silyl group such as a ^{t}butyldimethylsilyl group.

The hydrolysis may be performed using an acid, for example an inorganic acid such as hydrochloric acid in an organic solvent such as an ether e.g. diethylether, or an alcohol e.g. ethanol optionally in the presence of added water at a temperature from about ambient to 80°C.

Intermediates of formula (4) may be obtained by the cycloaddition of an intermediate of formula (5): 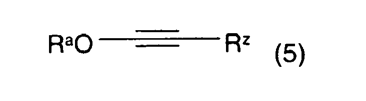 with a ketene of formula (6): 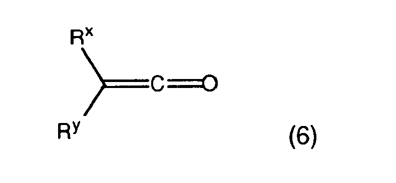 preformed or generated *in situ* during the cycloaddition reaction from an acid chloride of formula (7):

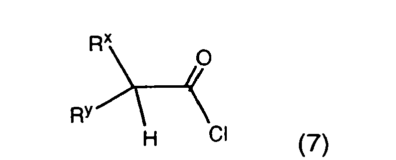

The reaction may be performed in the presence of an organic base such as an amine e.g. triethylamine or N,N-diisopropylethylamine or a cyclic amine such as pyridine or N-methylmorpholine optionally in an organic solvent such as an ether e.g. diethylether or diisopopylether.

Acid chlorides of formula (7) may be obtained from the corresponding acids by a convenient method of generating acid halides, for example by reaction with thionyl chloride or oxalyl chloride under such standard conditions as are well known in the art.

Compounds of formula (1a) in which R^{z} is for example a halogen atom may be obtained from compounds of formula (1a) in which R^{z} is a hydrogen atom by reaction with a halogen source such as bromine or a halosuccinamide e.g. chloro or bromosuccinamide. The reaction may be performed in a solvent such as an ether e.g. a cyclic ether such as tetrahydrofuran at a temperature from about 0° to 30°. When bromine is used as halogen source the reaction may optionally be performed in the presence of added base such as an amine e.g. triethylamine.

Further compounds of formula (1a) in which R^{z} is a group -L¹(Alk¹)ₙ(R³)ᵥ in which L¹ is for example a Se, S, O or N(R⁸) may be prepared by reaction of an intermediate of formula HL¹(Alk¹)ₙ(R³)ᵥ with a compound of formula (1a) in which R^{z} is a hydrogen atom. The reaction may be performed in an organic solvent such as an ether e.g. a cyclic ether such as tetrahydrofuran at around room temperature optionally in the presence of a base such as an amine e.g. triethylamine.

Intermediate compounds of formula (4) may also be obtained from squaric acid derivations by such well known methods in the art as those of MacDougall, J. M. *et al,* J. Org. Chem, 64 5979-83 (1999); Hergueta, R.

A., J. Org. Chem., 64, 5979-83, (1999); Heileman, M. J. *et al,* J. Am. Chem. Soc. 120, 3801-2, (1998); Yamamoto, Y. *et al,* J. Org. Chem, 62, 1292-8 (1997); Zhag, D. *et al,* J. Org. Chem. 61, 2594-5 (1996); Petasis, N. A. *et al,* Synlett, 155-6 (1996); Petasis, N. A. *et al,* Tetrahedron Lett., 36, 6001-4, (1995); Turnbull, P. *et al,* J. Org. Chem 60, 644-9 (1995); Yerxa, B. R. *et al,* Tetrahedron, 50, 6173-80 (1994); Ezcurra, J. E. *et al,* Tetrahedron Lett, 34, 6177-80, (1993); Ohno, M. *et al,* Tetrahedron Lett., 34, 4807-10, (1993); Yerxa, B. R. *et al,* Tetrahedron Lett. 33, 7811-14 (1992); Xu, S. L. *et al,* J. Org. Chem, 57, 326-8 (1992) and Kravs, J .L. *et al,* Tetrahedron Lett. 28, 1765-8 (1987).

Further compounds of the invention and intermediates thereto may be prepared by alkylation, arylation or heteroarylation. For example, compounds containing a -L¹H or -L²H group (where L¹ and L² is each a linker atom or group) may be treated with a coupling agent R³(Alk¹)ₙX¹ or Ar¹X¹ respectively in which X¹ is a leaving atom or group such as a halogen atom, e.g. a fluorine, bromine, iodine or chlorine atom or a sulphonyloxy group such as an alkylsulphonyloxy, e.g. trifluoro-methylsulphonyloxy or arylsulphonyloxy, e.g. p-toluenesulphonyloxy group.

The reaction may be carried out in the presence of a base such as a carbonate, e.g. caesium or potassium carbonate, an alkoxide, e.g. potassium t-butoxide, or a hydride, e.g. sodium hydride, or an organic amine e.g. triethylamine or N,N-diisopropylethylamine or a cyclic amine, such as N-methylmorpholine or pyridine, in a dipolar aprotic solvent such as an amide, e.g. a substituted amide such as dimethylformamide or an ether, e.g. a cyclic ether such as tetrahydrofuran.

Compounds of formula Ar¹X¹ may be prepared from alcohols of formula Ar¹OH by reaction with a halogenating agent, for example a phosphorous oxyhalide such as phosphorous oxychloride at an elevated temperature e.g. 110°C.

Intermediate alcohols of formula Ar¹OH in which, for example, Ar¹ represents a 2,6-naphthyridine may be prepared by methods well known to a person skilled in the art, e.g. by the method of Sakamoto, T, *et al* [Chem. Pharm. Bull. 33, 626-633, (1985)].

Alternatively alkylating agents of formula Ar¹X¹ in which, for example, Ar¹ represents a 2,6-naphthyridine may be prepared by reaction of a 2,6-naphthyridine N-oxide or N, N'-dioxide with a halogenating agent, e.g. a phosphorous oxyhalide such as phosphorous oxychloride to give a 1-halo or 1,5-dihalo-2,6-napthyridine respectively. In the case of 1,5-dihalo-2,6-napthyridines each halogen atom may be substituted separately by a reagent such as HL²Ar²AlkN(R²)H or HL³(Alk²)ₜL⁴(R⁴)ᵤ by the particular methods just described above.

L³ and L⁴, which may be the same or different, is each a covalent bond or a linker atom or group, t is zero or the integer 1, u is an integer 1, 2 or 3, Alk² is an optionally substituted aliphatic or heteroaliphatic chain and R⁴ is a hydrogen or halogen atom or a group selected from optionally substituted C₁₋₆alkyl or C₃₋₈cycloalkyl, -OR⁵ [where R⁵ is a hydrogen atom, an optionally substitued C₁₋₆alkyl or C₃₋₈cycloalkyl group], -SR⁵, -NR⁵R⁶ [where R⁶ is as just defined for R⁵ and may be the same or different], - NO₂, -CN, -CO₂R⁵, -SO₃H, -SOR⁵, -SO₂R⁵, -SO₃R⁵, -OCO₂R⁵, - CONR⁵R⁶, -OCONR⁵R⁶, -CSNR⁵R⁶, -COR⁵, -OCOR⁵, -N(R⁵)COR⁶, - N(R⁵)CSR⁶, -SO₂N(R⁵)(R⁶), -N(R⁵)SO₂R⁶, N(R⁵)CON(R⁶)(R⁷) [where R⁷ is a hydrogen atom, an optionally substituted C₁₋₆alkyl or C₃₋₈cycloalkyl group], -N(R⁵)CSN(R⁶)(R⁷) or -N(R⁵)SO₂N(R⁶)(R⁷), provided that when t is zero and each of L³ and L⁴ is a covalent bond then u is the integer 1 and R⁴ is other than a hydrogen atom. 2,6-Napthyridine N-oxides and N,N'-dioxides may be generated from the corresponding 2,6-napthyridines by the general methods of synthesis of N-oxides described below or they may be synthesised by the methods of Numata, A. *et al* (Synthesis, 1999, 306-311).

Further alkylating agents of formula Ar¹X¹ in which, for example, Ar¹ represents a 2,6-naphthyridine, may be prepared by the methods of Giacomello G. *et al* [Tetrahedron Letters, 1117-1121 (1965)], Tan, R. and Taurins, A. [Tetrahedron Lett., 2737-2744, (1965)], Ames, D. E. and Dodds, W. D. [J. Chem. Soc. Perkin 1, 705-710 (1972)] and Alhaique, F. *et al* [Tetrahedron Lett., 173-174 (1975)].

Intermediate alcohols of formula Ar¹OH in which Ar¹ represents an optionally substituted 2,7-naphthyridin-1-yl group may be prepared by methods well known to a person skilled in the art, e.g. by the method of Sakamoto,T. *et al* [Chem. Pharm. Bull. 33, 626-633, (1985)] or Baldwin, J, J. *et al* [J. Org. Chem, 43, 4878-4880, (1978)]. Thus for example the method of Baldwin may be modified to allow the synthesis of intermediate 3-substituted 2,7-naphthyridin-1-yl groups of formula Ar¹OH as depicted in Scheme 1.

Reaction of an optionally substituted 4-methyl-3-cyano pyridine of formula (8) with a N,N-dimethylformamide di-C₁₋₆alkyl acetal, e.g. N,N-dimethylformamide diethyl acetal, in a dipolar solvent such as an amide e.g. a substituted amide such as dimethylformamide at an elevated temperature e.g. 140-150° gives a compound of formula (9) or (10) or a mixture thereof depending on the nature of the group R¹⁶.

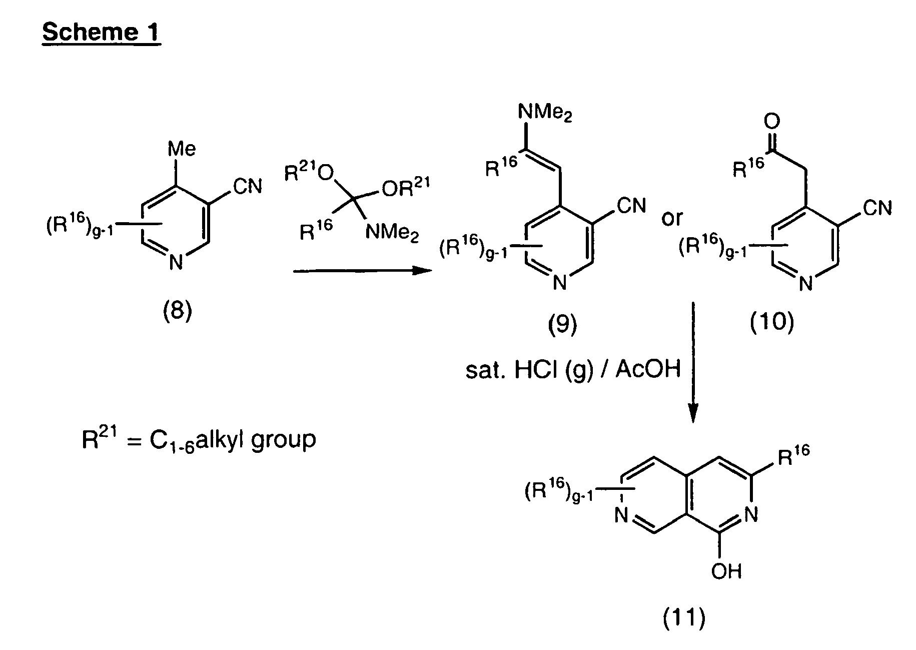

Compounds of formula (9) or (10) may be cyclised to 3-substituted 2,7-naphthyridin-1-yl alcohol of formula (11) by treatment with an acid e.g. an inorganic acid such as hydrochloric acid or hydrobromic acid or an acidic gas such as hydrogen chloride gas in an organic solvent e.g. an organic acid such as acetic acid optionally in the presence of water at a temperature from about ambient to 50⁰C.

Alternatively alkylating agents of formula Ar¹X¹ in which Ar¹ represents an optionally substituted 2,7-naphthyridin-yl group may be prepared by reaction of a 2,7-naphthyridine N-oxide or N,N'-dioxide with a halogenating agent, e.g. a phosphorous oxyhalide such as phosphorous oxychloride to give a 1-halo or 1,6-dihalo- and/or-1,8-dihalo-2,7-napthyridine respectively. In the case of 1,6-dihalo- and/or 1,8-dialo-2,6-napthyridines each halogen atom may be substituted separately by a reagent such as HL²Ar²AlkN(R²)H or HL³(Alk²)ₜL⁴(R⁴)ᵤ by the particular methods just described above. 2,7-Napthyridine N-oxides and N,N'-dioxides may be generated from the corresponding 2,7-napthyridines by the general methods of synthesis of N-oxides described below or they may be synthesised by the methods of Numata, A. *et al* (Synthesis, 1999, 306-311).

Further alkylating agents of formula Ar¹X¹ in which, for example, Ar¹ represents a 2,7-naphthyridin-1-yl, may be prepared by the methods of Wenkert E. *et al* J. Am. Chem. Soc. 89, 6741-5 (1967), and Aust. J. Chem. 433 (1972), and Sheffield D.J. J. Chem. Soc. Perkin. Trans I, 2506 (1972).

Intermediate alcohols of formula Ar¹OH in which Ar¹ represents a 3-substituted isoquinolin-1-yl group may be prepared by methods well known to a person skilled in the art, e.g. by the methods of Wu M.-J. *et al* Tetrahedron, 55, 13193-200 (1999), Hiebl J. *et al* Tetrahedron Lett. 40, 7935-8 (1999), Nagarajan A. *et al* Indian J. Chem., Sect. B, 28B, 67-78 (1989), Brun E. M. *et al* Synlett, 7, 1088-90 (1999) and Brun, E. M. *et al* Synthesis, 273-280 (2000).

Further alkylating agents of formula Ar¹X¹ in which, for example, Ar¹ represents a isoquinolin-1-yl group, may be prepared by the methods of Falk H. *et al* Monatsch. Chem. 25, 325-33 (1994), and Deady, L. W. *et al* Aust. J. Chem 42, 1029-34 (1989).

In a further example intermediates of formula R¹R²NH may be obtained by reaction of a compound of formula Ar¹L²H with a compound of formula X¹Ar²AlkN(R²)H under the reaction conditions just described

Compounds of formula Ar¹ L²H in which, for example Ar¹ represents a 2,6-naphthyridine and L² is a -N(R⁸)- group, may be prepared from substituted 4-cyano-3-cyanomethylpyridines by the methods of Alhaique, F. *et al* (*ibid* and Gazz. Chim. Ital. 1975, 105, 1001-1009) or from 3-fomylpyridines by the methods of Molina, P. at al (Tetrahedron 1992, 48, 4601-4616).

Compounds of formula Ar¹ L²H in which, for example Ar¹ represents a 2,7-naphthyridin-1-yl group and L² is a -N(R⁸)- group, may be prepared from substituted 4-formylpyridines by the methods of Molina, P. *et al* Tetrahedron, 48, 4601-4616, (1992), or by the methods described in US 3,938,367.

Compounds of formula Ar¹L²H in which, for example Ar¹ represents a 3-substituted isoquinolin-1-yl group and L² is a -N(R⁸)- group, may be prepared by the methods of Bordner, J. *et al* J. Med. Chem. 31, 1036-9 (1988), Tovar J. D. *et al* J. Org. Chem., 64, 6499-6504 (1999), Karser E. M. *et al* Synthesis, 11, 805-6 (1974), and Molino, P *et al* J. Chem. Soc. Perkin Trans. 1, 1727-31 (1990).

In another example, compounds containing a -L¹H or -L²H or group as defined above may be functionalised by acylation or thioacylation, for example by reaction with one of the alkylating agents just described but in which X¹ is replaced by a -C(O)X², -C(S)X², -N(R⁸)COX² or -N(R8)C(S)X2 group in which X² is a leaving atom or group as described for X¹. The reaction may be performed in the presence of a base, such as a hydride, e.g. sodium hydride or an amine, e.g. triethylamine or N-methylmorpholine, in a solvent such as a halogenated hydrocarbon, e.g. dichloromethane or carbon tetrachloride or an amide, e.g. dimethylformamide, at for example ambient temperature. Alternatively, the acylation may be carried out under the same conditions with an acid (for example one of the alkylating agents described above in which X¹ is replaced by a -CO₂H group) in the presence of a condensing agent, for example a diimide such as 1-(3-dimethylaminopropyl)-3-ethylcarbodiimide or N,N'-dicyclohexylcarbodiimide, advantageously in the presence of a catalyst such as a N-hydroxy compound e.g. a N-hydroxytriazole such as 1-hydroxybenzotriazole. Alternatively the acid may be reacted with a chloroformate, for example ethylchloroformate, prior to the desired acylation reaction.

In a further example compounds may be obtained by sulphonylation of a compound containing an -OH group by reaction with one of the above alkylating agents but in which X¹ is replaced by a -S(O)Hal or -SO₂Hal group [in which Hal is a halogen atom such as chlorine atom] in the presence of a base, for example an inorganic base such as sodium hydride in a solvent such as an amide, e.g. a substituted amide such as dimethylformamide at for example ambient temperature.

In another example, compounds containing a -L¹H or -L²H group as defined above may be coupled with one of the alkylation agents just described but in which X¹ is replaced by an -OH group in a solvent such as tetrahydrofuran in the presence of a phosphine, e.g. triphenylphosphine and an activator such as diethyl, diisopropyl- or dimethylazodicarboxylate.

In a further example, ester groups -CO₂R⁵, -CO₂R¹¹ or -CO₂Alk⁷ in the compounds may be converted to the corresponding acid [-CO₂H] by acid-or base-catalysed hydrolysis depending on the nature of the groups R⁵, R¹¹ or Alk⁷. Acid- or base-catalysed hydrolysis may be achieved for example by treatment with an organic or inorganic acid, e.g. trifluoroacetic acid in an aqueous solvent or a mineral acid such as hydrochloric acid in a solvent such as dioxan or an alkali metal hydroxide, e.g. lithium hydroxide in an aqueous alcohol, e.g. aqueous methanol.

In a further example, -OR⁵ or -OR¹⁴ groups [where R⁵ or R¹⁴ each represents an alkyl group such as methyl group] in compounds of formula (1) may be cleaved to the corresponding alcohol -OH by reaction with boron tribromide in a solvent such as a halogenated hydrocarbon, e.g. dichloromethane at a low temperature, e.g. around -78°C.

Alcohol [-OH] groups may also be obtained by hydrogenation of a corresponding -OCH₂R¹⁴ group (where R¹⁴ is an aryl group) using a metal catalyst, for example palladium on a support such as carbon in a solvent such as ethanol in the presence of ammonium formate, cyclohexadiene or hydrogen, from around ambient to the reflux temperature. In another example, -OH groups may be generated from the corresponding ester [CO₂Alk⁷ or CO₂R⁵] or aldehyde [-CHO] by reduction, using for example a complex metal hydride such as lithium aluminium hydride or sodium borohydride in a solvent such as methanol.

In another example, alcohol -OH groups in the compounds may be converted to a corresponding -OR⁵ or -OR¹⁴ group by coupling with a reagent R⁵OH or R¹⁴OH in a solvent such as tetrahydrofuran in the presence of a phosphine, e.g. triphenylphosphine and an activator such as diethyl-, diisopropyl-, or dimethylazodicarboxylate.

Aminosulphonylamino [-NHSO₂NHR³ or -NHSO₂NHAr¹] groups in the compounds may be obtained, in another example, by reaction of a corresponding amine [-NH₂] with a sulphamide R³NHSO₂NH₂ or Ar¹NHSO₂NH₂ in the presence of an organic base such as pyridine at an elevated temperature, e.g. the reflux temperature.

In another example compounds containing a -NHCSAr¹, -CSNHAr¹, - NHCSR³ or -CSNHR³ may be prepared by treating a corresponding compound containing a -NHCOAr¹, -CONHAr¹, -NHCOR³ or -CONHR³ group with a thiation reagent, such as Lawesson's Reagent, in an anhydrous solvent, for example a cyclic ether such as tetrahydrofuran, at an elevated temperature such as the reflux temperature.

In a further example amine (-NH₂) groups may be alkylated using a reductive alkylation process employing an aldehyde and a borohydride, for example sodium triacetoxyborohyride or sodium cyanoborohydride, in a solvent such as a halogenated hydrocarbon, e.g. dichloromethane, a ketone such as acetone, or an alcohol, e.g. ethanol, where necessary in the presence of an acid such as acetic acid at around ambient temperature.

In a further example, amine [-NH₂] groups in compounds of formula (1) may be obtained by hydrolysis from a corresponding imide by reaction with hydrazine in a solvent such as an alcohol, e.g. ethanol at ambient temperature.

In another example, a nitro [-NO₂] group may be reduced to an amine [-NH₂], for example by catalytic hydrogenation using for example hydrogen in the presence of a metal catalyst, for example palladium on a support such as carbon in a solvent such as an ether, e.g. tetrahydrofuran or an alcohol e.g. methanol, or by chemical reduction using for example a metal, e.g. tin or iron, in the presence of an acid such as hydrochloric acid.

Aromatic halogen substituents in the compounds may be subjected to halogen-metal exchange with a base, for example a lithium base such as n-butyl or t-butyl lithium, optionally at a low temperature, e.g. around - 78°C, in a solvent such as tetrahydrofuran and then quenched with an electrophile to introduce a desired substituent, for example, a formyl group may be introduced by using dimethylformamide as the electrophile; a thiomethyl group may be introduced by using dimethyldisulphide as the electrophile.

In another example, sulphur atoms in the compounds, for example when present in a linker group L¹ or L² may be oxidised to the corresponding sulphoxide or sulphone using an oxidising agent such as a peroxy acid, e.g. 3-chloroperoxybenzoic acid, in an inert solvent such as a halogenated hydrocarbon, e.g. dichloromethane, at around ambient temperature.

In another example compounds of formula Ar¹X¹ (where X¹ is a halogen atom such as a chlorine, bromine or iodine atom) may be converted to such compounds as Ar¹CO₂R²⁰ (in which R²⁰ is an optionally substituted alkyl, aryl or heteroaryl group), Ar¹CHO, Ar¹CHCHR²⁰, Ar¹CCR²⁰, Ar¹N(R²⁰)H, Ar¹N(R²⁰)₂, for use in the synthesis of for example compounds of formula Ar¹L²Ar²AlkN(R²)H, using such well know and commonly used palladium mediated reaction conditions as are to be found in the general reference texts *Rodd's Chemistry of Carbon Compounds,* Volumes 1-15 and Supplementals (Elsevier Science Publishers, 1989), *Fieser and Fieser's Reagents for Organic Synthesis,* Volumes 1-19 (John Wiley and Sons, 1999), *Comprehensive Heterocyclic Chemistry,* Ed. Katritzky *et al,* Volumes 1-8, 1984 and Volumes 1-11, 1994 (Pergamon), *Comprehensive Organic Functional Group Transformations,* Ed. Katritzky *et al,* Volumes 1-7, 1995 (Pergamon), *Comprehensive Organic Synthesis,* Ed. Trost and Flemming, Volumes 1-9, (Pergamon, 1991), *Encyclopedia of Reagents for Organic Synthesis,* Ed. Paquette, Volumes 1-8 (John Wiley and Sons, 1995), *Larock's Comprehensive Organic Transformations* (VCH Publishers Inc., 1989) and *March's Advanced Organic Chemistry* (John Wiley and Sons, 1992).

N-oxides of compounds of formula (1) may be prepared for example by oxidation of the corresponding nitrogen base using an oxidising agent such as hydrogen peroxide in the presence of an acid such as acetic acid, at an elevated temperature, for example around 70°C to 80°C, or alternatively by reaction with a peracid such as peracetic acid in a solvent, e.g. dichloromethane, at ambient temperature.

Salts of compounds of formula (1) may be prepared by reaction of a compound of formula (1) with an appropriate base in a suitable solvent or mixture of solvents e.g. an organic solvent such as an ether e.g. diethylether, or an alcohol, e.g. ethanol using conventional procedures.

Where it is desired to obtain a particular enantiomer of a compound of formula (1) this may be produced from a corresponding mixture of enantiomers using any suitable conventional procedure for resolving enantiomers.

Thus for example diastereomeric derivatives, e.g. salts, may be produced by reaction of a mixture of enantiomers of formula (1) e.g. a racemate, and an appropriate chiral compound, e.g. a chiral base. The diastereomers may then be separated by any convenient means, for example by crystallisation and the desired enantiomer recovered, e.g. by treatment with an acid in the instance where the diastereomer is a salt.

In another resolution process a racemate of formula (1) may be separated using chiral High Performance Liquid Chromatography. Alternatively, if desired a particular enantiomer may be obtained by using an appropriate chiral intermediate in one of the processes described above. Alternatively, a particular enantiomer may be obtained by performing an enantiomer specific enzymatic biotransformation e.g. an ester hydrolysis using an esterase and then purifying only the enantiomerically pure hydrolysed acid from the unreacted ester antipode.

Chromatography, recrystallisation and other conventional separation procedures may also be used with intermediates or final products where it is desired to obtain a particular geometric isomer of the invention.

The following Examples illustrate the invention. All temperatures are in °C.
The following abbreviations are used:
- NMM -: N-methylmorpholine;
- MeOH -: methanol;
- DCM -: dichloromethane;
- DIPEA -: diisopropylethylamine;
- Pyr -: pyridine;
- DMSO -: dimethylsulphoxide;
- Et₂O -: diethylether;
- THF -: tetrahydrofuran,
- FMOC -: 9-fluorenylmethoxycarbonyl;
- DBU -: 1,8-Diazabicyclo[5,4-0]undec-7-ene;
- DMAP -: 4-(dimethylamino)pyridine.
- HOBT -: 1-hydroxybenzotriazole
- EtOAc -: ethyl acetate;
- BOC -: butoxycarbonyl;
- AcOH -: acetic acid;
- EtOH -: ethanol;
- Ar -: aryl;
- iPr -: isopropyl;
- Me -: methyl;
- DMF -: N,N-dimethylformamide;

All NMR's were obtained either at 300MHz or 400MHz.

All Intermediates and Examples were named with the aid of Beilstein Autonom (available from MDL Information Systems GmbH, Therdor-Heuss-Allee 108D 60486, Frankfurt, Germany) or were given names that seemed consistent, with the exception that propanoates were named by the IUPAC name rather than the trivial name (propionate) and isonicotinoyl (trivial name) is used in place of pyridine-4-carbonyl.

### INTERMEDIATE 1

### (+/-) 3-Ethoxy-4-methyl-4-propyl-2-cyclobuten-1-one

The title compound was prepared using a modification of the method of Wasserman, H.H. *et al* [J. Org. Chem, 38, 1451-1455, (1973)]; to a solution of 2-methyl pentanoyl chloride (3.91ml) and ethyl ethynylether (5g, 40% solution in hexanes, 28.6mmol) in Et₂O (35ml) at room temperature was added triethylamine (9.9ml), with stirring. The reaction was warmed to 50º and stirred for 72h prior to cooling and filtration. The filtrate was concentrated *in vacuo* and the residual oil chromatographed (SiO₂; hexanes 80: EtOAc 20) to give the title compound as a colourless oil (3.71g, 17.9mmol, 77%). δH (CDCl₃, 300K), 4.84 (1H, s), 4.24-3.98 (2H, m), 2.04 (3H, s), 1.56-1.43 (4H, m), 1.30-1.26 (3H, m), 0.91 (3H, t, J 7.3Hz). m/z (ES⁺, 70V) 178.1 (MH⁺).

### INTERMEDIATE 2

### (+/-) 3-Hydroxy-4-methyl-4-proayl-2-cyclobuten-1-one

Intermediate 1 (1g, 59.5mmol) and conc. hydrochloric acid (2ml) were stirred vigorously at room temperature for 48h. The resulting slurry was filtered and the residue washed with water (3 x10ml) and dried under vacuum to give the title compound as an off-white powder (620mg, 44.2mmol, 74%). δH (CDCl₃, 300K) 3.79 (2H, s), 1.59-1.53 (2H, m), 1.41-1.27 (2H, m), 1.18 (3H, s), 0.85 (3H, t, J 7.3Hz). m/z (ES⁺, 70V) 140.9 (MH⁺).

### INTERMEDIATE 3

### 3-Cyano-4-(2-(N.N-dimethylamino)ethylen-1-yl)pyridine

A solution of 4-methyl-3-cyanopyridine [prepared acccording to Ref: J. Prakt. Chem. 338, 663 (1996)], (8.0g, 67.8mmol) and *N,N-*dimethylformamide diethyl acetal (11.0g, 74.8mmol) in dry DMF (50ml) was stirred at 140º under N₂ for 2 days. An additional portion of N,N,-dimethylformamide diethyl acetal (5g) was added and stirred at 140° for 4h. The volatiles were removed *in vacuo* and the obtained dark oil partitioned between EtOAc (300ml) and water (50ml). The phases were separated and the aqueous layer re-extracted with EtOAc (3x100ml). The combined organic extracts were washed with brine (30ml), dried (Na₂SO₄), treated with activated charcoal, filtered and evaporated in *vacuo* to afford essentially pure title compound as a dull orange solid (10.1g, 85%). δH (CDCl₃) 8.49 (1H, s), 8.25 (1h, d, J 5.9Hz), 7.29 (1H, d, J 13.2Hz), 7.09 (1H, d, J 5.9Hz), 5.25 (1 H, d, J 13.2Hz) and 2.99 (6H, s); m/z (ES⁺, 70V) 174 (MH⁺).

### INTERMEDIATE 4

### 1-Hydroxy-2,7-naphthyridine hydrochloride salt

HCl gas was bubbled through a stirred solution of Intermediate 3 (6.2g, 3.58mmol) in glacial acetic acid (50ml) and water (0.64ml, 3.55mmol) for 1-2min. The reaction mixture was stirred in a stoppered flask at 40º for 18h. The volatiles were removed *in vacuo* affording a dark residue, which was treated with water (3x20ml) and re-evaporated *in vacuo.* The obtained dark semi-solid was treated with 40ml warm ethanol, ice-cooled, and the undissolved solid collected by filtration affording the title compound as a green coloured solid (5.2g, 80%) δH (DMSO-d⁶) 12.5 (1H, br s), 9.38 (1H, s), 8.84 (1H, d, J 7.0Hz), 8.15 (1 H, d, J 7.0Hz), 7.89 (1H, br dd, J 7.0, 5.0Hz) and 6.85 (1H, d, J 7.0Hz); m/z (ES⁺, 70V), 147 (MH⁺).

### INTERMEDIATE 5

### 1-Chloro-2,7-naphthyridine

Intermediate 4 (5.2g, 28.5mmol) was stirred with phosphorous oxychloride (75ml) at 110° for 24h. The volatiles were removed *in vacuo* affording a dark oil which was poured into an ice-bath cooled mixture of saturated aqueous NaHCO₃ (100ml containing 20g solid NaHCO₃) and EtOAc (100ml). After thorough mixing the phases were separated and the aqueous layer re-extracted with EtOAc (2x75ml). The combined organic extracts were washed with brine (15ml), dried (Na₂SO₄) and evaporated *in vacuo* to afford the title compound as a yellow solid (4.0g, 85%) δH (CDCl₃) 9.45 (1H, s), 8.81 (1H, d, J 5.7Hz), 8.47 (1H, d, J 5.7Hz), 7.66 (1H, d, J 5.7Hz) and 7.60 (1H, d,J 5.7Hz); m/z (ES⁺, 70V) 165 and 167 (MH⁺).

### INTERMEDIATE 6

### Ethyl (2S)-2-amino-3-[4-(2,7-naphthyridin-1-ylamino)phenyl] propanoate

A solution of ethyl-(*S*)-3-[4-aminophenyl]-2-[*t*-butoxycarbonylamino] propanoate (638mg, 2.07mmol) and Intermediate 5 (310mg, 1.88mmol) in ethoxyethanol (2ml) was stirred at 120º for 15 min and at 100º for 1h under nitrogen. The volatiles were removed *in vacuo* and the dark residue partitioned between EtOAc (70ml) and saturated aqueous NaHCO₃ (10ml). The phases were separated and the aqueous layer re-extracted with EtOAc (2x30ml). The combined organic extracts were washed with brine (10ml), dried (Na₂SO₄) and evaporated *in vacuo* to afford a dark foam. Chromatography (SiO₂; 5 to 10% MeOH/DCM) afforded a mixture of ethyl-(*S*)-3-[4-(2,7-naphthyridin-1-ylamino)phenyl]-2-[(*t*-butoxycarbonyl) amino]propanoate and some of the title compound (730mg). This mixture was treated with a solution of trifluoroacetic acid (5ml) and DCM (5ml) at room temperature for 1h. The volatiles were removed *in vacuo* and the residue partitioned between EtOAc (75ml) and saturated aqueous NaHCO₃ (20ml). The phases were separated and the aqueous layer re-extracted with EtOAc (3x30ml). The combined organic extracts were washed with brine (10ml), dried (Na₂SO₄) and evaporated *in vacuo* to afford an orange solid. Chromatography (SiO₂; 10% MeOH/DCM) afforded the title compound as a straw-coloured solid (420mg, 60% over two steps). δH (CDCl₃) 10.70 (1H, s), 10.31 (1H, s), 9.44 (1H, d, J 5.6Hz), 8.94 (1H, d, J 5.6Hz), 8.55 (1H, d, J 7.3Hz), 8.54 (2H, d, J 8.5Hz), 8.46 (1H, d, J 5.6Hz), 7.94 (2H, d, J 8.5Hz), 4.84 (2H, q, J 7.1 Hz), 4.35 (1H, t, J 6.6Hz), 4.10 (2H, br s), 3.64 (1H, dd, J 13.5, 6.4Hz), 3.56 (1H, dd, J 13.5, 7.0Hz) and 1.95 (3H, t, J 7.1 Hz); m/z (ES⁺, 70V) 337 (MH⁺).

### INTERMEDIATE 7

### Methyl (2S)-2-amino3-[4-(2,7-naphthyridin-1-yloxy)phenyl]propanoate

A mixture of *N*-(BOC)-(*S*)-tyrosine methyl ester (1.71g, 5.80 mmol) potassium carbonate (0.80g, 5.80mmol) and Intermediate 5 (1.0g, 6.08mmol) in dry DMF (10ml) was stirred at room temperature for 18h, and at 40º for 18h. The DMF was removed *in vacuo* and the residue partitioned between EtOAc (80ml) and 10% aqueous Na₂CO₃ (20ml). The phases were separated and the aqueous layer re-extracted with EtOAc (2x20ml). The combined organic extracts were washed with brine (10ml), dried (Na₂SO₄) and evaporated *in vacuo* to afford a new colourless oil. Chromatography (silica; 2.5% MeOH/DCM) afforded reasonably pure *N*-t-butoxycarbonyl protected title compound (1.75g, 71%). This material was dissolved in EtOAc (40ml) and HCl gas was bubbled through the stirred solution for 1 min. then the mixture was stirred for an additional 0.5h. The volatiles were removed *in vacuo* affording a yellow solid which was partitioned between EtOAc (80ml) and saturated aqueous NaHCO₃ (20ml). The phases were separated and the aqueous layer re-extracted with EtOAc (2x20ml). The combined organic extracts were washed with brine (10ml), dried (Na₂SO₄) and evaporated *in vacuo.* The obtained oil was chromatographed (SiO₂; 5% MeOH/DCM) to afford the title compound as a near colourless oil (0.83g, 62%) δH (CDCl₃) 9.77 (1H, s), 8.75 (1H, d, J 5.8Hz), 8.10 (1H, d, J 5.8Hz), 7.58 (1H, d, J 5.8Hz), 7.29 (2H, d, J 8.4Hz), 7.25 (1H, d, J 5.9Hz), 7.21 (2H, d, J 8.4Hz), 3.80-3.70 (1H, obscured m), 3.72 (3H, s), 3.15 (1H, dd, J 13.6, 5.1 Hz), 2.88 (1H, dd, J 13.6, 8.0Hz) and 0.78 (2H, br s); m/z (ES⁺, 70V) 324 (MH⁺).

### INTERMEDIATE 8

### Ethyl (2S)-2-[(tert-butoxycarbonyl)amino]-3-{4-[(3-methyl[2,7]naphthyridin-1-yl)oxy]phenyl}propanoate

A mixture of *N*-*t*-butyloxycarbonyl-(*S*)-tyrosine ethyl ester (14.5g, 46.9mmol), caesium carbonate (14.05g, 43.1mmol) and Intermediate 3 (7.0g, 39.2mmol) in dry DMF (60ml) was stirred at room temperature for 48h. The reaction was diluted with Et₂O (150ml) and filtered off. The filtrate was evaporated under high vacuum and the residue was chromatographed (SiO₂; 40%-60% EtOAc/Hexane) which afforded the title compound as a viscous, straw-coloured oil (16.2g, 77%) δH (CDCl₃) 9.56 (1H, s), 8.58 (1H, d, J 5.8Hz), 7.39 (1H, d, J 5.8Hz), 7.15-7.10 (4H, m), 7.00 (1H, s), 4.99-4.91 (1H,m), 4.54-4.46 (1H, m), 4.09 (2H, q, J 7.1Hz), 3.10-2.99 (2H,m), 2.36 (3H, s), 1.34 (9H, s) and 1.15 (3H, t, J 7.1 Hz); m/z (ES⁺, 70V) 452 (MH⁺).

### INTERMEDIATE 9

### Ethyl (2S)-2-amino-3-{4-[(3-methyl[2,7]naphthyridin-1-yl)oxy]phenyl} propanoate

HCl gas was bubbled through a stirred solution of Intermediate 8 (16g) in EtOAc (300ml) until a persistent fine white precipitate formed (-2minutes). After stirring for 0.5h the volatiles were removed *in vacuo.* The obtained solid was partitioned between EtOAc (250ml) and saturated aqueous NaHCO₃ (80ml plus 5g solid NaHCO₃). The phases were separated and the aqueous layer re-extracted with EtOAc (5x50ml). The combined organic extracts were washed with brine (10ml), dried (Na₂SO₄) and evaporated *in vacuo* to afford an oil. Chromatography (SiO₂; 100% EtOAC-10% EtOH/EtOAc) afforded the title compound as a viscous oil (11.1g, 89%). δH (CDCl₃) 9.71 (1H, s), 8.70 (1H, d, J 5. Hz), 7.50 (1H, d, J 5.8Hz), 7.31-7.28 (4H,m), 7.11 (1H, s), 4.23 (2H, q, J 7.1Hz), 3.79-3.72 (1H, m), 3.14 (1H, dd, J 14.1, 5.4Hz), 2.94 (1H, dd, J 14.1, 7.8Hz), 2.47 (3H, s), 1.75-1.50 (2H, br s) and 1.30 (3H, t, J 7.1 Hz); m/z (ES⁺, 70V) 352 (MH⁺).

### INTERMEDIATE 10

### 1-Chloro-2,6-naphthyridine

1-Hydroxy-2,6-naphthyridine (550mg) [prepared according to the method of Sakamoto, T. *et al* Chem. Pharm. Bull. 33, 626, (1985)] was stirred with phosphorous oxychloride (10ml) at 110º for 5h. The volatiles were removed *in vacuo* and the residue treated carefully with ice. After diluting with water (to ~25ml), solid NaHCO₃ was added to effect neutralisation and the product extracted into EtOAc (2x80ml). The combined organic extracts were dried (MgSO₄), evaporated *in vacuo,* and the crude product chromatographed (SiO₂; EtOAc) affording the title compound as a slightly yellow solid (420mg, 68%). δH (CDCl₃) 9.35 (1H, s), 8.82 (1H, d, J 5.9Hz), 8.48 (1H, d, J 5.6Hz), 8.00 (1H, d, J 5.9Hz), 7.74 (1H, d, J 5.6Hz); m/z (ES⁺, 70V) 165 and 167 (MH⁺).

### INTERMEDIATE 11

### Ethyl (2S)-2-[(tert-butoxycarbonyl)amino]3-[4-([2,6]naphthyridin-1-ylamino)phenyl]propanoate

Ethyl (*S*)-3-(4-aminophenyl)-2-[N-(t-butyloxycarbonyl)amino]propanoate (600mg, 1.95mmol), Intermediate 10 (350mg, 2.13mmol) and DIPEA (276mg, 372µl, 2.13mmol) in 2-ethoxyethanol (0.5ml) were stirred at 130º under N₂ for several hours. The reaction was partitioned between EtOAc (70ml) and saturated aqueous NaHCO₃ (30ml). The phases were separated and the aqueous layer re-extracted with EtOAc (3x30ml). The combined organic extracts were washed with brine (10ml), dried (MgSO₄) and evaporated *in vacuo* to afford a dark oil. Chromatography (SiO₂; 3% MeOH/DCM) gave the title compound as a dull orange foam (360mg, 42%). δH (CDCl₃) 9.19 (1H, s), 8.67 (1H, d, J 5.9Hz), 8.24 (1H, d, J 5.8Hz), 7.66 (1H, d, J 5.9Hz), 7.65 (2H, d, J 8.5Hz), 7.21 (1H, d, J 5.8Hz), 7.16 (2H, d, J 8.5Hz), 7.15 (1H, obscured s), 5.05-4.97 (1H, m), 4.60-4.51 (1H, m), 4.19 (2H, q, J 7.1Hz), 3.17-3.04 (2H, m), 1.44 (9H, s), 1.27 (3H, t, J 7.1 Hz); m/z (ES⁺, 70V) 459 (MNa⁺), 437 (MH⁺).

### INTERMEDIATE 12

### Ethyl (2S)-2-amino-3-[4-([2,6]naphthyridin-1-ylamino)phenyl] propanoate

Intermediate 11 (360mg) was treated with a solution of trifluoroacetic acid (10ml) and DCM (10ml) and stirred at RT for 2h. The volatiles were removed *in vacuo* and the residue was partitioned between EtOAc (80ml) and saturated aqueous NaHCO₃ (30ml). The phases were separated and the aqueous layer re-extracted with EtOAc (3x30ml). The combined organic extracts were dried (MgSO₄) and evaporated *in vacuo* to afford the title compound as a dark orange viscous oil (280mg, 100%). δH (CDCl₃) 9.18 (1H, s), 8.66 (1H, d, J 5.9Hz), 8.22 (1H, d, J 5.8Hz), 7.67 (1H, d, J 5.9Hz), 7.64 (2H, d, J 8.5Hz), 7.22 (2H, d, J 8.5Hz), 7.19 (1H, d, J 5.8Hz), 4.20 (2H, q, J 7.1 Hz), 3.73 (1H, dd, J 7.9, 5.1 Hz), 3.10 (1H, dd, J 13.6, 5.2Hz), 2.87 (1H, dd, J 13.6, 7.9Hz), 1.70 (3H, br s), 1.28 (3H, t, 7.1 Hz); m/z (ES⁺, 70V) 337 (MH⁺).

### INTERMEDIATE 13

### 3,5-Dichloropyridine-4-carboxylic acid

A solution of 3,5-dichloropyridine (5.00g, 33.8mmol) in THF (25ml) was added to a solution of LDA [generated from nBuLi (2.5M solution in hexanes, 14.9ml, 37.2mmol) and diisopropylamine (4.10g, 5.7ml, 40.6mmol)] in THF (25ml) at -78º under nitrogen, to give a yellow/brown slurry. The reaction was stirred for 30min at -78º then CO₂ gas was bubbled through to give a clear brown solution that slowly gave a precipitate, warmed to RT over 2h, then quenched with water (20ml) and partitioned between Et₂O (100ml) and 1M NaOH (100ml). The aqueous layer was separated and acidified to pH 1 with concentrated hydrochloric acid and then extracted with 10% MeOH in DCM (100ml×3). The combined organic layers were dried (MgSO₄) and the solvent removed under vacuum to give a brown solid that was recrystallised from ethanol and dried under vacuum to give the title compound as pinkish crystals (2.63g, 41%). δH (DMSO-d⁶) 8.74 (2H, s). δC (DMSO-d⁶) 163.5, 147.7, 141.0, 126.7.

### INTERMEDIATE 14

### Ethyl (2S)-2-[(tert-butoxycarbonyl)amino]-3-{4-[(3,5-dichloroisonicotinoyl)amino]phenyl}propanoate

A slurry of the compound of Intermediate 13 (51.2g, 0.267mol) in DCM (195ml) and thionyl chloride (195ml, 2.67mol) was treated with DMF (5 drops) and heated to reflux for 4h. The reaction was concentrated in *vacuo* and azeotroped with toluene (2x50ml) to give a yellow solid which was used without further purification. A solution of ethyl-(*S*)-3-(4-aminophenyl)-2-(t-butoxycarbonyl amino)propionate (130.8g, 0.425mol) in DCM (800ml) was cooled to 0º and treated with NMM (56.0ml, 0.51mol), stirred for 5 minutes and then a solution of the acid chloride (98.3g, 0.468mol) in DCM (200ml) was added dropwise keeping the reaction temperature below 5º. The reaction was stirred for 1 h, quenched with NaHCO₃ solution (500ml), the organic layer separated, washed with NaHCO₃ solution (500ml), 10% citric acid solution (500ml) and NaHCO₃ solution (500ml), dried (MgSO₄) and concentrated *in vacuo* to give a yellow solid which was recrystallised (EtOAc/hexane) to give the title compound, (140g, 69%). δH (DMSO d⁶), 8.8 (2H, s), 7.55 (2H, d, J 8.5Hz), 7.23 (2H, d, J 8.5Hz), 4.0 (3H, m), 3.4 (2H, b s), 2.9 (1H, m), 2.8 (1H, m), 1.3 (9H, s), 1.25 (3H, t); m/z (ES⁺, 70V) 504 (MNa⁺).

### INTERMEDIATE 15

### Ethyl (2S)-2-amino-3-{4-[(3,5-dichloroisonicotinoyl)amino] phenyl}propanoate hydrochloride

A solution of the compound of Intermediate 14 (70g, 0.146mol) in EtOAc (500ml) and 1,4-dioxan (50ml) was treated with a solution of HCl in EtOAc (500ml, 3M), and stirred at room temperature for 4h. The reaction was concentrated *in vacuo* to give a yellow solid which was triturated with Et₂O then recrystallised (EtOAc/hexane) to give the title compound (59.3g, 92%). δH (DMSO d⁶), 11.10 (1H, s), 8.70 (2H, s), 7.55 (2H, d, J 8.4Hz), 7.25 (2H, d, J 8.4Hz), 4.10 (3H, m), 3.10 (2H, m), 1.10 (3H, m); m/z (ES⁺, 70V) 382 (MH⁺). (12.1g, 59%). δH (CDCl₃, 300K) 4.85 (1H, s), 4.23 (2H, q, J 7.1Hz), 3.89-3.75 (4H, m), 1.88-1.79 (4H, m), 1.47 (3H, t, J 7.1 Hz); m/z (ES⁺, 70V) 182.9 (MH⁺).

### INTERMEDIATE 16

### 3-Ethoxyspiro[3.6]decan-1-one.

A solution of cycloheptyl carbonyl chloride (10.0g, 0.062mol) and ethoxyacetylene (40%w/w solution in hexanes, 6.0g, 0.083mol, 12ml) in diethylether (50ml) was treated dropwise with triethylamine (20ml, 0.14mol) and the reaction stirred for 5d at room temperature. Filtration and concentration of the filtrate *in vacuo* followed by chromatography (SiO₂, 5:1 EtOAc:hexanes) gave the title compound as a pale yellow oil (10.5g, 0.054mol, 87%). δH (CDCl₃, 300K) 4.78 (1H, s), 4.20 (2H, q J 7.1 Hz), 1.94-1.87 (2H, m), 1.83-1.77 (2H, m), 1.71-1.66 (2H, m), 1.63-1.52 (6H, m), 1.45 (3H, t J 7.1Hz); m/z (ES⁺, 70V) 194.9 (MH⁺).

### INTERMEDIATE 17

### Spiro[3.6]decane-1,3-dione

Intermediate 16 (8.5g, 0.044mol) and 2M hydrochloric acid (30ml) was stirred vigorously for 24h at room temperature. The resulting slurry was extracted with EtOAc (3x100ml), the extracts combined and concentrated *in vacuo,* and the resulting solid was recrystallised from diethyl ether to give the title compound as an off-white powder (7.1g, 0.043mol, 95%). δH (DMSO d⁶, 300K) 4.58 (2H, s), 1.75-1.29 (12H, m); m/z (ES⁺, 70V) 166.9 (MH⁺).

### INTERMEDIATE 18

### 7-Acetyl-3-ethoxy-7-azaspiro[3.5]non-2-en-1-one.

A solution of 1-acetyl piperidine-4-carbonyl chloride (5.0g, 26.4mmol) and ethoxyacetylene (4.0g, 55.5mmol) in THF (60ml) was treated dropwise with triethylamine (7.6ml, 55.0mmol). The resulting slurry was stirred at room temperature for 5d prior to filtration and concentration of the filtrate *in vacuo.* Chromatography (SiO₂, 100% EtOAc to 95:5 EtOAc:MeOH) gave the title compound as a white powder (3.97g, 17.8mmol, 67%). δH (CDCl₃, 300K) 4.79 (1H, s), 4.17 (2H, q, J 7.1 Hz), 3.87-3.81 (1H, m), 3.56-3.42 (3H, m), 2.02 (3H, s), 1.85-1.67 (4H, m), 1.39 (3H, t, J 7.1 Hz); m/z (ES⁺, 70V) 223.9 (MH⁺).

### INTERMEDIATE 19

### 7-Acetyl-7-azaspiro[3.5]nonane-1,3-dione

Intermediate 18 (700mg, 0.31 mmol) and hydrochloric acid (2M, 5ml) were stirred at room temperature for 4h. Concentration of the resulting straw-coloured solution in vacuo gave the title compound as a pale brown watersoluble powder (535mg, 0.027mmol, 87%). m/z (ES⁺, 70V) 195.9 (MH⁺).

### INTERMEDIATE 20

### 3-Ethoxy-7-methoxyspiro[3.5]non-2-en-1-one

Was prepared from 4-methoxy cyclohexanecarbonyl chloride (10g, 52.1mmol) and ethoxyacetylene (7.5g, 0.10mol) according to the method of Intermediate 1 to give the title compound as an approx. 1:1 mixture of isomers, as a pale yellow oil (7.2g, 34.4mmol, 65%). δH (CDCl₃, 300K) 4.81-4.79 (1H, s), 4.22-4.20 (2H q, J 7.1Hz), 3.34-3.32 (3H, s), 3.31-3.22 (1H, m), 2.04-1.56 (8H, m), 1.44-1.43 (3H t, J 7.1Hz); m/z (ES⁺, 70V) 211.0 (MH⁺).

### INTERMEDIATE 21

### 7-Methoxyspiro[3.5]nonane-1,3-dione

Intermediate 20 (5.0g, 23.9mmol) and hydrochloric acid (2M, 20ml) were stirred at room temperature for 18h. The resulting slurry was then diluted with water (50ml) and extracted with EtOAc (3x25ml), the extracts were dried (MgSO₄), filtered, and concentrated *in vacuo.* Recrystallisation from diethylether gave the title compound as an off-white powder (4.06g, 22.4mmol, 94%). δH (CDCl₃, 300K) 3.81 (2H, s), 3.25 (4H, m) 1.96-1.90 (2H, m), 1.86-1.79 (2H, m), 1.73-1.66 (2H, m), 1.64-1.56 (2H, m); m/z (ES⁺, 70V) 182.9 (MH⁺).

### INTERMEDIATE 22

### Ethyl (2S)-2-amino-3-hydroxypropanoate hydrochloride

A mixture of (2*S*)-2-amino-3-hydroxypropanoate (25g, 238mmol) and acetyl chloride (34ml, 476mmol) in absolute ethanol (250ml) was stirred at 50° for 18hr. The volatiles were removed *in vacuo* until the volume was reduced to ~100ml. Upon cooling the resultant precipitate was collected, washed with ether and hexane to give the title compound as a white powder (26.3g, 65 %). δH NMR (DMSO d⁶) 8.47 (3H, br s), 5.58 (1H, dd), 4.20 (2H, q), 4.08 (1H, t), 3.81 (2H, dd), 1.23 (3H, t).

### INTERMEDIATE 23

### Ethyl (2S)-2-[(tert-butoxycarbonyl)amino]-3-hydroxypropanoate

Di-*tert*-butyl dicarbonate (10.26g, 47mmol) was added to a stirred mixture of Intermediate 22 (7.98g, 47mmol) and NaHCO₃ (8.70g, 2.2equiv.) in dioxan/water (1:1) (80ml) and stirred for 4.5hr. The bulk of the solvent was removed *in vacuo* and the resultant slurry was treated with EtOAc (150ml). The inorganics were removed by filtration with EtOAc. The filtrate was washed with 10% aq citric acid (30ml), water (30ml), saturated aq. NaHCO₃ (20ml) and brine (20ml) and dried (Na₂SO₄) and evaporared *in vacuo* to afford the title compound as a colourless oil (10.3g, 94%). δH (CDCl₃) 5.45 (1H, br), 4.36 (1H, br), 4.26 (2H, q), 3.94 (2H, br m), 1.47 (9H, s), 1.28 (3H, t); m/z (ES⁺, 70V) 233 (MH⁺), 256 (MNa⁺).

### INTERMEDIATE 24

### Ethyl (2S)-2-[(tert-butoxycarbonyl)amino]-3-[(methylsulfonyl)oxy]propanoate

Methanesulphonyl chloride (730µL, 9.43mmol) was added to a stirred, ice-bath cooled solution of Intermediate 23 (2.0g, 8.5mmol) and 4-methylmorpholine (1.13ml, 10.29mmol) in dry DCM (30ml) and stirred for 6hr. The solvent was removed *in vacuo* and the residue treated with EtOAc (150ml). The organics were washed with water (40ml), 10% aq citric acid (20ml), water (20ml), sat aq NaHCO₃ (20ml), water (20ml), brine (10ml), dried (Na₂SO₄) and evaporated *in vacuo* to afford a colourless glass which solidified on standing. This was treated with hexane and the solid was filtered, washed with hexane and dried under N₂ atmosphere to give the title compound (2.45g, 92 %). δH (CDCl₃), 5.38 (1H, br), 4.63 (3H, br m), 4.27 (2H, q), 3.03 (3H, s), 1.48 (9H, s), 1.33 (3H, t); m/z (ES⁺, 70V) 333 (MNa⁺).

### INTERMEDIATE 25

### Ethyl (2R)-2-[(tert-butoxycarbonyl)amino]-3-iodopropanoate

Intermediate 24 (1.0g, 3.21mmol) was stirred in acetone (10ml) in a foil covered flask with sodium iodide (723mg, 4.82mmol) at RT for 18 hr. The acetone was removed *in vacuo* and the residue partitioned between EtOAc (100ml) and water (30ml). The organics washed with brine (10ml), dried (Na₂SO₄) and evaporated *in vacuo* to afford a yellow oil. This was purified by chromatography (SiO₂; 30% Et₂O/hexane) to afford the title compound as a colourless oil which solidified to a white solid (597mg, 54%). δH (CDCl₃) 5.36 (1H, br), 4.50 (1H, br m), 4.27 (3H, m), 3.59 (2H, m), 1.48 (9H, s), 1.33 (3H, t); m/z (ES⁺, 70V) 365 (MNa⁺).

### INTERMEDIATE 26

### Ethyl (2S)-2-[(tert-butoxycarbonyl)amino]-3-(5-nitropyridin-2-yl) propanoate

Zinc dust (100mesh) (581 mg, 8.88mmol) was heated under vacuum and then cooled under N₂. 1,2-dibromoethane (32µL, 0.37mmol) and dry THF (1ml) were added with heating to boiling. Heating was stopped and the mixture stirred for 1 min. This heating and stirring was repeated twice more. TMSCI (66µL 0.52mmol) was added and stirred at 50° for ~10 mins. Intermediate 25. (2.54g, 7.40mmol) in dry THF (4ml) was added and stirred at -35-40° for 40 minutes. 2-bromo-5-nitropyridine (1.50g, 7.30mmol) and PdCl₂(PPh₃)₂ (260mg, 0.37mmol) and dry THF (2ml) were added and the reaction mixture stirred at 35° for 2hr. The reaction mixture was partitioned between EtOAc (150ml) and sat. aq. NH₄Cl (40ml). The phases were separated and the aqueous phase re-extracted with EtOAc (50ml). The combined organic extracts were washed with brine (10ml), dried (Na₂SO₄) and evaporated in vacuo to afford a dark straw coloured oil. Purification by chromatography (SiO₂; 30-70% Et₂O/hexane) afforded the title compound as a yellow oil (1.52g, 61%). δH (CDCl₃), 9.34 (1H, s), 8.39 (1H, d), 7.38 (1H, d), 5.58 (1H, br), 4.75 (1H, br m), 4.20 (2H, m), 3.47 (2H, m), 1.42 (9H, s), 1.23 (3H, t); m/z (ES⁺, 70V) 339 (MH⁺).

### INTERMEDIATE 27

### Ethyl (2S)-3-(5-aminopyridin-2-yl)-2-[(tert-butoxycarbonyl)amino]propanoate

A stirred solution of Intermediate 26 (1.16g, 3.42mmol) in absolute EtOH (20ml) was hydrogenated at atmospheric pressure with 10% Pd on charcoal (100mg) for 3.5hrs. The catalyst was removed by filtration through a celite pad with DCM. The filtrate was evaporated *in vacuo.* The crude title compound was obtained as a straw-coloured oil (1.03g, 98%) and used without further purification. δH (CDCl₃), 8.01 (1H, s), 6.92 (2H, s), 5.83 (1H, br), 4.59 (1H, br m), 4.13 (2H, m), 3.63 (2H, br), 3.15 (2H, br), 1.43 (9H, s), 1.21 (3H, t); m/z (ES⁺, 70V) 309 (MH⁺).

### INTERMEDIATE 28

### Ethyl (2S)-2-[(tert-butoxycarbonyl)amino]-3-{5-[(3,5-dichloroisonicotinoyl)amino]pyridin-2-yl}propanoate.

3,5-Dichloroisonicotinoyl chloride (0.51 ml, 3.61mmol) was added to a stirred, ice-bath cooled solution of Intermediate 27 (1.06g, 3.43mmol) and dry pyridine (0.55ml) in dry DCM (20ml) and stirred at RT for 1 hr. After evaporation of the solvent the residue was dissolved in EtOAc (80ml) and washed with satuarted sodium bicarbonate (20ml), water (10ml), brine (10ml), then dried (Na₂SO₄) filtered and concentrated *in vacuo* to a red-brown glass. Chromatography (silica, 75% Et₂O/DCM) afforded a the title compound as tan-coloured solid (1.25g, 75%). δH NMR (DMSO d⁶) 8.69 (2H, s), 8.58 (1H, s), 7.92 (1H, d), 7.20 (1H, d), 4.26 (1H, m), 3.97 (2H, m), 2.93 (2H, m), 1.21 (9H, s), 1.01 (3H, t); m/z (ES⁺, 70V) 483 (MH⁺).

### INTERMEDIATE 29

### Ethyl (2S)-2-amino-3-{5-[(3,5-dichloroisonicotinoyl)amino]pyridin-2-yl}propanoate

Acetyl chloride (6ml) was added to absolute EtOH (20ml) and stirred for 15 min., cooled to RT, then Intermediate 28 (2.74g, 5.67mmol) added with and stirring for 3.5hrs. The solvent removed *in vacuo.* The resultant yellow residue was treated with sat. sodium bicarbonate (10ml) and solid sodium bicarbonate till neutralised. Extraction with EtOAc (4 x 50 ml), drying (Na₂SO₄) and concentrated affordet the title compound as a straw-coloured foam (2.1 g, 97%). δH NMR (d⁶ DMSO) 8.67 (2H, s), 8.56 (1H, s). 7.85 (1H, d), 7.16 (1H, d), 3.89 (2H, q), 3.57 (1H, dd), 2.86 (1H, dd), 2.82 (1H, dd), 1.73 (2H, br), 1.00 (3H, t). m/z (ES⁺, 70V) 383 (MH⁺).

### INTERMEDIATE 30

### 3-Ethoxy-7,7-dioxo-7λ⁶-thia-spiro[3.5]non-2-en-1-one

A solution of 1,1-dioxo-hexahydro-1λ⁶-thiopyran-4-carboxylic acid (10.2g, 57.3mmol) [Prepared according to the procedure of [*Org. Prep. Proc. Int.* 1977, 94] and DMF (0.3ml) in DCM (120ml) at rt, was treated dropwise with oxalyl chloride and the resulting slurry stirred for 3d. The crude reaction was then concentrated *in vacuo* to give an oil which was redissolved in THF (100ml), treated with ethoxyacetylene (10ml, 50%w/w) and triethylamine (10ml) and the resulting slurry stirred for 10d at rt. Filtration and concentration of the filtrate *in vacuo* gave a crude oil which was purified by chromatography (SiO2, 30% EtOAc:hexanes) to give the title compound as a yellow oil (8.9g, 38.6mmol, 67%). δH (CDCl₃, 300K) 4.88 (1H, s), 4.27 (2H, q, J 7.1Hz), 3.44-3.37 (2H, m), 3.13-3.05 (2H, m), 2.47-2.40 (2H, m), 2.35-2.29 (2H, m), 1.48 (3H, t, J 7.1Hz). m/z (ES⁺, 70V) 230.9 (MH⁺).

### INTERMEDIATE 31

### 3-Hydroxy-7,7-dioxo-7λ⁶-thia-spiro[3.5]non-2-en-1-one

Intermediate 30 (8.6g, 37.4mmol) was stirred with 1M HCl (100ml) for 3d and the resulting solution concentrated *in vacuo.* The residual solid was triturated with EtOAc to give the title compound as an off-white solid (5.1g, 25.2mmol, 68%). m/z (ES⁺, 70V) 202.9 (MH⁺).

### INTERMEDIATE 32

### 3-Ethoxy-spiro[3.4]octa-2,6-dien-1-one

A solution of cyclopent-3-ene carboxylic acid (4.0g, 36.0mmol) and DMF (0.25ml) in DCM (30ml) at 0º was treated dropwise with oxalyl chloride (3.5ml, 39.0mmol). After 2h the reaction mixture was concentrated *in vacuo,* the residual slurry diluted with Et₂O (100ml) and the resulting precipitate removed by filtration and the filtrate concentrated *in vacuo.* The resulting oil was diluted with Et₂O (50ml), treated with ethoxyacetylene (40%w/w solution in hexanes, 10ml) followed dropwise with triethylamine (6ml, 44.0mmol) and the reaction stirred for 7d. Filtration and concentration of the filtrate *in vacuo* followed by chromatography (SiO₂, 5:1 EtOAc:hexanes) gave the title compound as a pale yellow oil (4.3g, 73%). m/z (ES⁺, 70V) 164.9 (MH⁺).

### INTERMEDIATE 33

### 3-Hydroxy-spiro[3.4]octa-2,6-dien-1-one

Intermediate 32 (2.0g, 12.0mmol) and 2M hydrochloric acid (5ml) were stirred vigorously for 24h at room temperature. The resulting solution was extracted with EtOAc (25ml), the extracts dried (MgSO₄), filtered and concentrated *in vacuo* to give the title compound as an pale brown powder (1.07g, 7.9mmol, 65%). δH (DMSO d⁶, 300K) 5.54 (4H, s), 4.57 (2H, s), 2.52 (2H, s). m/z (ES⁺, 70V) 136.9 (MH⁺).

### INTERMEDIATE 34

### 2-Cyclohexyl-3-triisopropylsilanyloxy-spiro[3.5]non-2-en-1-one;

To a stirred solution of the compound of example 47 (5.6g, 20 mmol) in t-butylmethyl ether (50ml) was added cyclohexylcarbonyl chloride (5.3ml, 40 mmol) and triethylamine (13 ml, 100mmol). The mixture was stirred under reflux for 24 hours, allowed to cool and filtered to remove triethylammonium chloride. The filtrate was concentrated under reduced pressure and chromatographed on silica gel, mobile phase 3% EtOAc in hexane to afford the title compound as a brown oil (5.8g, 74%). m/z (ES⁺, 70V) 235.2 (MH⁺ of desilylated compound).

### INTERMEDIATE 35

### 2-Cyclohexyl-spiro[3.5]nonane-1,3-dione

Intermediate 34 was stirred with 5 volumes of 2M hydrochloric acid for 14 days and worked up in a similar manner to Intermediate 4 to afford the title compound as a white crystalline solid in 40% yield. m/z (ES⁺, 70V) 235.0 (MH⁺).

### INTERMEDIATE 36

### 1-Butoxyprop-1-yne

Prepared according to the method of Nooi and Arens; Recl. Trav. Chim. Pays-Bas; 78; 1959; 284 - 287.

### INTERMEDIATE 37

### 1-Butoxypent-1-yne

Prepared in a similar manner to Intermediate 36 from the appropriate starting materials.

### INTERMEDIATE 38

### 3-Butoxy-2-methyl-spiro[3.5]non-2-en-1-one

Prepared in a similar manner to Intermediate 1 from Intermediate 36 in 23% yield. δH (CDCl₃) 4.34 (2H, t, J 6.5Hz), 1.77-1.25 (17H, m), 1.00 (3H, t, J 7.4Hz). m/z (ES⁺, 70V) 223.0 (MH⁺)

### INTERMEDIATE 39

### 3-Butoxy-2-propyl-spiro[3.5]non-2-en-1-one

Prepared in a similar manner to Intermediate 1 from Intermediate 37 in 67% yield. δH (CDCl₃) 4.31 (2H, t, J 6.4Hz), 2.07 (2H, t, J 7.2Hz), 1.80-1.40 (13H, m), 1.00 (3H, t, J 7.1 Hz), 0.93 (3H, t, J 7.3Hz). m/z (ES⁺, 70V) 251.1 (MH⁺)

### INTERMEDIATE 40

### 2-Methyl-spiro[3.5]nonane-1,3-dione

Prepared in a similar manner to Intermediate 2 from Intermediate 38 in 90% yield. δH (d⁶ DMSO) 1.56 (10H, m), 1.37 (3H, s). m/z (ES⁺, 70V) 166.9 (MH⁺).

### INTERMEDIATE 41

### 2-Propyl-spiro[3.5]nonane-1,3-dione

Prepared in a similar manner to Intermediate 2 from Intermediate 39 in 64% yield. δH (d⁶ DMSO) 1.82 (2H, t, J 7.2Hz), 1.58 (8H, m), 1.41 (2H, m), 1.39 (2H, q, J 7.4Hz), 0.85 (3H, t, J 7.3Hz). m/z (ES⁺, 70V) 195.1 (MH⁺).

### INTERMEDIATE 42

### 3-Butoxy-2-methyl-7-oxa-spiro[3.5]non-2-en-1-one

Prepared in a similar manner to Intermediate 1 from Intermediate 36 in 48% yield. δH (CDCl₃) 4.30 (2H, t, J 6.5Hz), 3.76 (4H, m), 1.70 (6H, m), 1.63 (3H, s), 1.36 (2H, m), 0.92 (3H, t, J 7.4Hz). m/z (ES⁺, 70V) 225.0 (MH⁺).

### INTERMEDIATE 43

### 3-Butoxy-2-propyl-7-oxa-spiro[3.5]non-2-en-1-one

Prepared in a similar manner to Intermediate 1 from Intermediate 37 in 79% yield. δH (CDCl₃) 4.33 (2H, t, J 6.4Hz), 3.81 (4H, m), 2.09 (2H, t, J 7.7Hz), 1.81 (6H, m), 1.50 (4H, m), 1.00 (3H, t, J 7.4Hz), 0.94 (3H, t, J 7.3Hz). m/z (ES⁺, 70V) 253.0 (MH⁺).

### INTERMEDIATE 44

### 2-Methyl-7-oxa-spiro[3.5]nonane-1,3-dione

Prepared in a similar manner to Intermediate 2 from Intermediate 42 in 51 % yield. δH NMR (d⁶ DMSO) 3.67 (4H, m), 1.68 (4H, m), 1.40 (3H, s). m/z (ES⁺, 70V) 168.9 (MH⁺).

### INTERMEDIATE 45

### 2-Propyl-7-oxa-spiro[3.5]nonane-1,3-dione

Prepared in a similar manner to Intermediate 2 from Intermediate 43 in 79% yield. m/z (ES⁺, 70V) 196.9 (MH⁺).

### INTERMEDIATE 46

### 4-Bromomethyl-5-methyl-2-oxo-1,3-dioxolene

Prepared according to the method of Sakamoto F., lkeda S. and Tsukamoto G., Chem. Pharm. Bull., 1984, 32, 2241-2248.

### INTERMEDIATE 47

### Ethyl (2S)-2-tert-Butoxycarbonylamino-3-{4-[(3,5-dichloro-1-oxypyridine-4-carbonyl)amino]phenyl} propanoate

Intermediate 14 (500mg, 1.04mmol) and mCPBA (493mg, 2.0mmol) in DCM (10ml) were stirred together at room temperature for 48hrs. After this time sodium sulfite (10% solution in water, 20ml) was added with stirring for 5 mins, prior to separating between DCM (50ml) and sodium bicarbonate solution (50 ml). The organics were washed with sodium bicarbonate solution (2x50ml) and water (1x50m1), dried (MgSO₄) and reduced in vacuo. The resulting orange solid was recrystalised from EtOAc/hexane to give title compound as a of pale yellow powder (350mg). δH (DMSO d6) 7.78 (2H, s), 6.78 (2H, d, J 8.3Hz), 6.46 (2H, d, J 8.4Hz), 3.55 (1H, m), 3.36 (2H, q, J 7.1Hz), 2.31 (1H, dd J 5.8Hz, 13.8Hz), 2.31 (1H, dd, J 13.6, 8.9Hz), 0.60 (9H, s), 0.43 (3H, t, 3H).

### INTERMEDIATE 48

### (S)-2-Amino-3-{4-[(3,5-dichloro-1-oxy-pyridine-4-carbonyl)-amino]-phenyl}-propionic acid ethyl ester

Intermediate 47 (330mg, 0.55mmol) and HCl in EtOAc (2.6M) were stirred together at room temperature overnight. After this time the formed precipitate was filtered off, washed with Et₂O, (3x50ml) and then made basic by separating between EtOAc (50ml) and sodium bicarbonate solution (50ml). The organics were dried (MgSO₄) and reduced *in vacuo* to give title compound as white solid (185mg). δH (CD₃OD) 8.40 (2H, s), 7.43 (2H, d, J 8.6Hz), 7.05 (2H, d, J 8.6Hz), 3.98 (2H, q, J 7.1 Hz), 2.85 (2H, m), 1.04 (3H, t, J 7.1 Hz).

### Reference EXAMPLE 1

### Ethyl (2S)-2-[(4,4-dimethyl-3-oxo-1-cyclobutenyl)amino]-3-[4-([2,7]naphthyridin-1-yloxy)phenyl]propanoate

A solution of 3-hydroxy-4,4-dimethyl-2-cyclobutenone (57mg, 0.51 mmol) [prepared according to the method of Wasserman, H.H. *et al* J. Org. Chem, 38, 1451-1455, (1973)] and the ethyl ester prepared according to the method used to prepare Intermediate 7 (164mg, 0.51mmol), in 1,2-dichloroethylene (5ml), was stirred at room temperature for 72h. The volatiles were removed *in vacuo* and the residue chromatographed (SiO₂; EtOAc) affording the title compound as a white solid (188mg, 0.45mmol, 89%). δH (CDCl₃, 300K) 9.92 (1H, s), 8.75 (1H, d, J 5.7Hz), 8.60 (1H, d, J 8.6Hz), 8.04 (1H, d, J 5.8Hz), 7.82 (1H, d, J 5.6Hz), 7.47 (1H, d, J 5.8Hz), 7.27 (2H, d, J 8.5Hz), 7.16 (2H, d, J 8.5Hz), 4.31 (1H, s), 4.30-4.21 (1H, m), 3.68-3.63 (2H, q, J 7.1 Hz), 3.17 (1H, dd. J 13.6, 9.4Hz), 2.95 (1H, dd, J 5.0, 13.6Hz), 1.01 (3H, s), 0.93 (3H, s). m/z (ES⁺, 70V) 418.1(MH+).

### Reference EXAMPLE 2

### (2S)-2-[(4,4-Dimethyl-3-oxo-1-cyclobutenyl)amino]-3-[4-([2,7]naphthyridin-1-yloxy)phenyl]propanoic acid

The compound of Reference Example 1 (127mg, 0.31mmol) in THF (5ml) was treated in a single portion with LiOH.H₂O (13mg, 0.32mmol) in H₂O (1ml) and the reaction stirred at room temperature for 2h. The reaction was then quenched by the addition of HOAc (glacial, 1ml) and the volatiles removed *in vacuo.* Water (10ml) was then added to the residual foam and stirred vigorously to effect precipitation. The precipitate was then collected by vacuum filtration and the residue washed with water (2 x 5ml). Drying under vacuum gave the title compound as a fine white solid (108mg, 0.27mmol, 88%). δH (DMSO d⁶, 300K) 9.67 (1H, s), 8.78 (1H, d, J 5.7Hz), 8.51 (1H, d, J 8.6Hz), 8.09 (1H, d, J 5.8Hz), 7.86 (1H, d, J 5.6Hz), 7.50 (1H, d, J 5.7Hz), 7.21 (2H, d, J 8.4Hz), 4.17 (2H, d, J 8.4Hz), 4.34 (1H, s), 4.18-4.14 (1H, m), 3.21 (1H, dd, J 4.9, 13.9Hz), 2.98 (1H, dd, J 13.9, 9.3Hz), 1.06 (3H, s), 0.99 (3H, s). m/z (ES⁺, 70V) 404.1 (MH⁺).

### Reference EXAMPLE 3

### Ethyl (2S)-2-[(4,4-dimethyl-3-oxo-1-cyclobutenyl)amino]-3-[4-([2,6]naphthyridin-1-ylamino)phenyl]propanoate

A solution of 3-hydroxy-4,4-dimethyl-2-cyclobutenone (58mg, 5.1mmol) and Intermediate 12 (1.01g, 2.7mmol) in DCM (15ml), was stirred at room temperature for 48h. The volatiles were removed *in vacuo* and the residue chromatographed (SiO₂; EtOAc) affording the title compound as a white powder (990mg, 2.3mmol, 88%). δH (CDCl₃, 300K) 9.33 (1H, s), 9.24 (1H, s), 8.69 (1H, d, J 5.9Hz), 8.63 (1H, d, J 8.5Hz), 8.42 (1H, dd, J 5.9, 0.8Hz), 8.15 (1H, dd, J 5.7, 1.3Hz), 7.85-7.80 (3H, m), 7.31-7.22 (4H, m), 4.39 (1H, s), 4.24-4.21 (1H, m), 4.17 (2H, q. J 7.1Hz), 3.15 (1H, dd, J 13.8, 5.6Hz), 3.00 (1H, dd, J 13.8, 9.0Hz), 1.19 (3H, t, J 7.1 Hz), 1.11 (3H, s), 1.05 (3H, s). m/z (ES⁺, 70V) 431.1 (MH⁺).

### Reference EXAMPLE 4

### Ethyl (2S)-2-[(4,4-dimethyl-3-oxo-1-cyclobutenyl)amino]-3-{4-[(3,5-dichloroisonicotinoyl)amino]phenyl}propanoate

A solution of 3-hydroxy-4,4-dimethyl-2-cyclobutenone (58mg, 0.52mmol) [prepared according to the method of Wasserman, H.H. *et al* J. Org. Chem, 38, 1451-1455, (1973)] and the free base of Intermediate 15 (200mg, 5.2mmol), in DCM (5ml), was stirred at room temperature for 48h. The volatiles were removed *in vacuo* and the residue chromatographed (SiO₂; EtOAc) to give the title compound as a white solid (230mg, 0.48mmol, 93%). δH (CDCl₃, 300K) 8.48 (2H, s), 8.10 (1H, s), 7.51 (2H, d, J 8.2Hz), 7.04 (2H, d, 8.2Hz), 5.91 (1H, s), 4.43 (1H, s), 4.22 (2H, q, J 7.1Hz), 3.17 (1H, dd, J 14.0, 5.1Hz), 3.05 (1H, dd, J 14.0, 5.8Hz), 1.28 (3H, t, J 7.1Hz), 1.15 (3H, s), 1.14 (3H, s). m/z (ES⁺, 70V) 476.0 and 478.0 (MH⁺).

### Reference EXAMPLE 5

### Ethyl (2S)-2-[(4R,S)-4-methyl-3-oxo-4-propyl-1-cyclobutenyl]amino-3-[4-([2,7]naphthyridin-1-yloxy)phenyl]propanoate

A solution of intermediate 2 (300mg, 2.1 mmol) and the ethyl ester of Intermediate 7 (724mg, 2.14mmol), in DCM (15ml), was stirred at room temperature for 24h. The reaction was then diluted with DCM (30ml) and distilled water (20ml) and washed successively with 1 M aqueous hydrochloric acid (30ml) water (30ml) and saturated, aqueous sodium hydrogen carbonate (30ml). The organic layer was then dried (MgSO₄), filtered and concentrated *in vacuo.* The residual foam was chromatographed (SiO₂; EtOAc) to give the title compound as a white powder (827mg, 1.8mmol, 84%) as an approx. 1:1 mixture of diastereomers. δ□H (CDCl₃, 300K) 9.72 (1H, s), 8.71 (1H, d, J 5.7Hz), 8.04 (1H, d, J 5.8Hz), 7.55 (1H, d, J 5.7Hz), 7.22-7.12 (5H, m), 5.80 (1H, d, J 7.6Hz), 4.57 (1H, s), 4.28-4.20 (3H, m), 3.25-3.07 (2H, m), 1.57-1.21 (7H, m), 1.18 and 1.17 (3H, s) 0.84-0.78 (3H, m). m/z (ES⁺, 70V) 460.1 (MH⁺) and 482.0 (MNa⁺).

### EXAMPLE 1

### Ethyl (2S)-2-[(3-oxospiro[3.5]non-1-en-1-yl)amino]-3-{4-[(3,5-dichloroisonicotinoyl)amino]phenyl}propanoate

Prepared from 1-keto-3-hydroxyspiro[3,5]-non-2-ene (400mg, 2.6mmol) [prepared according to the method of Wasserman, H.H. *et al,* J. Org. Chem., 38, 1451-1455 (1973)] and the free amine of Intermediate 15 (400mg, 1.04mmol), in a similar manner to the compound of Reference Example 5 to give the title compound as a white powder (512mg, 0.99mmol, 95%). δH (CDCl₃, 300K) 10.86 (1H, s), 8.78 (2H, s), 8.34 (1H, d, J 8.5Hz), 7.56 (2H, d, J 8.5Hz), 7.25 (2H, d, J 8.5Hz), 4.36 (1H, s), 4.20-4.11 (3H, m), 3.13 (1H, dd, J 13.8, 5.3Hz), 3.00 (1H, dd, J 9.2, 13.8Hz), 1.67-1.19 (10H, m), 1.17 (3H, t, J 4.1 Hz). m/z (ES⁺, 70V) 516.0 and 518.0 (MH⁺).

### EXAMPLE 2

### (2S)-2-[(3-Oxospiro[3.5]non-1-en-1-yl)amino]-3-{4-[(3,5-dichloroisonicotinoyl)amino]phenyl}propanoic acid

The compound of Example 1 (700mg, 1.36mmol) was hydrolysed in a similar manner to the method of Reference Example 2 to give the title compound as a fine white solid (627mg, 1.28mmol, 95%). δH (DMSO d⁶, 360K) 10.54 (1H, s), 8.73 (2H, s), 7.81 (1H, d, J 8.4Hz), 7.56 (2H, d, J 8.5Hz), 7.27 (2H, d, J 8.5Hz), 4.39 (1H, s), 4.12-4.05 (1H, m), 3.19 (1H, dd, J 13.9, 5.1Hz), 3.00 (1H, dd, J 13.9, 8.8Hz), 1.94-1.24 (10H, m). m/z (ES⁺, 70V) 488.0 and 490.0 (MH⁺).

### Example 3

### Ethyl (2S)-2-[(3-oxospiro[3.5]non-1-en-1-yl)amino]-3-{4-[(3-methyl[2,7]naphthyridin-1-yl)oxy]phenyl}propanoate

Prepared from 1-keto-3-hydroxyspiro[3,5]-non-2-ene (400mg, 2.6mmol) and Intermediate 9 (400mg, 1.14mmol), in a similar manner to the compound of Reference Example 5 to give the title compound as a white powder (497mg, 1.02mmol, 89%). δH (CDCl₃, 300K) 9.62 (1H, s), 8.72 (1H, d, J 5.7Hz), 7.99 (1H, d, J 8.6Hz), 7.73 (1H, dd, J 5.7, 0.9Hz), 7.37-7.34 (3H, m), 7.28-7.24 (2H, m), 4.42 (1H, s), 4.26-4.18 (3H, m), 3.25 (1H, dd, J 14.0, 5.6Hz), 3.12 (1H, dd, J 14.0, 9.1 Hz), 2.42 (3H, s), 1.72-1.55 (10H, m), 1.25 (3H, t, J 7.1 Hz). m/z (ES⁺, 70V) 486.1 (MH⁺).

### EXAMPLE 4

### (2S)-2-[(3-Oxospiro[3.5]non-1-en-1-yl)amino]-3-{4-[(3-methyl[2,7]naphthyridin-1-yl)oxy]phenyl}propanoic acid

The compound of Example 3 (300mg, 0.62mmol) was hydrolysed in a similar manner to the method of Reference Example 2 to give the title compound as a fine white solid (237mg, 0.52mmol, 84%). δH (DMSO d⁶, 360K) 9.62 (1H, s), 8.72 (1H, d, J 5.7Hz), 7.82 (1H, d, J 6.3Hz), 7.73 (1H, d, J 5.5Hz), 7.35 (2H, d, J 8.7Hz), 7.25 (2H, d, J 8.7Hz), 4.39 (1H, s), 4.12 (1H, dd, J 8.7, 13.2Hz), 3.34-3.12 (2H, m), 2.42 (3H, s), 1.72-1.53 (10H, m). m/z (ES⁺, 70V) 458.0 (MH⁺).

### EXAMPLE 5

### Ethyl (2S)-2-[(2-bromo-3-oxospiro[3.5]non-1-en-1-yl)amino]-3-{4-[(3,5-dichloroisonicotinoyl)amino]phenyl}propanoate

A solution containing the compound of Example 1 (500mg, 0.97mmol) and triethylamine (2eq, 270µl) in THF (10ml) at 0º was treated dropwise with a solution of bromine (1.1eq, 170mg) in THF (5ml). After 20mins the reaction was allowed to warm to room temperature prior to dilution with EtOAc (100ml). The crude reaction mixture was washed with saturated aqueous NaHCO₃ (20ml) and brine (20ml), dried (MgSO₄) filtered and concentrated *in vacuo.* The residual foam was chromatographed (SiO₂; EtOAc) to give the title compound as a white powder (511 mg, 0.86mmol, 95%). δH (CDCl₃, 300K) 8.48 (2H, s), 8.05 (1H, s br), 7.52 (2H, d J 8.4Hz), 7.04 (2H, d J 8.5Hz), 5.81 (1H, d br, J 8.3Hz), 4.98-4.91 (1H, m), 4.21 (2H, q, J 7.1Hz), 3.21 (2H, d J 5.3Hz), 1.70-1.66 (4H, m), 1.53-1.44 (4H, m), 1.28 (3H, t J 7.1Hz), 1.20-1.16 (2H, m). m/z (ES⁺, 70V) 597.9 and 595.0 (MH⁺).

### EXAMPLE 6

### (2S)-2-[(2-Bromo-3-oxospiro[3.5]non-1-en-1-yl)amino]-3-{4-[(3,5-dichloroisonicotinoyl)amino]phenyl}propanoic acid

The compound of Example 5 (511mg, 0.86mmol) was hydrolysed in a similar manner to the method of Reference Example 2 (1.3eq, 50mg), to give the title compound as a white powder (421 mg, 0.74mmol, 87%). δH (DMSO d⁶, 390K) 10.34 (1H, s), 8.67 (2H, s), 7.53 (2H, s br), 7.26 (2H, d J 8.26Hz), 4.67 (1H, m), 3.26-3.22 (1H, m), 3.13-3.08 (1H, m), 1.67-1.21 (10H, m). δC (DMSO-d⁶, 300K) 23.86, 25.30, 30.75, 37.79, 57.98, 61.94, 67.02, 119.73, 128.47, 130.38, 133.46, 136.86, 142.85, 148.10, 160.11, 171.80, 173.96,186.93. m/z (ES⁺, 70V) 569.9 and 567.9 (MH⁺).

### EXAMPLE 7

### Ethyl (2S)-2-[(3-oxospiro[3.5]non-1-en-1-yl)amino]-3-{4-[(2,7)naphthyridin-1-yloxyl]phenyl}propanoate

A solution of the ethyl ester of Intermediate 7 (565mg, 1.68mmol) and 1-keto-3-hydroxyspiro[3,5]-non-2-ene (280mg, 1.84mmol) in DCM (20ml) was stirred at roon temperature for 24h. Concentration *in vacuo* and chromatography (SiO₂; EtOAc) to give the title compound as a pale yellow powder (730mg, 1.55mmol, 92%). δH (CDCl₃, 300K) 9.82 (1H, s), 8.82 (1H, d J 5.7Hz), 8.14 (1H, d J 5.9Hz), 7.64 (1H, d J 5.8Hz), 7.25-7.17 (6H, m), 5.77 (1H, d J 7.6Hz), 4.60 (1H, s), 4.25 (2H, q J 7.1Hz), 3.30 (1H, dd J 5.5Hz 13.9Hz), 3.18 (1H, dd J 5.5Hz 13.9Hz), 1.84-1.53 (10H, m), 1.35 (3H, t J 7.1 Hz). m/z (ES⁺, 70V) 472.1 (MH⁺).

### EXAMPLE 8

### Ethyl (2S)-2-[(2-bromo-3-oxospiro[3.5]non-1-en-1-yl)amino]-3-{4-(2,7)naphthyridin-1-yloxylphenyl}propanoate

A stirred solution of the compound of Example 7 (300mg, 0.637mmol) and triethylamine (1.2eq, 100µl) at 0º was treated dropwise with a solution of bromine in DCM (2%wv/v, 2.1ml, 1.2eq). After 12h the reaction was diluted with DCM (50ml) and washed successively with saturated aqueous NaHCO₃, dried (MgSO₄) filtered and concentrated *in vacuo.* The residual foam was triturated with diisopropylether and the resulting solid collected and dried *in vacuo* to give the title compound as a pale yellow powder (325mg, 0.59mmol, 95%). δH (CDCl₃, 300K) 9.83 (1H, s), 8.78 (1H, d J 5.8Hz), 8.16 (1H, d J 5.8Hz), 7.69 (1H, d, J 5.7Hz), 7.32 (1H, d, J 5.8Hz), 7.27 (4H, s), 5.87 (1H, d, J 8.4Hz), 5.10-5.03 (1H, m), 4.30 (2H, q, J 7.1 Hz), 3.38-3.32 (2H, m), 1.85-1.69 (4H, m), 1.67-1.50 (6H, m), 1.36 (3H, t, J 7.1 Hz). m/z (ES⁺, 70V) 552.0 (MH⁺).

### EXAMPLE 9

### (2S)-2-[(2-Bromo-3-oxospiro[3.5]non-1-en-1-yl)amino]-3-{4-[(2,7)naphthyridin-1-yloxy]phenyl}propanoic acid

The compound of Example 8 (220mg, 0.40mmol) was hydrolysed in a similar manner to the method of Reference Example 2, to give the title compound as a white powder (125mg, 0.24mmol, 60%). δH (DMSO-d⁶, 300K) 9.27 (1H, s), 8.88 (1H, d J 9.4Hz), 8.83 (1H, d J 5.4Hz), 8.12 (1H, d J 5.8Hz), 7.90 (1H, d J 5.7Hz), 7.55 (1H, d J 5.8Hz), 7.38 (2H, d J 8.4Hz), 7.27 (2H, d J 8.4Hz), 4.83-4.79 (1H, m), 3.08-3.03 (2H, m), 1.80-1.37 (8H, m), 1.19-1.12 (2H, m). m/z (ES⁺, 70V) 523.9 (MH⁺).

### EXAMPLE 10

### Ethyl (2S)-2-[(3-oxo-7-oxaspiro[3.5]non-1-en-1-yl)amino]-3-{4-[(3,5-dichloroisonicotinoyl)amino]phenyl}propanoate

Prepared from 7-oxaspiro[3.5]nonane-1,3-dione (1.2g, 7.8mmol) and the free amine of Intermediate 15 (2.67g, 7.0mmol) in a similar manner to the method of Reference Example 5, to give the title compound (3.31g, 6.38mmol, 91%). δH (CDCl₃, 300K) 8.61 (1H, s), 8.33 (2H, s), 7.41 (2H, d J 5Hz), 6.94 (2H, d J 8.5Hz), 6.30 (1H, s br), 4.35 (1H, s), 4.11 (2H, q J 7.1 Hz) and (1H, m obscured), 5.72 (4H, m), 3.07 (1H, dd J 14.0, 5.0Hz), 2.94 (1H, dd J 14.0, 6.6Hz), 1.75-1.66 (2H, m), 155-1.48 (2H, m), 1.17 (3H, t J 7.1Hz). m/z (ES⁺, 70V) 517.9 (MH⁺).

### EXAMPLE 11

### Ethyl (2S)-2-[(2-bromo-3-oxo-7-oxaspiro[3,5]non-1-en-1-yl)amino]-3-{4-[(3,5-dichloroisonicotinoyl)amino]phenyl}propanoate

A solution of the compound of Example 10 (1.64g, 3.17mmol) and triethylamine (0.69g, 970µl, 6.8mmol) in THF (15ml) at 0º was treated dropwise with a solution of bromine (560mg, 3.1 mmol) in THF (2ml). After 1 h the resulting precipitate was removed by filtration, washed several times with cold EtOAc and dried to give the title compound as a white powder (1.53g, 2.56mmol, 81%). δH (DMSO d⁶, 300K) 10.90 (1H, s), 9.07 (1H, d J 9.0Hz), 8.81 (2H, s), 7.60 (2H, d J 8.4Hz), 7.28 (2H, d J 8.4Hz), 4.85-4.80 (1H, m), 4.21 (2H, q J 7.1 Hz), 3.81-3.76 (2H, m), 3.63-3.58 (2H, m), 3.23 (1H, dd J 13.8, 4.8Hz), 3.05 (1H, dd J 13.8, 9.4Hz), 2.07-1.94 (2H, m), 1.52-1.49 (1H, m), 1.34-1.31 (1H, m), 1.24 (3H, t J 7.1Hz). m/z (ES⁺, 70V) 597.9 and 599.9 (MH⁺).

### EXAMPLE 12

### (2S)-2-[(2-Bromo-3-oxo-7-oxaspiro[3.5]non-1-en-1-yl)amino]-3-{4-[(3,5-dichloroisonicotinoyl)amino]phenyl}propanoic acid

The compound of Example 11 (575mg, 0.96mmol) was hydrolysed in a similar manner to the method of Reference Example 2, to give the title compound as a white powder (283mg, 0.50mmol, 52%). δH (DMSO d⁶,390K) 10.88 (1H, s), 8.98 (1H, d J 9.2Hz), 8.81 (2H, s), 7.59 (2H, d J 8.5Hz), 7.27 (2H, d J 8.5Hz), 4.78-4.72 (1H, m), 3.82-3.75 (2H, m), 3.64-3.54 (2H, m), 3.24 (1H, dd J 13.9, 4.5Hz), 3.01 (1H, dd J 13.8, 9.5Hz), 2.08-1.93 (2H, m), 1.52-1.48 (1H, m), 1.30-1.26 (1H, m). m/z (ES⁺, 70V) 569.9 and 571.9 (MH⁺).

### EXAMPLE 13

### Methyl (2S)-2-{(3-oxospiro[3.5]non-1-en-1-yl)amino}-3-(2,6-dimethoxy[1,1'-biphenyl]-4-yl)propanoate

To a solution of methyl (2*S*)-2-amino-3-(2,6-dimethoxy[1,1'-biphenyl]-4-yl)propanoate (0.80g, 2.5mmol) in DCM (10ml) at room temperature was added 1-keto-3-hydroxyspiro[3,5]-non-2-ene (0.38g, 2.5mmol) and the mixture stirred for 48h. Volatiles were removed *in vacuo* and the residue purified by column chromatography (SiO₂; EtOAc) to give the title compound as a white solid (1.05g, 92%). δH (CDCl₃): 7.32-7.26 (3H, m), 7.12 (2H, d, J 8.2Hz), 6.92 (2H, d, J 8.3Hz), 5.90 (1H, br d, J 8.2Hz), 4.60 (1H, s), 4.33 (1H, br), 3.86 (3H,s), 3.73 (6H, s), 3.30 (1H, dd, J 13.9, 5.3Hz), 3.13 (1H, dd, J 13.9, 6.3Hz), 1.82-1.33 (10H, m). m/z (ES⁺, 70V) 450.1 (MH⁺).

### EXAMPLE 14

### (2S)-2-{(3-Oxospiro[3.5]non-1-en-1-yl)amino}-3-(2.6-dimethoxy[1,1'-biphenyl]-4-yl)propanoic acid

The compound of Example 13 (0.40g, 0.9mmol) was hydrolysed in a similar manner to the method of Reference Example 2, to give the title compound as a white solid (0.19g, 45%). δH (DMSO d⁶) 8.25 (1H, d, J 8.6Hz), 7.29-7.19 (3H, m), 7.07 (2H, d, J 7.9Hz), 6.70 (2H, d, J 8.4Hz), 4.32 (1H, s), 4.11 (1H, br), 3.61 (6H, s), 3.18 (1H, dd, J 13.7, 4.7Hz), 2.93 (1H, dd, J 13.7 9.9Hz), 1.67-1.16 (10H, m). m/z (ES⁺, 70V) 436.1 (MH⁺).

### EXAMPLE 15

### Methyl (2S)-2-{(2-bromo-3-oxospiro[3.5]non-1-en-1-yl)amino}-3-(2,6-dimethoxy[1,1'-biphenyl]-4-yl)propanoate

To a cooled solution (0-5°) of the compound of Example 13 (0.42g, 0.93mmol) and triethylamine (0.14ml, 1.03mmol) in THF (10ml) was added a solution of bromine (0.16g, 1.0mmol) in DCM (1ml). The mixture was stirred at this temperature for 1 h prior to partitioning between EtOAc (100ml) and sodium hydrosulfite (100ml, 5% aq.). The organics were separated, washed with water (50ml), brine (50ml), dried (Na₂SO₄), filtered and concentrated *in vacuo* to give the crude product as pale yellow foam. Column chromatography (SiO₂, 1:1 EtOAc: hexanes) gave the title compound as a white foam (0.45g, 92%). δH (CDCl₃) 7.32-7.26 (3H, m), 7.13 (2H, d, J 8.1 Hz), 6.66 (2H, d, J 8.4Hz), 5.80 (1H, br d, J 8.6Hz), 5.15-5.08 (1H, m), 3.87 (3H, s), 3.73 (6H, s), 3.35 (1H, d, J 10.0Hz), 3.31 (1H, d, J 4.9Hz), 1.80-1.33 (10H, m). m/z (ES⁺, 70V) 529.0 and 530.0 (MH⁺).

### EXAMPLE 16

### (2S)-2-{(2-Bromo-3-oxospiro[3.5]non-1-en-1-yl)amino}-3-(2,6-dimethoxy[1,1'-biphenyl]-4-yl)propanoic acid

The compound of Example 15 (0.36g, 0.7mmol) was hydrolysed in a similar manner to the method of Reference Example 2 to give the title compound as a white solid (0.23g, 58%). δH (DMSO d⁶) 8.83 (1H, d, J 9.4Hz), 7.28 (1H, d, J 8.4Hz), 7.24-7.20 (2H, m), 7.10 (2H, d, J 8.1Hz), 6.70 (2H, d, J 8.4Hz), 4.83-4.77 (1H, br), 3.61 (6H, s), 3.25 (1H, dd, J 13.8, 9.8Hz), 2.95 (1H, dd, J 13.8, 10.3Hz), 1.78-1.35 (10H, m). m/z (ES⁺, 70V) 516.0 and 517.0 (MH⁺).

### EXAMPLE 17

### Ethyl (2S)-2-[(3-oxospiro[3.6]dec-1-en-1-yl)amino]-3-{4-[(3,5-dichloroisonicotinoyl)amino]phenyl}propanoate

Prepared from intermediate 17 (400mg, 2.4mmol) and the free amine of Intermediate 15 (920mg, 2.4mmol) in a similar manner to the method of Reference Example 5, to give the title compound (1.1g, 20.7mmol, 86%). δH (CDCl₃, 300K) 8.57 (2H, s), 8.28 (1H, s), 7.61 (2H, d J 8.5Hz), 7.14 (2H, d J 8.5Hz), 5.76 (1H, d J 7.5Hz), 4.33-4.23 (3H, m), 3.25 (1H, dd J 5.3, 14.0Hz), 3.12 (1H, dd J 5.7, 13.9Hz), 1.95-1.89 (2H, m), 1.79-1.70 (4H, m), 1.71-1.50 (6H, m), 1.36 (3H, t J 7.1 Hz). m/z (ES⁺, 70V) 530.0 (MH⁺).

### EXAMPLE 18

### (2S)-2-[(3-Oxospiro[3.6]dec-1-en-1-yl)amino]3-{4-[(3,5-dichloroisonicotinoyl)amino]phenyl}propanoic acid

The compound of Example 17, (257mg, 0.57mmol) was hydrolysed in a similar manner to the method of Reference Example 2, to give the title compound as a white powder (257mg, 0.51mmol, 89%). δH (DMSO d⁶, 390K) 10.83 (1H, s), 8.84 (2H, s), 7.39 (2H, d J 8.5Hz), 7.29 (2H, d J 8.5Hz), 4.30 (1H, s), 4.12-3.98 (1H, m), 3.15 (1H, dd J 13.9, 5.2Hz), 2.97 (1H, dd J 13.8, 9.5Hz), 1.85-1.78 (1H, m), 1.77-1.38 (11H, m). m/z (ES⁺, 70V) 502.0 (MH⁺).

### EXAMPLE 19

### Ethyl (2S)-2-[(2-bromo-3-oxospiro[3.6]dec-1-en-1-yl)amino]-3-{4-[(3,5-dichloroisonicotinoyl)amino]phenyl}propanoate.

A solution of the compound of Example 17 (988mg, 1.87mmol) and triethylamine (520µl, 3.7mmol) in THF (20ml) at 0º was treated dropwise with a solution of bromine (330mg, 2.1mmol) in THF (2ml). After 1h the crude reaction mixture was diluted with EtOAc (50ml), saturated aqueous NaHCO₃ (15ml) and saturated aqueous sodium chloride (15ml) and the crude product extracted with EtOAc (3 x 20ml). The combined extracts were dried (MgSO₄), concentrated *in vacuo* and the crude residue chromatographed (SiO₂, 1:1 EtOAc:hexanes) to give the title compound as a white powder (965mg, 1.58mmol, 85%). δH (CDCl₃, 300K) 8.61 (2H, s), 8.45 (1H, d, J 3.1 Hz), 7.63 (2H, d, J 8.2Hz), 7.15 (2H, d, J 8.2Hz), 5.91 (1H, d, J 8.1 Hz), 5.05-5.00 (1H, m), 4.30 (2H, q, J 7.1 Hz), 3.30 (2H, d, J 5.4Hz), 1.98-1.90 (2H, m), 1.89-1.60 (10H, m), 1.22 (3H, t, J 7.1Hz). m/z (ES⁺, 70V) 609.9 and 611.9 (MH⁺).

### EXAMPLE 20

### (2S)-2-[(2-Bromo-3-oxospiro[3.6]dec-1-en-1-yl)amino]-3-{4-[(3,5-dichloroisonicotinoyl)amino]phenyl}propanoic acid

The compound of Example 19, (560mg, 0.92mmol) was hydrolysed in a similar manner to the method of Reference Example 2, to give the title compound as a white powder (412mg, 0.71 mmol, 77%). δH (DMSO d⁶, 380K) 10.40 (1H, s), 8.67 (2H, s), 7.55 (2H, d, J 8.5Hz), 7.26 (2H, d, J 8.5Hz), 4.52 (1H, br s), 3.22 (1H, dd, J 14.1, 5.3Hz), 3.11 (1H, dd, J 13.9, 8.0Hz), 1.82-1.29 (12H, m). m/z (ES⁺, 70V) 589.1 and 583.9 (MH⁺).

### EXAMPLE 21

### Ethyl (2S)-2-[(3-oxo-7-acetyl-7-azaspiro[3.5]non-1-en-1-yl)amino]-3-{4-[(3,5-dichloroisonicotinoyl)amino]phenyl}propanoate

Prepared from Intermediate 19 (150mg, 0.77mmol), and the free amine of Intermediate 15 (150mg, 0.39mmol) in a similar manner to the method of Reference Example 5, to give the title compound (143mg, 0.26mmol, 67%). δH (DMSO d⁶, 300K) 10.89 (1H, s), 8.89 (2H, s), 8.55-8.48 (1H, m), 7.58 (2H, d, J 7.9Hz), 7.25 (2H, d, J 7.9Hz), 4.47 (1H, s), 4.29-4.23 (1H, m), 4.16 (2H, q, J 7.1 Hz), 3.76-3.72 (1H, m), 3.15 (1H, dd, J 13.8, 5.2Hz), 3.01-2.89 (2H, m), 2.00 (3H, s), 1.90-1.37 (6H, m), 1.21 (3H q J 7.1Hz). m/z (ES⁺, 70V) 559.0 (MH⁺).

### EXAMPLE 22

### (2S)-2-[(3-Oxo-7-acetyl-7-azaspiro[3.5]non-1-en-1-yl)amino]-3-{4-[(3,5-dichloroisonicotinoyl)amino]phenyl}propanoic acid

The compound of Example 21 (200mg, 0.35mmol) was hydrolysed in a similar manner to the method of Reference Example 2, to give the title compound as a white powder (91 mg, 0.16mmol, 46%). δH (CD₃OD, 300K) 8.90 (2H, s), 7.60 (2H, d, J 8.2Hz), 7.30 (2H, J 8.2Hz), 4.49 (1H, s), 4.33-4.27 (2H, m), 3.85-3.77 (1H, m), 3.57-3.45 (1H, m), 3.37-3.31 (1H, m), 3.20-3.11 (1H, m), 3.05-2.99 (1H, m), 2.11 (3H, s), 1.97-1.52 (4H, m). m/z (ES⁺, 70V) 531.0 (MH⁺).

### EXAMPLE 23

### Ethyl(2S)-2-[(7-methoxy-3-oxospiro[3.5]non-1-en-1-yl)amino]-3-{4-[(3,5-dichloroisonicotinoyl)amino]phenyl}propanoate

Prepared from Intermediate 21 (500mg, 2.77mmol) and the free amine of Intermediate 15 (980mg, 2.6mmol) in a similar manner to the method of Reference Example 5, to give the title compound as an inseparable 1:1 mixture of isomers (1.23g, 2.25mmol, 87%). δH (CDCl₃, 300K, 2 isomers) 9.12/8.99 (1H, s), 8.51/8.50 (2H, s), 7.59/7.56 (2H, d, J 8.5Hz), 7.08 (2H, d, J 8.5Hz), 6.21/5.98 (1H, d, J 7.9Hz/7.6Hz), 4.46/4.43 (1H, s), 4.29/4.10 (3H, m), 3.13-3.08 (1H, m), 3.39 (1H, m), 3.30/3.29 (3H, s), 3.23-3.18 (1H, m), 3.13-3.08 (1H, m), 1.97-1.58 (8H, m), 1.35-1.34 (3H, t, J 7.1Hz). m/z (ES⁺, 70V) 546.0 (MH⁺).

### EXAMPLE 24

### (2S)-2-[(7-Methoxy-3-oxospiro[3.5]non-1-en-1-yl)amino]-3-{4-[(3,5-dichloroisonicotinoyl)amino]phenyl}propanoic acid

The compound of Example 23 (950mg, 1.7mmol) was hydrolysed in a similar manner to the method of Reference Example 2, to give the title compound as a white powder, as an approx. 1:1 mixture of isomers (812mg, 1.57mmol, 92%). δH (DMSO d⁶,300K) 10.57 (1H, s), 8.73 (2H, s), 7.93 (1H, br s), 7.56 (2H, d, J 8.2Hz), 7.29-7.21 (2H, m), 4.37 (1H, s), 4.08-4.04 (1H, m), 3.34 (1H, m), 3.25 (3H, s), 3.21-3.02 (2H, m), 1.92-1.34 (8H, m). m/z (ES⁺, 70V) 518.0 (MH⁺).

### EXAMPLE 25

### Ethyl (2S)-2-[(2-bromo-7-methoxy-3-oxospiro[3.5]non-1-en-1-yl)amino]-3-{4-[(3,5-dichloroisonicotinoyl)amino]phenyl}propanoate

Was prepared according to the method of Example 19 from the compound of Example 23 (1.0g, 1.83mmol) and bromine (322mg, 2.0mmol) to give the title compound as a powder (778mg, 1.24mmol, 70%). [Separation of isomers at this stage was achieved chromatographically (SiO₂; 1:1 EtOAc:hexanes to 100% EtOAc)]. δH (CDCl₃, 300K, fast eluting isomer) 10.65 (1H, s), 10.74 (1H, d, J 9.2Hz), 8.58 (2H, s), 7.36 (2H, d, J 8.6Hz), 7.06 (2H, d, J 8.6Hz) 4.54-4.48 (1H, m), 3.18 (1H, m), 3.03-2.98 (1H, m), 3.00 (3H, s), 2.78 (1H, dd, J 13.9, 10.0Hz), 1.18-1.65 (2H, m), 1.61-1.44 (4H, m), 1.18-1.15 (1H, m), 0.92 (1H, m). m/z (ES⁺, 70V) 625.9 (MH⁺).

### EXAMPLE 26

### (2S)-2-[(2-Bromo-7-methoxy-3-oxospiro[3.5]non-1-en-1-yl)amino]-3-{4-[(3,5-dichloroisonicotinoyl)amino]phenyl}propanoic acid

The compound of Example 25 (650mg, 1.04mmol) was hydrolysed in a similar manner to the method Reference Example 2, to give the title compound as a white powder (512mg, 0.86mmol, 83%). δH (DMSO d⁶, 300K) 10.86 (1H, s), 9.11 (1H, d, J 8.9Hz), 8.81 (2H, s), 7.50 (2H, d, J 8.2Hz), 7.21 (2H, d, J 8.2Hz), 4.96-4.92 (1H, br s), 3.13 (1H, dd, J, 13.8, 4.5Hz), 2.94 (1H, dd, J 13.6, 8.7Hz), 1.22 (3H, s), 1.14 (3H, s). m/z (ES⁺, 70V) 597.9 (MH⁺).

### EXAMPLE 27

### Ethyl (2S)-2-[(2-bromo-3-oxospiro[3.5]non-1-en-1-yl)amino]-3-(4-[(3-methyl[2.7]naphthyridin-1-yl)oxy]phenyl}propanoate

To the compound of Example 3 (0.54g, 1.1mmol) in THF (10ml) at room temperature was added triethylamine (0.2ml, 1.4mmol) and a solution of bromine (224mg, 1.4mmol) in DCM (1ml). The mixture was stirred overnight and then partitioned between EtOAc (50ml) and water (50ml). The organics were separated, washed with sodium hydrosulfite (2 x 50ml, 5% aq.), water (50ml), brine (50ml), dried (Na₂SO₄), filtered and concentrated *in vacuo.* The crude product was subjected to column chromatography (SiO₂; EtOAc) to give the title compound as a white solid (0.46g, 73%). δH (CDCl₃) 9.75 (1H, s), 8.69 (2H, d, J 5.9Hz), 7.64 (2H, d, J 6.0Hz), 7.25 (2H, d, J 8.2Hz), 7.20 (2H, d, J 8.2Hz), 5.89 (1H, d, J 8.3Hz), 5.06 (1H, dt, J 5.4, 8.2Hz), 4.30 (2H, q, J 7.1Hz), 3.35 (2H, m), 2.50 (3H,s), 1.84-1.33 (10H, m). m/z (ES⁺, 70V) 566.1 and 567.1 (MH⁺).

### EXAMPLE 28

### (2S)-2-{(2-Bromo-3-oxospiro[3.5]non-1-en-1-yl)amino}-3-{4-[(3-methyl[2.7]naphthyridin-1-yl)oxy]phenyl}propanoic acid

The compound of Example 27 (0.32g, 0.6mmol) was hydrolysed in a similar manner to the method of Reference Example 2, to give the title compound as a white solid (0.20g, 66%). δH (DMSO d⁶) 9.61 (1H, s), 8.88 (1H, d, J 9.5Hz), 8.72 (1H, d, J 5.7Hz), 7.74 (1H, d, J 5.8Hz), 7.35 (3H, c), 7.24 (2H, d, J 8.6Hz), 4.77 (1H. m), 3.18 (1H. dd, J 13.7, 4.70Hz), 3.01 (1H, dd, J 13.7, 10.4Hz), 2.49 (3H, s), 1.78-1.12 (10H, m). m/z (ES⁺, 70V) 537.1 and 538.1 (MH⁺).

### EXAMPLE 29

### Ethyl (2S)-2[(2-chloro-3-oxospiro[3.5]non-1-en-1-yl)amino]-3-{4-[(3,5-dichloroisonicotinoyl)amino]phenyl}propanoate

A solution of the compound of Example 1 (366mg, 0.71 mmol) in THF (25ml), at room temperature, was treated portionwise with N-chloro succinimide (100mg, 0.75mmol). After 30min the reaction mixture was poured into a mixture of EtOAc (150ml) and saturated aqueous NaHCO₃ solution (50ml). The organic layer was extracted and washed with brine (25ml), dried (MgSO₄), filtered and concentrated *in vacuo.* Chromatography (SiO₂; 70% EtOAc:hexanes) gave the title compound as a white powder (312mg, 0.56mmol, 80%). δH (CDCl₃) 8.50 (2H, s), 7.73 (1H, s), 7.53 (1H, d, J 8.4Hz), 7.04 (2H, d, J 8.4Hz), 5.73 (1H, d, J 8.0Hz), 4.88-4.81 (1H, m), 4.21 (2H, q, J 7.1Hz), 3.21-3.16 (2H, m), 1.79-1.65 (4H, m), 1.51-1.36 (6H, m), 1.28 (3H, t, J 7.1Hz). m/z (ES⁺,70V) 550.0 (MH⁺).

### EXAMPLE 30

### (2S)-2-[(2-Chloro-3-oxospiro[3.5]non-1-en-1-yl)amino]-3-{4-[(3,5-dichloroisonicotinoyl)amino]phenyl}propanoic acid

Hydrolysis of the compound of Example 29 (300mg, 0.54 mmol) with lithium hydroxide (60mg, 1.4mmol), according to the method of Reference Example 2, gave the title compound. δH (DMSO d⁶,390K) 10.44 (1H, br s), 8.69 (2H, s), 8.05-7.85 (1H, s br), 7.54 (2H, d, J 7.8Hz), 7.25 (2H, d, J 7.8Hz), 4.60-4.49 (1H, m), 3.21 (1H, dd, J 14.0, 5.3Hz), 3.04 (1H, dd, J 14.0, 5.1 Hz), 1.80-1.21 (10H, m). m/z (ES⁺, 70V) 521.9, 525.9 (MH⁺).

### EXAMPLE 31

### Ethyl (2S)-2-[(2-iodo-3-oxospiro[3.5]non-1-en-1-yl)amino]-3-{4-{(3,5-dichloroisonicotinoyl)amino]phenyl}propanoate

To a stirred solution of the compound of Example 1 (1.0g, 1.9mmol) in THF (10ml) at room temperature was added N-iodosuccinamide (460mg, 2.0mmol) in one portion. After 5 minutes the mixture was concentrated *in vacuo* and the residue triturated with a mixture of ether (10ml) and water (10ml), filtered and washed with ether and water. Oven drying gave the title compound (802mg, 66%) as a yellow solid. δH (DMSO d⁶) 8.90 (1H, d, J 9.1 Hz), 8.78 (2H, s), 7.58 (2H, d, J 8.5Hz), 7.25 (2H, d, J 8.5Hz), 4.91 (1H, m), 4.20 (2H, q, J 7.1 Hz), 3.30-3.00 (2H, m), 1.80-1.24 (10H, m), 1.21 (3H, t, J 7.1 Hz). m/z (ES⁺, 70V) 642.0 (MH⁺).

### EXAMPLE 32

### Ethyl (2S)-3-{4-[(3,5-dichloro-pyridine-4-carbonyl)-amino]-phenyl}-2-(3-oxo-2-pyridin-3-yl-spiro[3.5]non-1-en-1-ylamino)-propanoate

A mixture of the compound of Example 31 (1.0g, 1.6mmol), 10% palladium on charcoal (15mg), triphenylphosphine (100mg, 0.32mmol), copper (1) iodide (30mg, 0.16mmol), 3-pyridyl tributylstannane (560µl, 1.7mmol) in DMF (10ml) was heated to 100º under a nitrogen atmosphere fro 2 hours. The slvent was removed by evaporation in vacuo and the residue purified by column chromatography (SiO₂; 666:333:1 EtOAc:hexane;triethylamine) to give the title compound as a yellow oil (378mg, 41%). δH (DMSO d⁶) 8.76 (2H, s), 8.60 (1H, m), 8.30 (2H, br. s), 7.94 (1H, d, J 8.0Hz), 7.54 (2H, m), 7.34 (2H, m), 7.10 (1H, d, J 8.4Hz), 4.34 (1H, m), 4.24 (2H, q, J 5.3Hz), 3.25-2.95 (2H, m), 1.86-1.40 (10H, m), 1.26 (3H, t, J 5.3Hz). m/z (ES⁺, 70V) 593.0 (MH⁺).

### EXAMPLE 33

### (2S)-3-{4-[(3,5-Dichloro-pyridine-4-carbonyl)-amino]-phenyl}-2-(3-oxo-2-pyridin-3-yl-spiro[3.5]non-1-en-1-ylamino)-propanoic acid

The compound of Example 32 was hydrolysed in a similar manner to the method of Reference Example 2 to give the title compound as a white solid (76%). δH (DMSO d⁶, 400K) 10.28 (1H, s), 8.66 (2H, s), 8.59 (1H, s), 8.34 (1H, m), 7.80 (1H, m), 7.69 (1H, m), 7.51 (2H, m), 7.24 (4H, m), 4.28 (1H, m), 3.25-3.07 (2H, m), 1.90-1.50 (10H, m). m/z (ES⁺, 70V) 565.0 (MH⁺).

### Reference EXAMPLE 6

### Ethyl (2S)-3-{4-[(3,5-Dichloro-pyridine-4-carbonyl)-amino]-phenyl)-2-(2-iodo-4,4-dimethyl-3-oxo-cyclobut-1-enylamino)propanoate

Prepared in a similar manner to the compound of Example 31 from the compound of Reference Example 4 to give the title compound as a white solid (72%). δH (DMSO d⁶) 9.17 (1H, d, J 9.1 Hz), 8.79 (2H, s), 7.58 (2H, d, J 8.5Hz), 7.29 (2H, d, J 8.5Hz), 4.94 (1H, m), 4.20 (2H, q, J 7.1 Hz), 3.25-3.00 (2H, m), 1.23 (3H. t, J 7.1 Hz), 1.12 (3H, s), 1.03 (3H, s). m/z (ES⁺. 70V) 601.8 (MH⁺).

### EXAMPLE 34

### (2S)-2-[(2-iodo-3-oxospiro[3.5]non-1-en-1-yl)amino]-3-{4-[(3,5 dichloroisonicotinoyl)amino]phenyl}propanoic acid

The compound of Example 31 was hydrolysed in a similar manner to the method of Reference Example 2 to give the title compound as a white solid (98%). δH (DMSO d⁶) 10.87 (1H, s), 8.84 (1H, d, J 9.3Hz), 8.79 (2H, s), 7.58 (2H, d, J 8.5Hz), 7.27 (2H, d, J 8.5Hz), 4.87 (1H, m), 3.25 (1H, m), 3.02 (1H, m), 1.70-1.25 (10H, m). m/z (ES⁺, 70V) 613.8 (MH⁺).

### Reference EXAMPLE 7

### (2S)-3-{4-[(3,5-Dichloro-pyridine-4-carbonyl)-amino]-phenyl}-2-(2-iodo-4,4-dimethyl-3-oxo-cyclobut-1-enylamino)propanoic acid

The compound of Reference Example 6 was hydrolysed in a similar manner to the method of Reference Example 2 to give the title compound as a white solid (95%). δH (DMSO d⁶) 10.87 (1H, s), 9.08 (1H, d, J 9.3Hz), 8.78 (2H, s), 7.58 (2H, d, J 8.5Hz), 7.26 (2H, d, J 8.5Hz), 4.88 (1H, m), 3.25 (1H, m), 3.04 (1H, m), 1.12 (3H, s), 1.01 (3H, s). m/z (ES⁺, 70V) 573.8 (MH⁺).

### EXAMPLE 35

### Ethyl (2S)-3-{5-[(3,5-dichloroisonicotinoyl)amino]pyridin-2-yl}-2-[(3-oxaspiro[3.5]non-1-en-1-yl)amino]propanoate

To a solution of Intermediate 29 (470mg, 1.22mmol) in DCM (10ml) was added spiro[3.5]nonane-1, 3-dione (187mg, 1.23mmol) with stirred for 18hr. After evaporation of the solvent the crude product was pyrified by chromatography (silica, 3-4 % MeOH/DCM) to afford the title compound as a white foam (610mg, 96%). δH NMR (d⁶ DMSO) 8.81 (2H, s), 8.70 (1H, s), 8.33 (1H, d), 8.02 (1H. d), 7.32 (1H. d), 4.35 (1H, m), 4.13 (2H, m), 3.23 (2H, m), 1.53 (8H, br), 1.37 (2H, br), 1.17 (3H, t). m/z (ES⁺, 70V) 517 (MH⁺).

### EXAMPLE 36

### Ethyl (2S)-2-[(2-bromo-3-oxaspiro[3.5]non-1-en-1-yl)amino]-3-{5-[(3,5-dichloroisonicotinoyl)amino]pyridin-2-yl}propanoate

A solution of NBS (169mg, 0.94mmol) in dry DCM (5ml) was added to a stirred solution of the compound of Example 35 (490 mg, 0.94 mmol) in DCM (10ml) at 0°C (ice-water bath). After 30 min the solvent was evaporated *in vacuo* and the residue partitioned between Et₂O (80ml) and saturated sodium bicarbonate (20ml). The phases were separated and the aqueous layer re-extracted with Et₂O (40ml). The combined organics were washed with water (2 x 10ml), brine (10ml), dried (Na₂SO₄) and evaporated *in vacuo* and the residue purified by chromatography (silica, 50-80 % Et₂O/hexane) to give the title compound as a colourless glass foam (501mg, 88%). δH NMR (d⁶ DMSO) 11.17 (1H. s), 8.83 (2H, s), 8.73 (1H, s), 8.01 (1H, d), 7.34 (1H, d), 5.06 (1H, dd), 4.20 (2H, q), 3.39-3.20 (2H, brm), 1.73 (1H, m), 1.57 (8H, br), 1.34 (1H, br), 1.22 (3H, t). m/z (ES⁺, 70V) 596 (MH⁺).

### EXAMPLE 37

### Ethyl (2S)-2-[(2-bromo-3-oxaspiro[3.5]non-1-en-1-yl)amino]-3-{5-[(3,5-dichloroisonicotinoyl)amino]pyridin-2-yl}propanoate hydrochloride

The compound of Example 36 (300mg, 0.50mmol) was dissolved in EtOAc (20ml) and HCl gas bubbled through for a short time. The resulting white precipitate was collected by filtration, washed with Et₂O and dried to give the title compound as a white powder (155 mg, 48 %). δH NMR (d⁶ DMSO) 11.32 (1H, s), 8.84 (2H, s), 8.81 (1H, s), 8.13 (1H, d), 7.43 (1H, d), 5.06 (1H, dd), 4.19 (2H, q), 3.39 (1H, m), 3.28 (1H, m), 1.74 (1H. m), 1.57 (8H, br), 1.35 (1H, br), 1.22 (3H, t). m/z (ES⁺, 70V) 631 (MH⁺).

### EXAMPLE 38

### (2S)-2-[(2-bromo-3-oxaspiro[3.5]non-1-en-1-yl)amino]-3-{5-[(3,5-dichloroisonicotinoyl)amino]pyridin-2-yl}propanoic acid

The compound of Example 36 (370mg, 0.62mmol) was hydrolysed in a similar manner to the method of Reference Example 2 to give the title compound as a white solid (200 mg, 56 %) as light yellow solid. δH NMR (d⁶ DMSO) 11.16 (1H. s), 8.83 (2H, s), 8.73 (1H, s), 8.05 (1H, d), 7.35 (1H. d), 5.00 (1H. dd), 2.76 (2H, brm), 1.55 (8H, m), 1.27 (1H, br), 1.12 (1H, br). m/z (ES⁺, 70V) 568 (MH⁺).

### EXAMPLE 39

### Ethyl (2S)-2-[(2-chloro-3-oxaspiro[3.5]non-1-en-1-yl)amino]-3-{5-[(3,5-dichloroisonicotinoyl)amino]pyridin-2-yl)propanoate

A solution of NCS (247mg, 1.85mmol) in dry THF (10ml) was added to a stirred (ice-water bath cooled) solution of the compound of Example 35 (800mg, 1.54mmol) in THF (10ml) and DCM (10ml). After 2hr the solvent was evaporated *in vacuo* and the residue partitioned between Et₂O (250ml) and saturated sodium bicarbonate (30ml). The phases were separated and the organic layer was washed with brine (10ml), dried (Na₂SO₄) and evaporated *in vacuo* and the residue purified by chromatography (silica, 70-100 % Et₂O/hexane) to give the title compound as white foam (620mg, 72%). δH NMR (d⁶ DMSO) 8.96 (2H, s), 8.86 (1H, s), 8.20 (1H, d), 7.50 (1H, d), 5.08 (1H, m), 4.32 (2H, q), 3.53-3.31 (2H, brm), 1.72 (9H, m), 1.50 (1H, br), 1.34 (3H, t). m/z (ES⁺, 70V) 551 (MH⁺).

### EXAMPLE 40

### Ethyl (2S)-2-[(2-chloro-3-oxaspiro[3.5]non-1-en-1-yl)amino]-3-{5-[(3,5-dichloroisonicotinoyl)amino]pyridin-2-yl}propanoate hydrochloride

The compound of Example 39 (269mg, 0.48mmol) was dissolved in EtOAc (20ml) and HCl gas bubbled through for a short time. The resulting precipitate was collected by filtration, washed with Et₂O and dried to give the title compound (230 mg, 80.3 %). δH NMR (d⁶ DMSO) 11.21 (1H, s), 8.83 (2H, s), 8.75 (1H, s), 8.08 (1H, d), 7.39 (1H, d), 4.95 (1H, m), 4.20 (2H, q), 3.36 (1H, m), 3.26 (1H, m), 1.71 (1H, m), 1.57 (8H, br), 1.35 (1H, m), 1.21 (3H, t). m/z (ES⁺, 70V) 587 (MH⁺).

### EXAMPLE 41

### (2S)-2-[(2-chloro-3-oxaspiro[3.5]non-1-en-1-yl)amino]-3-{5-[(3,5-dichloroisonicotinoyl)amino]pyridin-2-yl}propanoic acid

The compound of Example 36 (250mg, 0.45mmol) was hydrolysed in a similar manner to the method of Reference Example 2 to give the title compound as a white powder (142mg, 60 %). δH NMR (d⁶ DMSO) 11.19 (1H, s), 8.83 (2H, s), 8.74 (1H, s), 8.07 (1H, d), 7.35 (1H, d), 4.90 (1H, m), 3.37 (1H, m), 3.19 (1H, m), 1.71-1.28 (10H, brm). m/z (ES⁺, 70V) 523 (MH⁺).

### EXAMPLE 42

### Ethyl (2S)-3-{5-[(3,5-dichloroisonicotinoyl)amino]pyridin-2-yl}-2-{[(2-(methylsulfanyl)-3-oxaspiro[3.5]non-1-en-1-yl)amino}propanoate

Sulphuryl chloride (49µL) was added dropwise to a stirred ice-bath cooled solution of dimethyl sulfide (74µL) in THF (5ml). The ice bath was removed and the solution stirred for 45 min. This solution was added to a stirred solution of the compound of Example 35 (700mg, 1.35mmol) in THF (10ml) and DCM (10ml) and stirred at RT. The reaction was worked up in a similar manner to that of Example 43 to give the title compound.

### EXAMPLE 43

### Ethyl (2S)-3-{5-[(3,5-dichloroisonicotinoyl)amino]pyridin-2-yl}-2-{[(2-(isopropylsulfanyl)-3-oxaspiro[3.5]non-1-en-1-yl)amino}propanoate.

Sulphuryl chloride (218µL) was added dropwise to a stirred ice-bath cooled solution of isopropyl disulphide (432µL) in THF (10ml). The ice bath removed and the mixture stood for 35 min. 5ml of this solution was added to a stirred solution of the compound of Example 35 (700mg, 1.35mmol) in THF (10ml) and DCM (10ml) and stirred at RT for 15 min. The solvent removed and the residue was partitioned between Et₂O (130ml) and saturated sodium bicarbonate (30ml). The phases were separated and the organic layer was washed with brine (10ml), dried (Na₂SO₄) and evaporated *in vacuo* and the residue purified by chromatography (silica, 2-3% EtOH/DCM) to give the title compound as a white foam. m/z (ES⁺, 70V) 591 (MH⁺).

### EXAMPLE 44

### Ethyl (2S)-3-{4-[(3,5-Dichloroisonicotinoyl)amino]phenyl}-2-[2-(1-methyl-pyridin-3-yl)-3-oxo-spiro[3.5]non-1-en-1-ylamino]-propanoate iodide salt

To a stirred solution of the compound of Example 32 (126mg, 0.21 mmol) in DMF (1ml) was added iodomethane (14mL, 0.23mmol). After 18 hrs the solvent was removed *in vacuo* to give the crude title compound which was used without further purification. m/z (ES⁺, 70V) 607.0 (MH⁺).

### EXAMPLE 45

### Ethyl (2S)-3-{4-[(3,5-Dichloroisonicotinoyl)amino]phenyl]-2-[2-(1-methyl-piperidin-3-yl)-3-oxo-spiro[3.5]non-1-en-1-ylamino]propanoate

The compound of Example 44 (127mg, 0.21 mmol) was dissolved in EtOH (10ml) and hydrogenated over platinum dioxide (50mg) at room temperature and 1 atmosphere hydrogen for 5 days. The catalyst was removed by evaporation in vacuo to afford the title compound as a yellow oil (129mg, 100%). δH NMR (d⁶ DMSO) 10.48 (1H, br s), 8.70 (2H, s), 7.59 (2H, d, J 8.1 Hz), 7.30 (2H, d, J 8.1 Hz), 4.25 (1H, m), 4.22 (2H, q, J 4.0Hz), 3.23 (1H, m), 3.08 (1H, m), 1.70-1.50 (22H, m), 1.26 (3H, m). m/z (ES⁺, 70V) 613.2 (MH⁺).

### EXAMPLE 46

### (2S)-3-{4-[(3,5-Dichloroisonicotinoyl)amino]-phenyl}-2-[2-(1-methyl-piperidin-3-yl)-3-oxo-spiro[3.5]non-1-en-1-ylamino]propanoic acid

The compound of Example 45 was hydrolysed in a similar manner to the method of Reference Example 2. The product was purified by passage through a short column (RP-18-silica; 5% aqueous acetonitrile) to give the title compound as a yellow solid (52%). δH NMR (d⁶ DMSO) 10.47 (1H, br s), 8.70 (2H, s), 7.57 (2H, d, J 7.7Hz), 7.27 (2H, d, J 7.7Hz), 4.13 (1H, m), 3.19 (1H, m), 3.02 (1H, m), 2.27 (3H, s), 1.70-1.30 (10H, m). m/z (ES⁺, 70V) 585.1 (MH⁺).

### EXAMPLE 47

### (2S)-Ethyl-2-[(2-chloro-3-oxo-7-oxa-sairo[3.5]non-1-en-1-yl)amino]-3-{4-[(3,5-dichloroisonicotinoyl)amino]phenyl}propanoate

A stirred solution of the compound of Example 10 (800mg, 1.54mmol) in THF (25ml), at rt was treated in several portions with N-chloro succinimide (226mg, 1.69mmol). After 1 h the crude reaction was partitioned between EtOAc (150ml) and brine (100ml). The organic layer was removed and washed with a further 100ml of brine and the organic phase dried (MgSO₄), filtered and concentrated *in vacuo.* Chromatography (SiO₂; 50% EtOAc:hexanes) gave the title compound as a white powder (625mg, 1.13mmol, 67%). δH (DMSO d⁶, 390K) 10.39 (1H, br s), 8.69 (2H, s), 8.39 (1H, d, J 8.8Hz), 7.57 (2H, s), 7.29 (2H, d, J 8.4Hz), 4.72 (1H, m), 4.24 (2H, q, J 7.1 Hz), 3.83-3.77 (2H, m), 3.73-3.62 (2H, m), 3.28 (1H, dd, J 5.5, 14.2Hz), 2.04-1.93 (2H, m), 1.54-1.51 (1H, m), 1.44-1.42 (1H, m), 1.27 (3H, t, J 7.1Hz). m/z (ES⁺,70V) 554 (MH⁺).

### EXAMPLE 48

### (2S)-2-[(2-Chloro-3-oxo-7-oxa-spiro[3.5]non-1-en-1-yl)amino]-3-{4-[(3,5-dichloroisonicotinoyl)-amino]phenyl}propanoic acid

Hydrolysis of the compound of Example 47 (525mg, 0.95mmol) with lithium hydroxide (80mg, 1.7mmol), according to the method of Reference Example 2, gave the title compound (412mg, 0.79mol, 83%). δH (DMSO d⁶, 390K) 10.40 (1H, s), 8.68 (2H, s), 8.30 (1H, br s), 7.55 (2H, d, J 5.8Hz), 7.27 (2H, d, J 5.8Hz), 4.63 (1H, m), 3.80-3.73 (2H, m), 3.69-3.61 (2H, m), 3.26 (1H, dd, J 4.9, 14.1 Hz), 3.07 (1H, dd, J 9.1, 14.1 Hz), 1.97-1.90 (2H, m), 1.51-1.48 (1H, m), 1.40-1.37 (1H, m). m/z (ES⁺, 70V) 524.0 (MH⁺).

### EXAMPLE 49

### (2S)-Ethyl-2-[(2-Chloro-3-oxo-spiro[3.6]dec-1-en-1-yl)amino]-3-(4-[(3,5-dichloro-isonicotinoyl)-amino]-phenyl}propanoate

Was prepared according to the method of Example 47 from the compound of Example 17 (800mg, 1.51 mmol) and N-chloro succinimide (222mg, 1.66mmol) to give the title compound as a white powder (625mg, 1.11 mmol, 74%). δH (DMSO d⁶, 390K) 10.40 (1H, s), 8.70 (2H, s), 8.11 (1H, s br), 7.57 (2H, s br), 7.29 (2H, d, J 8.3Hz), 4.68 (1H, m), 4.24 (2H, q, J 7.1 Hz), 3.28 (1H. dd, J 5.5, 14.3Hz), 3.12 (1H, dd, J 9.1, 14.3Hz), 1.82-1.52 (12H, m), 1.27 (3H, t, J 7.1 Hz). m/z (ES⁺, 70V) 566.0 (MH⁺).

### EXAMPLE 50

### (2S)-2-[(2-Chloro-3-oxo-spiro[3.6]dec-1-en-1-yl)amino]-3-{4-[(3.5-dichloroisonicotinoyl)amino]phenyl}-propanoic acid

Hydrolysis of the compound of Example 49 (600mg, 1.07mmol) with lithium hydroxide (80mg, 1.7mmol), according to the method of Reference Example 2, gave the title compound (512mg, 0.95mol, 89%). δH (DMSO d⁶, 390K) 10.37 (1H, s), 8.67 (2H, s), 7.52 (2H, m), 7.25 (2H, d, J 8.3Hz), 4.44 (1H, m), 3.22 (1H, dd, J 5.2, 14.0Hz), 3.13 (1H, dd, J 8.0, 13.9Hz), 1.98-1.41 (12H, m). m/z (ES⁺, 70V) 536.0 (MH⁺).

### EXAMPLE 51

### (2S)-Ethyl-2-[(3,7,7-trioxo-7λ⁶-thia-spiro[3.5]non-1-en-1-yl)amino]-3-{4-[(3,5-dichloroisonicotinoyl)amino]phenyl}-propanoate

Prepared from Intermediate 31 (1.1g, 5.4mmol) and the free base of Intermediate 15 (2.08mg, 5.5mmol), in a similar manner to the compound of Reference Example 5 to give the title compound as a white powder (712mg, 1.25mmol, 23%). δH (CDCl₃, 300K) 8.51 (1H, s), 8.33 (2H, s), 7.37 (2H, d, J 8.2Hz), 6.96 (2H, d, J 8.2Hz), 4.25 (1H, s), 4.10 (2H q, J 7.1 Hz), 4.01 (1H, m), 3.40-3.33 (2H, m), 3.06 (1H, dd, J 4.5, 14.2Hz), 2.90 (1H, dd, J 14.1, 8.0Hz), 2.79-2.75 (2H, m), 2.38-2.31 (2H, m), 1.99-1.96 (1H, m), 1.86-1.81 (1H, m), 1.16 (3H, t, J 7.1Hz). m/z (ES⁺, 70V) 565.9 (MH⁺).

### EXAMPLE 52

### (2S)-Ethyl-2-[(2-bromo-3,7,7-trioxo-7λ⁶-thia-spiro[3.5]non-1-en-1-yl)amino]-3-{4-[(3,5-dichloroisonicotinoyl)amino]phenyl}-propanoate

A solution of the compound of Example 51 (435mg, 0.77mmol) in THF (18ml) at 0º was treated portionwise with N-bromo succinimide (151mg, 0.85mmol). After 10min the reaction was partitioned between EtOAc (100ml) and saturated aqueous sodium hydrogencarbonate solution (50ml), the organic phase removed, dried (MgSO₄) and concentrated *in vacuo.* Chromatography (SiO₂, 50% EtOAc:hexanes) gave the title compound as a white powder (411 mg, 0.64mmol, 83%). δH (DMSO d⁶, 390K) 10.43 (1H, s), 9.00 (1H, d, J 8.4Hz), 8.69 (2H, s), 7.58 (2H, d, J 6.8Hz), 7.28 (2H, d, J 6.8Hz), 4.85 (1H, m), 4.23 (2H, q, J 7.1 Hz), 3.37-3.25 (3H, m, overlapping), 3.13-3.03 (3H, m, overlapping), 2.56-2.45 (2H, m), 2.09-1.89 (2H, m), 1.27 (3H, t, J 7.1 Hz). m/z (ES⁺, 70V) 645.9 (MH⁺).

### EXAMPLE 53

### (2S)-2-[(2-Bromo-3,7,7-trioxo-7λ⁶-thia-spiro[3.5]non-1-en-1-yl)amino]-3-{4-[(3,5-dichloroisonicotinoyl)amino]phenyl}-propanoic acid

Hydrolysis of the compound of Example 52 (410mg, 0.63mmol) with lithium hydroxide (31 mg, 0.7mmol), according to the method of Reference Example 2, gave the title compound (371 mg, 0.60mol, 95%). δH (DMSO d⁶, 390K) 10.41 (1H, s), 9.35 (1H, d, J 2.6Hz), 8.69 (2H, s), 7.55 (2H, d, J 6.7Hz), 7.27 (2H, d, J 6.7Hz), 4.70 (1H, m), 3.37-3.25 (5H, m), 3.05 (1H, dd, J 5.4, 13.3Hz), 2.3 (2H, m), 2.02 (1H, m), 1.92 (1H, m). m/z (ES⁺, 70V) 617.8 (MH⁺).

### EXAMPLE 54

### (2S)-Ethyl-2-[(3-thioxo-spiro[3.5]non-1-en-1-yl)amino]-3-{4-[(3,5-dichloroisonicotinoyl)amino]-phenyl}propanoate

A solution of the compound of Example 1 (700mg, 1.36mmol) and Lawesson's reagent [2,4-bis(4-methoxyphenyl)-1,2,3,4-dithiadiphosphetane 2,4-disulphide] (561mg, 1.38mmol) in toluene (25ml) was heated to 80º for 24h. The crude reaction was then partitioned between EtOAc (100ml) and brine (100ml). The organic phase was separated, dried (MgSO₄) and concentrated *in vacuo.* Chromatography (SiO₂, EtOAc) gave the title compound as a yellow powder (621 mg, 1.17mmol, 86%). δH (DMSO d⁶, 390K) 10.84 (1H, s), 8.96 (1H, d, J 8.9Hz), 8.78 (2H, s), 7.56 (2H, d, J 7.9Hz), 7.25 (2H, d, J 7.9Hz), 5.48 (1H, s), 4.37 (1H, m), 4.18 (2H, q, J 7.1Hz), 3.20 (1H, dd, J 4.9, 13.9Hz), 3.04 (1H, dd, J 9.9, 13.9Hz), 1.96-1.87 (2H, m), 1.63-1.42 (8H, m), 1.21 (3H, t, J 7.1Hz). m/z (ES⁺, 70V) 532.0 534.0 (MH⁺).

### EXAMPLE 55

### (2S)-2-[(3-Thioxo-spiro[3.5]non-1-en-1-yl)amino]-3-{4-[(3,5-dicloroisonicotinoyl)amino]phenyl}-propanoic acid

Hydrolysis of the compound of Example 54 (340mg, 0.66mmol) with lithium hydroxide (30mg, 0.67mmol), according to the method of Reference Example 2, gave the title compound (287mg, 0.57mol, 86%). δH (DMSO d⁶, 390K) 10.84 (1H, s), 8.87 (1H, d, J 8.8Hz), 8.77 (2H, s), 7.54 (2H, d, J 8.3Hz), 7.24 (2H, d, J 8.3Hz), 5.45 (1H, s), 4.23 (1H, m), 3.21 (1H, dd, J 4.4, 13.9Hz), 3.00 (1H, dd, J 9.9, 13.9Hz), 1.96-1.87 (2H, m), 1.67-1.41 (8H, m). m/z (ES⁺, 70V) 562.0 (MH⁺).

### EXAMPLE 56

### (2S)-2-[(3-oxo-spiro[3.4]oct-1-en-1-yl)amino]-3-{4-[(3,5-dichloroisonicotinoyl)amino]phenyl}-propanoate

A solution of 3-hydroxy-spiro[3.4]oct-2-en-1-one (330mg, 2.39mmol) [prepared according to the method of Wasserman, H.H. *et al* J. Org. Chem, 38, 1451-1455, (1973)] and the free base of Intermediate 15 (911mg, 2.39mmol), in DCM (5ml), was stirred at rt for 48h. The volatiles were removed *in vacuo* and the residue chromatographed (SiO₂; EtOAc) to give the title compound as a white solid (1.03g, 2.05mmol, 86%). δH (CDCl₃, 300K) 8.97 (1H, s), 8.41 (2H, s), 7.51 (2H, d, J 8.5Hz), 7.01 (2H, d, J 8.5Hz), 5.89 (1H, d, J 7.5Hz), 4.39 (1H, s), 4.21 (3H, obscured m), 3.15 (1H, dd, J 5.3, 14.0Hz), 3.03 (1H, dd, J 5.8, 14.0Hz), 1.74-1.49 (10H, m), 1.27 (3H, t, J 7.1 Hz). m/z (ES⁺, 70V) 502.0 (MH⁺).

### EXAMPLE 57

### (2S)-2-[(3-Oxo-spiro[3.4]oct-1-en-1-yl)amino]-3-{4-[(3,5-dichloroisonicotinoyl)amino]-phenyl}propanoic acid

Hydrolysis of the compound of Example 56 (150mg, 0.30mmol) with lithium hydroxide (30mg, 0.67mmol), according to the method of Reference Example 2, gave the title compound (112mg, 0.23mol, 79%). δH (DMSO d⁶, 390K) 13.08 (1H, s), 10.87 (1H, s), 8.79 (2H, s), 8.39 (1H, d, J 8.5Hz), 7.57 (2H, d, J 8.2Hz), 7.26 (2H, d, J 8.2Hz), 4.39 (1H, s), 4.14 (1H, m), 3.16 (1H, dd, J 4.7, 13.8Hz), 2.98 (1H, dd, J 9.4, 13.8Hz), 1.73-1.58 (10H, m). m/z (ES⁺, 70V) 473.9 (MH⁺).

### EXAMPLE 58

### (2S)-Ethyl-2-[(2-chloro-3-oxo-spiro[3.4]oct-1-en-1-yl)amino]-3-{4-[(3,5-dichloroisonicotinoyl)amino]phenyl}-propanoate

Prepared from the compound of Example 56 (1.25g, 2.49 mmol) and N-chloro succinimide (333mg, 2.7mmol), according to the method of Example 29, to give the title compound as a white powder (1.13g, 2.1mmol, 84%). δH (DMSO d⁶, 390K) 10.41 (1H, s), 8.68 (2H, s), 8.33 (1H, d, J 5.9Hz), 7.57 (2H, m), 7.27 (2H, m), 4.66 (1H, m), 4.21 (2H, q, J 7.1Hz), 3.26 (1H, dd, J 5.3, 14.1Hz), 3.11 (1H, dd, J 9.0, 14.1Hz), 1.98-1.58 (10H, m), 1.23 (3H, t, J 7.1 Hz). m/z (ES⁺, 70V) 535.9 (MH⁺).

### EXAMPLE 59

### (2S)-2-[(2-Chloro-3-oxo-spiro[3.4]oct-1-en-1-yl)amino]-3-{4-[(3,5-dichloro-pyridine-4-carbonyl)amino]phenyl}-propanoic acid

Hydrolysis of the compound of Example 58 (1.10g, 2.05mmol) with lithium hydroxide (100mg, 2.2mmol), according to the method of Reference Example 2, gave the title compound (976mg, 1.92mol, 94%). δH (DMSO d⁶, 390K) 10.41 (1H, s), 8.69 (2H, s), 8.26 (1H, s), 7.57 (2H, d, J 6.2Hz), 7.28 (2H, d, J 6.2Hz), 4.61 (1H, m), 3.26 (1H, dd, J 5.0, 14.1 Hz), 3.08 (1H, dd, J 9.1, 14.1 Hz), 1.92-1.60 (10H, m). m/z (ES⁺, 70V) 509.9 (MH⁺).

### EXAMPLE 60

### (2S)-Ethyl-2-[(2-bromo-3-oxo-spiro[3.4]oct-1-en-1-yl)amino]-3-{4-[(3,5-dichloroisonicotinoyl)amino]phenyl}-propanoate

A solution of the compound of Example 56 (1.25g, 2.49mmol) in THF (25ml) at rt was treated with N-bromo succinimide (443mg, 2.49mmol).

After 30min the reaction was diluted with EtOAc (100ml), washed with saturated aqueous sodium hydrogencarbonate solution (50ml) and the organic phase separated, dried (MgSO₄) and concentrated *in vacuo.* Chromatography (SiO₂, EtOAc) gave the title compound as a white powder (1.27g, 2.18mmol, 87%). δH (DMSO d⁶, 390K) 10.43 (1H, s), 8.69 (2H, s), 8.42 (1H, d, J 8.9Hz), 7.58 (2H, d, J 6.7Hz), 7.29 (2H, d, J 6.7Hz), 4.77 (1H, s), 4.22 (2H, q, J 7.1Hz), 3.26 (1H, dd, J 5.4, 14.1 Hz), 3.12 (1H, dd, J 9.0, 14.1 Hz), 1.98-1.62 (8H, m), 1.25 (3H, t, J 7.1 Hz). m/z (ES⁺, 70V) 582.0 (MH⁺).

### EXAMPLE 61

### (2S)-2-[(2-Bromo-3-oxo-spiro[3.4]oct-1-en-1-yl)amino]-3-{4-[(3,5-dichlorolsonicotinoyl)amino]phenyl}-propanoic acid

Hydrolysis of the compound of Example 60 (900mg, 1.54mmol) with lithium hydroxide (100mg, 2.2mmol), according to the method of Reference Example 2, gave the title compound (721 mg, 1.3mmol, 84%). δH (DMSO d⁶, 390K) 10.39 (1H, s), 8.68 (2H, s), 8.12 (1H, s br), 7.54 (2H, d, J 8.2Hz), 7.27 (2H, d, J 8.2Hz), 4.64 (1H, m), 3.25 (1H, dd, J 5.1, 14.1Hz), 3.11 (1H, dd, J 8.6, 14.1 Hz), 1.92-1.62 (8H, m). m/z (ES⁺, 70V) 553.9 (MH⁺).

### EXAMPLE 62

### (2S)-Ethyl-2-[(2-methylsulfanyl-3-oxo-spiro[3.5]non-1-en-1-yl)amino]-3-{4-[(3,5-dichloroisonicotinoyl)amino]phenyl}propanoate

A solution of the compound of Exa"mple 1 (1.0g, 1.94mmol) in THF (25ml) at rt was treated dropwise with a solution of methanesulfenyl chloride in DCM (2.13ml, 1.0M) [prepared according to the method of Still, I. W. J., *et al.* J. Org. Chem., *1982,* 47, 560]. After 20min the reaction was diluted with EtOAc (100ml) and washed with saturated aqueous sodium hydrogencarbonate solution (50ml). The organic phase was separated, dried (MgSO₄), filtered and concentrated *in vacuo.* Chromatography (SiO₂, 60% EtOAc:hexanes) gave the title compound as a white powder (1.03g, 1.83mmol, 94%). δH (DMSO d⁶, 390K) 10.86 (1H, s), 8.78 (2H, s), 8.70 (1H, d, J 9.2Hz), 7.57 (2H, d, J 8.4Hz), 7.26 (2H, d, J 8.4Hz), 5.11 (1H, m), 4.18 (2H, q, J 7.1Hz), 3.20 (1H, dd, J 4.6, 13.9Hz), 3.00 (1H, dd, J 9.8, 13.9Hz), 1.93 (3H, s), 1.66-1.33 (10H, m), 1.21 (3H, t, J 7.1Hz). m/z (ES⁺, 70V) 562.1 (MH⁺).

### EXAMPLE 63

### (2S)-2-[(2-Methylsulfanyl-3-oxo-spiro[3.5]non-1-en-1-yl)amino]-3-{4-[(3,5-dichloroisonicotinoyl)amino]phenyl}-propanoci acid

Hydrolysis of the compound of Example 62 (600mg, 1.07mmol) Reference with lithium hydroxide (100mg, 2.2mmol), according to the method of Reference Example 2, gave the title compound as a white powder (421 mg, 0.79mmol, 73%). δH (DMSO d⁶, 390K) 10.84 (1H, s), 8.77 (2H, s), 8.44 (1H, d, J 8.8Hz), 7.54 (2H, d, J 8.4Hz), 7.23 (2H, d, J 8.4Hz), 4.90 (1H, m), 3.19 (1H, dd, J 4.4, 13.7Hz), 2.98 (1H, dd, J 9.1, 13.7Hz), 1.93 (3H, s), 1.79-1.54 (8H, m), 1.36-1.22 (2H, m). m/z (ES⁺, 70V) 534.0 (MH⁺).

### EXAMPLE 64

### (2S)-Ethyl-2-[(2-fluoro-3-oxo-spiro[3.5]non-1-en-1-yl)amino]-3-{4-[(3,5-dichloroisonicotinoyl)amino]phenyl}-propanoate

A solution of the compound of Example 1 (2.02g, 3.91 mmol) in THF (55ml) was treated with Selectfluor^{™} reagent (1.38g, 3.89mmol) and heated to 70º. After 48h the reaction was diluted with EtOAc (300ml) and washed with saturated aqueous sodium hydrogencarbonate solution (50ml). The organic phase was dried (MgSO₄), filtered and concentrated *in vacuo.* Chromatography (SiO₂, 60% EtOAc:hexanes) gave the title compound as a white powder (1.87g, 3.50mmol, 89%). δH (DMSO d⁶, 390K) 10.89 (1H, s), 8.81 (2H, s), 8.47 (1H, d, J 8.7Hz), 7.59 (12H, d, J 8.5Hz), 7.27 (2H, d, J 8.5Hz), 4.26 (1H, m), 4.19 (2H, q, J 7.1 Hz), 3.21 (1H, dd, J 4.9, 13.8Hz), 2.98 (1H, dd, J 9.8, 13.8Hz), 1.70-1.38 (10H, m), 1.22 (3H, t, J 7.1 Hz). m/z (ES⁺, 70V) 534.1 (MH⁺).

### EXAMPLE 65

### (2S)-2-(2-Fluoro-3-oxo-spiro[3.5]non-1-en-1-ylamino)3-{4-[(3,5-dichloroisonicotinoyl)amino]phenyl}-propanoic acid

Hydrolysis of the compound of Example 64 (273mg, 0.51 mmol) with lithium hydroxide (60mg, 1.3mmol), according to the method of Reference Example 2, gave the title compound as a white powder (197mg, 0.39mmol, 76%). δH (DMSO d⁶, 390K) 10.87 (1H, s), 8.80 (2H, s), 8.30 (1H, d, J 8.7Hz), 7.58 (2H, d, J 8.2Hz), 7.25 (2H, d, J 8.2Hz), 4.15 (1H, m), 3.19 (1H, dd, J 4.5, 13.8Hz), 2.96 (1H, dd, J 9.5, 13.8Hz), 1.92-1.49 (8H, m), 1.37-1.16 (2H, m). m/z (ES⁺, 70V) 506.0 (MH⁺).

### EXAMPLE 66

### (2S)-2-[(2-Fluoro-3-oxo-7-oxa-spiro[3.5]non-1-en-1-yl)amino]-3-[4-[(3,5-dichloroisonicotinoyl)amino]phenyl}-propanoic acid

A mixture of the compound of Example 10 (1.0g, 1.93mmol) and Selectfluor^{™} reagent (1.0g, 2.8mmol) in THF (25ml) was heated to reflux for 72h. The crude reaction was then diluted with EtOAc (100ml) and washed with saturated aqueous sodium hydrogencarbonate solution (50ml). The organic phase was dried (MgSO₄), filtered and concentrated *in vacuo* to give a gum. This was then redissolved in THF (20ml) and treated with lithium hydroxide (80mg, 1.78mmol) and water (1ml) and stirred for 24h at rt. Acidification with a few drops of AcOH followed by concentration *in vacuo* and chromatography (SiO₂, 10:90:3:2 MeOH:DCM:AcOH:H2O) gave the title compound as a white powder (561 mg, 1.1 mmol, 57%). δH (DMSO d⁶, 390K) 10.89 (1H, s), 8.80 (2H, s), 8.58 (1H, d, J 8.8Hz), 7.59 (2H, d, J 8.4Hz), 7.27 (2H, d, J 8.4Hz), 4.23 (1H, m), 3.76 (2H, m), 3.60 (2H, m), 3.23 (1H, dd, J 4.4, 13.9Hz), 2.96 (1H, dd, J 9.8, 13.9Hz), 1.98-1.93 (2H, m), 1.47 (1H, m), 1.30 (1H, m). m/z (ES⁺, 70V) 508.0 (MH⁺).

### EXAMPLE 67

### (2S)-Ethyl-2-[(2-Isopropylsulfanyl-3-oxo-spiro[3.5]non-1-en-1-yl)amino]-3-{4-[(3,5-dichloroisonicotinoyl)amino]phenyl}-propanoate

A stirred solution of the compound of Example 1 (1.0g, 1.93mmol) in THF (50ml) at rt was treated dropwise with a 1.0M solution of isopropyl sulfenyl chloride in DCM until a yellow colouration of the reaction mixture just persisted. The reaction was then diluted with EtOAc (200ml) and washed with saturated aqueous sodium hydrogencarbonate solution (50ml). The organic phase was separated, dried (MgSO₄), filtered and concentrated *in vacuo.* Chromatography (SiO₂, 100% EtOAc) gave the title compound as a pale yellow powder (987mg, 87%). δH (DMSO d⁶, 390K) 10.85 (1H, s), 8.79 (2H, s), 8.73 (1H, d, J 9.5Hz), 7.56 (2H, d, J 8.5Hz), 7.25 (2H, d, J 8.5Hz), 5.20 (1H, m), 4.17 (2H, q, J 7.1Hz), 3.18 (1H, dd, J 4.3, 13.8Hz), 2.97 (1H, dd, J 10.2, 13.8Hz), 2.65 (1H, m), 1.73-1.57 (8H, m), 1.36-1.33 (1H, m), 1.21 (3H, t, J 7.1Hz), 1.17-1.14 (1H, m), 1.02 (6H, d, J 6.6Hz). m/z (ES⁺, 70V) 590.0 (MH⁺).

### EXAMPLE 68

### (2S)-2-[(2-Isopropylsulfanyl-3-oxo-spiro[3.5]non-1-en-1-yl)amino]-3-{4-[(3,5-dichloroisonicotinoyl)amino]phenyl}-propanoic acid

Hydrolysis of the compound of Example 67 (400mg, 0.68mmol) with lithium hydroxide (100mg, 2.2mmol), according to the method of Reference Example 2, gave the title compound as a white powder (333mg, 0.59mmol, 89%). δH (DMSO d⁶, 390K) 10.84 (1H, s), 8.78 (2H, s), 8.62 (1H, d, J 9.4Hz), 7.55 (2H, d, J 8.1 Hz), 7.25 (1H, d, J 8.1 Hz), 5.12 (1H, m), 3.20 (1H, dd, J 3.6, 13.7Hz), 2.94 (1H, dd, J 10.2, 13.7Hz), 2.62 (1H, m), 1.91-1.64 (8H, m), 1.59-1.56 (1H, m), 1.36-1.33 (1H, m), 1.02 (6H, d, J 6.6Hz). m/z (ES⁺, 70V) 562.0 (MH⁺).

### Reference EXAMPLE 8

### (2S)-Ethyl-2-[(2-isopropylsulfanyl-4,4-dimethyl-3-oxo-cyclobut-1-enyl)amino]-3-[4-([2,7]naphthyridin-1-ylamino)phenyl] propanoate

A solution of 3-hydroxy-4,4-dimethyl-2-cyclobutenone (333mg, 2.97mmol) and Intermediate 6 (1.0g, 2.98mmol) in THF (25ml), was stirred at rt for 48h. The volatiles were removed *in vacuo* and the residue chromatographed (SiO₂; EtOAc) affording 970mg of the coupled product of approx 90% purity. This intermediate was redissolved in THF (35ml) and treated with a 1.0M solution of isopropyl sulfenyl chloride in DCM (3.0ml, 3.0mmol) at rt. After 60min the reaction was diluted with EtOAc (100ml) and washed with saturated aqueous sodium carbonate solution (50ml), the organic phase was dried (MgS04), filtered and concentrated *in vacuo.* Chromatography (SiO₂, EtOAc) gave the title compound as a white powder (817mg, 1.62mmol, 54%). δH (DMSO d⁶, 390K) 9.83 (1H, s), 9.52 (1H, s), 9.01 (1H, d, J 9.4Hz), 8.65 (1H, d, J 5.7Hz), 8.16 (1H, d, J 5.7Hz), 7.81 (2H, d, J 8.5Hz), 7.68 (1H, d, J 5.7Hz), 7.23 (2H, d, J 8.5Hz), 7.13 (1H, d, J 5.7Hz), 5.24 (1H, m), 4.19 (2H, q, J 7.1 Hz), 3.20 (1H, dd, J 4.5, 13.8Hz), 2.97 (1H, dd, J 10.0, 13.8Hz), 2.76 (1H, m), 1.22 (3H, t, J 7.1Hz), 1.13 (3H, s), 1.05 (6H, d, J 6.7Hz), 1.04 (3H, s). m/z (ES⁺, 70V) 505.2 (MH⁺).

### EXAMPLE 69

### (2S)-Ethyl-2-[(2-isopropylsulfanyl-3-oxo-spiro[3.5]non-1-en-1-yl)amino]-3-[4-([2.7]naphthyridin-1-ylamino)phenyl]-propanoate

Was prepared in two steps from Intermediate 6 (1.0g, 2.98mmol), 1-keto-3-hydroxyspiro[3,5]-non-2-ene (452mg, 2.97mmol) [prepared according to the method of Wasserman, H.H. *et al,* J. Org. Chem., 38, 1451-1455 (1973)] and a 1.0M solution of isopropyl sulfenyl chloride in DCM (3.5ml, 3.5mmol) according to the method of Reference Example 18 to give the title compound as a yellow powder (931 mg, 1.71mmol, 58%). (DMSO d⁶, 390K) 9.89 (1H, s), 9.58 (1H, s), 8.82 (1H, d, J 9.4Hz), 8.71 (1H, d, J 5.6Hz), 8.21 (1H, d, J 5.6Hz), 7.85 (2H, d, J 8.3Hz), 7.74 (1H, d, J 5.6Hz), 7.28 (2H, d, J 8.3Hz), 7.18 (1H, d, J 5.6Hz), 5.31 (1H, m), 4.24 (2H, q, J 7.1 Hz), 3.24 (1H, dd, J 4.1, 13.7Hz), 3.03 (1H, dd, J 10.3, 13.7Hz), 2.80 (1H, m), 1.80-1.71 (8H, m), 1.46-1.43 (1H, m), 1.28 (3H, t, J 7.1Hz), 1.24-1.21 (1H, m), 1.11 (6H, d, J 6.5Hz). m/z (ES⁺, 70V) 545.2 (MH⁺).

### EXAMPLE 70

### (2S)-2-[(2-Isopropylsulfanyl-3-oxo-spiro[3.5]non-1-en-1-yl)amino]-3-[4-([2.7]naphthyridin-1-ylamino)-phenyl]-propanoic acid

Hydrolysis of the compound of Example 69 (900mg, 1.62mmol) with lithium hydroxide (150mg, 3.3mmol), according to the method of Reference Example 2, gave the title compound as a bright yellow powder (790mg, 1.53mmol, 94%). δH (DMSO d⁶, 390K) 9.83 (1H, s), 9.51 (1H, s), 8.67 (1H, d, J 5.6Hz), 8.65 (1H, d, J 5.6Hz), 8.15 (1H, d, J 5.7Hz), 7.77 (2H, d, J 8.4Hz), 7.68 (1H, d, J 5.7Hz), 7.23 (2H, d, J 8.4Hz), 7.12 (1H, J 5.7Hz), 5.20 (1H, m), 3.22 (1H, dd, J 3.9, 13.8Hz), 2.94 (1H, dd, J 10.4, 13.8Hz), 1.79-1.66 (8H, m), 1.40-1.35 (1H, m), 1.20-1.15 (1H, m), 1.05 (3H, d, J 6.3Hz), 1.03 (3H, d, J 6.3Hz). m/z (ES⁺, 70V) 517.2 (MH⁺).

### EXAMPLE 71

### (2S)-Ethyl-2-[(2-isopropylsulfanyl-3-oxo-7-oxa-spiro[3.5]non-1-en-1-yl)amino]-3-[4-([2,7]naphthyridin-1-ylamino)-phenyl]-propanoate

Was prepared in two steps from Intermediate 6 (1.96g, 5.8mmol), 7-oxaspiro[3.5]nonane-1,3-dione (1.0g, 6.5mmol) [prepared according to the method of Wasserman, H.H. *et al,* J. Org. Chem., 38, 1451-1455 (1973)] and a 1.0M solution of isopropyl sulfenyl chloride in DCM (5.4ml, 5.4mmol) according to the method of 118 to give the title compound as a yellow powder (1.15g, 2.1mmol, 36%). δH (DMSO d⁶, 390K) 9.83 (1H, s), 9.52 (1H, s), 8.94 (1H, d, J 9.5Hz), 8.65 (1H, d, J 5.6Hz), 8.15 (1H, d, J 5.7Hz), 7.78 (2H, d, J 8.5Hz), 7.68 (1H, d, J 5.6Hz), 7.23 (2H, d. J 8.5Hz), 7.12 (1H, d, J 5.7Hz), 5.26 (1H, m), 4.19 (2H, q, J 7.1 Hz), 3.81-3.76 (2H, m), 3.64-3.55 (2H, m), 3.20 (1H, dd, J 4.3, 13.8Hz), 2.96 (1H, dd, J 10.3, 13.8Hz), 2.81-2.74 (1H, m), 2.06-1.93 (2H, m), 1.50-1.47 (1H, m), 1.32-1.28 (1H, m), 1.23 (3H, t, J 7.1 Hz), 1.07 (3H, d, J 6.6Hz), 1.05 (3H. d, J 6.6Hz). m/z (ES⁺, 70V) 547.2 (MH⁺).

### EXAMPLE 72

### (2S)-2-[(2-Isopropylsulfanyl-3-oxo-7-oxa-spiro[3.5]non-1-en-1-yl)amino]-3-[4-([2,7]naphthyridin-1-ylamino)-phenyl]-propanoic acid

Hydrolysis of the compound of Example 71 (906mg, 1.66mmol) with lithium hydroxide (150mg, 3.3mmol), according to the method of Reference Example 2, gave the title compound as a pale yellow powder (801 mg, 1.54mmol, 93%). δH (DMSO d⁶, 390K) 9.82 (1H, s), 9.51 (1H, s), 8.86 (1H, d, J 9.5Hz), 8.65 (1H, d, J 5.5Hz), 8.14 (1H, d, J 5.6Hz), 7.76 (2H, d, J 8.1 Hz), 7.68 (1H, d, J 5.5Hz), 7.28 (2H, d, J 8.1Hz), 7.12 (1H, d, J 5.6Hz), 5.24-5.19 (1H, m), 3.78 (2H, m), 3.61 (2H, m), 3.21 (1H, dd, J 3.5, 13.8Hz), 2.91 (1H, dd, J 10.7, 13.8Hz), 2.77-2.71 (1H, m), 2.05-1.91 (2H, m), 1.49-1.46 (1H, m), 1.30-1.26 (1H, m), 1.07 (3H, s), 1.03 (3H, s). m/z (ES⁺, 70V) 519.1 (MH⁺).

### EXAMPLE 73

### (2S)-Ethyl-2-[(3-oxo-spiro[3.4]octa-1,6-dien-1-yl)amino]-3-{4-[(3,5-dichloroisonicotinoyl)amino]phenyl}propanoate

A solution of Intermediate 33 (1.0g, 7.3mmol) and the free base of Intermediate 15 (2.48g, 7.3mmol), in DCM (25ml), was stirred at room temperature for 48h. The volatiles were removed *in vacuo* and the residue chromatographed (SiO₂; EtOAc) to give the title compound as a white solid (2.14g, 4.28mmol, 59%). δH (CDCl₃, 300K) 9.02 (1H, s), 8.38 (2H, s), 7.49 (2H, d, 8.5Hz), 7.00 (2H, d, J 8.5Hz), 6.03 (1H, d, J 7.8Hz), 5.54 (2H, s), 4.41 (1H, s), 4.21 (2H, q, J 7.1 Hz), 4.20 (1H, m), 3.15 (1H, dd, J 5.2, 14.0Hz), 3.03 (1H, dd, J 6.1, 14.0Hz), 1.56-1.51 (2H, m), 2.38-2.34 (2H, m), 1.18 (3H, t, J 7.1 Hz). m/z (ES⁺, 70V) 500.0 (MH⁺).

### EXAMPLE 74

### (2S)-2-[(3-Oxo-spiro[3.4]octa-1,6-dien-1-yl)amino]-3-{4-[(3,5-dichloroisonicotinoyl)amino]phenyl}-propanoic acid

Hydrolysis of the compound of Example 73 (970mg, 1.94mmol) with lithium hydroxide (100mg, 2.2mmol), according to the method of Reference Example 2, gave the title compound (896mg, 1.90mol, 98%). δH (DMSO d⁶, 390K) 10.87 (1H, s), 8.80 (2H, s), 8.45 (1H, d, J 8.4Hz), 7.57 (2H, d, J 8.5Hz), 7.27 (2H, d, J 8.5Hz), 5.63 (2H, s), 4.41 (1H, s), 4.11 (1H, m), 3.17 (1H, dd, J 4.8, 13.9Hz), 2.98 (1H, dd, J 9.3, 13.9Hz), 2.46-2.42 (2H, m), 2.36-2.25 (2H, m). m/z (ES⁺, 70V) 471.9 (MH⁺).

### EXAMPLE 75

### (2S)-Ethyl-2-[(2-bromo-3-oxo-spiro[3.4]octa-1,6-dien-1-yl)amino]-3-{4-[(3,5-dichloroisonicotinoyl)amino]phenyl}-propanoate

Bromination of the compound of Example 73 (206mg, 0.41 mmol) with N-bromo succinimide (75mg, 0.57mmol), according to the method of Example 8 afforded the title compound as a white solid (116mg, 0.20mmol, 50%). δH (DMSO d⁶, 390K) 10.36 (1H, s), 8.67 (2H, s), 8.45 (1H, d, J 9.0Hz), 7.58 (2H, d, J 8.5Hz), 7.28 (2H, d, J 8.5Hz), 5.67-5.63 (2H, m), 4.50 (1H, s br), 4.23 (2H, q, J 7.1 Hz), 3.28 (1H, dd, J 6.3, 14.1Hz), 3.15 (1H, dd, J 9.0, 14.1Hz), 2.52-2.42 (4H, m), 1.28 (3H, t, J 7.1 Hz). m/z (ES⁺, 70V) 579.0 (MH⁺).

### EXAMPLE 76

### (2S)-2-[(2-Bromo-3-oxo-spiro[3.4]octa-1,6-dien-1-yl)amino]-3-{4-[(3,5-dichloroisonicotinoyl)amino]phenyl}-propanoic acid

Hydrolysis of the compound of Example 75 (190mg, 0.33mmol) with lithium hydroxide (60mg, 1.3mmol), according to the method of Reference Example 2, gave the title compound (162mg, 0.29mol, 88%). δH (DMSO d⁶, 390K) 10.89 (1H, s), 9.09-9.04 (1H, m), 8.81 (2H, s), 7.62 (2H, d, J 8.5Hz), 7.29 (2H, d, J 8.5Hz), 5.69-5.60 (2H, m), 4.72 (1H, m), 3.03 (1H, dd, J 9.0, 14.1Hz), 2.98 (1H, dd, J 6.7, 14.1Hz), 2.62-2.20 (4H, m). m/z (ES⁺, 70V) 551.8 (MH⁺).

### EXAMPLE 77

### (2S)-Ethyl-2-[(7-acetyl-2-bromo-3-oxo-7-aza-spiro[3.5]non-1-en-1-yl)amino]-3-{4-[(3,5-dichloroisonicotinoyl)amino]phenyl}-propanoate

A solution of the compound of Example 21 (100mg, 0.18mmol) in THF (10ml) at rt was treated with N-bromo succinimide (32mg, 0.19mmol). After 30min the reaction was diluted with EtOAc (50ml) and saturated aqueous sodium hydrogencarbonate solution (30ml), the organic phase separated and dried (MgSO₄), filtered and concentrated *in vacuo.* Chromatography (SiO₂, EtOAc) gave the title compound as a white powder (79mg, 0.12mmol, 67%). δH (CDCl₃, 300K) 9.54 (1H, s), 8.65 (1H, d, J 9.4Hz), 8.19 (2H, s), 7.49 (2H, d, J 8.4Hz), 7.07 (2H, d, J 8.4Hz), 4.91-4.81 (1H, m), 4.11 (2H, q, J 7.1 Hz), 3.49-3.44 (1H, m), 3.25-2.66 (5H, m), 1.89 (3H, s), 1.86-1.25 (4H, m), 1.18 (3H, t, J 7.1 Hz). m/z (ES⁺, 70V) 639.0 (MH⁺).

### EXAMPLE 78

### (2S)-2[(7-Acetyl-2-bromo-3-oxo-7-aza-spiro[3.5]non-1-en-1-yl)amino]-3-{4-[(3,5-dichloroisonicotinoyl)amino]phenyl}-propanoic acid

The compound of Example 77 (50mg, 0.078mmol) was hydrolysed in a similar manner to the method of Reference Example 2, to give the title compound as a white powder (21mg, 0.034mmol, %). δH(DMSO d⁶, 390K) 10.38 (1H, s), 8.96 (2H, s), 7.57 (2H, s br), 7.28 (2H, d, J 7.9Hz), 4.78 (1H, m), 3.99-3.96 (2H, m), 3.30-3.06 (4H, m), 2.00 (3H, s), 1.94-1.84 (2H, m), 1.48-1.21 (2H, m). m/z (ES⁺, 70V) 610.9 (MH⁺).

### EXAMPLE 79

### Isopropyl (2S) 2-(2-Bromo-3-oxo-spiro[3.5]non-1-en-1-ylamino)-3-{4-[(3,5-dichloroisonicotinoyl)amino]phenyl} propanoate

To a solution of the compound of Example 6 (0.5g, 0.9mmol) in DMF (5ml) was added EDC (185mg, 1.1eq), HOBT (135mg, 1.1eq) and *iso*propanol (0.5ml). The mixture was stirred at room temperature for 48h. then partitioned between EtOAc (100ml) and water (50ml). The aqueous was separated and the organics washed with water (5 x 30ml), brine (30ml), dried (Na₂SO₄), filtered and concentrated *in vacuo* to give a crude solid. The crude was triturated with diethyl ether to give the title compound as a white powder (0.35g, 65%) δH NMR (300MHz, DMSO d⁶) 10.69 (1H, br), 8.68 (1H, d, J 9.1 Hz), 8.60 (2H, s), 7.39 (2H, d, J 8.5Hz), 7.08 (2H, d, J 8.5Hz), 4.78 (1H, sep, J 6.3Hz), 4.53 (1H, m), 3.01 (1H, dd, J 4.9, 13.8Hz), 2.83 (1H, dd, J 9.5, 13.9Hz), 1.36-1.60 (9H, m), 1.19 (1H, d, J 12.7Hz), 0.98-1.05 (6H, dd). m/z (ESI, 70V) MH⁺ 608.

### EXAMPLE 80

### (2S) 2-(2-Bromo-3-oxo-spiro[3.5]non-1-en-1-ylamino)-3-{4-[(3.5-dichloro-isonicotinoyl)amino]phenyl}propanoic acid 1-methyl-piperidin-4-yl ester

Using a similar procedure to that for the preparation the compound of Example 79 from the compound of Example 6 (0.5g, 0.89mmol), EDC (185mg, 1.1eq), HOBT (135mg, 1.1eq), 4-hydroxy-1-methylpiperidine (0.5ml) and DMF (2ml) was prepared the title compound (0.21g, 36%). δH NMR (300MHz, DMSO d⁶) 11.01 (1H, br), 9.01 (1H, d, J 9.4Hz), 8.92 (2H, s), 7.71 (2H, d, J 8.5Hz), 7.40 (2H, d, J 8.5Hz), 4.90 (1H, br), 3.33 (1H, dd, J 13.8, 4.8Hz), 3.16 (1H, dd, J 13.8, 9.6Hz), 2.55 (2H, br), 2.40 (2H, br), 2.24 (3H, d, J 8.0Hz), 1.64-1.95 (12H, m), 1.50 (2H, d, J 12.1 Hz), 1.23 (2H, br). m/z (ESI, 70V) MH+ 664.

### EXAMPLE 81

### (2S) 2-(2-Bromo-3-oxo-spiro[3.5]non-1-en-1-ylamino)-3-{4-[(3,5-dichloroisonicotinoyl)amino]phenyl} propanoic acid cyclopentyl ester

Using a similar procedure to that for the preparation the compound of Example 79 from the compound of Example 6 (0.5g, 0.89mmol, EDC (600mg, 3eq), HOBT (400mg, 3eq), cyclopentanol (0.5ml) and DMF (5ml)] was prepared the title compound. δH NMR (400MHz, DMSO d⁶) 10.88 (1H, s), 8.85 (1H, d, J 9.0Hz), 8.78 (2H, s), 7.58 (2H, d, J 8.5Hz), 7.26 (2H, d, J 8.5Hz), 5.15 (1H, m), 4.71 (1H, m), 3.18 (1H, dd, J 5.2, 13.9Hz), 3.02 (1H, dd, J 9.5, 13.9Hz), 1.17-1.84 (18H, m), 1.14 (1H, m). m/z (ESI, 70V) MH⁺ 634.

### EXAMPLE 82

### (2S) 2-(2-Bromo-3-oxo-spiro[3.5]non-1-en-1-ylamino)-3-{4-[(3,5-dichloro-isonicotinoyl)amino]phenyl}propanoic acid tetrahydrofuran-3-yl ester

Using a similar procedure to that for the preparation the compound of Example 79 from the compound of Example 6 acid (0.5g, 0.89mmol), EDC (600mg, 3eq), HOBT (400mg, 3eq), (S)-3-hydroxytetrahydrofuran (0.5ml) and dimethylformamide (5ml)] was prepared the title compound. δH NMR (300MHz, DMSO d⁶) 8.87 (1H, d, J 8.9Hz), 8.79 (2H, s), 7.58 (2H, d, J 8.4Hz), 7.27 (2H, d, J 8.5Hz), 5.30 (1H, m), 4.77 (1H, m), 3.72-3.83 (3H, m), 3.65 (1H, d, J 10.4Hz), 3.20 (1H, dd, J 14.0, 5.1 Hz), 3.04 (1H, dd, J 14.0, 9.7Hz), 2.11-2.27 (1H, m), 1.87-1.99 (1H, m), 1.37-1.78 (9H, m), 1.07-1.17 (1H, m). m/z (ESI, 70V) MH⁺ 636.

### EXAMPLE 83

### 2-(2-Bromo-3-oxo-spiro[3.5]non-1-en-1-ylamino)-3-{4-[(3,5-dichloroisonicotinoyl)amino]phenyl}propanoic acid 1-methyl-pyrrolidin-3-yl ester

Using a similar procedure to that for the preparation the compound of Example 79 from the compound of Example 6 (0.5g, 0.89mmol), EDC (600mg, 3eq), HOBT (400mg, 3eq), 1-methyl-3-pyrrolidinol (0.6ml) and DMF (5ml) was prepared the title compound. δH NMR (400MHz, DMSO d⁶) 10.88 (1H, s), 8.87 (1H, d, J 9.0Hz), 8.78 (2H, s), 7.58 (2H, d, J 8.3Hz), 7.26 (2H, d, J 8.5Hz), 5.13 (1H, m), 4.74 (1H, m), 3.16 (1H, m), 3.04 (1H, m), 2.43-2.73 (2H, m), 2.07-2.26 (2H, m), 1.55-1.75 (11H, m), 1.11 (1H, m). m/z (ESI, 70V) MH⁺ 649.

### EXAMPLE 84

### 2-(2-Bromo-3-oxo-spiro[3.5]non-1-en-1-ylamino)-3-{4-[(3.5-dichloropyridine-4-carbonyl)-amino]-phenyl}-propanoic acid phenyl ester

Using a similar procedure to that for the preparation the compound of Example 79 from the compound of Example 6 (0.5g, 0.89mmol, EDC (500mg, 2.6eq), HOBT (350mg, 2.6eq), phenol (0.5g) and DMF (5ml)] was prepared the title compound (0.17g, 29%). δH (300MHz, DMSO d⁶) 11.10 (1H, br) ), 9.24 (1H, d, J 8.8Hz), 8.98 (2H, s), 7.81 (2H, d, J 8.5Hz), 7.47-7.67 (5H, c), 7.31 (2H, dd, J 8.4, 0.9Hz), 6.93 (1H, d, J 8.1 Hz), 5.20 (1H, br), 3.59 (1H, dd, J 9.3, 6.0Hz), 3.40 (1H, dd, J 14.0, 9.3Hz), 1.60-1.97 (10H, m). m/z (ESI, 70V) MH⁺ 644.

### EXAMPLE 85

### 2-(2-Bromo-3-oxo-spiro[3.5]non-1-en-1-ylamino)-3-{4-[(3,5-dichloroisonicotinoyl)amino]phenyl}propanoic acid pyridin-4-ylmethyl ester

Using a similar procedure to that for the preparation the compound of Example 79 from the compound of Example 6 (0.60g, 1.1mmol), EDC (222mg, 1.1eq), HOBT (162mg, 1.1eq), 4-pyridinemethanol (0.36g, 3.2mmol) and DMF (5ml)] was prepared the title compound (0.48g, 66%). δH NMR (400MHz, DMSO d⁶, 380K) 10.43 (1H, br), 8.69 (2H, s), 8.57 (2H, d, J 6.0Hz), 8.38 (1H, d, J 8.1Hz), 7.57 (2H, m), 7.30 (4H, m), 5.26 (2H, s), 4.95 (1H, br), 3.32 (1H, dd, J 14.2, 5.4Hz), 3.17 (1H, dd, J 14.1, 9.1Hz), 1.46-1.71 (9H, m), 1.21 (1H, br m). m/z (ESI, 70V) MH⁺ 657.

### EXAMPLE 86

### Methyl (2S) 2-[(3-oxo-7-oxaspiro[3.5]non-1-en-yl)amino]-3-(2,6-dimethoxy[1.1'-biphenyl]-4-yl)propanoate

Using a similar procedure to that for the preparation the compound of Example 10 from methyl (2*S*)-2-amino-3-(2,6-dimethoxy[1,1'-biphenyl]-4-yl)propanoate (2.0g, 6.3mmol) and 7-oxaspiro[3.5]nonane-1,3-dione (1.1g, 1.1 eq) in DCM (30ml) was prepared the title compound as a white foam (2.4g, 86%). δH NMR (300MHz, DMSO d⁶) 8.53 (1H, d, J 8.7Hz), 7.20-7.30 (3H, m), 7.08 (2H, d, J 8.1 Hz), 6.70 (2H, d, J 8.5Hz), 4.44 (1H, s), 4.30 (1H, m), 3.72 (3H, s), 3.61 (6H, s), 3.22 (1H, dd, J 13.7, 5.0Hz), 2.98 (1H, dd, J 13.8, 10.1 Hz), 1.74-1.99 (2H, m), 1.48 (1H, d, J 13.6Hz), 1.31 (1H, d, J 13.0Hz). m/z (ESI, 70V) MH⁺ 439.

### EXAMPLE 87

### Methyl (2S) 2-{[2-(isopropylsulfanyl)-3-oxo-7-oxaspiro[3.5]non-1-en-yl)]amino}-3-(2,6-dimethoxy[1,1'-biphenyl]-4-yl)propancate

The title compound was prepared in a similar manner to the compound of Reference Example 7 from the bromo derivative of the compound of Example 86 (0.5g, 1.1 mmol) as a white foam (0.42g, 73%). δH NMR (400MHz, DMSO d⁶) 8.91 (1H. d, J 9.4Hz), 7.29-7.21 (3H, m), 7.09 (2H, d, J 8.2Hz), 6.70 (2H,d, J 8.4Hz), 5.32 (1H, br), 3.74-3.80 (5H, m), 3.54-3.64 (8H, m), 3.28 (1H. dd, J 4.2, 16.4Hz), 2.97 (1H. dd, J 10.8, 13.6Hz), 2.76 (1H, sep, J 6.7Hz), 1.99 (1H, dt, J 11.5, 4.8Hz), 1.84 (1H, dt, J 4.8, 13.0Hz), 1.48 (1H, d, J 12.1 Hz), 1.25 (1H, d, J 12.2Hz), 1.05 (3H, d, J 6.7Hz), 1.02 (3H, d, J 6.7Hz). m/z (ESI, 70V) MH⁺ 526.

### EXAMPLE 88

### (2S) 2-{[2-(isopropylsulfanyl)-3-oxo-7-oxaspiro[3.5]non-1-en-yl) ]amino}-3-(2,6-dimethoxy[1,1'-biphenyl]-4-yl propanoic acid

Prepared from the compound of Example 87 (0.25g, 0.46mmol) in a similar manner to that of Reference Example 2 to give the title compound as a white powder (0.21 g, 89%). δH NMR (400MHz, DMSO d⁶) 8.80 (1H, d, J 9.5Hz), 7.21-7.28 (3H, m), 7.08 (2H, d, J 8.1 Hz), 6.70 (2H, d, J 8.4Hz), 5.25 (1H, m), 3.76 (2H, m), 3.59 (8H, m), 3.30 (1 H), 2.93 (1H, dd, J 10.9, 13.6Hz), 2.76 (1H, sep, J 6.8Hz), 1.99 (1H, m), 1.86 (1H, m), 1.46 (1H, d, J 13.1 Hz), 1.23 (1H, d, J 12.7Hz), 1.06 (3H, d, J 7.8Hz), 1.03 (3H, d, J 7.8Hz). m/z (ESI, 70V) MH⁺ 512.

### EXAMPLE 89

### Ethyl (2S)-2-[(2-Cyclohexyl-3-oxospiro[3.5]non-1-en-1-yl)amino]-3-{4-[(3,5-dichloroisonicotinoyl)amino]phenyl} propanoate

A mixture of the free amine of Intermediate 15 (1000mg, 2.61mmol), Intermediate 35 (611mg, 2.61 mmol) and nitromethane (1 0ml) were heated at reflux for 5 hours. The solvent was removed by evaporation under reduced pressure and the residue chromatographed (SiO₂, 3:2 hexane:EtOAc), to afford the title compound as a colorless oil (310mg, 20%). δH NMR (CDCl₃) 8.51 (2H, s), 7.80 (1H, br s), 7.52 (2H, d, J 8.5Hz), 7.07 (2H, d, J 8.5Hz), 5.15 (1H, d, J 7.6Hz), 4.32 (1H, m), 4.20 (2H, q, J 7.1 Hz), 3.07 (2H, d, J 5.6Hz), 1.75-1.40 (20H, m), 1.19 (3H, t, J 7.1Hz).

### EXAMPLE 90

### (2S) 2-[(2-Cyclohexyl-3-oxo-spiro[3.5]non-1-en-1-yl)amino]-3-{4-[(3,5 dichloroisonicotinoyl)amino]phenyl} propanoic acid

The compound of Example 89 was hydrolysed by the method of Reference Example 2, giving the title compound in 78% yield. δH NMR (d⁶ DMSO) 10.84 (1H, br s), 8.78 (2H, s), 7.60 (1H, s), 7.56 (2H, d, J 8.2Hz), 7.25 (2H, d, J 7.9Hz), 4.06 (1H, m), 3.12 (1H, dd, J 3.9, 13.6Hz), 2.92 (1H, dd, J 9.8, 13.4Hz), 1.80-1.00 (21H, m). m/z (ES⁺, 70V) 570.1 (MH⁺)

### EXAMPLE 91

### Ethyl (2S) 3-{4-[(3,5-Dichloroisonicotinoyl)amino]phenyl}-2-(2-methyl-3-oxo-spiro[3.5]non-1-en-1-ylamino) propanoate

Prepared in a similar manner to the compound of Example 89 from Intermediate 40 to give the title compound in 68% yield. δH NMR (d⁶ DMSO) 8.79 (2H, s), 7.93 (2H, d, J 8.9Hz), 7.58 (2H, d, J 8.4Hz), 7.29 (2H, d, J 8.4Hz), 4.33 (1H, m), 4.16 (2H, q, J 7.1Hz), 3.10 (1H, m), 2.99 ( 1H, m), 1.60 (10H, m), 1.37 (3H, s), 1.18 (3H, t, J 7.1Hz). m/z (ES⁺, 70V) 530.1 (MH⁺).

### EXAMPLE 92

### (2S) 3-{4-[(3,5-Dichloroisonocotinoyl)amino]phenyl}-2-(2-methyl-3-oxo-spiro[3.5]non-1-en-1-ylamino) propanoic acid

The compound of Example 91 was hydrolysed by the method of Reference Example 2 to afford the title compound in 86% yield. δH NMR (d⁶ DMSO) 8.82 (2H, s), 7.81 (1H, d, J 9.1 Hz), 7.57 (2H, d, J 8.4Hz), 7.30 (2H, d, J 8.4Hz), 4.28 (1H, m), 3.13 (1H, m), 2.96 (1H, m), 1.70 - 1.49 (8H, m), 1.38 (3H, s), 1.14 (2H, m). m/z (ES⁺, 70V) 501.9 (MH⁺).

### EXAMPLE 93

### Ethyl (2S) 3-{4-[(3,5-Dichloroisonicotinoyl)amino]phenyl}-2-(3-oxo-2-propyl-spiro[3.5]non-1-en-1-ylamino) propanoate

Prepared in a similar manner to the compound of Example 89 from Intermediate 41 to give the title compound in 76% yield. δH NMR (CDCl₃) 8.58 (2H, s), 8.44 (1H, s), 7.65 (2H, d, J 8.5Hz), 7.16 (2H, d, J 8.4Hz), 5.38 (1H, m), 4.46 (1H, m), 4.30 (2H, q, J 7.1Hz), 3.17 (2H, m), 1.85 - 1.42 (14H, m), 1.38 (3H, t, J 7.1Hz), 0.90 (3H, t, J 7.3Hz). m/z (ES⁺, 70V) 558.1 (MH⁺).

### EXAMPLE 94

### (2S) 3-{4-[(3,5-Dichloroisonicotinoyl)amino]phenyl}-2-(3-oxo-2-propyl-spiro[3.5]non-1-en-1-ylamino) propanoic acid

The compound of Example 93 was hydrolysed by the method of Reference Example 2 to afford the title compound in 84% yield. δH NMR (d⁶ DMSO, 380K) 10.42 (1H, br s), 8.69 (2H, s), 7.57 (2H, d, J 7.3Hz), 7.28 (d, 2H, J 8.3Hz), 7.12 (1H, d, J 9.1 Hz), 4.21 (1H, m), 3.20 (1H, dd, J 4.9, 14.0Hz), 3.06 (1H, dd, J 8.8, 14.0Hz), 1.87 (2H, m), 1.72 - 1.45 (10H, m), 1.36 (2H, m), 1.23 (1H, m), 0.82 (3H, t, J 7.4Hz). m/z (ES⁺, 70V) 530.1 (MH⁺).

### EXAMPLE 95

### Ethyl (2S) 3-{4-[(3,5-Dichloroisonicotinoyl)amino]phenyl}-2-(2-methyl-3-oxo-7-oxa-spiro[3.5]non-1-en-1-ylamino) propanoate

Prepared in a similar manner to the compound of Example 89 from Intermediate 44 to give the title compound in 71% yield. δH NMR (d⁶ DMSO) 8.80 (2H, s), 8.11 (1H, d, J 9.1Hz), 7.59 (2H, d, J 8.4Hz), 7.30 (2H, d, J 8.4Hz), 4.38 (1H, m), 4.17 (2H, q, J 7.1Hz), 3.75 (2H, m), 3.60 (2H, m), 3.15 (1H, dd, J 5.1, 13.7Hz), 2.99 (1H, dd, J 9.2, 13.6Hz), 1.90 (2H, m), 1.39 (3H, s), 1.19 (3H, t, J 7.1 Hz). m/z (ES⁺, 70V) 532.0 (MH⁺).

### EXAMPLE 96

### (2S)3-{4-[(3,5-Dichloroisonicotinoyl)amino]phenyl}-2-(2-methyl-3-oxo-7-oxa-spiro[3.5]non-1-en-1-ylamino) propanoic acid

The compound of Example 95 was hydrolysed by the method of Reference Example 2 to afford the title compound in 89% yield. δH NMR (d⁶ DMSO) 8.81 (2H, s), 8.05 (1H, d, J 9.2Hz), 7.60 (2H, d, J 8.4Hz), 7.32 (2H, d, J 8.4Hz), 4.25 (1H, m), 3.75 (2H, m), 3.58 (2H, m), 3.13 (1H, dd, J 4.6, 13.8Hz), 2.98 (1H, dd, J 9.5, 13.8Hz), 1.94 (2H, m), 1.40 (3H, s), 1.29 (2H, m). m/z (ES⁺, 70V) 504.0 (MH⁺).

### EXAMPLE 97

### Ethyl (2S) 3-{4-[(3.5]-Dichloroisonicotinoyl)amino]phenyl}-2-(3-oxo-2-propyl-7-oxa-spiro[3.5]non-1-en-1-ylamino) propanoate

Prepared in a similar manner to the compound of Example 89 from Intermediate 45 to give the title compound in 59% yield. δH NMR (d⁶ DMSO) 8.78 (2H, s), 8.08 (1H, d, J 8.8Hz), 7.55 (2H, d, J 8.4Hz), 7.29 (2H, d, J 8.5Hz), 4.23 (1H, m), 4.16 (2H, q, J 7.1 Hz), 3.76 (2H, m), 3.58 (2H, m), 3.15 (1H, dd, J 4.8, 13.6Hz), 2.98 (1H, dd, J 9.7, 13.6Hz), 1.80 (2H, m), 1.18 (6H, m), 0.73 (3H, t, J 7.4Hz). m/z (ES⁺, 70V) 560.0 (MH⁺).

### EXAMPLE 98

### Ethyl (2S) 3-{4-[(3,5-Dichloroisonicotinoyl)amino]phenyl}-2-(3-oxo-2-propyl-7-oxa-spiro[3.5]non-1-en-1-ylamino) propanoate

The compound of Example 97 was hydrolysed by the method of Reference Example 2 to afford the title compound in 82% yield. δH NMR (d⁶ DMSO, 380K) 10.39 (1H, br s), 8.68 (2H, s), 7.57 (2H, m), 7.33 (3H, m), 4.23 (1H, m), 3.70 (4H, m), 3.21 (1H, dd, J 4.8, 14.0Hz), 3.04 (1H, dd, J 9.0, 14.0Hz), 2.10 (1H, s), 1.88 (4H, m), 1.87 (3H, t, J 7.0Hz), 1.40 (4H, m). m/z (ES⁺, 70V) 532.1 (MH⁺).

### EXAMPLE 99

### (2S) 2-(2-Bromo-3-oxo-spiro[3.5]non-1-en-1-ylamino)-3-{4-[(3,5-dichloroisonicotinoyl)amino]phenyl}-propanoic acid 2-imidazol-1-yl-ethyl ester

Prepared in a similar manner to the compound of Example 79 from N-2-hydroxyethylimidazole [prepared according to the method of Yoshino *et al,* J. C. S. Perkin Trans. 1, 1977, 1266-72] to give the title compound in 48% yield. δH NMR (d⁶ DMSO) 10.88 (1H, s), 8.88 (1H, d, J 9.1 Hz), 8.79 (2H, s), 7.66 (1H, s), 7.58 (2H, d, J 8.5Hz), 7.29 (2H, d, J 8.5Hz), 6.89 (1H, s), 4.84 (1H, m), 4.39 (2H, m), 4.29 (2H, m), 3.16 (1H, dd, J 4.6, 13.9Hz), 2.96 (1H, dd, J 9.7, 13.9Hz), 1.75-1.45 (8H, m), 1.35 (1H, m), 1.13 (1H, m). m/z (ES⁺, 70V) 662.1 (MH⁺).

### EXAMPLE 100

### 2-(2-Bromo-3-oxo-7-oxa-spiro[3.5]non-1-en-1-ylamino)-3-{4-[(3-methyl-[2,7]naphthyridine-1-yl)amino]phenyl}propanoic acid

Prepared by the methods as described herein in 77% yield. δH NMR (d⁶ DMSO) 8.77 (1H, s), 7.67 (1H. d, J 6.3Hz), 6.96 (2H, d, J 8.5Hz), 6.78 (1H, d, J 5.8Hz), 6.45 (2H, d, J 8.5Hz), 6.14 (1H, s), 4.19 (1H m), 3.05 - 2.85 (4H, m), 2.59 (1H, dd, J 4.4, 14.0Hz), 2.25 (1H. dd, J 9.7, 13.9Hz), 1.66 (3H, s), 1.28 (1H, m), 1.16 (1H, m), 0.83 (1H, d, J 13.5Hz), 0.66 (1H, d, J 13.5Hz). m/z (ES⁺, 70V) 537.9 (MH⁺).

### EXAMPLE 101

### Ethyl 3-{4-[(3,5-Dichloroisonicotinoyl)amino]phenyl}-2-(2-[1,3]dithian-2-yl-3-oxo-spiro[3.5]non-1-en-1-ylamino) propanoate

To a solution of the compound of Example 1 (1.5g, 2.9mmol) in DCM (100ml) was added 1,3-dithienium tetrafluorborate (3g, 14mmol) [prepared by the method of Paterson I; Price L.G. Tet. Lett.1981, 22 (29), 2829]. The mixture was stirred overnight and then partitioned between EtOAc (200ml) and sodium carbonate (100ml, sat. aq.), the organics were separated, washed with water (3 x 50ml), brine (50ml), dried (Na₂SO₄), filtered and concentrated *in vacuo* to give a crude product which was purified by column chromatography (SiO₂: 4:1, EtOAc: hexane) to give the title compound as a pale yellow solid (0.6g, 86%) δH NMR (400MHz, d6 DMSO, 300K) 08.67 (2H, s), 8.15 (1H, d, J 9.5Hz), 7.67 (2H, d, J 8.5Hz), 7.12 (2H, d, J 8.5Hz), 5.06 (1H, m), 4.65 (1H, s),1.10 (1H, m), 4.08 (2H, t, J 7.1Hz), 3.17-2.72 (3H, m), 2.65 (2H, m), 1.95 (1H, m), 1.87 (1H, m), 1.78-1.46 (11H, m), 1.25 (1H, d, J 12.3Hz). m/z (ESI, 70V) MH⁺ 634.

### EXAMPLE 102

### (2S) 3-{4-[(3,5-Dichloroisonicotinoyl)amino]phenyl}-2-(2-[1,3]dithian-2-yl-3-oxo-spiro[3.5]non-1-en-1-ylamino) propanoic acid

To a solution of the compound of Example 101 (0.25g, 0.4mmol) in THF (2ml) was added a solution of lithium hydroxide (25mg, 0.6mmol) in water (1ml). The mixture was stirred at room temperature overnight, concentrated *in vacuo* and the residue dissolved in the minimum amount of water. HCl (2M, aq.) was added until the pH of the solution was 4, the resulting solid was filtered, washed with ether and ethyl acetate to give the product as a pale yellow solid (0.15g, 63%). δH NMR (400MHz, d⁶ DMSO, 300K) □10.85 (1H, s), 8.78 (2H, s), 8.28 (1H, d, J 9.9Hz), 7.55 (2H, d, J 8.5Hz), 7.31 (2H, d, J 8.5Hz), 5.06 (1H, m), 4.75 (1H, s), 3.21-2.78 (3H, m), 2.67 (2H, m), 1.99 (1H, m), 1.75 (1H, m), 1.57 (8H, m), 1.22 (1H, d, J 11.9Hz), 1.08 (1H, m). m/z (ESI, 70V) MH⁺ 606.

### EXAMPLE 103

### (2S)-2-[2-(Methylsulfanyl)-3-oxospiro[3.5]non-1-en-1-yl]amino-3-[4-([2,7]naphthyridin-1-ylamino)phenyl] propanoic acid

The ethyl ester of the title compound was prepared in two steps from the free amine of Intermediate 6 (1.40g, 0.41 mmol), spiro[3.5]nonane-1,3-dione (650mg, 0.42mmol) [prepared according to the method of Wasserman, H.H. *et al,* J. Org. Chem., 38, 1451-1455 (1973)] and a 1.0M solution of methyl sulfenyl chloride in DCM (0.5ml, 0.5mmol) according to the method of Example 62 (1.53g, 0.30mmol, 71%). This ester was then subjected to hydrolysis according to the method of Reference Example 2 to give the title compound as a yellow powder (1.15g, 0.23mmol, 57%). δH (DMSO d⁶, 390K) 9.80 (1H, s), 8.61 (1H, d, J 5.6Hz), 8.13 (1H, d, J 5.4Hz), 7.67 (2H, d, J 8.3Hz), 7.60 (1H, d, J 5.5Hz), 7.18 (1H, d, J 8.3Hz), 7.05 (1H, d, J 5.5Hz), 4.37 (1H, m), 3.22-3.12 (2H, m), 2.14 (3H, s), 1.85-1.10 (10H, m). m/z (ES⁺, 70V) 489.1 (MH⁺).

### EXAMPLE 104

### Ethyl-(2S)-2-[2-(methylsulfanyl)-3-oxo-7-oxaspiro[3.5]non-1-en-1-yl]amino-3-[4-([2,7]naphthyridin-1-ylamino)phenyl]propanoate

Was prepared in two steps from the free amine of Intermediate 12 (1.40g, 0.41mmol), 7-oxaspiro[3.5]nonane-1,3-dione (650mg, 0.42mmol) [prepared according to the method of Wasserman, H.H. *et al,* J. Org. Chem., 38, 1451-1455 (1973)] and a 1.0M solution of methyl sulfenyl chloride in DCM (0.5ml, 0.5mmol) according to the method of Example 62 to give the title compound as a yellow powder (1.21g, 0.23mmol, 55%). δH (DMSO d⁶, 390K) 9.82 (1H, s), 9.55 (1H, s), 8.93 (1H, d, J 9.2Hz), 8.65 (1H, d, J 5.6Hz), 8.15 (1H, d, J 5.7Hz), 7.78 (2H, d, J 8.5Hz), 7.68 (1H, d, J 5.6Hz), 7.23 (2H, d, J 8.5Hz), 7.13 (1H, d, J 5.7Hz), 5.16-5.10 (1H, m), 4.20 (2H, q, J 7.1Hz), 3.77 (2H, m), 3.59-3.52 (2H, m), 3.21 (1H, dd, J 4.5Hz 13.8Hz), 2.98 (1H, dd, J 10.2Hz 13.8Hz), 1.96 (3H, s), 1.48-1.32 (4H, m), 1.23 (3H, t, J 7.1 Hz). m/z (ES⁺, 70V) 519.1 (MH⁺).

### EXAMPLE 105

### (2S)-2-[2-(Methylsulfanyl)-3-oxo-7-oxaspiro[3.5]non-1-en-1-yl]amino-3-[4-([2,7]naphthyridin-1-ylamino)phenyl]propanoic acid

Hydrolysis of the compound of Example 104 (650mg, 0.12mmol) with lithium hydroxide (30mg, 0.7mmol), according to the method of Reference Example 2, gave the title compound as a pale yellow powder (501 mg, 0.10mmol, 85%). δH (DMSO d⁶, 390K) 9.83 (1H, s), 9.54 (1H, s), 8.70 (1H, d, J 8.9Hz), 8.65 (1H, d, J 5.6Hz), 8.14 (1H, d, J 5.7Hz), 7.75 (2H, d, J 8.5Hz), 7.68 (1H, d, J 5.6Hz), 7.21 (2H, d, J 8.5Hz), 7.11 (1H, d, J 5.7Hz), 4.92 (1H, m), 3.76-3.73 (2H, m), 3.62-3.54 (2H, m), 3.23 (1H, d, J 3.9Hz 13.6Hz), 2.94 (1H, dd, J 9.9Hz, 13.6Hz), 1.91 (3H, s), 1.47-1.28 (4H, m). m/z (ES⁺. 70V) 491.1 (MH⁺).

### EXAMPLE 106

### N⁴-(4-[3-(2-Bromo-3-oxospiro[3.5]non-1-en-1-yl)-5-oxo-1.3-oxazolan-4-yl]methylphenyl)-3,5-dichloroisonicotinamide

A solution containing the compound of Example 6 (1.0g, 1.76mmol), finely ground potassium carbonate (500mg) and DMAP (50mg, 0.4mmol), in DMF (14ml) was treated dropwise with chloromethyl pivalate (0.5ml) at room temperature. After 24h the reaction was diluted with EtOAc (150ml), washed with brine (3x50ml), dried (MgSO₄) and concentrated *in vacuo.* Chromatography (SiO₂, 1:1 EtOAc:hexanes) gave the title compound as a white powder (475mg, 0.82mmol, 47%). δH (DMSO d⁶, 390K) 10.97 (1H, s), 8.80 (2H, s), 7.63 (2H, d, J 8.5Hz), 7.17 (2H, d, J 8.5Hz), 5.41 (1H, d, J 3.9Hz), 4.95 (1H, m), 4.69 (1H, d, J 3.9Hz), 3.39-3.29 (2H, m), 1.99-1.06 (10H, m). m/z (ES⁺, 70V) 580.9 (MH⁺).

### EXAMPLE 107

### 3-[2-(Isopropylsulfanyl)-3-oxospiro[3.5]non-1-en-1-yl]-4-[4-([2,7]naphthyridin-1-ylamino)benzyl]-1,3-oxazolan-5-one

Prepared from the compound of Example 70 (450mg, 0.88mmol) in a similar manner to the compound of Example 106 to give the title compound as an off-white powder (390mg, 0.73mmol, 84%). δH (DMSO d⁶, 390K) 9.57 (1H, s), 8.64 (1H, d, J 5.7Hz), 8.24 (1H, d, J 5.8Hz), 8.17 (1H, s), 7.74 (2H, d, J 8.4Hz), 7.53 (1H, d, J 5.6Hz), 7.16 (2H, d, J 8.4Hz), 7.06 (1H, d, J 5.8Hz), 5.20 (1H, br s), 4.93 (1H, br s), 4.26 (1H, br s), 3.58 (1H, br s), 3.33 (1H, br s), 3.27 (1H, m), 1.99-1.06 16H, m). m/z (ES⁺, 70V) 529.2 (MH⁺).

### EXAMPLE 108

### Neopentyl (2S)-2-[(2-bromo-3-oxospiro[3.5]non-1-en-1-yl)amino]-3{4-[(3,5-dichloroisonicotinoyl)amino]phenyl}propanoate

Using a similar procedure to that for the preparation the compound of Example 79 from the compound of Example 6 (0.5g, 0.89mmol), EDC (191mg, 1.0mmol), HOBT (120mg, 0.89mmol), neopentyl alcohol (0.4g, 4.5mmol) and DMF (15ml) was prepared, after purification by chromatography (SiO₂, EtOAc), the title compound as a white powder (400mg, 0.63mmol, 71%). δH (DMSO d⁶, 390K) 10.87 (1H, s), 8.92 (1H, d, J 9.1 Hz), 8.79 (2H, s), 7.58 (2H, d, J 8.5Hz), 7.32 (2H, d, J 8.5Hz), 4.88-4.82 (1H, m), 3.86 (1H, d, J 10.4Hz), 3.80 (1H, d, J 10.4Hz), 3.26 (1H, dd, J 13.9, 4.8Hz), 3.03 (1H, dd, J 13.9, 10.1Hz), 1.99-1.06 (10H, m), 0.91 (9H, br s). m/z (ES⁺, 70V) 638.0 (MH⁺).

### EXAMPLE 109

### Isopropyl (2S) 3-{4-[(3,5-dichloroisonicotinoyl)amino]phenyl}-2-(2-methyl-3-oxo-7-oxa-spiro[3.5]non-1-en-1-ylamino)propanoate

Prepared in a similar manner to the compound of Example 79 from the compound of Example 96 to give the title compound in 79% yield. δH (DMSO d⁶) 10.87 (1H, s), 8.80 (2H, s), 8.10 (1H, d, J 8.9Hz), 7.59 (d, 2H, J 8.2Hz), 7.31 (d, 2H, J 8.2Hz), 4.98 (1H, m), 4.30 (1H, m), 3.76 (2H, m), 3.60 (2H, m), 3.11 (1H, dd, J 13.7, 5.3Hz), 3.00 (1H, dd, J 13.2, 9.1Hz), 2.00-1.80 (2H, m), 1.42 (3H, s), 1.17 (6H, m). m/z (ES⁺, 70V) 546.1 (MH⁺).

### EXAMPLE 110

### 5-Methyl-2-oxo-[1,3]dioxol-4-ylmethyl (2S) 2-(2-bromo-3-oxo-spiro[3.5]non-1-en-1-ylamino)-3-{4-[(3,5-dichloroisonicotinoyl)amino]phenyl} propanoate

To a stirred solution of the compound of Example 6 (1.0g, 1.76 mmol) and potassium carbonate (484mg, 3.52mmol) in DMF (20ml) at 0°C was added Intermediate 46 (408mg, 2.12mmol) in one portion. The ice-bath was removed and the mixture allowed to stir at room temperature for 3 hours. The mixture was poured into ice/water and extracted with EtOAc. The extract was washed three times with brine, dried (MgSO₄) and the solvent removed *in vacuo* to afford a yellow solid. Chromatography (SiO₂, 1:1 hexane:EtOAc) gave the title compound as a white solid (686mg, 57%). δH (DMSO d⁶) 10.90 (1H, s), 8.95 (1H, d, J 8.9Hz), 8.80 (2H, s), 7.60 (2H, d, J 8.3Hz), 7.26 (2H, d, J 8.3Hz), 4.86 (1H, m), 3.22 (1H, m, J 4.0, 13.6Hz), 3.06 (1H, m, J 13.2, 10.7Hz), 2.17 (3H, s), 1.74-1.38 (10H, m). m/z (ES⁺, 70V) 680.0 (MH⁺).

### EXAMPLE 111

### 2,3-Dihydroxy-propyl (2S) 2-(2-bromo-3-oxo-spiro[3.5]non-1-en-1-ylamino)-3-{4-[(3,5-dichloroisonicotinoyl)amino]phenyl} propanoate

Prepared in a similar manner to the compound of Example 79 from the compound of Example 6 and glycerol to give the title compound in 48% yield after chromatography on silica gel. δH (DMSO d⁶) 10.91 (1H, s), 8.91 (1H, d, J 9.2Hz), 8.80 (2H, s), 7.60 (2H, d, J 8.5Hz), 7.28 (2H, d, J 8.3Hz), 5.01 (1H, m), 4.85 (1H, m), 4.71 (1H, m), 4.20 (1H, m), 4.09 (1H, m), 3.71 (1H, m), 3.57 (1H, m), 3.26 (1H, dd, J 13.8, 3.9Hz), 3.04 (1H, dd, J 13.8, 9.3Hz), 1.80-1.45 (10H, m). m/z (ES⁺, 70V) 640.0 (MH⁺).

### EXAMPLE 112

### Tetrahydro-furan-3-ylmethyl (2S)-2-(2-bromo-3-oxo-spiro[3.5]non-1-en-1-ylamino)-3-{4-[(3.5-dichloroisonicotinoyl)amino]phenyl} propanoate

Prepared in a similar manner to the compound of Example 79 from the compound of Example 6 and tetrahydrofurfuryl alcohol to give the title compound in 52% yield after chromatography on silica gel. δH (DMSO d⁶) 10.88 (1H, s), 8.93 (1H, d, J 9.1Hz), 8.80 (2H, s), 7.60 (2H, d, J 8.3Hz), 7.28 (2H, d, J 6.9Hz), 4.84 (1H, m), 4.15 (2H, m), 4.05 (1H, m), 3.23 (1H, dd, J 13.8, 4.4Hz), 3.04 (1H, dd, J 13.6, 9.6,Hz), 2.00-1.50 (14H, m). m/z (ES⁺, 70V) 650.1(MH⁺).

### EXAMPLE 113

### Tetrahydropyran-4-yl (2S)-2-(2-bromo-3-oxo-spiro[3.5]non-1-en-1-ylamino)-3-{4-[(3,5-dichloroisonicotinoyl)amino]-phenyl} propanoate

Prepared in a similar manner to the compound of Example 79 from the compound of Example 6 (0.5g, 0.89mmol), EDC (300mg), HOBT (200mg) and 4-hydroxytetrahydropyran (0.8ml) in DMF (5ml) to give the title compound (0.42g, 74%). δH (300MHz, DMSO d⁶) 11.02 (1H, br), 9.04 (1H, d, J 9.0Hz), 8.92 (2H, s), 7.73 (2H, d, J 8.4Hz), 7.42 (2H, d, J 8.5Hz), 5.13 (1H, br), 4.93 (1H, br), 3.87 (2H, br), 3.60 (2H, br), 3.36 (1H, dd, J 14.0, 5.1Hz), 3.18 (1H, dd, J 13.9, 9.3Hz), 1.61-2.06 (12H, m), 1.52 (2H, d, J 12.6Hz), 1.26 (2H, br). *m*/*z* (ES⁺, 70V) 652 (MH⁺).

### Example 114

### Isopropyl (2S)-2-(2-bromo-3-oxo-7-oxa-spiro[3.5]non-1-en-1-ylamino)-3-{4-[(3,5-dichloroisonicotinoyl)amino]phenyl} propanoate

To a solution of the isopropyl ester of the compound of Example 12 (0.4g, 8.1 mmol) [prepared in a similar manner to the compound of Example 138] in THF (5ml) at room temperature was added NBS (0.3g). The mixture was stirred for 2h and then partitioned between water (100ml) and EtOAc (100ml), the organics were separated and washed with water (3x50ml), brine (50ml), dried (Na₂SO₄), filtered and concentrated *in vacuo* to give a crude oil. Purification by column chromatography (3:2, hexane:EtOAc) gave the title compound as a white solid (0.24g, 52%). δH (400MHz, DMSO d⁶) 9.02 (1H, d, J 9.0Hz), 8.79 (2H, s), 7.59 (2H, d, J 8.5Hz), 7.27 (2H, d, J 8.5Hz), 4.97 (1H, m), 4.75 (1H, m), 3.80 (2H, m), 3.58 (2H, q, J 11.7Hz), 3.20 (1H, dd, J 13.0, 5.0Hz), 3.03 (1H, dd, J 13.0, 9.4,Hz), 1.97 (2H, m), 1.49 (1H, dd, J 13.0, 1.6Hz), 1.33 (1H, dd, J 13.2, 1.6Hz), 1.23 (3H, d, J 11.3Hz), 1.19 (3H, d, J 11.4Hz). *m*/*z* (ES⁺, 70V) MH⁺ 612.

### EXAMPLE 115

### Ethyl (2S)-3-{4-[(3,5-dichloro-1-oxy-pyridine-4-carbonyl)amino]phenyl}-2-(3-oxo-spiro[3.5]non-1-en-1-ylamino) propanoate

Intermediate 48 (170mg, 0.42 mmol) and 1-keto-3-hydroxy[3,5]-non-2-ene (200mg, 1.3mmol) were stirred together at room temperature overnight in THF (5ml) The reaction mixture was diluted with DCM (50ml), washed with sodium bicarbonate solution (saturated, 2x50ml), dried (MgSO₄) and reduced *in vacuo* to give a yellow solid. The residue was chromatographed (SiO₂ DCM:methanol, 98:2) to give the title compound as a white powder (120mg). δH (CDCl₃) 9.03 (1H, br s), 8.11 (2H, s), 7.64 (2H, d, J 7.9Hz) 7.18 (2H, d, J 7.5Hz), 5.01 (1H, m), 4.22 (1H, m), 4.21 (2H, q, J 7.1Hz), 3.12 (2H, m), 1.45 (10H, m), 1.30 (3H, t, J 7.1Hz). *m*/*z* (ES⁺, 70V) 532.0 (MH⁺).

### Example 116

### (2S)-3-{4-[(3,5-Dichloro-1-oxy-pyridine-4-carbonyl)amino]phenyl}-2-(3-oxo-spiro[3.5]non-1-en-1-ylamino) propanoic acid

The compound of Example 115 (30mg, 0.056mmol) was hydrolysed by the method of Reference Example 2 to afford the title compound as white powder (20mg). δH (CD₃OD) 8.46 (2H, s), 7.47 (2H, d, J 8.5Hz), 7.18 (2H, d, J 8.5Hz), 4.14 (1H, m), 3.21 (1H, m), (obscured by MeOH/water) 2.83 (1H, dd, J 9.6, 4.2Hz), 1.80-1.10 (9H, m), 1.07 (3H, t, J 7.1 Hz). *m*/*z* (ES⁺, 70V) 505.0 (MH⁺).

### EXAMPLE 117

### Ethyl (2S)-2-(2-bromo-3-oxo-spiro[3.5]non-1-en-1-ylamino)-3-{4-[(3.5-dichloro-1-oxy-pyridine-4-carbonyl)amino]phenyl} propanoate

Prepared from the compound of Example 115 (85mg, 0.160 mmol) in a similar manner to the compound of Example 5 to give the title compound (80mg). δH (CDCl3) 8.94 (1H, br s), 8.14 (2H, s), 7.62 (2H, d, J 8.4Hz), 7.13 (2H, d, J 8.3Hz), 5.88 (1H, m), 5.00 (1H, m), 4.26 (2H, q, J 7.1Hz), 3.26 (2H, m), 2.03-1.41 (10H, m), 1.35 (3H, t, J 7.1Hz). *m*/*z* (ES⁺, 70V) 612.0 (MH⁺).

### EXAMPLE 118

### (2S)-2-(2-Bromo-3-oxo-spiro[3.5]non-1-en-1-ylamino)-3-{4-[(3.5-dichloro-1-oxy-pyridine-4-carbonyl)amino]phenyl} propanoic acid

The compound of Example 117 was hydrolysed by the method of Reference Example 2 to afford the title compound. δH (DMSO d⁶) 10.80 (1H, s) 8.73 (2H, s), 7.55 (2H, d, J 8.0Hz), 7.24 (2H, d, J 8.4Hz), 4.65 (1H, m), 3.22 (1H, dd, J 13.8, 4.4Hz,), 3.00 (1H, dd, J 13.7, 4.4Hz), 1.82-1.00 (11H, m).

### EXAMPLE 119

### Ethyl (2S)-2-(2-chloro-3-oxo-spiro[3.5]non-1-en-1-ylamino)-3-{4-[(3,5-dichloro-1-oxy-pyridine-4-carbonyl)amino]phenyl} propanoate

Prepared in a similar manner to the compound of Example 29 from the compound of Example 115 (500mg, 0.94 mmol) and N-chlorosuccinimide (150mg, 1.13mmol) to give the title compound as a white powder (220mg). δH 10.85 (1H,s), 8.84 (1H, d, J 9.0Hz), 8.75 (2H, s), 7.58 (2H, d, J 8.5Hz) 7.27 (2H, d, J 8.5Hz), 4.68 (1H, m) 4.20 (2H, q, J 7.0Hz) 3.22 (1H, dd, J 13.8, 4.7Hz) 3.01 (1H, dd, J 13.7, 9.7Hz) 1.74-1.55 (9H, m) 1.38 (1H, m) 1.23 (3H, m, J 7.1 Hz) 1.13 (1H, m). *m*/*z*(ES⁺, 70V) 568.0 (MH⁺).

### EXAMPLE 120

### (2S)-2-(2-Chloro-3-oxo-spiro[3.5]non-1-en-1-ylamino)-3-{4-[(3,5-dichloro-1-oxy-pyridine-4-carbonyl)amino]phenyl}propanolc acid

The compound of Example 119 was hydrolysed by the method of Reference Example 2 to afford the title compound. δH (DMSO d⁶) 10.83 (1H, s), 8.75 (m, 3H), 7.57 (2H, d, J 8.4Hz) 7.26 (2H, d, J 8.5Hz), 4.63 (1H, m) 3.22 (1H, dd, J 13.8, 4.5Hz) 3.00 (1H, m), 1.64-1.55 (9H, m) 1.35 (1H, m), 1.15 (1H, m). *m*/*z* (ES⁺, 70V) 538.0 (MH⁺).

### EXAMPLE 121

### Ethyl (2S)-3-[4-(2,6-Dichlorobenzoylamino)phenyl]-2-(3-oxo-spiro[3.5]non-1-en-1-ylamino) propanoate

The title compound was prepared in a similar manner to that of the compound of Example 1 (coupling of amino acid ethyl ester (1.68mmol), dione (1.68mmol) in DCM (5ml)) to give the title compound as a yellow powder (1.1 mmol, 66%). δH (CD₃OD) 7.83 (2H,d, J 8.3Hz), 7.55-7.35 (4H, m), 7.47 (2H, d, J 8.4Hz), 4.62 (1H, s), 4.50 (3H, m), 3.50 (1H, m), 3.08 (1H, m) 2.05-1.55 (11H, m) 1.49 (3H, t, J 7.1Hz). *m*/*z* (ES⁺, 70V) 501.0 (MH⁺).

### EXAMPLE 122

### Ethyl (2S)-2-(2-bromo-3-oxo-spiro[3.5]non-1-en-1-ylamino)-3-[4-(2,6-dichlorobenzoylamino)phenyl] propanoate

Prepared in a similar manner to the compound of Example 36 from the compound of Example 121 (1.08mmol) to give the title compound as yellow powder (0.86mmol, 80%). δH (CD₃0D) 7.53 (2H, d, J 8.4Hz), 7.38-7.15 (4H, m), 7.17 (2H, d, J 8.4Hz), 5.32 (1H, m) 4.65 (1H, m), 3.22 (1H, dd, J 13.9, 4.4Hz), 3.18 (q, 2H, J 7.1 Hz) 2.95 (1H, dd J 13.9, 9.5Hz,),1.85-1.20 (14H, m). ). *m*/*z* (ES⁺, 70V) 581.0 (MH).

### EXAMPLE 123

### (2S)-2-(2-Bromo-3-oxo-spiro[3.5]non-1-en-1-ylamino)-3-[4-(2,6-dichlorobenzoylamino)-phenyl] propanoic acid

Prepared in a similar manner to the compound of Reference Example 2 from the compound of Example 122 (0.85mmol) to give the title compound as white powder (0.60mmol, 60%). δH (DMSO d⁶) 13.39 (1H, br s), 10.70 (1H, d, J 6.1 Hz), 8.81 (1H, d, J 9.2Hz), 7.61 (3H, m), 7.56 (1H, m), 7.23 (2H, d, J 8.2Hz), 3.18 (1H, dd, J 13.9, 4.4Hz), 2.98(1H, dd J 13.8, 9.6Hz), 2.89-1.20 (11H, m). *m*/*z* (ES⁺, 70V) 567.0 (MH⁺).

### EXAMPLE 124

### Isopropyl (2S)-2-(2-bromo-3-oxo-spiro[3.5]non-1-en-1-ylamino)-3-[4-(2,6-dichlorobenzoylamino)phenyl] propanoate

Prepared in a similar manner to the compound of Example 79 from the compound of Example 123 (0.78 mmol) to give the title compound (0.49mmol, 63%). δH (DMSO d⁶) 10.71 (1H, s), 8.89(1H, d, J 9.0Hz), 7.62-7.47 (5H, cm), 4.97,(1H,m), 3.17 (1H, dd, J 4.8Hz, 13.8Hz), 3.00 (1H, dd J 9.7Hz, 13.8Hz), 1.79-1.50 (8H, c m) 1.35 (1H,m) 1.24-1.11 (7H,m). *m*/*z* (ES⁺, 70V) 609.0 (MH⁺).

### EXAMPLE 125

### Ethyl (2S)-3-[4-(3-methyl-[2,7]naphthyridin-1-ylamino)phenyl]-2-(3-oxo-spiro[3.5]non-1-en-1-ylamino) propanoate

Prepared in a similar manner to that of the compound of Reference Example 3 to give the title compound as a yellow powder (1.5mmol, 76%). δH (CDCl3) 9.56 (1H, s), 8.53 (1H, d, J 5.8Hz), 7.78 (2H, d, J 8.4Hz), 7.45 (1H, d, J 5.8Hz), 7.08 (2H, d, J 8.5Hz), 6.89 (1H, s), 5.77 (1H, m), 4.57 (1H, s), 4.27 (2H, q, J 7.1 Hz), 3.10 (2H, m), 2.54 (3H, s), 1.84-1.23 (14H, m). *m*/*z* (ES⁺, 70V) 485.2 (MH⁺).

### EXAMPLE 126

### Ethyl (2S)-2-(2-bromo-3-oxo-spiro[3.5]non-1-en-1-ylamino)-3-[4-(3-methyl-[2,7]naphthyridin-1-ylamino)phenyl] propanoate

Prepared in a similar manner to the compound of Example 5 from the compound of Example 125 (0.62mmol), bromine (0.81 mmol) and triethylamine (0.81mmol) in DCM (5ml)) to give the title compound as yellow powder (0.25mmol, 40%). δH (CD₃OD) 9.44 (1H, s), 8.34 (1H, d, J 5.8Hz), 7.43 (1H, d, J 5.8Hz), 7.09 (2H, d, J 8.5 Hz), 6.81 (1H, s), 4.87 (1H, m), 4.89 (1H, m), 4.13 (2H, q, J 7.1 Hz), 3.22 (1H, m), (obscured mostly by MeOH), 2.92 (1H, dd, J 14.0, 9.7Hz), 2.34 (3H, s), 1.58-1.26 (10H, m), 1.18 (3H, t, J 7.1 Hz). *m*/*z* (ES⁺, 70V) 564.2 (MH⁺).

### EXAMPLE 127

### Ethyl (2S)-2-(2-bromo-3-oxo-spiro[3.5]non-1-en-1-ylamino)-3-[4-(3-methyl-[2,7]naphthyridin-1-ylamino)phenyl]propanoate

Prepared in a similar manner to to the compound of Reference Example 2 from the compound of Example 124 (0.22mmol) to give the title compound as yellow powder (0.20mmol, 90%). δH (DMSO d⁶) 9.76 (1H, s), 9.70 (1H, s), 8.87 (1H, s, J 9.5Hz), 8.56 (1H, d, J 5.6Hz), 7.87 (2H, d, J 8.4Hz), 7.56 (1H, d, J 5.6Hz), 7.20 (2H, d, J 8.4Hz), 6.96 (1H, s), 4.73 (1H, m), 3.22 (1H, dd, J 13.9, 4.0Hz), 2.93 (1H, dd J 13.5, 10.1 Hz,), 2.42 (3H, s), 1.80-1.00 (11H, m). *m*/*z* (ES⁺, 70V) 535.0 (MH⁺).

### EXAMPLE 128

### (2S)-3-[4-(3-Methyl-[2,7]naphthyridin-1-ylamino)phenyl]-2-(3-oxo-spiro[3.5]non-1-en-1-ylamino) propanoic acid

Prepared in a similar manner to the compound of Reference Example 2 from the compound of Example 125 (0.62mmol) to give the title compound as white powder (0.27mmol, 43%). δH (DMSO d⁶) 9.81 (1H,s), 9.52 (1H, s), 8.58 (1H, d, J 5.6Hz), 8.30 (1H, J 8.6Hz), 7.86 (2H, d, J 8.4Hz), 7.57(1H, d, J 5.6Hz), 7.22 (2H, d, J 8.5Hz), 6.97(1H, s), 4.08 (1H, m), 4.32 (1H, s), 3.15 (1H, dd, J 13.7, 4.7Hz), 2.97 (1H, dd, J 13.7, 9.5Hz), 2.44 (3H, s), 1.74-1.45 (9H, m), 1.24-1.15 (2H, m). *m*/*z* (ES⁺, 70V) 457.1 (MH⁺).

### EXAMPLE 129

### Ethyl (S)-3-[4-([2,7]naphthyridin-1-ylamino)phenyl]-2-(3-oxo-spiro[3.5]non-1-en-1-ylamino) propanoate

Prepared in a similar manner to the compound of Reference Example 3 to give the title compound as a yellow powder (1.4 mmol, 73%) δH (CDCl3) 9.61 (1H,s), 8.65 (1H, d, J 5.7Hz), 8.25 (1H, d, J 5.8Hz), 7.71 (2H, d, J 8.4Hz), 7.63 (1H, d, J 8.5Hz), 7.12 (2H, d, J 8.5Hz), 7.05 (1H, d, J 5.8Hz), 5.80 (1H, m), 4.55 (1H, s), 4.29 (2H, q, J 7.2Hz), 3.13 (2H, m), 1.87-1.25 (14H, m). *m*/*z* (ES⁺, 70V) 471.1 (MH⁺).

### EXAMPLE 130

### Ethyl (2S)-2-(2-bromo-3-oxo-spiro[3.5]non-1-en-1-ylamino)-3-[4-([2,7]naphthyridin-1-ylamino)-phenyl] propanoate

Prepared in a similar manner to that of Example 5 from the compound of Example 129 (0.64mmol) to give the title compound as a yellow powder (0.45mmol, 76%). δH (CDCl3) 9.81 (1H, s), 8.64 (1H, d, J 5.7Hz), 8.29 (1H, d, J 5.8Hz), 7.75 (2H, d, J 8.3Hz), 7.60 (1H, d, J 5.8Hz), 7.12 (2H, d, J 8.4Hz), 7.08 (1H, d, J 5.7Hz), 5.91 (1H. m), 5.03 (1H, m), 4.28 (2H, q, J 7.1 Hz), 3.29 (2H, m), 1.81-1.39 (10H, m), 1.35 (3H, t, J 7.1Hz). *m*/*z* (ES⁺, 70V) 550.0 (MH⁺).

### EXAMPLE 131

### (S)-2-(2-Bromo-3-oxo-spiro[3.5]non-1-en-1-ylamino)-3-[4-([2,7]naphthyridin-1-ylamino)-phenyl]-propionic acid

Prepared in a similar manner to the compound of Reference Example 2 from the compound of Example 130 (0.40mmol) to give the title compound as white powder (0.25mmol, 64%) δH (DMSO d⁶, 300 K) 9.90 (1H, s), 9.56 (1H, s), 8.86 (1H,d, J 9.3Hz), 8.66 (1H, d, J 5.6Hz), 8.17 (1H, d, J 5.7Hz), 7.81 (2H, d, J 8.2Hz), 7.70 (1H, d, J 5.6Hz), 7.24 (2H, d, J 8.4Hz), 7.14 (1H, d, J 5.7Hz), 4.78 (1H, m) 3.23 (1H, dd, J 13.9, 4.1 Hz), 2.99 (1H, dd, J 13.7, 10.0Hz), 1.81-1.04 (11H, m). *m*/*z* (ES⁺, 70V) 522.0 (MH⁺).

### EXAMPLE 132

### (2S)-3-[4-([2,7]Naphthyridin-1-ylamino)phenyl]-2-(3-oxo-spiro[3.5]non-1-en-1-ylamino) propanoic acid

Prepared in a similar manner to the compound of reference Example 2 from the compound of Example 128 (0.64mmol) to give the title compound as white powder (0.21 mmol, 33%). δH (DMSO d⁶, 300K) 9.85 (1H, s), 9.54 (1H, s), 8.67 (1H,d, J 5.6Hz), 8.28 (1H, d, J 8.6Hz), 8.18 (1H, d, J 5.6Hz), 7.78 (2H, d, J 8.3Hz), 7.70 (1H, d, J 5.6Hz), 7.23 (2H,d, J 8.4Hz), 7.14 (1H, d, J 5.7Hz), 4.34 (1H, s), 4.08 (1H, m), 3.15 (1H, dd, J 13.8, 4.8Hz), 2.95 (1H, dd, J 13.8, 9.4Hz), 1.74-1.39 (9H, m), 1.20 (2H, m). *m*/*z* (ES⁺, 70V) 443.1 (MH⁺).

### EXAMPLE 133

### (2S)-3-[4-([2,7]Naphthyridin-1-yloxy)phenyl]-2-(3-oxo-spiro[3.5]non-1-en-1-ylamino) propanoic acid

Prepared in a similar manner to the compound of Reference Example 2 (hydrolysis from the compound of Example 7 (0.70 mmol) to give the title compound as a white powder (0.56 mmol, 80%). δH (DMSO d⁶, 300K) 9.70 (1H, s), 8.81 (1H, d, J 5.7Hz), 8.30 (1H, d, J 8.8Hz), 8.10 (1H, d J 5.8Hz), 7.89 (1H, d, J 5.7Hz), 7.53 (1H, d, J 5.9Hz), 7.34 (1H, d, J 8.5Hz), 7.23 (2H, d, J 8.5Hz), 4.34 (1H, s), 4.15 (1H, m), 3.21 (1H, dd, J 14.0, 4.8Hz), 3.00 (1H, dd, J 13.8, 9.7Hz), 1.71-1.50 (11H, m). *m*/*z* (ES⁺, 70V) 444.6 (MH⁺).

### EXAMPLE 134

### Ethyl (2S)-3-{4-[(3,5-dichloroisonicotinoyl)amino]phenyl}-2-(2-hydroxy-3-oxo-spiro[3.5]non-1-en-1-ylamino)-propionic acid ethyl ester

To a solution of the compound of Example 1 (1.0g, 1.9mmol) in DCM (40ml) at -40°C was added lead tetraacetate (0.94g, 1.1 eq). The mixture was allowed to warm to 0°C and stirred at this temperature for 8h. The reaction mixture was partitioned between EtOAc (200ml) and water (100ml), the organics were separated washed with water (2x100ml), brine (50ml), dried (MgSO₄), filtered and concentrated *in vacuo* to give a crude oil. The crude was dissolved in ethanol (10ml) and treated with NaH (100mg). The mixture was stirred at room temperature until TLC analysis indicated that all starting material had been consumed. The reaction was quenched by the addition of NH₄Cl (5ml, sat. aq.), EtOAc (2x20ml) extraction of the mixture followed by washing with water (10ml), brine (10ml), drying (MgSO₄), filtering and concentration *in vacuo* to give a crude product which was purified by column chromatography (SiO₂, EtOAc:Hexane 1:1) to give the title compound as a white foam (0.89g, 86%). δH (DMSO d⁶, 400MHz) 10.83 (1H, br), 8.78 (2H, s), 7.51 (2H, d, J 8.5Hz), 7.12 (2H, d, J 8.5Hz), 4.94 (1H, dd, J 11.4, 5.0Hz), 4.10 (2H, m), 3.33 (1H, dd, J 14.1, 4.9Hz), 3.14 (1H, dd, J 14.0, 11.4Hz), 1.40-1.63 (4H, m), 1.19-1.33 (6H, m), 1.16 (3H, t, J 7.1Hz). *m*/*z* (ES⁺, 70V) 532 (MH⁺).

### EXAMPLE 135

### Ethyl (2S)-3-{4-[(3,5-Dichloroisonicotinoyl)amino]phenyl}-2-(2-methoxy-3-oxo-spiro[3.5]non-1-en-1-ylamino) propanoate

To a solution of the compound of Example 134 (0.8g, 1.5mmol) in acetone (15ml) was added K₂CO₃ (5g) and methyl iodide (2.5ml). The mixture was stirred at room temperature for 5 days. The mixture was filtered and concentrated *in vacuo* and the residue purified by column chromatography (SiO₂, EtOAc:Hexane 1:1) to give the title compound as a white solid (0.45g, 55%). δH (DMSO d⁶, 400MHz) 8.50 (2H, d, J 4.8Hz), 7.21 (2H, d, J 8.4Hz), 7.04 (2H, d, J 8.4Hz), 4.87 (1H, dd, J 11.8, 4.9Hz), 4.00-4.16 (2H, m), 3.34 (3H, s), 3.26 (1H, dd, J 13.9, 4.9Hz), 3.07 (1H, dd, J 13.9, 11.6Hz), 1.15-1.66 (10H, m), 1.12 (3H, t, J 7.0Hz). *m*/*z* (ES⁺, 70V) 546 (MH⁺).

### EXAMPLE 136

### Ethyl (2S)-2-(2-bromo-3-oxo-spiro[3.5]non-1-en-1-ylamino)-3-(2,4,6-trimethoxy[1,1'-biphenyl]-4-yl) propanoate

The title compound was prepared by the methods as described herein. δH (CDCl₃) 7.19 (2H, d, J 8.1 Hz), 7.04 (2H, d, J 8.1 Hz), 6.14 (2H, s), 5.84 (1H, d, J 8.6Hz), 4.98 (1H, m), 4.20 (2H, q, J 7.1Hz), 3.78 (3H, s), 3.62 (6H, s), 3.21 (2H, d), 1.97-1.40 (10H, m), 1.24 (3H, t, J 7.1 Hz). *m*/*z* (ES⁺, 70V) 572 (MH⁺).

### EXAMPLE 137

### (2S)-2-(2-Bromo-3-oxo-spiro[3.5]non-1-en-1-ylamino)-3-(2,4,6-trimethoxy[1,1'-biphenyl]-4-yl) propanoic acid

Prepared from the compound of Example 136 by the method of Reference Example 2 to give the title compound. δH (DMSO d⁶) 12.30 (1H, br s), 8.79 (2H, d, J 10.0Hz), 7.19 (2H, d, J 8.1Hz), 7.08 (2H, d, J 8.1Hz), 6.29 (2H, s), 4.78 (1H, m), 3.81 (3H, s), 3.61 (6H, s), 3.27 (1H, m), 2.98 (1H, dd, J 13.4, 10.2Hz), 1.95-1.00 (10H, m). *m*/*z* (ES⁺, 70V) 544 (MH⁺).

### EXAMPLE 138

### Tetrahydro-furan-2-ylmethyl (2S)-2-(2-Bromo-3-oxo-spiro[3.5]non-1-en-1-ylamino)-3-{4-[(3.5-dichloro-pyridine-4-carbonyl)amino]phenyl}propanoate

Using a similar procedure to that for the preparation of the compound of Example 79 form the compound of Example 6 (0.25g, 0.44mmol), EDC (150mg), HOBT (100mg) and tetrahydrofurfurylalcohol (0.5ml) in DMF (2ml) was prepared the title compound (0.15g, 52%). δH (400MHz, DMSO d⁶) 10.88 (1H, s), 8.93 (1H, d, J 9.1 Hz), 8.80 (2H, s), 7.60 (2H, d, J 8.3Hz), 7.28 (2H, d, J 6.9Hz), 4.84 (1H, m), 4.15 (2H, m), 4.05 (1H, m), 3.23 (1H, dd, J 13.8, 4.4Hz), 3.04 (1H, dd, J 13.6, 9.6Hz), 1.50-2.00 (14H, m). *m*/*z* (ES⁺, 70V) 650 (MH⁺).

The following assays can be used to demonstrate the potency and selectivity of the compounds according to the invention. In each of these assays an IC₅₀ value was determined for each test compound and represents the concentration of compound necessary to achieve 50% inhibition of cell adhesion where 100% = adhesion assessed in the absence of the test compound and 0% = absorbance in wells that did not receive cells.

### α₄β₁ Integrin-dependent Jurkat cell adhesion to VCAM-Ig

96 well NUNC plates were coated with F(ab)₂ fragment goat anti-human IgG Fcγ-specific antibody [Jackson Immuno Research 109-006-098: 100 µl at 2 µg/ml in 0.1M NaHCO₃ pH 8.4], overnight at 4°. The plates were washed (3x) in phosphate-buffered saline (PBS) and then blocked for 1h in PBS/1% BSA at room temperature on a rocking platform. After washing (3x in PBS) 9 ng/ml of purified 2d VCAM-lg diluted in PBS/1% BSA was added and the plates left for 60 minutes at room temperature on a rocking platform. The plates were washed (3x in PBS) and the assay then performed at 37º for 30 min in a total volume of 200 µl containing 2.5 x 105 Jurkat cells in the presence or absence of titrated test compounds.

Each plate was washed (2x) with medium and the adherent cells were fixed with 100µl methanol for 10 minutes followed by another wash. 100µl 0.25% Rose Bengal (Sigma R4507) in PBS was added for 5 minutes at room temperature and the plates washed (3x) in PBS. 100µl 50% (v/v) ethanol in PBS was added and the plates left for 60min after which the absorbance (570nm) was measured.

### α₄β₇ Integrin-dependent JY cell adhesion to MAdCAM-Ig

This assay was performed in the same manner as the α₄β₁ assay except that MAdCAM-Ig (150ng/ml) was used in place of 2d VCAM-Ig and a subline of the β-lympho blastoid cell-line JY was used in place of Jurkat cells. The IC₅₀ value for each test compound was determined as described in the α₄β₁ integrin assay.

### α₅β₁ Integrin-dependent K562 cell adhesion to fibronectin

96 well tissue culture plates were coated with human plasma fibronectin (Sigma F0895) at 5µg/ml in phosphate-buffered saline (PBS) for 2 hr at 37°C. The plates were washed (3x in PBS) and then blocked for 1 h in 100µl PBS/1% BSA at room temperature on a rocking platform. The blocked plates were washed (3x in PBS) and the assay then performed at 37°C in a total volume of 200µl containing 2.5x 10⁵ K562 cells, phorbol-12-myristate-13-acetate at 10ng/ml, and in the presence or absence of titrated test compounds. Incubation time was 30 minutes. Each plate was fixed and stained as described in the α₄β₁ assay above.

### αₘβ₂-dependent human polymorphonuclear neutrophils adhesion to plastic

96 well tissue culture plates were coated with RPMI 1640/10% FCS for 2h at 37°C. 2 x 10⁵ freshly isolated human venous polymorphonuclear neutrophils (PMN) were added to the wells in a total volume of 200µl in the presence of 10ng/ml phorbol-12-myristate-13-acetate, and in the presence or absence of test compounds, and incubated for 20min at 37ºC followed by 30min at room temperature. The plates were washed in medium and 100µl 0.1% (w/v) HMB (hexadecyl trimethyl ammonium bromide, Sigma H5882) in 0.05M potassium phosphate buffer, pH 6.0 added to each well. The plates were then left on a rocker at room temperature for 60 min. Endogenous peroxidase activity was then assessed using tetramethyl benzidine (TMB) as follows: PMN lysate samples mixed with 0.22% H₂O₂ (Sigma) and 50µg/ml TMB (Boehringer Mannheim) in 0.1M sodium acetate/citrate buffer, pH 6.0 and absorbance measured at 630nm.

### αIIb/β₃ -dependent human platelet aggregation

Human platelet aggregation was assessed using impedance aggregation on the Chronolog Whole Blood Lumiaggregometer. Human platelet-rich plasma (PRP) was obtained by spinning fresh human venous blood anticoagulated with 0.38% (v/v) tri-sodium citrate at 220xg for 10 min and diluted to a cell density of 6 x 10⁸/ml in autologous plasma. Cuvettes contained equal volumes of PRP and filtered Tyrode's buffer (g/liter: NaCl 8.0; MgCl₂.H₂O 0.427; CaCl₂ 0.2; KCI 0.2; D-glucose 1.0; NaHCO₃ 1.0; NaHPO₄.2H₂O 0.065). Aggregation was monitored following addition of 2.5µM ADP (Sigma) in the presence or absence of inhibitors.

In the above assays the preferred compounds of the invention such as the compounds of the Examples generally have IC₅₀ values in the α₄β₁ and assay of 1 µM and below and in the α₄β₇ assay of 5µM and below. In the other assays featuring α integrins of other subgroups the same compounds had IC₅₀ values of 50µM and above thus demonstrating the potency and selectivity of their action against α₄ integrins.

Additionally, compounds of the invention, such as the compounds of the Examples, possess advantageous absorption properties as determined by standard tests, which make the compounds particularly suitable for oral dosing.

## Claims

1. A compound of formula (1): 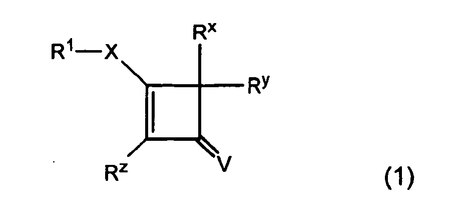 wherein
R¹ is a group Ar¹L²Ar²Alk- in which:
Ar¹ is an optionally substituted aromatic or heteroaromatic group; wherein the optional substituents are selected from one, two or three halogen atoms, and/or C₁₋₆alkyl, C₃₋₈cycloalkyl, C₁₋₆hydroxyalkyl, carboxyC₁₋₆alkyl, C₁₋₆alkylthio, carboxyC₁₋₆alkylthio, C₁₋₆alkoxy, hydroxyC₁₋₆alkoxy, haloC₁₋₆alkyl, haloC₁₋₆alkoxy, C₁₋₆alkylamino, -NH₂, aminoC₁₋₆alkyl, C₁₋₆dialkylamino, C₁₋₆alkylaminoC₁₋₆alkyl, C₁₋₆dialkylaminoC₁₋₆alkyl, aminoC₁₋₆alkoxy, C₁₋₆alkylaminoC₁₋₆alkoxy, C₁₋₆dialkylaminoC₁₋₆alkoxy, nitro, cyano, amidino, -OH, HC(O)-, -CO₂H, -CO₂R⁵, C₁₋₆alkanoyl, -SH, thioC₁₋₆alkyl, -SO₃H, -SO₃R⁵, C₁₋₆alkylsulphinyl, C₁₋₆alkylsulphonyl, -SO₂NH₂, C₁₋₆alkylaminosulphonyl, C₁₋₆dialkylaminosulphonyl, phenylamino-sulphonyl, -CONH₂, C₁₋₆alkylaminocarbonyl, C₁₋₆dialkylaminocarbonyl, aminoC₁₋₆alkylaminocarbonyl, C₁₋₆alkylaminoC₁₋₆alkylaminocarbonyl, C₁₋₆dialkylaminoC₁₋₆alkylaminocarbonyl, aminocarbonylamino, C₁₋₆alkylaminocarbonylamino, C₁₋₆dialkylamino-carbonylamino, C₁₋₆alkylaminocabonylC₁₋₆alkylamino, aminothiocarbonylamino, C₁₋₆alkylaminothiocarbonylamino, C₁₋₆dialkylaminothiocarbonylamino, C₁₋₆alkylaminothiocarbonylC₁₋₆alkylamino, C₁-₆alkylsulphonylamino, C₁₋₆dialkylsulphonylamino, -NHSO₂NH₂, C₁₋₆alkylaminosulphonylamino, C₁₋₆dialkylaminosulphonylamino, C₁₋₆alkanoylamino, aminoC₁₋₆alkanoylamino, C₁₋₆dialkylaminoC₁₋₆alkanoylamino, C₁₋₆alkanoyl-aminoC₁₋₆alkyl, C₁₋₆alkanoylaminoC₁₋₆alkylamino or C₁₋₆alkoxycarbonyl-amino groups;
R⁵ is a hydrogen atom, or a C₁₋₆alkyl or C₃₋₈cycloalkyl group;
L² is a covalent bond or a group L^{2a} or -(Alk³)L^{2a}, where Alk³ is an optionally substituted aliphatic or heteroaliphatic chain and L^{2a} is a -O- or -S- atom or a -C(O)-, -C(O)O-, -OC(O)-, -C(S)-, -S(O)-, -S(O)₂-, -N(R⁸)-[where R⁸ is a hydrogen atom or a straight or branched C₁₋₆alkyl group], -CON(R⁸)-, -OC(O)N(R⁸)-, -CSN(R⁸)-, -N(R⁸)CO-, -N(R⁸)C(O)O-, -N(R⁸)CS-, -S(O)₂N(R⁸)-, -N(R⁸)S(O)₂-, -N(R⁸)O-, -ON(R⁸)-, -N(R⁸)N(R⁸)-, -N(R⁸)CON(R⁸)-, -N(R⁸)CSN(R⁸)-, or -N(R⁸)SO₂N(R⁸)- group; optional substituents that may be present on Alk³ are selected from one, two or three substituents where each substituent may be the same or different and is selected from halogen atoms, or -OH, -CO₂H, -CO₂R⁹, where R⁹ is a straight or branched C₁₋₆alkyl group, -CONHR⁹, -CON(R⁹)₂, -COR⁹, C₁₋₆alkoxy, thiol, -S(O)R⁹, -S(O)₂R⁹, C₁₋₆alkylthio, amino, -NHR⁹ or -N(R⁹)₂ groups;
Ar² is an optionally substituted arylene or heteroarylene group; wherein the optional substituents which may be present on Ar² are selected from halogen atoms, or C₁₋₆alkyl, haloC₁₋₆alkyl, C₁₋₆alkoxy, haloC₁₋₆alkoxy, -CN, -CO₂CH₃, -NO₂, -NH₂, -NR⁵R⁶ and -N(R⁵)COCH₃ groups;
R⁶ is as defined for R⁵;
and Alk is a chain
-CH₂-CH(R)-, -CH=C(R)- or 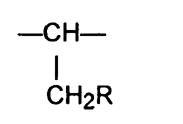 in which R is a carboxylic acid (-CO₂H) or a tetrazole, phosphonic acid, phosphinic acid, sulphonic acid, sulphinic acid, boronic acid or an acylsulphonamide biostere thereof or a -CO₂Alk⁷ or -CONR⁵R⁶ group; Alk⁷ is straight or branched C₁₋₈alkyl, C₂₋₈ alkenyl, C₂₋₈alkynyl, C₃₋₈cycloalkyl, C₃₋₈heterocycloalkyl, 1-methylpyrrolidinyl, 1-methylpiperidinyl, 5-methyl-2-oxo-[1,3]dioxol-4-yl, C₃₋₈cycloalkylC₁₋₈alkyl, C₃₋₈heterocycloalkylC₁₋₈alkyl, C₁₋₆alkyloxyC₁₋₆alkyl, hydroxyC₁₋₆alkyl, C₁₋₆alkylthioC₁₋₆alkyl, C₁₋₆alkylsulfinylC₁₋₆alkyl, C₁₋₆alkylsulfonylC₁₋₆alkyl, C₃₋₈cycloalkyloxyC₁₋₆alkyl, C₃₋₈cycloalkylthioC₁₋₆alkyl, C₃₋₈cycloalkylsulfinylC₁₋₆alkyl, C₃₋₈cycloalkylsulfonylC₁₋₆alkyl, C₁₋₆alkyloxycarbonylC₁₋₆alkyl, C₁₋₆alkyloxycarbonylC₁₋₆alkenyl, C₁₋₆alkyloxycarbonyloxyC₁₋₆alkyl, C₁₋₆alkyloxycarbonyloxyC₁₋₆alkenyl, C₃₋₈cycloalkyloxycarbonyloxyC₁₋₆alkyl, N-di-C₁₋₈alkylaminoC₁₋₈alkyl, N-C₆₋₁₂aryl-N-C₁₋₆alkylaminoC₁₋₆alkyl, N-di-C₁₋₈alkyl-carbamoylC₁₋₈alkyl, C₆₋₁₂arylC₁₋₆alkyl, heteroC₆₋₁₀arylC₁₋₆alkyl, C₆₋₁₂aryl, C₆₋₁₂aryloxyC₁₋₈alkyl, C₆₋₁₂arylthioC₁₋₈alkyl, C₆₋₁₂arylsulfinylC₁₋₈alkyl, C₆₋₁₂aryisulfonylC₁₋₈ alkyl, C₁₋₈alkanoyloxyC₁₋₈alkyl, C₄₋₈imidoC₁₋₈alkyl, C₆₋₁₂aroyloxyC₁₋₈ alkyl, or a triglyceride;
X is an -N(R²)- group in which:
R² is a hydrogen atom or a C₁₋₆alkyl group;
V is an oxygen (O) atom;
R^{z} is a hydrogen or halogen atom or a C₁₋₆alkyl group, or a group L¹(Alk¹)ₙR³ in which:
L¹ is a covalent bond or an -O-, -S- or -Se- atom or -S(O)- or - N(R⁸)- group;
Alk¹ is a C₁₋₆alkylene chain;
R³ is a hydrogen atom or C₃₋₁₀cycloaliphatic group, or an optionally substituted C₃₋₁₀heterocycloaliphatic, C₆₋₁₂aromatic or C₁₋₉heteroaromatic group; the optional substituents which may be present on such heterocycloaliphatic groups are the R^{x} and R^{y} spiro-linked heterocycloaliphatic group substituents; optional substituents which may be present on such aromatic and heteroaromatic groups are selected from halogen atoms, or C₁₋₆alkyl, haloC₁₋₆alkyl, C₁₋₆alkoxy or haloC₁₋₆alkoxy groups;
n is zero or the integer 1;
R^{x} and R^{y} are joined together to form an optionally substituted spiro linked C₃₋₁₀cycloaliphatic or C₃₋₁₀heterocycloaliphatic group; optional substituents which may be present on such spiro linked groups are selected from halogen atoms, C₁₋₆alkyl groups, C₁₋₆alkoxy groups, haloC₁₋₆alkoxy groups, -CN, -CO₂CH₃, -NO₂ and -N(R¹¹)₂; when the spiro linked heterocycloaliphatic group contains a nitrogen atom this may be substituted by a group -(L⁶)ₚ(Alk⁵)_{q}R¹² where L⁶ is -C(O)- or -S(O)₂-, Alk⁵ is a C₁₋₆alkylene chain, or a heteroC₁₋₆alkylene chain and R¹² is a hydrogen atom or an optionally substituted phenyl ring, wherein the optional phenyl substituents are selected from halogen atoms, or C₁₋₆alkyl, haloC₁₋₆alkyl, C₁₋₆alkoxyl, haloC₁₋₆alkoxy, -CN, -CO₂CH₃, -NO₂, - NH₂, -NR⁵R⁶, -N(R⁵)COCH₃, or phenyl, furyl, thienyl, imidazolyl, pyridyl or pyrimidinyl groups;
R¹¹ is a hydrogen atom, or a C₁₋₆alkyl or C₃₋₈cycloalkyl group;
and the salts, solvates, hydrates and N-oxides thereof.

2. A compound according to claim 1 in which Alk is a -CH₂CH(R)- or - CH(CH₂R)- chain.

3. A compound according to claim 1 or claim 2 in which R is a carboxylic acid (-CO₂H) group.

4. A compound according to claim 1 or claim 2 in which R is an esterified carboxyl group of formula -CO₂Alk⁷.

5. A compound according to claim 1 in which R² is a hydrogen atom.

6. A compound according to any one of claims 1 to 5 in which Ar² is an optionally substituted phenylene group or an optionally substituted pyridinediyl group of formula: 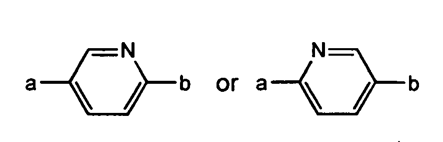 where a and b signify the points of attachment of L² and Alk respectively.

7. A compound according to claim 6 wherein Ar² is 1,4-phenylene.

8. A compound according to any one of claims 1 to 7 in which Ar¹ is an optionally substituted phenyl or five-, six- or ten-membered heteroaromatic group.

9. A compound according to claim 8 in which Ar¹ is an optionally substituted pyridyl, pyrimidinyl, naphthyridinyl, quinolinyl or isoquinolinyl group.

10. A compound according to claim 9 wherein Ar¹ is 2,7-naphthyridinyl.

11. A compound according to any one of claims 1 to 10 in which R^{z} is a halogen atom.

12. A compound according to claim 1 wherein R^{z} is a bromine atom.

13. A compound according to any one of claims 1 to 10 in which R^{z} is an optionally substituted C₁₋₈alkyl group.

14. A compound according to any one of claims 1 to 10 in which R^{z} is a group -L¹(Alk¹)ₙR³ in which L¹ is an -O-, -S- or -Se- atom or -S(O)- or - N(R⁸)- group.

15. A compound according to any one of claims 1 to 10 in which R^{z} is a group -L¹(Alk¹)ₙR³ in which L¹ is a covalent bond.

16. A compound according to claim 14 or claim 15 in which n is zero.

17. A compound according to claim 14 or claim 15 in which n is the integer 1 and Alk¹ is an optionally substituted C₁₋₆alkylene chain.

18. A compound according to Claim 1 which is:
(2*S*)-2-[(3-Oxospiro[3.5]non-1-en-1-yl)amino]-3-{4-[(3,5-dichloroisonicotinoyl)amino]phenyl}propanoic acid
(2*S*)-2-[(3-Oxospiro[3.5]non-1-en-1-yl)amino]-3-{4-[(3-methyl[2,7]naphthyridin-1-yl)oxy]phenyl}propanoic acid
(2*S*)-2-[(2-Bromo-3-oxospiro[3.5]non-1-en-1-yl)amino]-3-{4-[(3,5-dichloroisonicotinoyl)amino]phenyl}propanoic acid
(2*S*)-2-[(2-Bromo-3-oxospiro[3.5]non-1-en-1-yl)amino]-3-{4-[(2,7)naphthyridin-1-yloxy]phenyl}propanoic acid
(2*S*)-2-[(2-Bromo-3-oxo-7-oxaspiro[3.5]non-1-en-1-yl)amino]-3-{4-[(3,5-dichloroisonicotinoyl)amino]phenyl}propanoic acid
(2*S*)-2-{(2-Bromo-3-oxospiro[3.5]non-1-en-1-yl)amino}-3-(2,6-dimethoxy[1,1'-biphenyl]-4-yl)propanoic acid
(2*S*)-2-[(3-Oxospiro[3.6]dec-1-en-1-yl)amino]3-{4-[(3,5-dichloroisonicotinoyl)amino]phenyl}propanoic acid
(2*S*)-2-[(2-Bromo-3-oxospiro[3.6]dec-1-en-1-yl)amino]-3-{4-[(3,5-dichloroisonicotinoyl)amino]phenyl}propanoic acid
(2*S*)-2-[(2-Bromo-7-methoxy-3-oxospiro[3.5]non-1-en-1-yl)amino]-3-{4-[(3,5-dichloroisonicotinoyl)amino]phenyl}propanoic acid
(2*S*)-2-{(2-Bromo-3-oxospiro[3.5]non-1-en-1-yl)amino}-3-{4-[(3-methyl[2,7]naphthyridin-1-yl)oxy]phenyl}propanoic acid
(2*S*)-2-[(2-Chloro-3-oxospiro[3.5]non-1-en-1-yl)amino]-3-{4-[(3,5-dichloroisonicotinoyl)amino]phenyl}propanoic acid
(2*S*)-3-{4-[(3,5-Dichloroisonicotinoyl)amino]phenyl}-2-(3-oxo-2-pyridin-3-yl-spiro[3.5]non-1-en-1-ylamino)propanoic acid
(2*S*)-2-[(2-lodo-3-oxospiro[3.5]non-1-en-1-yl)amino]-3-{4-[(3,5-dichloroisonicotinoyl)amino]phenyl}propanoic acid
(2*S*)-2-[(2-Bromo-3-oxospiro[3.5]non-1-en-1-yl)amino]-3-{5-[(3,5-dichloroisonicotinoyl)amino]pyridin-2-yl}propanoic acid
(2*S*)-2-[(2-Chloro-3-oxospiro[3.5]non-1-en-1-yl)amino]-3-{5-[(3,5-dichloroisonicotinoyl)amino]pyridin-2-yl}propanoic acid
(2*S*)-2-[(2-Chloro-3-oxo-7-oxa-spiro[3.5]non-1-en-1-yl)amino]-3-{4-[(3,5-dichloroisonicotinoyl)amino]phenyl}propanoic acid
(2S)-2-[(2-Chloro-3-oxo-spiro[3.6]dec-1-en-1-yl)amino]-3-{4-[(3,5-dichloroisonicotinoyl)amino]phenyl}propanoic acid
(2*S*)-2-[(2-Bromo-3,7,7-trioxo-7λ⁶-thia-spiro[3.5]non-1-en-1-yl)amino]-3-{4-[(3,5-dichloroisonicotinoyl)amino]phenyl}propanoic acid
(2*S*)-2-[(2-Chloro-3-oxo-spiro[3.4]oct-1-en-1-yl)amino]-3-{4-[(3,5-dichloro-isonicotinoyl)amino]phenyl}propanoic acid
(2*S*)-2-[(2-Bromo-3-oxo-spiro[3.4]oct-1-en-1-yl)amino]-3-{4-[(3,5-dichloroisonicotinoyl)amino]phenyl}propanoic acid
(2*S*)-2-[(2-Methylsulfanyl-3-oxo-spiro[3.5]non-1-en-1-yl)amino]-3-{4-[(3,5-dichloroisonicotinoyl)amino]phenyl}propanoic acid
(2*S*)-2-(2-Fluoro-3-oxo-spiro[3.5]non-1-en-1-ylamino)3-{4-[(3,5-dichloroisonicotinoyl)amino]phenyl}propanoic acid
(*2S*)-2-[(2-Fluoro-3-oxo-7-oxa-spiro[3.5]non-1-en-1-yl)amino]-3-{4-[(3,5-dichloroisonicotinoyl)amino]phenyl}propanoic acid
(2*S*)-2-[(2-Isopropylsulfanyl-3-oxo-spiro[3.5]non-1-en-1-yl)amino]-3-{4-[(3,5-dichloroisonicotinoyl)amino]phenyl}propanoic acid
(2*S*)-2-[(2-Isopropylsulfanyl-3-oxo-spiro[3.5]non-1-en-1-yl)amino]-3-[4-([2,7]naphthyridin-1-ylamino)phenyl]propanoic acid
(2*S*)-2-[(2-Isopropylsulfanyl-3-oxo-7-oxa-spiro[3.5]non-1-en-1-yl)amino]-3-[4-([2,7]naphthyridin-1-ylamino)phenyl]propanoic acid
(2*S*)-2-[(2-Bromo-3-oxo-spiro[3.4]octa-1,6-dien-1-yl)amino]-3-{4-[(3,5-dichloroisonicotinoyl)amino]phenyl}propanoic acid
(2*S*)-2-[(7-Acetyl-2-bromo-3-oxo-7-aza-spiro[3.5]non-1-en-1-yl)amino]-3-{4-[(3,5-dichloroisonicotinoyl)amino]phenyl}propanoic acid
(2*S*)-2-{[2-(Isopropylsulfanyl)-3-oxo-7-oxaspiro[3.5]non-1-en-yl)]amino}-3-(2,6-dimethoxy[1,1'-biphenyl]-4-yl)propanoic acid
(2*S*)-2-[(2-Cyclohexyl-3-oxo-spiro[3.5]non-1-en-1-yl)amino]-3-{4-[(3,5-dichloroisonicotinoyl)amino]phenyl}propanoic acid
(2*S*)-3-{4-[(3,5-Dichloroisonicotinoyl)amino]phenyl}-2-(2-methyl-3-oxo-spiro[3.5]non-1-en-1-ylamino)propanoic acid
(2*S*)-3-{4-[(3,5-Dichloroisonicotinoyl)amino]phenyl}-2-(3-oxo-2-propyl-spiro[3.5]non-1-en-1-ylamino)propanoic acid
(*2S*)-3-{4-[(3,5-Dichloroisonicotinoyl)amino]phenyl}-2-(2-methyl-3-oxo-7-oxa-spiro[3.5]non-1-en-1-ylamino)propanoic acid
(*2S*)-3-{4-[(3,5-Dichloroisonicotinoyl)amino]phenyl}-2-(3-oxo-2-propyl-7-oxa-spiro[3.5]non-1-en-1-ylamino)propanoic acid
(2S)-3-{4-[(3,5-Dichloroisonicotinoyl)amino]phenyl}-2-(2-[1,3]dithian-2-yl-3-oxo-spiro[3.5]non-1-en-1-ylamino)propanoic acid
(*2S*)-3-{4-[(3,5-Dichloroisonicotinoyl)amino]phenyl}-2-(2-ethyl-3-oxo-spiro[3.5]non-1-en-1-ylamino)propanoic acid
(*2S*)-3-{4-[(3,5-Dichloro-1-oxy-pyridine-4-carbonyl)amino]phenyl}-2-(3-oxo-spiro[3.5]non-1-en-1-ylamino)propanoic acid
(2*S*)-2-(2-Bromo-3-oxo-spiro[3.5]non-1-en-1-ylamino)-3-{4-[(3,5-dichloro-1-oxy-pyridine-4-carbonyl)amino]phenyl}propanoic acid
(2*S*)-2-(2-Chloro-3-oxo-spiro[3.5]non-1-en-1-ylamino)-3-{4-[(3,5-dichloro-1-oxy-pyridine-4-carbonyl)amino]phenyl}propanoic acid
(*2S*)-2-(2-Bromo-3-oxo-spiro[3.5]non-1-en-1-ylamino)-3-[4-(3-methyl-[2,7]naphthyridin-1-ylamino)phenylJpropanoic acid
(2*S*)-2-(2-Bromo-3-oxo-spiro[3.5]non-1-en-1-ylamino)-3-[4-([2,7]naphthyridin-1-ylamino)phenyl]propanoic acid
(2*S*)-2-[(2-Bromo-3-oxo-spiro[3.4]oct-1-en-1-yl)amino]-3-{4-[(3,5-dichloroisonicotinoyl)amino]phenyl}propanoic acid
and the salts, solvates, hydrates, N-oxides and carboxylic acid esters thereof.

19. A compound according to Claim 1 which is:
(2*S*)-2-[(2-Bromo-3-oxospiro[3.5]non-1-en-1-yl)amino]-3-{4-[(3,5-dichloroisonicotinoyl)amino]phenyl}propanoic acid
(2*S*)-2-[(2-Bromo-3-oxo-7-oxaspiro[3.5]non-1-en-1-yl)amino]-3-{4-[(3,5-dichloroisonicotinoyl)amino]phenyl}propanoic acid
(2*S*)-2-[(2-Bromo-3-oxospiro[3.6]dec-1-en-1-yl)amino]-3-{4-[(3,5-dichloroisonicotinoyl)amino]phenyl}propanoic acid
(2*S*)-2-{(2-Bromo-3-oxospiro[3.5]non-1-en-1-yl)amino}-3-{4-[(3-methyl[2,7]naphthyridin-1-yl)oxy]phenyl}propanoic acid
(2*S*)-2-[(2-Chloro-3-oxospiro[3.5]non-1-en-1-yl)amino]-3-{4-[(3,5-dichloroisonicotinoyl)amino]phenyl}propanoic acid
(2S)-3-{4-[(3,5-Dichloroisonicotinoyl)amino]phenyl}-2-(3-oxo-2-pyridin-3-yl-spiro[3.5]non-1-en-1-ylamino)propanoic acid
(2*S*)-2-[(2-Iodo-3-oxospiro[3.5]non-1-en-1-yl)amino]-3-{4-[(3,5-dichloroisonicotinoyl)amino]phenyl}propanoic acid
(2*S*)-2-[(2-Bromo-3-oxospiro[3.5]non-1-en-1-yl)amino]-3-{5-[(3,5-dichloroisonicotinoyl)amino]pyridin-2-yl}propanoic acid
(2*S*)-2-[(2-Chloro-3-oxospiro[3.5]non-1-en-1-yl)amino]-3-{5-[(3,5-dichloroisonicotinoyl)amino]pyridin-2-yl}propanoic acid
(2*S*)-2-[(2-Chloro-3-oxo-7-oxa-spiro[3.5]non-1-en-1-yl)amino]-3-{4-[(3,5-dichloroisonicotinoyl)amino]phenyl}propanoic acid
(2S)-2-[(2-Chloro-3-oxo-spiro[3.6]dec-1-en-1-yl)amino]-3-{4-[(3,5-dichloroisonicotinoyl)amino]phenyl}propanoic acid
(2*S*)-2-[(2-Bromo-3,7,7-trioxo-7λ⁶-thia-spiro[3.5]non-1-en-1-yl)amino]-3-{4-[(3,5-dichloroisonicotinoyl)amino]phenyl}propanoic acid
(2*S*)-2-[(2-Chloro-3-oxo-spiro[3.4]oct-1-en-1-yl)amino]-3-{4-[(3,5-dichloroisonicotinoyl)amino]phenyl}propanoic acid
(2*S*)-2-[(2-Bromo-3-oxo-spiro[3.4]oct-1-en-1-yl)amino]-3-{4-[(3,5-dichloroisonicotinoyl)amino]phenyl}propanoic acid
(2*S*)-2-[(2-Methylsulfanyl-3-oxo-spiro[3.5]non-1-en-1-yl)amino]-3-{4-[(3,5-dichloroisonicotinoyl)amino]phenyl}propanoic acid
(2*S*)-2-(2-Fluoro-3-oxo-spiro[3.5]non-1-en-1-ylamino)3-{4-[(3,5-dichloroisonicotinoyl)amino]phenyl}propanoic acid
(2*S*)-2-[(2-Fluoro-3-oxo-7-oxa-spiro[3.5]non-1-en-1-yl)amino]-3-{4-[(3,5-dichloroisonicotinoyl)amino]phenyl}propanoic acid
(2*S*)-2-[(2-Isopropylsulfanyl-3-oxo-spiro[3.5]non-1-en-1-yl)amino]-3-{4-[(3,5-dichloroisonicotinoyl)amino]phenyl}propanoic acid
(2*S*)-2-[(2-Isopropylsulfanyl-3-oxo-spiro[3.5]non-1-en-1-yl)amino]-3-[4-([2,7]naphthyridin-1-ylamino)phenyl)propanoic acid
(2*S*)-2-[(2-Isopropylsulfanyl-3-oxo-7-oxa-spiro[3.5]non-1-en-1-yl)amino]-3-[4-([2,7]naphthyridin-1-ylamino)phenyl]propanoic acid
(2*S*)-2-[(2-Bromo-3-oxo-spiro[3.4]octa-1,6-dien-1-yl)amino]-3-{4-[(3,5-dichloroisonicotinoyl)amino]phenyl}propanoic acid
(2*S*)-2-[(7-Acetyl-2-bromo-3-oxo-7-aza-spiro[3.5]non-1-en-1-yl)amino]-3-{4-[(3,5-dichloroisonicotinoyl)amino]phenyl}propanoic acid
(2*S*)-2-[(2-Cyclohexyl-3-oxo-spiro[3.5]non-1-en-1-yl)amino]-3-{4-((3,5-dichloroisonicotinoyl)amino]phenyl}propanoic acid
(2*S*)3-{4-[(3,5-Dichloroisonicotinoyl)amino]phenyl}-2-(2-methyl-3-oxo-spiro[3.5]non-1-en-1-ylamino)propanoic acid
(2*S*)-3-{4-[(3,5-Dichloroisonicotinoyl)amino]phenyl}-2-(3-oxo-2-propyl-spiro[3.5]non-1-en-1-ylamino)propanoic acid
(2S)-3-{4-[(3,5-Dichloroisonicotinoyl)amino]phenyl}-2-(2-[1,3]dithian-2-yl-3-oxo-spiro[3.5]non-1-en-1-ylamino)propanoic acid
(2*S*)-2-(2-Bromo-3-oxo-spiro[3.5]non-1-en-1-ylamino)-3-{4-[(3,5-dichloro-1-oxy-pyridine-4-carbonyl)amino]phenyl}propanoic acid
2-(2-Chloro-3-oxo-spiro[3.5]non-1-en-1-ylamino)-3-{4-[(3,5-dichloro-1-oxy-pyridine-4-carbonyl)amino]phenyl}propanoic acid
and the salts, solvates, hydrates, N-oxides and carboxylic acid esters thereof.

20. A compound according to Claim 1 which is:
Ethyl (2S)-2-(2-bromo-3-oxo-spiro[3.5]non-1-en-1-ylamino)-3-{4-[(3,5-dichloroisonicotinoyl)amino]phenyl}propanoate
Isopropyl (2S)-2-(2-bromo-3-oxo-spiro[3.5]non-1-en-1-ylamino)-3-{4-[(3,5-dichloroisonicotinoyl)amino]phenyl}propanoate
t-Butyl (2S)-2-(2-bromo-3-oxo-spiro[3.5]non-1-en-1-ylamino)-3-{4-[(3,5-dichloroisonicotinoyl)amino]phenyl}propanoate
1-Methyl-piperidin-4-yl (*2S*)-2-(2-bromo-3-oxo-spiro[3.5]non-1-en-1-ylamino)-3-{4-[(3,5-dichloroisonicotinoyl)amino]phenyl}propanoate
Phenyl (2S)-2-(2-bromo-3-oxo-spiro[3.5]non-1-en-1-ylamino)-3-{4-[(3,5-dichloroisonicotinoyl)amino]phenyl}propanoate
Cyclopentyl (2S)-2-(2-bromo-3-oxo-spiro[3.5]non-1-en-1-ylamino)-3-{4-[(3,5-dichloroisonicotinoyl)amino]phenyl}propanoate
2-Imidazol-1-yl-ethyl (2*S*)-2-(2-bromo-3-oxo-spiro[3.5]non-1-en-1-ylamino)-3-{4-[(3,5-dichloroisonicotinoyl)amino]phenyl}propanoate
Neopentyl (*2S*)-2-(2-bromo-3-oxo-spiro[3.5]non-1-en-1-ylamino)-3-{4-[(3,5-dichloroisonicotinoyl)amino]phenyl}propanoate
Tetrahydro-furan-3-yl (*2S*)-2-(2-bromo-3-oxo-spiro[3.5]non-1-en-1-ylamino)-3-{4-[(3,5-dichloroisonicotinoyl)amino]phenyl}propanoate
Pyridin-4-ylmethyl (*2S*)-2-(2-bromo-3-oxo-spiro[3.5]non-1-en-1-ylamino)-3-{4-[(3,5-dichloroisonicotinoyl)amino]phenyl}propanoate
Tetrahydropyran-4-yl (*2S*)-2-(2-bromo-3-oxo-spiro[3.5]non-1-en-1-ylamino)-3-{4-[(3,5-dichloroisonicotinoyl)amino]phenyl}propanoate
5-Methyl-2-oxo-[1,3]dioxol-4-ylmethyl (*2S*)-2-(2-bromo-3-oxo-spiro[3.5]non-1-en-1-ylamino)-3-{4-[(3,5-dichloroisonicotinoyl)amino]-phenyl}propanoate
1-Methyl-pyrrolidin-3-yl (2*S*)-2-(2-bromo-3-oxo-spiro[3.5]non-1-en-1-ylamino)-3-{4-[(3,5-dichloroisonicotinoyl)amino]phenyl}propanoate
2,3-Dihydroxypropyl (2*S*)-2-(2-bromo-3-oxo-spiro[3.5]non-1-en-1-ylamino)-3-{4-[(3,5-dichloroisonicotinoyl)amino]phenyl}propanoate
Tetrahydrofuran-2-ylmethyl (*2S*)-2-(2-bromo-3-oxo-spiro[3.5]non-1-en-1-ylamino)-3-{4-[(3,5-dichloroisonicotinoyl)amino]phenyl}propanoate
and the salts, solvates, hydrates and N-oxides thereof.

21. A pharmaceutical composition comprising a compound according to claim 1 together with one or more pharmaceutically acceptable carriers, excipients or diluents.

22. Ethyl (*2S*)-2-(2-bromo-3-oxo-spiro[3.5]non-1-en-1-ylamino)-3-[4-([2,7]naphthyridin-1-ylamino)phenyl]propanoate according to claim 1.

23. A pharmaceutical composition comprising ethyl (*2S*)-2-(2-bromo-3-oxo-spiro[3.5]non-1-en-1-ylamino)-3-[4-([2,7]naphthyridin-1-ylamino)-phenyl]propanoate according to claim 22 in association with a pharmaceutically acceptable carrier.

24. The use of ethyl (*2S*)-2-(2-bromo-3-oxo-spiro[3.5]non-1-en-1-ylamino)-3-[4-([2,7]naphthyridin-1-ylamino)phenyl]propanoate according to claim 22 for the manufacture of a medicament for the treatment and/or prevention of rheumatoid arthritis, multiple sclerosis, asthma or inflammatory bowel diseases.

## Patentansprüche

1. Verbindung der Formel (1): 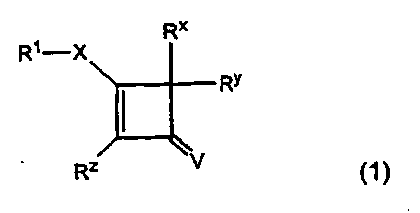 wobei
R¹ eine Gruppe Ar¹L²Ar²Alk- ist, in welcher
Ar¹ eine gegebenenfalls substituierte aromatische oder heteroaromatische Gruppe ist, wobei die optionalen Substituenten aus einem bzw. einer, zwei oder drei Halogenatomen und/oder C₁₋₆-Alkyl-, C₃₋₈-Cycloalkyl-, C₁₋₆-Hydroxyalkyl-, Carboxy-C₁₋₆-alkyl-, C₁₋₆-Alkylthio-, Carboxy-C₁₋₆alkylthio-, C₁₋₆-Alkoxy-, Hydroxy-C₁₋₆-alkoxy-, Halogen-C₁₋₆-alkyl-, Halogen-C₁₋₆-alkoxy-, C₁₋₆-Alkylamino-, -NH₂-, AminO-C₁₋₆-alkyl-, C₁₋₆-Dialkylamino-, C₁₋₆-Alkylamino-C₁₋₆-alkyl-, C₁₋₆-Dialkylamino-C₁₋₆-alkyl-, Amino-C₁₋₆-alkoxy-, C₁₋₆-Alkylamino-C₁₋₆-alkoxy-, C₁₋₆-Dialkylamino-C₁₋₆-alkoxy-, Nitro-, Cyano-, Amidino-, -OH-, HC(O)-, -CO₂H-, -CO₂R⁵-, C₁₋₆-Alkanoyl-, -SH-, Thio-C₁₋₆-alkyl-, -SO₃H-, -SO₃R⁵-, C₁₋₆-Alkyl-sufinyl-, C₁₋₆-Alkylsulfonyl-, -SO₂NH₂-, C₁₋₆-Alkylaminosulfonyl-, C₁₋₆-Dialkylaminosulfonyl-, Phenylaminosulfonyl-, -CONH₂-, C₁₋₆-Alkylaminocarbonyl-, C₁₋₆-Dialkylaminocarbonyl-, Amino-C₁₋₆-alkylaminocarbonyl-, C₁₋₆-Alkylamino-C₁₋₆-alkylaminocarbonyl-, C₁₋₆-Dialkylamino-C₁₋₆-alkylaminocarbonyl-, Aminocarbonylamino-, C₁₋₆-Alkylaminocarbonylamino-, C₁₋₆-Dialkylaminocarbonylamino-, C₁₋₆-Alkylaminocarbonyl-C₁₋₆-alkylamino-, Aminothiocarbonylamino-, C₁₋₆-Alkylaminothiocarbonylamino-, C₁₋₆-Dialkylaminothiocarbonylamino-, C₁₋₆-Alkylaminothiocarbonyl-C₁₋₆-alkyl-amino-, C₁₋₆-Alkylsulfonylamino-, C₁₋₆-Dialkylsulfonylamino-, -NHSO₂NH₂-, C₁₋₆-Alkylaminosulfonylamino-, C₁₋₆-Dialkylaminosulfonylamino-, C₁₋₆-Alkanoylamino-, Amino-C₁₋₆-alkanoylamino-, C₁₋₆-Dialkylamino-C₁₋₆-alkanoylamino-, C₁₋₆-Alkanoylamino-C₁₋₆-alkyl-, C₁₋₆-Alkanoylamino-C₁₋₆-alkylamino- oder C₁₋₆-Alkoxycarbonylaminogruppen ausgewählt sind,
R⁵ ein Wasserstoffatom oder eine C₁₋₆-Alkyl- oder C₃₋₈-Cycloalkyl-Gruppe ist,
L² eine kovalente Bindung oder eine Gruppe L^{2a} oder -(Alk³)L^{2a} ist, wobei Alk³ eine gegebenenfalls substituierte aliphatische oder heteroaliphatische Kette ist und L^{2a} ein -O- oder -S-Atom oder eine -C(O)-, -C(O)O-, -OC(O)-, -C(S)-, -S(O)-, -S(O)₂-, -N(R⁸)- [wobei R⁸ ein Wasserstoffatom oder eine geradkettige oder verzweigtkettige C₁₋₆-Alkylgruppe ist], -CON(R⁸)-, -OC(O)N(R⁸)-, -CSN(R⁸)-, -N(R⁸)CO-, -N(R⁸)C(O)O-, -N(R⁸)CS-, -S(O)₂N(R⁸)-, -N(R⁸)S(O)₂-, -N(R⁸)O-, -ON(R⁸)-, -N(R⁸)N(R⁸)-, -N(R⁸)CON(R⁸)-, -N(R⁸)CSN(R⁸)- oder -N(R⁸)SO₂N(R⁸)-Gruppe ist, wobei optionale Substituenten, die am Alk³ vorhanden sein können, aus einem, zwei oder drei Substituenten ausgewählt sind, wobei jeder Substituent gleich oder verschieden sein kann und aus Halogenatomen oder -OH-, -CO₂H-, -CO₂R⁹-, wobei R⁹ eine geradkettige oder verzweigtkettige C₁₋₆-Alkylgruppe ist, -CONHR⁹-, -CON(R⁹)₂-, -COR⁹-, C₁₋₆-Alkoxy-, Thiol-, -S(O)R⁹-, -S(O)₂R⁹-, C₁₋₆-Alkylthio-, Amino-, -NHR⁹- oder -N(R⁹)₂-Gruppen ausgewählt ist,
Ar² eine gegebenenfalls substituierte Arylen- oder Heteroarylengruppe ist, wobei die optionalen Substituenten, die an dem Ar² vorhanden sein können, aus Halogenatomen oder C₁₋₆-Alkyl-, Halogen-C₁₋₆-alkyl-, C₁₋₆-Alkoxy-, Halogen-C₁₋₆-alkoxy-, -CN-, -CO₂CH₃-, -NO₂-, -NH₂-, -NR⁵R⁶- und -N(R⁵)COCH₃-Gruppen ausgewählt sind,
R⁶ wie für R⁵ definiert ist,
und Alk eine Kette -CH₂-CH(R)-, -CH=C(R)- oder 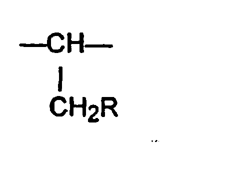 ist, in welcher R eine Carbonsäure (-CO₂H) oder eine Tetrazol-, Phosphonsäure-, Phosphinsäure, Sulfonsäure-, Sulfinsäure-, Boronsäuregruppe oder ein Acylsulfonamidbioster davon oder eine -CO₂Alk⁷ oder -CONR⁵R⁶-Gruppe ist,
Alk⁷ geradkettiges oder verzweigtkettiges C₁₋₈-Alkyl, C₂₋₈-Alkenyl, C₂₋₈-Alkinyl, C₃₋₈-Cycloalkyl, C₃₋₈-Heterocycloalkyl, 1-Methylpyrrolidinyl, 1-Methylpiperidinyl, 5-Methyl-2-oxo-[1,3]dioxol-4-yl, C₃₋₈-Cycloalkyl-C₁₋₈-alkyl, C₃₋₈-Heterocycloalkyl-C₁₋₈-alkyl, C₁₋₆-Alkyloxy-C₁₋₆-alkyl, Hydroxy-C₁₋₆-alkyl, C₁₋₆-Alkylthio-C₁₋₆-alkyl, C₁₋₆-Alkylsulfinyl-C₁₋₆-alkyl, C₁₋₆-Alkylsulfonyl-C₁₋₆-alkyl, C₃₋₈-Cycloalkyloxy-C₁₋₆-alkyl, C₃₋₈-Cycloalkylthio-C₁₋₆-alkyl, C₃₋₈-Cycloalkylsulfinyl-C₁₋₆-alkyl, C₃₋₈-Cycloalkylsulfonyl-C₁₋₆-alkyl, C₁₋₆-Alkytoxycarbonyl-C₁₋₆-alkyl, C₁₋₆-Alkyloxycarbonyl-C₁₋₆-alkenyl, C₁₋₆-Alkyloxycarbonyloxy-C₁₋₆-alkyl, C₁₋₆-Alkyloxycarbonyloxy-C₁₋₆-alkenyl, C₃₋₈-Cycloalkyloxycarbonyloxy-C₁₋₆-alkyl, N-Di-C₁₋₈-alkylamino-C₁₋₈-alkyl, N-C₆₋₁₂-Aryl-N-C₁₋₆-alkylamino-C₁₋₆-alkyl, N-Di-C₁₋₈-alkylcarbamoyl-C₁₋₈-alkyl, C₆₋₁₂-Aryl-C₁₋₆-alkyl, Hetero-C₆₋₁₀-aryl-C₁₋₆-alkyl, C₆₋₁₂-Aryl, C₆₋₁₂-Aryloxy-C₁₋₈-alkyl, C₆₋₁₂-Arylthio-C₁₋₈-alkyl, C₆₋₁₂-Arylsulfinyl-C₁₋₈-alkyl, C₆₋₁₂-Arylsulfonyl-C₁₋₈-alkyl, C₁₋₈-Alkanoyloxy-C₁₋₈-alkyl, C₄₋₈-Imido-C₁₋₈-alkyl, C₆₋₁₂-Aroyloxy-C₁₋₈-alkyl oder ein Triglycerid ist,
X eine -N(R²)-Gruppe ist, in welcher
R² ein Wasserstoffatom oder eine C₁₋₆-Alkylgruppe ist,
V ein Sauerstoffatom (O) ist,
R^{z} ein Wasserstoff- oder Halogenatom oder eine C₁₋₆-Alkylgruppe oder eine Gruppe L¹(Alk¹)ₙR³ ist, in welcher
L¹ eine kovalente Bindung oder ein -O-, -S- oder -Se-Atom oder eine -S(O)-oder -N(R⁸)-Gruppe ist,
Alk¹ eine C₁₋₆-Alkylenkette ist,
R³ ein Wasserstoffatom oder eine C₃₋₁₀-cycloaliphatische Gruppe oder eine gegebenenfalls substituierte C₃₋₁₀-heterocyloaliphatische, C₆₋₁₂-aromatische oder C₁₋₉-heteroaromatische Gruppe ist, wobei die optionalen Substituenten, welche an solchen heterocycloaliphatischen Gruppen vorhanden sein können, die R^{x}-und R^{y}-spiroverknüpften heterocycloaliphatischen Gruppensubstituenten sind, wobei die optionalen Substituenten, welche an solchen aromatischen und heteroaromatischen Gruppen vorhanden sein können, aus Halogenatomen oder C₁₋₆-Alkyl-, Halogen-C₁₋₆-alkyl-, C₁₋₆-Alkoxy- oder Halogen-C₁₋₆-alkoxygruppen ausgewählt sind,
n Null oder die ganze Zahl 1 ist,
R^{x} und R^{y} miteinander verbunden sind, um eine gegebenenfalls substituierte, spiroverknüpfte C₃₋₁₀-cycloaliphatische oder C₃₋₁₀-heterocycloaliphatische Gruppe zu bilden, wobei optionale Substituenten, welche an solchen spiroverknüpften Gruppen vorhanden sein können, aus Halogenatomen, C₁₋₆-Alkylgruppen, C₁₋₆-Alkoxygruppen, Halogen-C₁₋₆-alkoxygruppen, -CN, -CO₂CH₃, -NO₂ und -N(R¹¹)₂ ausgewählt sind; wenn die spiroverknüpfte heterocycloaliphatische Gruppe ein Stickstoffatom enthält, kann dieses durch eine Gruppe -(L⁶)ₚ(Alk⁵)_{q}R¹² substituiert sein, wobei L⁶ -C(O)- oder -S(O)₂- ist, wobei Alk⁵ eine C₁₋₆-Alkylenkette oder eine Hetero-C₁₋₆-alkylenkette ist und R¹² ein Wasserstoffatom oder ein gegebenenfalls substituierter Phenylring ist, wobei die optionalen Phenylsubstituenten aus Halogenatomen oder C₁₋₆-Alkyl-, Halogen-C₁₋₆-alkyl-, C₁₋₆-Alkoxyl-, Halogen-C₁₋₆-alkoxy-, -CN-, -CO₂CH₃-, -NO₂-, -NH₂-, -NR⁵R⁶-, -N(R⁵)COCH³-, oder Phenyl-, Furyl-, Thienyl-, Imidazolyl , Pyridyl-oder Pyrimidinylgruppen ausgewählt sind,
R¹¹ ein Wasserstoffatom oder eine C₁₋₆-Alkyl- oder C₃₋₈-Cycloalkylgruppe ist, und die Salze, Solvate, Hydrate und N-Oxide davon.

2. Verbindung nach Anspruch 1, in welcher Alk eine -CH₂CH(R)- oder -CH(CH₂R)-Kette ist.

3. Verbindung nach Anspruch 1 oder Anspruch 2, in welcher R eine Carbonsäuregruppe (-CO₂H) ist.

4. Verbindung nach Anspruch 1 oder Anspruch 2, in welcher R eine veresterte Carboxylgruppe der Formel -CO₂Alk⁷ ist.

5. Verbindung nach Anspruch 1, in welcher R² ein Wasserstoffatom ist.

6. Verbindung nach einem der Ansprüche 1 bis 5, in welcher Ar² eine gegebenenfalls substituierte Phenylengruppe oder eine gegebenenfalls substituierte Pyridindiylgruppe der Formel: 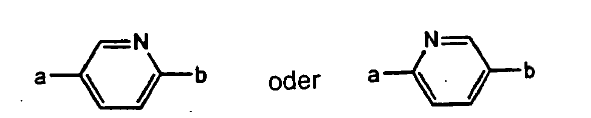 ist, wobei a und b Anknüpfungspunkte von L² bzw. Alk darstellen.

7. Verbindung nach Anspruch 6, wobei Ar² 1,4-Phenylen ist.

8. Verbindung nach einem der Ansprüche 1 bis 7, in welcher Ar¹ eine gegebenenfalls substituierte Phenyl- oder fünf-, sechs- oder zehngliedrige heteroaromatische Gruppe ist.

9. Verbindung nach Anspruch 8, in welcher Ar¹ eine gegebenenfalls substituierte Pyridyl-, Pyrimidinyl-, Naphthyridinyl-, Chinolinyl- oder Isochinolinylgruppe ist.

10. Verbindung nach Anspruch 9, wobei Ar¹ 2,7-Naphthyridinyl ist.

11. Verbindung nach einem der Ansprüche 1 bis 10, in welcher R^{z} ein Halogenatom ist.

12. Verbindung nach Anspruch 1, wobei R^{z} ein Bromatom ist.

13. Verbindung nach einem der Ansprüche 1 bis 10, in welcher R^{z} eine gegebenenfalls substituierte C₁₋₈-Alkylgruppe ist.

14. Verbindung nach einem der Ansprüche 1 bis 10, in welcher R^{z} eine Gruppe -L¹(Alk¹)ₙR³ ist, in welcher L¹ ein -O-, -S- oder -Se- Atom oder eine -S(O)- oder -N(R⁸)-Gruppe ist.

15. Verbindung nach einem der Ansprüche 1 bis 10, in welcher R^{z} eine Gruppe - L¹(Alk¹)ₙR³ ist, in welcher L¹ eine kovalente Bindung ist.

16. Verbindung nach Anspruch 14 oder Anspruch 15, in welcher n Null ist.

17. Verbindung nach Anspruch 14 oder Anspruch 15, in welcher n die ganze Zahl 1 ist und Alk¹ eine gegebenenfalls substituierte C₁₋₆-Alkylenkette ist.

18. Verbindung nach Anspruch 1, welche
(2*S*)-2-[(3-Oxospiro[3.5]non-1-en-1-yl)amino]-3-{4-[(3,5-dichlorisonicotinoyl)amino]phenyl}propansäure
(2*S*)-2-[(3-Oxospiro[3.5]non-1-en-1-yl)amino]-3-{4-[(3-methyl[2,7]naphthyridin-1-yl)oxy]phenyl}propansäure
(2*S*)-2-[(2-Brom-3-oxospiro[3.5]non-1-en-1-yl)amino]-3-{4-[(3,5-dichlorisonicotinoyl)amino]phenyl}propansäure
(2*S*)-2-[(2-Brom-3-oxospiro[3.5]non-1-en-1-yl)amino]-3-{4-[(2,7)naphthyridin-1-yloxy]phenyl}propansäure
(2*S*)-2-[(2-Brom-3-oxo-7-oxaspiro[3.5]non-1-en-1-yl)amino]-3-{4-[(3,5-dichlorisonicotinoyl)amino]phenyl}propansäure
(2*S*)-2-{(2-Brom-3-oxospiro[3.5]non-1-en-1-yl)amino}-3-(2,6-dimethoxy[1,1'-biphenyl]-4-yl)propansäure
(2*S*)-2-[(3-Oxospiro[3.6]dec-1-en-1-yl)amino]-3-{4-[(3,5-dichlorisonicotinoyl)amino]phenyl}propansäure
(2*S*)-2-[(2-Brom-3-oxospiro[3.6]dec-1-en-1-yl)amino]-3-{4-[(3,5-dichlorisonicotinoyl)amino]phenyl}propansäure
(2*S*)-2-[(2-Brom-7-methoxy-3-oxospiro[3.5]non-1-en-1-yl)amino]-3-{4-[(3,5-dichlorisonicotinoyl)amino]phenyl}propansäure
(2*S*)-2-{(2-Brom-3-oxospiro[3.5]non-1-en-1-yl)amino}-3-{4-[(3-methyl[2,7]naphthyridin-1-yl)oxy]phenyl}propansäure
(2*S*)-2-[(2-Chlor-3-oxospiro[3.5]non-1-en-1-yl)amino]-3-{4-[(3,5-dichlorisonicotinoyl)amino]phenyl}propansäure
(2*S*)-3-{4-[(3,5-Dichlorisonicotinoyl)amino]phenyl}-2-(3-oxo-2-pyridin-3-yl-spiro[3.5]non-1-en-1-ylamino)propansäure
(2*S*)-2-[(2-lod-3-oxospiro[3.5]non-1-en-1-yl)amino]-3-{4-[(3,5-dichlorisonicotinoyl)amino]phenyl}propansäure
(2*S*)-2-[(2-Brom-3-oxospiro[3.5]non-1-en-1-yl)amino]-3-{5-[(3,5-dichlorisonicotinoyl)amino]pyridin-2-yl}propansäure
(2*S*)-2-[(2-Chlor-3-oxospiro[3.5]non-1-en-1-yl)amino]-3-{5-[(3,5-dichlorisonicotinoyl)amino]pyridin-2-yl}propansäure
(2*S*)-2-[(2-Chlor-3-oxo-7-oxa-spiro[3.5]non-1-en-1-yl)amino]-3-{4-[(3,5-dichlorisonicotinoyl)amino]phenyl}propansäure
(2*S*)-2-[(2-Chlor-3-oxospiro[3.6]dec-1-en-1-yl)amino]-3-{4-[(3,5-dichlorisonicotinoyl)amino]phenyl}propansäure
(2*S*)-2-[(2-Brom-3,7,7-trioxo-7λ⁶-thia-spiro[3.5]non-1-en-1-yl)amino]-3-{4-[(3,5-dichlorisonicotinoyl)amino]phenyl}propansäure
(2*S*)-2-[(2-Chlor-3-oxo-spiro[3.4]oct-1-en-1-yl)amino]-3-{4-[(3,5-dichlorisonicotinoyl)amino]phenyl}propansäure
(2*S*)-2-[(2-Brom-3-oxo-spiro[3.4]oct-1-en-1-yl)amino]-3-{4-[(3,5-dichlorisonicotinoyl)amino]phenyl}propansäure
(2*S*)-2-[(2-Methylsulfanyl-3-oxo-spiro[3.5]non-1-en-1-yl)amino]-3-{4-[(3,5-dichlorisonicotinoyl)amino]phenyl}propansäure
(2*S*)-2-(2-Fluor-3-oxo-spiro[3.5]non-1-en-1-ylamino)-3-{4-[(3,5-dichlorisonicotinoyl)amino]phenyl}propansäure
(2*S*)-2-[(2-Fluor-3-oxo-7-oxa-spiro[3.5]non-1-en-1-yl)amino]-3-{4-[(3,5-dichlorisonicotinoyl)amino]phenyl}propansäure
(2*S*)-2-[(2-Isopropylsulfanyl-3-oxo-spiro[3.5]non-1-en-1-yl)amino]-3-{4-[(3,5-dichlorisonicotinoyl)amino]phenyl}propansäure
(2*S*)-2-[(2-Isopropylsulfanyl-3-oxo-spiro[3.5]non-1-en-1-yl)amino]-3-[4-([2,7]naphthyridin-1-ylamino)phenyl]propansäure
(2*S*)-2-[(2-Isopropylsulfanyl-3-oxo-7-oxa-spiro[3.5]non-1-en-1-yl)amino]-3-[4-([2,7]naphthyridin-1-ylamino)phenyl]propansäure
(2*S*)-2-[(2-Brom-3-oxo-spiro[3.4]octa-1,6-dien-1-yl)amino]-3-{4-[(3,5-dichlorisonicotinoyl)amino]phenyl}propansäure
(2*S*)-2-[(7-Acetyl-2-brom-3-oxo-7-aza-spiro[3.5]non-1-en-1-yl)amino]-3-{4-[(3,5-dichlorisonicotinoyl)amino]phenyl}propansäure
(2*S*)-2-{[2-(Isopropylsulfanyl)-3-oxo-7-oxaspiro[3.5]non-1-en-1-yl)]amino}-3-(2,6-dimethoxy[1,1'-biphenyl]-4-yl)propansäure
(2*S*)-2-[(2-Cyclohexyl-3-oxo-spiro[3.5]non-1-en-1-y1)amino]-3-{4-[(3,5-dichlorisonicotinoyl)amino]phenyl}propansäure
(2*S*)-3-{4-[(3,5-Dichlorisonicotinoyl)amino]phenyl}-2-(2-methyl-3-oxo-spiro[3.5]non-1-en-1-ylamino)propansäure
(2*S*)-3-{4-[(3,5-Dichlorisonicotinoyl)amino]phenyl}-2-(3-oxo-2-propyl-spiro[3.5]non-1-en-1-ylamino)propansäure
(2*S*)-3-{4-[(3,5-Dichiorisonicotinoyl)amino]phenyl}-2-(2-methyl-3-oxo-7-oxa-spiro[3.5]non-1-en-1-ylamino)propansäure
(2*S*)-3-{4-[(3,5-Dichlorisonicotinoyl)amino]phenyl}-2-(3-oxo-2-propyl-7-oxa-spiro[3.5]non-1-en-1-ylamino)propansäure
(2*S*)-3-{4-[(3,5-Dichlorisonicotinoyl)amino]phenyl}-2-(2-[1,3]dithian-2-yl-3-oxo-spiro[3.5]non-1-en-1-ylamino)propansäure
(2*S*)-3-{4-[(3,5-Dichlorisonicotinoyl)amino]phenyl}-2-(2-ethyl-3-oxo-spiro[3.5]non-1-en-1-ylamino)propansäure
(2*S*)-3-{4-[(3,5-Dichlor-1-oxypyridin-4-carbonyl)amino]phenyl}-2-(3-oxo-spiro[3.5]non-1-en-1-ylamino)propansäure
(2*S*)-2-(2-Brom-3-oxo-spiro[3.5]non-1-en-1-ylamino)-3-{4-[(3,5-dichlor-1-oxy-pyridin-4-carbonyl)amino]phenyl}propansäure
(2*S*)-2-(2-Chlor-3-oxo-spiro[3.5]non-1-en-1-ylamino)-3-{4-[(3,5-dichlor-1-oxy-pyridin-4-carbonyl)amino]phenyl}propansäure
(2*S*)-2-(2-Brom-3-oxo-spiro[3.5]non-1-en-1-ylamino)-3-[4-(3-methyl-[2,7]naphthyridin-1-ylamino)phenyl]propansäure
(2*S*)-2-(2-Brom-3-oxo-spiro[3.5]non-1-en-1-ylamino)-3-[4-([2,7]naphthyridin-1-ylamino)phenyl]propansäure
(2*S*)-2-[(2-Brom-3-oxo-spiro[3.4]oct-1-en-1-yl)amino]-3-{4-[(3,5-dichlorisonicotinoyl)amino]phenyl}propansäure
und die Salze, Solvate, Hydrate, N-Oxide und Carbonsäureester davon ist.

19. Verbindung nach Anspruch 1, welche
(2*S*)-2-[(2-Brom-3-oxospiro[3.5]non-1-en-1-yl)amino]-3-{4-[(3,5-dichlorisonicotinoyl)amino]phenyl}propansäure
(2*S*)-2-[(2-Brom-3-oxo-7-oxaspiro[3.5]non-1-en-1-yl)amino]-3-{4-[(3,5-dichlorisonicotinoyl)amino]phenyl}propansäure
(2*S*)-2-[(2-Brom-3-oxospiro[3.6]dec-1-en-1-yl)amino]-3-{4-[(3,5-dichlorisonicotinoyl)amino]phenyl}propansäure
(2*S*)-2-{(2-Brom-3-oxospiro[3.5]non-1-en-1-yl)amino}-3-{4-[(3-methyl[2,7]naphthyridin-1-yl)oxy]phenyl}propansäure
(2*S*)-2-[(2-Chlor-3-oxospiro[3.5]non-1-en-1-yl)amino]-3-{4-[(3,5-dichlorisonicotinoyl)amino]phenyl}propansäure
(2*S*)-3-{4-[(3,5-Dichlorisonicotinoyl)amino]phenyl}-2-(3-oxo-2-pyridin-3-yl-spiro[3.5]non-1-en-1-ylamino)propansäure
(2*S*)-2-[(2-lod-3-oxospiro[3.5]non-1-en-1-yl)amino]-3-{4-[(3,5-dichlorisonicotinoyl)amino]phenyl}propansäure
(2*S*)-2-[(2-Brom-3-oxospiro[3.5]non-1-en-1-yl)amino]-3-{5-[(3,5-dichlorisonicotinoyl)amino]pyridin-2-yl}propansäure
(2*S*)-2-[(2-Chlor-3-oxospiro[3.5]non-1-en-1-yl)amino]-3-{5-[(3,5-dichlorisonicotinoyl)amino]pyridin-2-yl}propansäure
(2*S*)-2-[(2-Chlor-3-oxo-7-oxa-spiro[3.5]non-1-en-1-yl)amino]-3-{4-[(3,5-dichlorisonicotinoyl)amino]phenyl}propansäure
(2*S*)-2-[(2-Chlor-3-oxo-spiro[3.6]dec-1-en-1-yl)amino]-3-{4-[(3,5-dichlorisonicotinoyl)amino]phenyl}propansäure
(2*S*)-2-[(2-Brom-3,7,7-trioxo-7λ⁶-thia-spiro[3.5]non-1-en-1-yl)amino]-3-{4-[(3,5-dichlorisonicotinoyl)amino]phenyl}propansäure
(2*S*)-2-[(2-Chlor-3-oxo-spiro[3.4]oct-1-en-1-yl)amino]-3-{4-[(3,5-dichlorisonicotinoyl)amino]phenyl}propansäure
(2*S*)-2-[(2-Brom-3-oxo-spiro[3.4]oct-1-en-1-yl)amino]-3-{4-[(3,5-dichlorisonicotinoyl)amino]phenyl}propansäure
(2*S*)-2-[(2-Methylsulfanyl-3-oxo-spiro[3.5]non-1-en-1-yl)amino]-3-{4-[(3,5-dichlorisonicotinoyl)amino]phenyl}propansäure
(2*S*)-2-(2-Fluor-3-oxo-spiro[3.5]non-1-en-1-ylamino)3-{4-[(3,5-dichlorisonicotinoyl)amino]phenyl}propansäure
(2*S*)-2-[(2-Fluor-3-oxo-7-oxa-spiro[3.5]non-1-en-1-yl)amino]-3-{4-[(3,5-dichlorisonicotinoyl)amino]phenyl}propansäure
(2*S*)-2-[(2-Isopropylsulfanyl-3-oxo-spiro[3.5]non-1-en-1-yl)amino]-3-{4-[(3,5-dichlorisonicotinoyl)amino]phenyl}propansäure
(2*S*)-2-[(2-Isopropylsulfanyl-3-oxo-spiro[3.5]non-1-en-1-yl)amino]-3-[4-([2,7]naphthyridin-1-ylamino)phenyl]propansäure
(2*S*)-2-[(2-Isopropylsulfanyl-3-oxo-7-oxa-spiro[3.5]non-1-en-1-yl)amino]-3-[4-([2,7]naphthyridin-1-ylamino)phenyl]propansäure
(2*S*)-2-[(2-Brom-3-oxo-spiro[3.4]octa-1,6-dien-1-yl)amino]-3-{4-[(3,5-dichlorisonicotinoyl)amino]phenyl)propansäure
(2*S*)-2-[(7-Acetyl-2-brom-3-oxo-7-aza-spiro[3.5]non-1-en-1-yl)amino]-3-{4-[(3,5-dichlorisonicotinoyl)amino]phenyl}propansäure
(2*S*)-2-[(2-Cyclohexyl-3-oxo-spiro[3.5]non-1-en-1-yl)amino]-3-{4-[(3,5-dichlorisonicotinoyl)amino]phenyl}propansäure
(2*S*)3-{4-[(3,5-Dichlorisonicotinoyl)amino]phenyl}-2-(2-methyl-3-oxo-spiro[3.5]non-1-en-1-ylamino)propansäure
(2*S*)-3-{4-[(3,5-Dichlorisonicotinoyl)amino]phenyl}-2-(3-oxo-2-propyl-spiro[3.5]non-1-en-1-ylamino)propansäure
(2*S*)-3-{4-[(3,5-Dichlorisonicotinoyl)amino]phenyl}-2-(2-[1,3]dithian-2-yl-3-oxo-spiro[3.5]non-1-en-1-ylamino)propansäure
(2*S*)-2-(2-Brom-3-oxo-spiro[3.5]non-1-en-1-ylamino)-3-{4-[(3,5-dichlor-1-oxy-pyridin-4-carbonyl)amino]phenyl}propansäure
2-(2-Chlor-3-oxo-spiro[3.5]non-1-en-1-ylamino)-3-{4-[(3,5-dichlor-1-oxy-pyridin-4-carbonyl)amino]phenyl}propansäure
und die Salze, Solvate, Hydrate, N-Oxide und Carbonsäureester davon ist.

20. Verbindung nach Anspruch 1, welche
Ethyl-(2*S*)-2-(2-brom-3-oxo-spiro[3.5]non-1-en-1-ylamino)-3-{4-[(3,5-dichlorisonicotinoyl)amino]phenyl}propanoat
Isopropyl-(2*S*)-2-(2-brom-3-oxo-spiro[3.5]non-1-en-1-ylamino)-3-{4-[(3,5-dichlorisonicotinoyl)amino]phenyl}propanoat
t-Butyl-(2*S*)-2-(2-brom-3-oxo-spiro[3.5]non-1-en-1-ylamino)-3-{4-[(3,5-dichlorisonicotinoyl)amino]phenyl}propanoat
1-Methylpiperidin-4-yl-(2*S*)-2-(2-brom-3-oxo-spiro[3.5]non-1-en-1-ylamino)-3-{4-[(3,5-dichlorisonicotinoyl)amino]phenyl}propanoat
Phenyl-(2*S*)-2-(2-brom-3-oxo-spiro[3.5]non-1-en-1-ylamino)-3-{4-[(3,5-dichlorisonicotinoyl)amino]phenyl}propanoat
Cyclopentyl-(2*S*)-2-(2-brom-3-oxo-spiro[3.5]non-1-en-1-ylamino)-3-{4-[(3,5-dichlorisonicotinoyl)amino]phenyl}propanoat
2-Imidazol-1-yl-ethyl-(2*S*)-2-(2-brom-3-oxo-spiro[3.5]non-1-en-1-ylamino)-3-{4-[(3,5-dichlorisonicotinoyl)amino]phenyl}propanoat
Neopentyl-(2*S*)-2-(2-brom-3-oxo-spiro[3.5]non-1-en-1-ylamino)-3-{4-[(3,5-dichlorisonicotinoyl)amino]phenyl}propanoat
Tetrahydrofuran-3-yl-(2*S*)-2-(2-brom-3-oxo-spiro[3.5]non-1-en-1-ylamino)-3-{4-[(3,5-dichlorisonicotinoyl)amino]phenyl}propanoat
Pyridin-4-ylmethyl-(2*S*)-2-(2-brom-3-oxo-spiro[3.5]non-1-en-1-ylamino)-3-{4-[(3,5-dichlorisonicotinoyl)amino]phenyl}propanoat
Tetrahydropyran-4-yl-(2*S*)-2-(2-brom-3-oxo-spiro[3.5]non-1-en-1-ylamino)-3-{4-[(3,5-dichlorisonicotinoyl)amino]phenyl}propanoat
5-Methyl-2-oxo-[1,3]d ioxol-4-ylmethyl-(2*S*)-2-(2-brom-3-oxo-spiro[3.5]non-1-en-1-ylamino)-3-{4-[(3,5-dichlorisonicotinoyl)amino]phenyl}propanoat
1-Methylpyrrolidin-3-yl-(2*S*)-2-(2-brom-3-oxo-spiro[3.5]non-1-en-1-ylamino)-3-{4-[(3,5-dichlorisonicotinoyl)amino]phenyl}propanoat
2,3-Dihydroxypropyl-(2*S*)-2-(2-brom-3-oxo-spiro[3.5]non-1-en-1-ylamino)-3-{4-[(3,5-dichlorisonicotinoyl)amino]phenyl}propanoat
Tetrahydrofuran-2-ylmethyl-(*2S*)-2-(2-brom-3-oxo-spiro[3.5]non-1-en-1-ylamino)-3-{4-[(3,5-dichlorisonicotinoyl)amino]phenyl}propanoat
und die Salze, Solvate, Hydrate und N-Oxide davon ist.

21. Pharmazeutische Zusammensetzung, umfassend eine Verbindung nach Anspruch 1 zusammen mit einem oder mehreren pharmazeutisch verträglichen Trägern, Bindemitteln oder Verdünnungsmitteln.

22. Ethyl-(2*S*)-2-(2-brom-3-oxo-spiro[3.5]non-1-en-1-ylamino)-3-[4-([2,7]naphthyridin-1-ylamino)phenyl]propanoat nach Anspruch 1.

23. Pharmazeutische Zusammensetzung, umfassend Ethyl-(2*S*)-2-(2-brom-3-oxo-spiro[3.5]non-1-en-1-ylamino)-3-[4-([2,7]naphthyridin-1-ylamino)phenyl]propanoat nach Anspruch 22 in Verbindung mit einem pharmazeutisch verträglichen Träger.

24. Verwendung von Ethyl-(2*S*)-2-(2-brom-3-oxo-spiro[3.5]non-1-en-1-ylamino)-3-[4-([2,7]naphthyridin-1-ylamino)phenyl]propanoat nach Anspruch 22 für die Herstellung eines Medikaments für die Behandlung und/oder Vorbeugung von rheumatischer Arthritis, Multipler Sklerose, Asthma oder Entzündungs-Darmerkrankungen.

## Revendications

1. Composé de formule (1) : 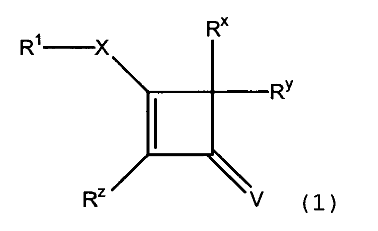 dans lequel
R¹ est un groupe Ar¹L²Ar²Alk- dans lequel :
Ar¹ est un groupe aromatique ou hétéroaromatique éventuellement substitué ; dans lequel les éventuels substituants sont choisis parmi un, deux ou trois atomes d'halogène, et/ou les groupes alkyle en C₁₋₆, cycloalkyle en C₃₋₈, hydroxyalkyle en C₁₋₆, carboxy-alkyle en C₁₋₆, alkylthio en C₁₋₆, carboxy-alkylthio en C₁₋₆, alcoxy en C₁₋₆, hydroxy-alcoxy en C₁₋₆, halo-alkyle en C₁₋₆, halo-alcoxy en C₁₋₆, alkylamino en C₁₋₆, -NH₂, amino-alkyle en C₁₋₆, dialkylamino en C₁₋₆, alkylamino en C₁₋₆-alkyle en C₁₋₆, dialkylamino en C₁₋₆-alkyle en C₁₋₆, amino-alcoxy en C₁₋₆, alkylamino en C₁₋₆-alcoxy en C₁₋₆, dialkylamino en C₁₋₆-alcoxy en C₁₋₆, nitro, cyano, amidino, -OH, HC(O)-, -CO₂H, -CO₂R⁵, alcanoyle en C₁₋₆, -SH, thio-alkyle en C₁₋₆, -SO₃H, -SO₃R⁵, alkylsulfinyle en C₁₋₆, alkylsulfonyle en C₁₋₆, - SO₂NH₂, alkylaminosulfonyle en C₁₋₆, dialkylaminosulfonyle en C₁₋₆, phénylamino-sulfonyle, -CONH₂, alkylaminocarbonyle en C₁₋₆, dialkylaminocarbonyle en C₁₋₆, amino-alkylaminocarbonyle en C₁₋₆, alkylamino en C₁₋₆-alkylaminocarbonyle en C₁₋₆, dialkylamino en C₁₋₆-alkylaminocarbonyle en C₁₋₆, aminocarbonylamino, alkylamino en C₁₋₆-carbonylamino, dialkylamino en C₁₋₆-carbonylamino, alkylamino en C₁₋₆-carbonyl-alkylamino en C₁₋₆, aminothiocarbonylamino, alkylaminothiocarbonylamino en C₁₋₆, dialkylaminothiocarbonylamino en C₁₋₆, alkylaminothiocarbonyle en C₁₋₆-alkylamino en C₁₋₆, alkylsulfonylamino en C₁₋₆, dialkylsulfonylamino en C₁₋₆, -NHSO₂NH₂, alkylaminosulfonylamino en C₁₋₆, dialkylaminosulfonylamino en C₁₋₆, alcanoylamino en C₁₋₆, amino-alcanoylamino en C₁₋₆, dialkylamino en C₁₋₆-alcanoylamino en C₁₋₆, alcanoyle en C₁₋₆-amino-alkyle en C₁₋₆, alcanoylamino en C₁₋₆-alkylamino en C₁₋₆ ou alcoxycarbonyl en C₁₋₆-amino ;
R⁵ est un atome d'hydrogène, ou un groupe akyle en C₁₋₆ ou cycloalkyle en C₃₋₈ ;
L² est une liaison covalente ou un groupe L^{2a} ou -(Alk³)L^{2a}, où Alk³ est une chaîne aliphatique ou hétéroaliphatique éventuellement substituée et L^{2a} est un atome -O- ou -S- ou un groupe -C(O)-, -C(O)O-, - OC(O)-, -C(S)-, -S(O)-, -S(O)₂-, -N(R⁸)- [où R⁸ est un atome d'hydrogène ou un groupe alkyle en C₁₋₆ linéaire ou ramifié], -CON(R⁸)-, -OC(O)N(R⁸)-, - CSN(R⁸)-, -N(R⁸)CO-, -N(R⁸)C(O)O-, N(R⁸)CS-, -S(O)₂N(R⁸)-, -N(R⁸)S(O)₂-, -N(R⁸)O-, - ON(R⁸)-, -N(R⁸)N(R⁸)-, -N(R⁸)CON(R⁸)-, -N(R⁸)CSN(R⁸)- ou - N(R⁸)SO₂N(R⁸)- ; les éventuels substituants qui peuvent être presents sur Alk³ sont choisis parmi un, deux ou trois substituants, chaque substituant pouvant être identique ou différent et étant choisi parmi les atomes d'halogène, ou les groupes -OH, -CO₂H, -CO₂R⁹, où R⁹ est un groupe alkyle en C₁₋₆ linéaire ou ramifié, -CONHR⁹, -CON(R⁹)₂, -COR⁹, alcoxy en C₁₋₆, thiol, -S(O)R⁹, - S(O)₂R⁹, alkylthio en C₁₋₆, amino, -NHR⁹ ou -N(R⁹)₂ ;
Ar² est un groupe arylène ou hétéroarylène éventuellement substitué ; les éventuels substituants qui peuvent être présents sur Ar² étant choisis parmi les atomes d'halogène ou les groupes alkyle en C₁₋₆, halo-alkyle en C₁₋₆, alcoxy en C₁₋₆, halo-alcoxy en C₁₋₆, -CN, -CO₂CH₃, -NO₂, -NH₂, -NR⁵R⁶ et -N(R⁵)COCH₃ ;
R⁶ est tel que défini pour R⁵ ;
et Alk est une chaîne
-CH₂-CH(R)-, -CH=C(R)- ou 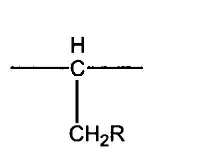 dans laquelle R est un acide carboxylique (-CO₂H) ou un tétrazole, un acide phosphonique, un acide phosphinique, un acide sulfonique, un acide sulfinique, un acide boronique ou un biostère d'acylsulfonamide de ceux-ci ou un groupe -CO₂Alk⁷ ou -CONR⁵R⁶ ; Alk⁷ est un groupe linéaire ou ramifié alkyle en C₁₋₈, alcényle en C₂₋₈, alcynyle en C₂₋₈, cycloalkyle en C₃₋₈, hétérocycloalkyle en C₃₋₈, 1-méthylpyrrolidinyle, 1-méthylpipéridinyle, 5-méthyl-2-oxo-[1,3]dioxol-4-yle, cycloalkyle en C₃₋₈-akyle en C₁₋₈, hétérocycloalkyle en C₃₋₈-alkyle en C₁₋₈, alkyloxy en C₁₋₆-alkyle en C₁₋₆, hydroxy-alkyle en C₁₋₆, alkylthio en C₁₋₆-alkyle en C₁₋₆, alkylsulfinyle en C₁₋₆-alkyle en C₁₋₆, alkylsulfonyle en C₁₋₆-alkyle en C₁₋₆, cycloalkyloxy en C₃₋₈-alkyle en C₁₋₆, cycloalkylthio en C₃₋₈-alkyle en C₁₋₆, cycloalkylsulfinyle en C₃₋₈-alkyle en C₁₋₆, cycloalkylsulfonyle en C₃₋₈-alkyle en C₁₋₆, alkyloxycarbonyle en C₁₋₆-alkyle en C₁₋₆, alkyloxycarbonyle en C₁₋₆-alcényle en C₁₋₆, alkyloxycarbonyloxy en C₁₋₆-alkyle en C₁₋₆, alkyloxycarbonyloxy en C₁₋₆-alcényle en C₁₋₆, cycloalkyloxycarbonyloxy en C₃₋₈-alkyle en C₁₋₆, N-di-alkylamino en C₁₋₈-alkyle en C₁₋₈, N-aryle en C₆₋₁₂-N-alkylamino en C₁₋₆-alkyle en C₁₋₆, N-di-alkyle en C₁₋₈-carbamoyle-alkyle en C₁₋₈, aryle en C₆₋₁₂-alkyle en C₁₋₆, hétéro-aryle en C₆₋₁₀-aryl-alkyle en C₁₋₆, aryle en C₆₋₁₂, aryloxy en C₆₋₁₂-alkyle en C₁₋₈, arylthio en C₆₋₁₂-alkyle en C₁₋₈, arylsulfinyle en C₆₋₁₂-alkyle en C₁₋₈, arylsulfonyle en C₆₋₁₂-alkyle en C₁₋₈, alcanoyloxy en C₁₋₈-alkyle en C₁₋₈, imido en C₄₋₈-alkyle en C₁₋₈, aroyloxy en C₆₋₁₂-alkyle en C₁₋₈, ou un triglycéride ;
X est un groupe -N(R²)- dans lequel :
R² est un atome d'hydrogène ou un groupe alkyle en C₁₋₆ ;
V est un atome d'oxygène (O) ;
R^{Z} est un atome d'hydrogène ou d'halogène ou un groupe alkyle en C₁₋₆, ou un groupe L¹(Alk¹)ₙR³ dans lequel :
L¹ est une liaison covalente ou un atome -O-, -S- ou -Se- ou un groupe -S(O)- ou -N(R⁸)- ;
Alk¹ est une chaîne alkylène en C₁₋₆,
R³ est un atome d'hydrogène ou un groupe cycloaliphatique en C₃₋₁₀, ou un groupe hétérocycloaliphatique en C₃₋₁₀, aromatique en C₆₋₁₂ ou hétéroaromatique en C₁₋₉ éventuellement substitué ; les éventuels substituants qui peuvent être présents sur de tels groupes hétérocycloaliphatiques sont les substituants des groupes hétérocycloaliphatiques liés par un spiro R^{x} et R^{y} ; d'éventuels substituants qui peuvent être présents sur de tels groupes aromatiques ou hétéroaromatiques sont choisis parmi les atomes d'halogène ou les groupes alkyle en C₁₋₆, halo-alkyle en C₁₋₆, alcoxy en C₁₋₆ ou halo-alcoxy en C₁₋₆ ;
n est égal à zéro ou est le nombre entier 1 ;
R^{x} et R^{y} sont joints l'un à l'autre pour former un groupe cycloaliphatique en C₃₋₁₀ ou hétérocycloaliphatique en C₃₋₁₀ lié par un spiro éventuellement substitué ; les éventuels substituants qui peuvent être présents sur de tels groupes liés par un spiro sont choisis parmi les atomes d'halogène, les groupes alkyle en C₁₋₆, les groupes alcoxy en C₁₋₆, les groupes halo-alcoxy en C₁₋₆, -CN, -CO₂CH₃, -NO₂ et -N(R¹¹)₂ ; lorsque le groupe hétérocycloaliphatique lié par un spiro contient un atome d'azote, celui-ci peut être substitué par un groupe -(L⁶)ₚ(Alk⁵)_{q}R¹² où L⁶ est -C(O)-ou -S(O)₂-, Alk⁵ est une chaîne alkylène en C₁₋₆, ou une chaîne hétéro-alkylène en C₁₋₆ et R¹² est un atome d'hydrogène ou un cycle phényle éventuellement substitué, les éventuels substituants du phényle étant choisis parmi les atomes d'halogène ou les groupes alkyle en C₁₋₆, halo-alkyle en C₁₋₆, alcoxy en C₁₋₆, halo-alcoxy en C₁₋₆, -CN, -CO₂CH₃, -NO₂, -NH₂, -NR⁵R⁶, - N(R⁵)COCH₃ ou phényle, furyle, thiényle, imidazolyle, pyridyle ou pyrimidinyle ;
R¹¹ est un atome d'hydrogène ou un groupe alkyle en C₁₋₆ ou cycloalkyle en C₃₋₈ ; et sels, solvates, hydrates et N-oxydes de celui-ci.

2. Composé selon la revendication 1, dans lequel Alk est une chaîne -CH₂CH(R)- ou -CH(CH₂R)-.

3. Composé selon la revendication 1 ou la revendication 2, dans lequel R est groupe acide carboxylique (-CO₂H).

4. Composé selon la revendication 1 ou la revendication 2, dans lequel R est un groupe carboxyle estérifié de formule -CO₂Alk⁷.

5. Composé selon la revendication 1, dans lequel R² est un atome d'hydrogène.

6. Composé selon l'une quelconque des revendications 1 à 5, dans lequel Ar² est un groupe phénylène éventuellement substitué ou un groupe pyridinediyle éventuellement substitué de formule : 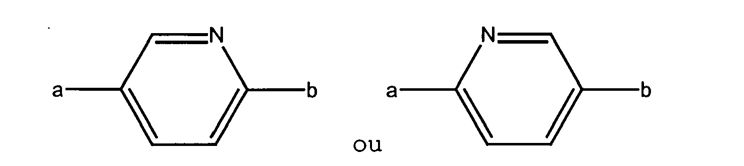 dans laquelle a et b désignent les points de fixation de L² et d'Alk respectivement.

7. Composé selon la revendication 6, dans lequel Ar² est le 1,4-phénylène.

8. Composé selon l'une quelconque des revendications 1 à 7, dans lequel Ar¹ est un groupe phényle ou hétéroaromatique à cinq, six ou dix chaînons éventuellement substitué.

9. Composé selon la revendication 8, dans lequel Ar¹ est un groupe pyridyle, pyrimidinyle, naphtyridinyle, quinolinyle ou isoquinolinyle éventuellement substitué.

10. Composé selon la revendication 9, dans lequel Ar¹ est le 2,7-naphtyridinyle.

11. Composé selon l'une quelconque des revendications 1 à 10, dans lequel R^{Z} est un atome d'halogène.

12. Composé selon la revendication 1, dans lequel R^{Z} est un atome de brome.

13. Composé selon l'une quelconque des revendications 1 à 10, dans lequel R^{Z} est un groupe alkyle en C₁₋₈ éventuellement substitué.

14. Composé selon l'une quelconque des revendications 1 à 10, dans lequel R^{Z} est un groupe -L¹(Alk¹)ₙR³ dans lequel L¹ est un atome -O-, -S- ou -Se-ou un groupe -S(O)- ou -N(R⁸)-.

15. Composé selon l'une quelconque des revendications 1 à 10, dans lequel R^{Z} est un groupe -L¹(Alk¹)ₙR³ dans lequel L¹ est une liaison covalente.

16. Composé selon la revendication 14 ou la revendication 15, dans lequel n est égal à zéro.

17. Composé selon la revendication 14 ou la revendication 15, dans lequel n est le nombre entier 1 et Alk¹ est une chaîne alkylène en C₁₋₆ éventuellement substituée.

18. Composé selon la revendication 1, qui est :
l'acide (2*S*)-2-[(3-oxospiro[3.5]non-1-én-1-yl)amino]-3-{4-[(3,5-dichloroisonicotinoyl)amino]phényl}propanoïque
l'acide (2*S*)-2-[(3-oxospiro[3.5]non-1-én-1-yl)amino]-3-(4-[(3-méthyl[2,7]naphtyridin-l-yl)oxy]phényl}propanoïque
l'acide (2*S*)-2-[(2-bromo-3-oxospiro[3.5]non-1-én-1-yl)amino]-3-{4-[(3,5-dichloroisonicotinoyl)amino]phényl}propanoïque
l'acide (2*S*)-2-[(2-bromo-3-oxospiro[3.5]non-1-én-1-yl)amino]-3-{4-[(2,7)naphtyridin-1-yloxy]phényl}propanoïque
l'acide (2*S*)-2-[(2-bromo-3-oxo-7-oxaspiro[3.5]non-1-én-1-yl)amino]-3-{4-[(3,5-dichloroisonicotinoyl)amino]phényl}propanoïque
l'acide (2*S*)-2-{(2-bromo-3-oxospiro[3.5]non-1-én-1-yl)amino}-3-(2,6-diméthoxy[1,1'-biphényl]-4-yl)propanoïque
l'acide (2*S*)-2-[(3-oxospiro[3.6]déc-1-én-1-yl)amino]-3-{4-[(3,5-dichloroisonicotinoyl)amino]phényl} propanoïque
l'acide (2*S*)-2-[(2-bromo-3-oxospiro[3.6]déc-1-én-1-yl)amino]-3-{4-[(3,5-dichloroisonicotinoyl)amino]phényl}propanoïque
l'acide (2*S*)-2-[(2-bromo-7-méthoxy-3-oxospiro[3.5]non-1-én-1-yl)amino]-3-{4-[(3,5-dichloroisonicotinoyl)amino]phényl}propanoïque
l'acide (2*S*)-2-{(2-bromo-3-oxospiro[3.5]non-1-én-1-yl)amino}-3-{4-[(3-méthyl[2,7]naphtyridin-1-yl)oxy]phényl} propanoïque
l'acide (2*S*)-2-[(2-chloro-3-oxospiro[3.5]non-1-én-1-yl)amino]-3-{4-[(3,5-dichloroisonicotinoyl)amino]phényl}propanoïque
l'acide (2*S*)-3-{4-[(3,5-dichloroisonicotinoyl)amino]phényl} -2-(3-oxo-2-pyridin-3-yl-spiro[3.5]non-1-én-1-ylamino)propanoïque,
l'acide (2*S*)-2-[(2-iodo-3-oxospiro[3.5]non-1-én-1-yl)amino]-3-{4-[(3,5-dichloroisonicotinoyl)amino]phényl}propanoïque,
l'acide (2*S*)-2-[(2-bromo-3-oxospiro[3.5]non-1-en-1-yl)amino]-3-{5-[(3,5-dichloroisonicotinoyl)amino]pyridin-2-yl}propanoïque,
l'acide (2*S*)-2-[(2-chloro-3-oxospiro[3.5]non-1-én-1-yl)amino]-3-{5-[(3,5-dichloroisonicotinoyl)amino]pyridin-2-yl}propanoïque,
l'acide (2*S*)-2-[(2-chloro-3-oxo-7-oxa-spiro[3.5]non-1-én-1-yl)amino]-3-{4-[(3,5-dichloroisonicotinoyl)amino]phényl}propanoïque,
l'acide (2*S*)-2-[(2-chloro-3-oxo-spiro[3.6]déc-1-én-1-yl)amino]-3-{4-[(3,5-dichloroisonicotinoyl)amino]phényl}propanoïque,
l'acide (2*S*)-2-[(2-bromo-3,7,7-trioxo-7λ⁶-thia-spiro[3.5]non-1-én-1-yl)amino]-3-{4-[(3,5-dichloroisonicotinoyl)amino]phényl}propanoïque,
l'acide (2*S*)-2-[(2-chloro-3-oxo-spiro[3.4]oct-1-én-1-yl)amino]-3-{4-[(3,5-dichloro-isonicotinoyl)amino]phényl}propanoïque,
l'acide (2*S*)-2-[(2-bromo-3-oxo-spiro[3.4]oct-1-én-1-yl)amino]-3-{4-[(3,5-dichloroisonicotinoyl)amino]phényl}propanoïque,
l'acide (2*S*)-2-[(2-méthylsulfanyl-3-oxo-spiro[3.5]non-1-én-1-yl)amino]-3-{4-[(3,5-dichloroisonicotinoyl)amino]phényl}propanoïque,
l'acide (2*S*)-2-(2-fluoro-3-oxo-spiro[3 .5]non-1-én-1-ylamino)3-(4-[(3,5-dichloroisonicotinoyl)amino]phényl}propanoïque,
l'acide (2*S*)-2-[(2-fluoro-3-oxo-7-oxa-spiro[3.5]non-1-én-1-yl)amino]-3-{4-[(3,5-dichloroisonicotinoyl)amino]phényl}propanoïque,
l'acide (2*S*)-2-[(2-isopropylsulfanyl-3-oxo-spiro[3.5]non-1-én-1-yl)amino]-3- {4-[(3,5-dichloroisonicotinoyl)amino]phényl}propanoïque,
l'acide (2*S*)-2-[(2-isopropylsulfanyl-3-oxo-spiro[3.5]non-1-én-1-yl)amino]-3-[4-([2,7]naphtyridin-1-ylamino)phényl]propanoïque,
l'acide (2*S*)-2-[(2-isopropylsulfanyl-3-oxo-7-oxa-spiro[3.5]non-1-én-1-yl)amino]-3-[4-([2,7]naphtyridin-1-ylamino)phényl]propanoïque,
l'acide (2*S*)-2-[(2-bromo-3-oxo-spiro[3.4]octa-1,6-dién-1-yl)amino]-3-{4-[(3,5-dichloroisonicotinoyl)amino]phényl}propanoïque,
l'acide (2*S*)-2-[(7-acétyl-2-bromo-3-oxo-7-aza-spiro[3.5]non-1-én-1-yl)amino]-3-{4-[(3,5-dichloroisonicotinoyl)amino]phényl}propanoïque,
l'acide (2*S*)-2-{[2-(isopropylsulfanyl)-3-oxo-7-oxaspiro[3.5]non-1-én-yl)]amino}-3-(2,6-diméthoxy[1,1'-biphényl]-4-yl)propanoïque,
l'acide (2*S*)-2-[(2-cyclohexyl-3-oxo-spiro[3.5]non-1-én-1-yl)amino]-3-{4-[(3,5-dichloroisonicotinoyl)amino]phényl}propanoïque,
l'acide (2*S*)-3-{4-[(3,5-dichloroisonicotinoyl)amino]phényl}-2-(2-méthyl-3-oxo-spiro[3.5]non-1-én-1-ylarnino)propanoïque,
l'acide (2*S*)-3-{4-[(3,5-dichloroisonicotinoyl)amino]phényl}--2-(3-oxo-2-propyl-spiro[3.5]non-1-én-1-ylamino)propanoïque,
l'acide (2*S*)-3-{4-[(3,5-dichloroisonicotinoyl)amino]phényl}-2-(2-méthyl-3-oxo-7-oxa-spiro[3.5]non-1-én-1-ylamino)propanoïque,
l'acide (2*S*)-3-{4-[(3,5-dichloroisonicotinoyl)amino]phényl}-2-(3-oxo-2-propyl-7-oxa-spiro[3.5]non-1-én-1-ylamino)propanoïque,
l'acide (2*S*)-3-{4-[(3,5-dichloroisonicotinoyl)amino]phényl}-2-(2-[1,3]dithian-2-yl-3-oxo-spiro[3.5]non-1-én-1-ylamino)propanoïque,
l'acide (2*S*)-3-{4-[(3,5-dichloroisonicotinoyl)amino]phényl}-2-(2-éthyl-3-oxo-spiro[3.5]non-1-én-1-ylamino)propanoïque,
l'acide (2*S*)-3-{4-[(3,5-dichloro-1-oxy-pyridine-4-carbonyl)amino]-phényl}-2-(3-oxo-spiro[3.5]non-1-én-1-ylamino)propanoïque,
l'acide (2*S*)-2-(2-bromo-3-oxo-spiro[3.5]non-1-én-1-ylamino)-3-{4-[(3,5-dichloro-1-oxy-pyridine-4-carbonyl)amino]phényl}propanoïque,
l'acide (2*S*)-2-(2-chloro-3-oxo-spiro[3.5]non-1-én-1-ylamino)-3-{4-[(3,5-dichloro-1-oxy-pyridine-4-carbonyl)amino]phényl}propanoïque,
l'acide (2*S*)-2-(2-bromo-3-oxo-spiro[3.5]non-1-én-1-ylamino)-3-[4-(3-méthyl-[2,7]naphtyridin-1-ylamino)phényl]propanoïque,
l'acide (2*S*)-2-(2-bromo-3-oxo-spiro[3.5]non-1-én-1-ylamino)-3-[4-([2,7]naphtyridin-1-ylamino)phényl]propanoïque,
l'acide (2*S*)-2-[(2-bromo-3-oxo-spiro[3.4]oct-1-én-1-yl)amino)-3-{4-[(3,5-dichloroisonicotinoyl)amino]phényl}propanoïque,
et sels, solvates, hydrates, N-oxydes et esters d'acide carboxylique de celui-ci.

19. Composé selon la revendication 1, qui est :
l'acide (2S)-2-[(2-bromo-3-oxospiro[3.5]non-1-én-1-yl)amino]-3-{4-[(3,5-dichloroisonicotinoyl)amino]phényl}propanoïque,
l'acide (2*S*)-2-[(2-bromo-3-oxo-7-oxaspiro[3.5]non-1-én-1-yl)amino]-3- {4-[(3,5-dichloroisonicotinoyl)amino]phényl}propanoïque,
l'acide (2*S*)-2-[(2-bromo-3-oxospiro[3.6]déc-1-én-1-yl)amino]-3-{4-[(3,5-dichloroisonicotinoyl)amino]phényl}propanoïque,
l'acide (2*S*)-2-{(2-bromo-3-oxospiro[3.5]non-1-én-1-yl)amino}-3-{4-[(3-méthyl[2,7]naphtyridin-1-yl)oxy]phényl}propanoïque,
l'acide (2*S*)-2-[(2-chloro-3-oxospiro[3.5]non-1-én-1-yl)amino]-3-{4-[(3,5-dichloroisonicotinoyl)amino]phényl}propanoïque,
l'acide (2*S*)-3-{4-[(3,5-dichloroisonicotinoyl)amino]phényl}-2-(3-oxo-2-pyridin-3-yl-spiro[3.5]non-1-én-1-ylamino)propanoïque,
l'acide (2*S*)-2-[(2-iodo-3-oxospiro[3.5]non-1-én-1-yl)amino]-3-{4-[(3,5-dichloroisonicotinoyl)amino]phényl}propanoïque,
l'acide (2*S*)-2-[(2-bromo-3-oxospiro[3.5]non-1-én-1-yl)amino]-3-{5-[(3,5-dichloroisonicotinoyl)amino]pyridin-2-yl}propanoïque,
l'acide (2*S*)-2-[(2-chloro-3-oxospiro[3.5]non-1-én-1-yl)amino]-3-{5-[(3,5-dichloroisonicotinoyl)amino]pyridin-2-yl}propanoïque,
l'acide (2*S*)-2-[(2-chloro-3-oxo-7-oxa-spiro[3.5]non-1-én-1-yl)amino]-3- {4-[(3,5-dichloroisonicotinoyl)amino]phényl}propanoïque,
l'acide (2*S*)-2-[(2-chloro-3-oxo-spiro[3.6]déc-1-én-1-yl)amino]-3-{4-[(3,5-dichloroisonicotinoyl)amino]phényl}propanoïque,
l'acide (2*S*)-2-[(2-bromo-3,7,7-trioxo-7λ⁶-thia-spiro[3.5]non-1-én-1-yl)amino]-3- {4-[(3,5-dichloroisonicotinoyl)amino]phényl}propanoïque,
l'acide (2*S*)-2-[(2-chloro-3-oxo-spiro[3.4]oct-1-én-1-yl)amino]-3-{4-[(3,5-dichloroisonicotinoyl)amino]phényl}propanoïque,
l'acide (2*S*)-2-[(2-bromo-3-oxo-spiro[3.4]oct-1-én-1-yl)amino]-3-{4-[(3,5-dichloroisonicotinoyl)amino]phényl}propanoïque,
l'acide (2*S*)-2-[(2-méthylsulfanyl-3-oxo-spiro[3.5]non-1-én-1-yl)amino]-3- {4-[(3,5-dichloroisonicotinoyl)amino]phényl}propanoïque,
l'acide (2*S*)-2-(2-fluoro-3-oxo-spiro[3.5]non-1-én-1-ylamino)3-{4-[(3,5-dichloroisonicotinoyl)amino]phényl}propanoïque,
l'acide (2*S*)-2-[(2-fluoro-3-oxo-7-oxa-spiro[3.5]non-1-én-1-yl)amino]-3-{4-[(3,5-dichloroisonicotinoyl)amino]phényl}propanoïque,
l'acide (2*S*)-2-[(2-isopropylsulfanyl-3-oxo-spiro[3.5]non-1-én-1-yl)amino]-3-{4-[(3,5-dichloroisonicotinoyl)amino]phényl}propanoïque,
l'acide (2*S*)-2-[(2-isopropylsulfanyl-3-oxo-spiro[3.5]non-1-én-1-yl)amino]-3-[4-([2,7]naphtyridin-1-ylamino)phényl]propanoïque,
l'acide (2*S*)-2-[(2-isopropylsulfanyl-3-oxo-7-oxa-spiro[3.5]non-1-én-1-yl)amino]-3-[4-([2,7]naphtyridin-1-ylamino)phényl]propanoïque,
l'acide (2*S*)-2-[(2-bromo-3-oxo-spiro[3.4]octa-1,6-dién-1-yl)amino]-3-{4-[(3,5-dichloroisonicotinoyl)amino]phényl}propanoïque,
l'acide (2*S*)-2-[(7-acétyl-2-bromo-3-oxo-7-aza-spiro[3.5]non-1-én-1-yl)amino]-3-{4-[(3,5-dichloroisonicotinoyl)amino]phényl}propanoïque,
l'acide (2*S*)-2-[(2-cyclohexyl-3-oxo-spiro[3.5]non-1-én-1-yl)amino]-3-{4-[(3,5-dichloroisonicotinoyl)amino]phényl}propanoïque,
l'acide (2*S*)3-{4-[(3,5-dichloroisonicotinoyl)amino]phényl}-2-(2-méthyl-3-oxo-spiro[3.5]non-1-én-1-ylamino)propanoïque,
l'acide (2*S*)-3-{4-[(3,5-dichloroisonicotinoyl)amino]phényl}-2-(3-oxo-2-propyl-spiro[3.5]non-1-én-1-ylamino)propanoïque,
l'acide (2*S*)-3-{4-[(3,5-dichloroisonicotinoyl)amino]phényl}-2-(2-[1,3]dithian-2-yl-3-oxo-spiro[3.5]non-1-én-1-ylamino)propanoïque,
l'acide (2*S*)-2-(2-bromo-3-oxo-spiro[3.5]non-1-én-1-ylamino)-3-{4-[(3,5-dichloro-1-oxy-pyridine-4-carbonyl)amino]phényl}propanoïque,
l'acide 2-(2-chloro-3-oxo-spiro[3.5]non-1-én-1-ylamino)-3-{4-[(3,5-dichloro-1-oxy-pyridine-4-carbonyl)amino]phényl} propanoïque,
et sels, solvates, hydrates, N-oxydes et esters d'acide carboxylique de celui-ci.

20. Composé selon la revendication 1, qui est :
le (2*S*)-2-(2-bromo-3-oxo-spiro[3.5]non-1-én-1-ylamino)-3-{4-[(3,5-dichloroisonicotinoyl)amino]phényl}propanoate d'éthyle,
le (2*S*)-2-(2-bromo-3-oxo-spiro[3.5]non-1-én-1-ylamino)-3-{4-[(3,5-dichloroisonicotinoyl)amino]phényl}propanoate d'isopropyle,
le (2*S*)-2-(2-bromo-3-oxo-spiro[3.5]non-1-én-1-ylamino)-3-{4-[(3,5-dichloroisonicotinoyl)amino]phényl}propanoate de t-butyle,
le (2*S*)-2-(2-bromo-3-oxo-spiro[3.5]non-1-én-1-ylamino)-3-{4-[(3, 5-dichloroisonicotinoyl)amino]phényl}propanoate de 1-méthyl-pipéridin-4-yle,
le (2*S*)-2-(2-bromo-3-oxo-spiro[3.5]non-1-én-1-ylamino)-3-{4-[(3,5-dichloroisonicotinoyl)amino]phényl}propanoate de phényle,
le (2*S*)-2-(2-bromo-3-oxo-spiro[3.5]non-1-én-1-ylamino)-3-{4-[(3,5-dichloroisonicotinoyl)amino]phényl}propanoate de cyclopentyle,
le (2*S*)-2-(2-bromo-3-oxo-spiro[3.5]non-1-én-1-ylamino)-3-{4-[(3,5-dichloroisonicotinoyl)amino]phényl}propanoate de 2-imidazol-1-yl-éthyle,
le (2*S*)-2-(2-bromo-3-oxo-spiro[3.5]non-1-én-1-ylamino)-3-{4-[(3,5-dichloroisonicotinoyl)amino]phényl}propanoate de néopentyle,
le (2*S*)-2-(2-bromo-3-oxo-spiro[3.5]non-1-én-1-ylamino)-3-{4-[(3,5-dichloroisonicotinoyl)amino]phenyl}propanoate de tétrahydrofuran-3-yle,
le (2*S*)-2-(2-bromo-3-oxo-spiro[3.5]non-1-én-1-ylamino)-3-{4-[(3,5-dichloroisonicotinoyl)amino]phényl}propanoate de pyridin-4-ylméthyle,
le (2*S*)-2-(2-bromo-3-oxo-spiro[3.5]non-1-én-1-ylamino)-3-{4-[(3,5-dichloroisonicotinoyl)amino]phényl}propanoate de tétrahydropyran-4-yle,
le (2*S*)-2-(2-bromo-3-oxo-spiro[3.5]non-1-én-1-ylamino)-3-{4-[(3,5-dichloroisonicotinoyl)amino]phényl}propanoate de 5-méthyl-2-oxo-[1,3]dioxol-4-ylméthyle,
le (2*S*)-2-(2-bromo-3-oxo-spiro[3.5]non-1-én-1-ylamino)-3-{4-[(3,5-dichloroisonicotinoyl)amino]phényl}propanoate de 1-méthyl-pyrrolidin-3-yle,
le (2*S*)-2-(2-bromo-3-oxo-spiro[3.5]non-1-én-1-ylamino)-3-(4-[(3,5-dichloroisonicotinoyl)amino]phényl)propanoate de 2,3-dihydroxypropyle,
le (2*S*)-2-(2-bromo-3-oxo-spiro[3.5]non-1-én-1-ylamino)-3-{4-[(3,5-dichloroisonicotinoyl)amino]phényl}propanoate de tétrahydrofuran-2-ylméthyle
et sels, solvates, hydrates et N-oxydes de celui-ci.

21. Composition pharmaceutique comprenant un composé selon la revendication 1 conjointement avec un ou plusieurs supports, excipients ou diluants pharmaceutiquement acceptables.

22. (2S)-2-(2-bromo-3-oxo-spiro[3.5]non-1-én-1-ylamino)-3-[4-([2,7]naphtyridin-1-ylamino)phényl]propanoate d'éthyle selon la revendication 1.

23. Composition pharmaceutique comprenant le (2S)-2-(2-bromo-3-oxo-spiro[3.5]non-1-én-1-ylamino)-3-[4-([2,7]naphtyridin-1-ylamino)-phényl]propanoate d'éthyle selon la revendication 22 en association avec un support pharmaceutiquement acceptable.

24. Utilisation du (2S)-2-(2-bromo-3-oxo-spiro[3.5]non-1-én-1-ylamino)-3-[4-([2,7]naphtyridin-1-ylamino)phényl]propanoate d'éthyle selon la revendication 22 pour la fabrication d'un médicament pour le traitement et/ou la prévention de la polyarthrite rhumatoïde, de la sclérose en plaques, de l'asthme ou des maladies inflammatoires chroniques de l'intestin.
